# EUROPEAN PATENT APPLICATION

(11) **EP 2 096 111 A1**
(43) Date of publication of application: **02.09.2009**
(21) Application number: 07832068.6
(22) Date of filing: 13.11.2007
(51) Int. Cl.: C07D 231/40, A61K 31/4155, A61K 31/4178, A61K 31/4192, A61K 31/42, A61K 31/422, A61K 31/4245, A61K 31/427, A61K 31/433, A61K 31/437, A61K 31/4439, A61K 31/454, A61K 31/506, A61K 31/5377, A61P 3/10, A61P 43/00

(54) **PYRAZOLES AND USE THEREOF AS DRUGS**

(30) Priority: 20.11.2006 JP 2006313238; 28.11.2006 US 861368 P
(71) Applicant: Japan Tobacco Inc., Tokyo 105-8422 (JP)
(72) Inventor: TAKAGI, Masaki, Takatsuki-shi Osaka 569-1125 (JP); NAKAMURA, Takeshi, Takatsuki-shi Osaka 569-1125 (JP); MATSUDA, Isamu, Takatsuki-shi Osaka 569-1125 (JP); KIGUCHI, Toshihiro, Takatsuki-shi Osaka 569-1125 (JP); OGAWA, Naoki, Takatsuki-shi Osaka 569-1125 (JP); OZEKI, Hidekazu, Takatsuki-shi Osaka 569-1125 (JP)
(74) Representative: Vossius & Partner
(86) International application number: PCT/JP2007/072338
(87) International publication number: WO 2008/062739

(57) **Abstract**

The pyrazole compound of the present invention is represented by the following general formula (I). The pyrazole compound of present invention or a salt thereof or a solvate thereof potently inhibits liver glycogen phosphorylase, and, therefore, is useful as a therapeutic or prophylactic agent for diabetes. 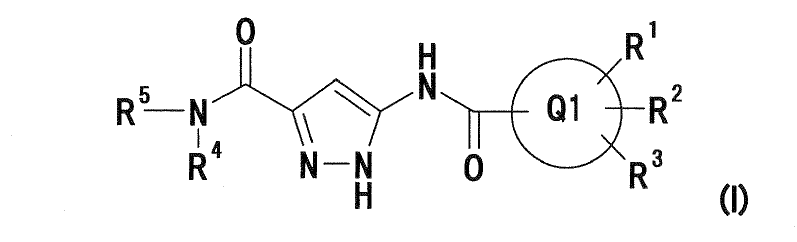 wherein each symbol denotes as described in the specification.

## Description

### Technical Field

The present invention relates to a pyrazole compound having a liver glycogen phosphorylase inhibitory activity or a salt thereof or a solvate thereof, and a pharmaceutical composition, a therapeutic agent for diabetes, and the like comprising the same.

### Background Art

Diabetes is a chronic disease characterized by impaired metabolism of sugars (glucose), lipids, and amino acids resulting from lack of the insulin action. Untreated conditions lead to hyperglycemia and urinary sugar.

Diabetes is roughly classified into type I diabetes characterized by hyposecretion of insulin which is attributed to destruction of the pancreatic islet cells primarily caused by production of autoantibodies against the pancreatic islets (insulin dependent diabetes mellitus, IDDM) and type II diabetes caused by insulin secretory dysfunction of the pancreas and insulin resistance in peripheral tissues such as muscle tissues and the liver (noninsulin independent diabetes mellitus, NIDDM). Since insulin secretory capacity is lost in patients with insulin dependent diabetes, ketonemia and acidosis are likely to occur, and diabetic coma occurs if the condition is left untreated. Diet and oral blood sugar lowering agent have no therapeutic effect, and IDDM can be treated only by using insulin. On the other hand, although the insulin action is lower than normal in patients with insulin independent diabetes (NIDDM), ketonemia and acidosis are not very likely, and insulin is not necessarily required in treatment. About 90 to 95% of diabetic patients have insulin independent diabetes.

Diabetes is a disease in which a high blood sugar condition persistent for a long period causes complications such as neuropathy, retinopathy, and microangiopathy in the kidneys, and the like and further leads to fatal complications such as coronary artery diseases and cerebral apoplexy.

Examples of hypoglycemic drugs currently used for the purpose of treatment of hyperglycemia include insulin preparations, sulfonylurea agents (for example, glibenclamide and tolbutamide), biguanide agents (for example, metformin), insulin resistance improving agents (for example, pioglitazone), and α-glucosidase inhibitors (for example, acarbose).

Insulin preparations are used for treatment of insulin dependent diabetes mellitus and reliably lower blood sugar levels, but they must be administered by injection and may cause hypoglycemia.

Sulfonylurea agents stimulate β cells of the pancreas and promote secretion of endogenous insulin from β cells of the pancreas, but the timing and the amount of insulin secretion are determined by the timing and the dose of a drug regardless of blood sugar levels. Therefore, hypoglycemia attributable to a sustained drug action occurs as an adverse drug reaction. Furthermore, digestive symptoms such as anorexia develop. Sulfonylurea agents are contraindicated in patients with severe ketosis or hepatic or renal dysfunction.

Biguanide drugs do not stimulate P cells of the pancreas and do not cause hypoglycemia in normal subjects or diabetic patients when administered alone. The mechanism of action is thought to involve increased sugar utilization due to an anaerobic glycolytic action, inhibition of gluconeogenesis, suppression of sugar absorption by the intestinal tract, and the like. As an adverse drug reaction, relatively severe lactic acidosis is likely to occur.

Examples of insulin resistance improving drugs (insulin sensitizers) include thiazolidine derivatives. While compounds derived from thiazolidine enhance the insulin action without exerting an insulinotropic action, activate insulin receptor kinase, promote increases in sugar uptake of peripheral tissues, improve excess liver sugar production, and so forth, digestive symptoms, edema, and the like occur as adverse drug reactions. Furthermore, these drugs are known to decrease the erythrocyte count, hematocrit, and hemoglobin and increase LDH.

α-Glucosidase inhibitors delay digestion and absorption of carbohydrates in the gastrointestinal tract and suppress elevation of blood sugar levels after meal, but adverse drug reactions such as flatus, borborygmus, and diarrhea are problematic (for example, refer to Non-Patent Document 1).

Thus, use of these drugs is restricted due to occurrence of some adverse drug reactions and existence of non-responder patients, and hypoglycemic drugs having a novel action mechanism have been awaited.

Recent reports have shown that the amount of sugar released from the liver increases in fasting in NIDDM patients as compared with healthy individuals. It is suggested that this phenomenon of sugar release from the liver may be a target for drug treatment of NIDDM.

According to the elucidation of diabetic conditions in recent years, abnormal function of pancreatic β cells and increased sugar release from the liver are closely associated with onset and progression of diabetes (for example, refer to Non-Patent Document 2). The sugar release from the liver is represented by the sum of products from two pathways, liver glycogenolysis and gluconeogenesis. It has been reported that liver glycogenolysis increases in diabetic conditions (for example, refer to Non-Patent Documents 3 and 4). Furthermore, it has been reported that blood sugar levels are lowered in diabetic animals or normal individuals upon stimulation of glucagon by inhibiting liver glycogenolysis (for example, refer to Non-Patent Document 5). These findings suggest that increased liver glycogenolysis is involved in diabetic conditions.

Meanwhile, it is known that this glycogenolysis is catalyzed by liver glycogen phosphorylase, and glucose 1-phosphate (G-1-P) and glycogen (n-1 units of glucose) are generated by subjecting glycogen (n units of glucose) to phosphorolysis.

Accordingly, development of therapeutic agents for diabetes with a novel mechanism having an inhibitory action on liver glycogen phosphorylase closely involved in this glycogenolysis has been promoted. However, no drug that satisfies requirements regarding activity has yet been found in actual situations. Furthermore, while it is well known that liver glycogen phosphorylase inhibitors are effective as therapeutic or prophylactic agents for diabetes, it has been reported that they have other various effects.

For example, it has been reported that compounds having an inhibitory activity against liver glycogen phosphorylase are "useful for treatment of diabetes, insulin resistance diseases, diabetic neuropathy, diabetic nephropathy, diabetic retinopathy, cataract, hyperglycemia, hypercholesterolemia, hypertension, hyperinsulinism, hyperlipemia, atherosclerosis, tissue ischemia, and myocardial ischemia" (for example, refer to Patent Document 1).

Furthermore, it has been reported that compounds having a liver glycogen phosphorylase inhibitory activity are effective as appetite-regulating agents and therapeutic agents for obesity and beneficial in therapeutic or prophylactic treatment of diseases in which the decrease in blood lipid content is effective such as, for example, dyslipidemia, hypertriglyceridemia, hyperlipemia, hyperlipoproteinemia, cardiovascular diseases, and hypertension (for example, refer to Patent Document 2).

Furthermore, it has been reported that liver glycogen phosphorylase inhibitors are effective in therapeutic and prophylactic treatment of diseases associated with the glucose metabolism, for example, diabetes, especially noninsulin independent diabetes (NIDDM) including long-term complications thereof (e.g., retinopathy, neuropathy, nephropathy, microangiopathy, and macroangiopathy) (for example, refer to Patent Document 3).

Furthermore, three isoforms of glycogen phosphorylase, i.e., a liver isoform, a muscle isoform, and a brain isoform have been identified in humans, and it has also been reported that these isoforms are three discrete gene products whose 80 to 83% of amino acid sequences are homologous, that glycogen phosphorylase also exists in bacteria, and that use of compounds having a liver glycogen phosphorylase inhibitory activity is a method for therapeutic or prophylactic treatment of infections, for example, bacterial, fungal, parasitic, and viral infections and are effective for therapeutic and prophylactic treatment of the above-mentioned infections (for example, refer to Patent Document 4).

Therefore, in addition to effectiveness as therapeutic and prophylactic agents for diabetes, liver glycogen phosphorylase inhibitors are expected to have efficacy as therapeutic agents for insulin resistance diseases, diabetic neuropathy, diabetic nephropathy, diabetic retinopathy, cataract, hypercholesterolemia, hypertension, hyperinsulinism, hyperlipemia, atherosclerosis, tissue ischemia, and myocardial ischemia; appetite regulating agents and therapeutic agents for obesity; and therapeutic agents for infections such as bacterial, fungal, parasitic, and viral infections.

Meanwhile, the following compounds are known as pyrazole compounds.

For example, 4-bromo-5-(2-chloro-benzoylamino)-1H-pyrazole-3-carboxylic acid amide derivatives are described in WO2004/098589 A1 (refer to Patent Document 5), and 1H-pyrazole-3-carboxylic acid amide derivatives similar thereto are also described in WO2004/098590 A1 (refer to Patent Document 6) and WO2004/099155 A2 (refer to Patent Document 7). However, these compounds are all bradykinin B1 receptor antagonists for treatment of inflammatory diseases and are different from the liver glycogen phosphorylase inhibitor of the present invention. [Non-Patent Document 1] Joslin's Diabetes Mellitus, 13th Edition, pp.521-522
[Non-Patent Document 2] Withers D.J., Endocrinology, 141; pp.1917-1921, 2000,
[Non-Patent Document 3] Tayek J.A., Am. J. Physiol., 270; pp.E709-E717, 1996,
[Non-Patent Document 4] Diraison F., Diabetologa, 41; pp.212-220, 1998,
[Non-Patent Document 5] William H. Martin, Proc. Natl. Acad. Sci. USA, 95, pp.1776-1781, 1998; Judith L., Abstracts from the ADA 61st Scientific Session
[Patent Document 1] Japanese Patent Laid-Open No. 2004-002311
[Patent Document 2] National Publication of International Patent Application No. 2002-536410
[Patent Document 3] National Publication of International Patent Application No. 2002-515064
[Patent Document 4] Japanese Patent Laid-Open No. 2001-247565
[Patent Document 5] WO2004/098589 A1
[Patent Document 6] WO2004/098590 A1
[Patent Document 7] WO2004/099155 A2

### Disclosure of the Invention

Many therapeutic agents for diabetes currently used have four targets: the pancreas (promotion of insulin secretion), peripheral tissues (improvement of insulin resistance), small intestine (inhibition of sugar absorption), or insulin itself. In recent years, however, development of therapeutic agents for diabetes based on a novel mechanism of action is desired, and development of therapeutic agents for diabetes by inhibiting liver glycogen phosphorylase is attracting attention. However, no drug has been found that satisfies requirements with regard to liver glycogen phosphorylase inhibitory activity, blood sugar lowering action, oral absorption, metabolic stability, and the like.

Therefore, development of a liver glycogen phosphorylase inhibitor has been strongly desired that comprises an active ingredient having a chemical structure different from those of conventional therapeutic agents for diabetes, has a potent activity, causes no adverse drug reactions, and is superior in oral absorption and metabolic stability.

### Means for Solving the Problems

To achieve the above objectives, the inventors of the present invention assiduously studied to search for a useful therapeutic agent for diabetes having an excellent liver glycogen phosphorylase inhibitory activity. As a result, they found that a pyrazole compound represented by the following general formula (I) had a potent liver glycogen phosphorylase inhibitory activity, and accomplished the present invention.

Details thereof are as described in the following 1 to 15.
1. A pyrazole compound represented by the following general formula (I) or a salt thereof or a solvate thereof: 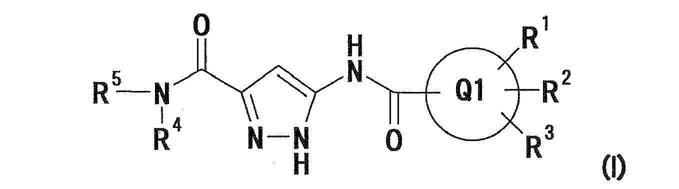 [wherein
   ring Q1 represents
   (1) an aryl group, or
   (2) a monocyclic aromatic heterocyclic ring group; R¹, R², and R³ are the same or different and each represent

   (1) a halogen atom, or
   (2) a C₁₋₆ alkyl group;
   R⁴ represents
   (1) a hydrogen atom, or
   (2) a C₁₋₆ alkyl group;
   R⁵ represents a substituent represented by the following general formula (II): 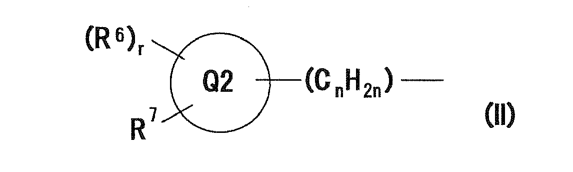 {wherein
   n is an integer of 1 to 4;
   r is 0 or an integer of 1 or 2;
   ring Q2 represents a monocyclic aromatic heterocyclic ring group;
   when r is 1, R⁶ represents
   (1) a C₁₋₆ alkyl group, or
   (2) a C₃₋₈ cycloalkyl group;
   when r is 2, R^{6'}s are the same or different and each represent
   (1) a C₁₋₆ alkyl group, or
   (2) a C₃₋₈ cycloalkyl group;
   R⁷ represents a substituent selected from the following (1) to (11);
   (1) -COOR⁸
      (R⁸ represents a hydrogen atom, a C₁₋₆ alkyl group, or an aralkyl group),
   (2) -S(O)₁-NR^{9a}R^{9b}
      (1 is an integer of 1 or 2, and R^{9a} and R^{9b} are the same or different and each represent a hydrogen atom or a C₁₋₆ alkyl group),
   (3) -O-R¹⁰
      (R10 represents an aralkyl group),
   (4) a monocyclic aromatic heterocyclic ring group substituted with 1 to 3 substituents that are the same or different and selected from the following group A:
      [group A]
      a. a C₁₋₆ alkyl group which may be substituted with 1 to 3 halogen atoms that are the same or different, and
      b. a halogen atom,
   (5) an aryl group substituted with 1 to 5 substituents that are the same or different and selected from the following group B:
      [group B]
      a. a C₁₋₆ alkyl group which may be substituted with 1 to 3 halogen atoms that are the same or different,
      b. a halogen atom,
      c. a cyano group,
      d. -NR^{11a}R^{11b}
         (R^{11a} and R^{11b} are the same or different and each represent a hydrogen atom or a C₁₋₆ alkyl group),
      e. -S(O)ₘ-R¹²
         (m is an integer of 1 or 2, and R12 represents a C1-6 alkyl group),
      f. -O-R¹³
         (R¹³ represents a hydrogen atom or a C₁₋₆ alkyl group),
      g. a nitro group, and
      h. an aryl group,
   (6) a C₃₋₈ cycloalkyl group,
   (7) an adamantyl group,
   (8) -S-R¹⁴
      (R¹⁴ represents a C₁₋₆ alkyl group or an aryl group),
   (9) -CONR^{15a}R^{15b}
      (R^{15a} represents a hydrogen atom or a C₁₋₆ alkyl group, and R^{15b} represents an aralkyl group, or R^{15a} and R^{15b}, together with an adjacent nitrogen atom, may form a monocyclic nonaromatic heterocyclic ring),
   (10) a C₁₋₆ alkyl group substituted with 1 to 3 substituents that are the same or different and selected from the following group C:
      [group C]
      a. -COOR¹⁶
         (R¹⁶ represents a hydrogen atom, a C₁₋₆ alkyl group, or an aralkyl group),
      b. -CONR^{17a}R^{17b}
         (R^{17a} and R^{17b} are the same or different and each represent a hydrogen atom, a C₁₋₆ alkyl group, or an aralkyl group, or R^{17a} and R^{17b}, together with an adjacent nitrogen atom, may form a monocyclic nonaromatic heterocyclic ring),
      c. a monocyclic aromatic heterocyclic ring group which may be substituted with 1 to 3 substituents that are the same or different and selected from the above group A,
      d. an aryl group which may be substituted with 1 to 5 substituents that are the same or different and selected from the above group B,
      e. -S-R¹⁸
         (R¹⁸ represents a hydrogen atom, a C₁₋₆ alkyl group, or an aryl group),
      f. -O-R¹⁹
         (_{R}¹⁹ represents a hydrogen atom, an aryl group, an aralkyl group, a C₁₋₆ alkyl group, or a C₁₋₆ alkyl group substituted with 1 to 3 substituents that are the same or different and selected from the following group D)
         [group D]

      a'. a halogen atom,
      b'. -COOR²⁰
         (R²⁰ represents a hydrogen atom or a C₁₋₆ alkyl group), and
      c'. -CONR^{21a}R^{21b}
         (R^{21a} and R^{21b} are the same or different and each represent a hydrogen atom, a C₁₋₆ alkyl group, or an aralkyl group, or R^{21a} and R^{21b}, together with an adjacent nitrogen atom, may form a monocyclic nonaromatic heterocyclic ring), g. -O-CO-R²²
         (R²² represents a C₁₋₆ alkyl group, an aryl group, a C₃-₈ cycloalkyl group, or a monocyclic aromatic heterocyclic ring group),
      h. -NR²³-CO-R²⁴
         (R²³ represents a hydrogen atom or a C₁₋₆ alkyl group, and R²⁴ represents a C₁₋₆ alkyl group or an aryl group [the aryl group may be substituted with 1 to 5 halogen atoms that are the same or different]), and
      i. -NR²⁵-S(O)ₚ-R²⁶
         (R²⁵ represents a hydrogen atom or a C₁₋₆ alkyl group, p is an integer of 1 or 2, and R²⁶ represents an aryl group which may be substituted with 1 to 5 C₁₋₆ alkyl groups that are the same or different), and
   (11) a polycyclic aromatic heterocyclic ring group} or R⁵ and R⁴, together with an adjacent nitrogen atom, may form a substituent represented by the following general formula (III): 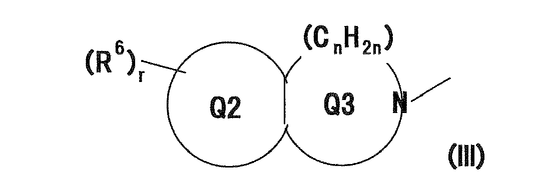 (wherein
      ring Q3 represents a monocyclic nonaromatic heterocyclic ring;
      ring Q2, n, r and R⁶ have the same meanings as defined above)].
2. The pyrazole compound represented by the following general formula (IV) or a salt thereof or a solvate thereof according to the above 1: 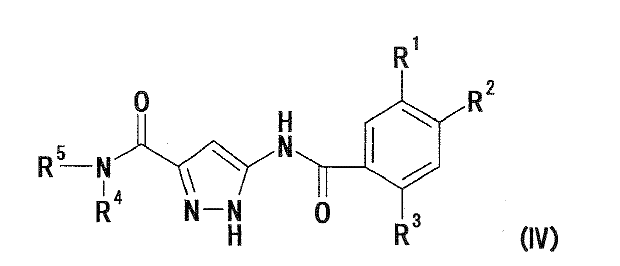 [wherein
   R¹ and R² are the same or different and each represent a halogen atom;
   R³ represents a halogen atom or a C₁₋₆ alkyl group;
   R⁴ and R⁵ have the same meanings as defined in claim 1].
3. The pyrazole compound represented by the following general formula (V) or a salt thereof or a solvate thereof according to the above 2: 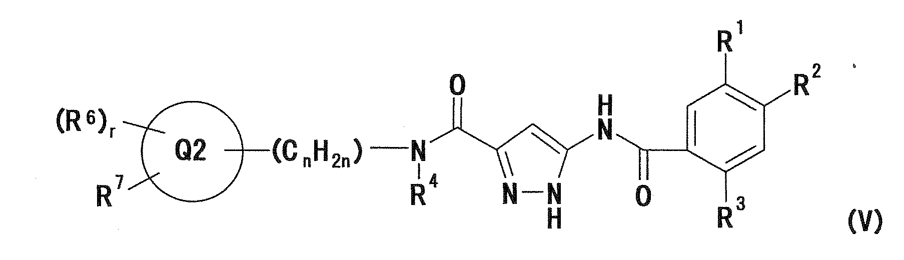 [wherein
   R¹ and R² are the same or different and each represent a halogen atom;
   R³ represents a halogen atom or a C₁₋₄ alkyl group;
   R⁴ represents a hydrogen atom;
   n is an integer of 1;
   r is 0 or an integer of 1 or 2; ring Q2 represents a monocyclic aromatic heterocyclic ring group;
   when r is 1, R⁶ represents
   (1) a C₁₋₄ alkyl group, or
   (2) a C₃₋₈ cycloalkyl group;
      when r is 2, R^{6'}S are the same or different and each represent

   (1) C₁₋₄ alkyl group;
      R⁷ represents a substituent selected from the following (1) to (11);
   (1) -COOR⁸
      (R⁸ represents a hydrogen atom, a C₁₋₄ alkyl group, or an aralkyl group),
   (2) -S(O)₁-NR^{9a}R^{9b}
      (l is an integer of 1 or 2, and R^{9a} and R^{9b} are the same or different and each represent a C₁₋₄ alkyl group),
   (3) -O-R¹⁰
      (R¹⁰ represents an aralkyl group),
   (4) a monocyclic aromatic heterocyclic ring group substituted with 1 to 3 substituents that are the same or different and selected from the following group A:
      [group A]
      a. a C₁₋₄ alkyl group which may be substituted with 1 to 3 halogen atoms that are the same or different, and
      b. a halogen atom,
   (5) an aryl group substituted with 1 to 5 substituents that are the same or different and selected from the following group B:
      [group B]
      a. a C₁₋₄ alkyl group which may be substituted with 1 to 3 halogen atoms that are the same or different,
      b. a halogen atom,
      c. a cyano group,
      d. -NR^{11a}R^{11b}
         (R^{11a} and R^{11b} are the same or different and each represent a C₁₋₄ alkyl group,
      e. -S(O)ₘ-R¹²
         (m is an integer of 1 or 2, and R¹² represents a C₁₋₄ alkyl group),
      f. -O-R¹³
         (R¹³ represents a hydrogen atom or a C₁₋₄ alkyl group),
      g. a nitro group, and
      h. an aryl group,
   (6) a C₃₋₈ cycloalkyl group,
   (7) an adamantyl group,
   (8) -S-R¹⁴
      (R¹⁴ represents an aryl group),
   (9) -CONR^{15a}R^{15b}
      (R^{15a} represents a hydrogen atom, and R^{15b} represents an aralkyl group, or R^{15a} and R^{15b}, together with an adjacent nitrogen atom, may form a monocyclic nonaromatic saturated heterocyclic ring),
   (10) a C₁₋₄ alkyl group substituted with 1 to 3 substituents that are the same or different and selected from the following group C:
      [group C]
      a. -COOR¹⁶
         (R¹⁶ represents a hydrogen atom or a C₁₋₄ alkyl group),
      b. -CONR^{17a}R^{17b}
         (R^{17a} and R^{17b} represent a hydrogen atom),
      c. a monocyclic aromatic heterocyclic ring group which may be substituted with 1 to 3 substituents that are the same or different and selected from the above group A,
      d. an aryl group which may be substituted with 1 to 5 substituents that are the same or different and selected from the above group B,
      e. -S-R¹⁸
         (R¹⁸ represents a C₁₋₄ alkyl group),
      f. -O-R¹⁹
         (R¹⁹ represents a hydrogen atom, an aryl group, an aralkyl group, a C₁₋₄ alkyl group, or a C₁₋₄ alkyl group substituted with 1 to 3 substituents that are the same or different and selected from the following group D)
         [group D]
      a'. a halogen atom,
      b'. -COOR²⁰
         (R²⁰ represents a hydrogen atom or a C₁₋₄ alkyl group),
         and
      c'_{.} -CONR^{21a}R^{21b}
         (R^{21a} and R^{21b} are the same or different and each represent a hydrogen atom, a C₁₋₄ alkyl group, or an aralkyl group, or R^{21a} and R^{21b}, together with an adjacent nitrogen atom, may form a monocyclic nonaromatic saturated heterocyclic ring),
      g. -O-CO-R²²
         (R²² represents a C₁₋₄ alkyl group, an aryl group, a C₃-₈ cycloalkyl group, or a monocyclic aromatic heterocyclic ring group),
      h. -NR²³-CO-R²⁴
         (R²³ represents a hydrogen atom, and R²⁴ represents a C₁₋₄ alkyl group or an aryl group [the aryl group may be substituted with 1 to 5 halogen atoms that are the same or different]), and
      i. -NR²⁵-S(O)ₚ-R²¹
         (R²⁵ represents a hydrogen atom, p is an integer of 1 or 2, and R²⁶ represents an aryl group which may be substituted with 1 to 5 C₁₋₄ alkyl groups that are the same or different),
   (11) a polycyclic aromatic heterocyclic ring group obtained by condensation of a 5- or 6-membered monocyclic aromatic heterocyclic ring and 1 or 2 benzene rings, or R⁴ and R⁷, together with an adjacent nitrogen atom and ring Q2, may form a substituent represented by the following general formula (VI): 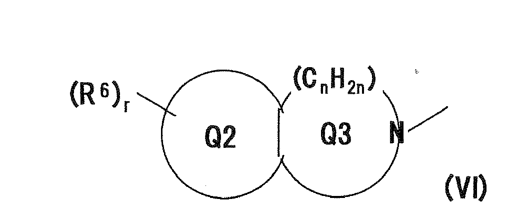 (wherein
      ring Q3 represents a monocyclic nonaromatic heterocyclic ring; and
      ring Q2, n, r and R⁶ have the same meanings as defined above)].
4. The pyrazole compound or a salt thereof or a solvate thereof according to the above 3, wherein R¹, R², R³, R⁴, R⁶, R⁷, n, r, and ring Q2 in the general formula (V) each represent as follows:
   R¹ and R² represent a fluorine atom;
   R³ represents a chlorine atom or a methyl group;
   R⁴ represents a hydrogen atom;
   n is an integer of 1;
   r is 0 or an integer of 1 or 2;
   ring Q2 represents a furyl group, a thienyl group, a thiazolyl group, an imidazolyl group, an isoxazolyl group, an oxadiazolyl group, a triazolyl group, a pyrazolyl group, a thiadiazolyl group, an oxazolyl group, or a pyrimidinyl group;
   when r is 1, R⁶ represents
   (1) a C₁₋₄ alkyl group, or
   (2) a C₃₋₈ cycloalkyl group;
   when r is 2, R⁶'s are the same or different and each represent
   (1) a C₁₋₄ alkyl group;
      R⁷ represents a substituent selected from the following (1) to (11);
   (1) -COOR⁸
      (R⁸ represents a hydrogen atom, a C₁₋₄ alkyl group, or a benzyl group),
   (2) -S(O)₁-NR^{9a}R^{9b}
      (1 is an integer of 2, and R^{9a} and R^{9b} are the same or different and each represent a C₁₋₄ alkyl group),
   (3) -O-R¹⁰
      (R¹⁰ represents a benzyl group),
   (4) a monocyclic aromatic heterocyclic ring group substituted with 1 to 3 substituents that are the same or different and selected from the following group A (the aromatic heterocyclic ring group is a thiazolyl group, a thienyl group, an isoxazolyl group, or a pyridyl group)
      [group A]
      a. a C₁₋₄ alkyl group which may be substituted with 1 to 3 halogen atoms that are the same or different, and
      b. a halogen atom,
   (5) a phenyl group substituted with 1 to 5 substituents that are the same or different and selected from the following group B:
      [group B]
      a. a C₁₋₄ alkyl group which may be substituted with 1 to 3 halogen atoms that are the same or different,
      b. a halogen atom,
      c. a cyano group,
      d. -NR^{11a}R^{11b}
         (R^{11a} and R^{11b} are the same or different and each represent a C₁₋₄ alkyl group,
      e. -S(O)ₘ-R¹²
         (m is an integer of 2, and R¹² represents a C₁₋₄ alkyl group),
      f. -O-R¹³
         (R¹³ represents a hydrogen atom or a C₁₋₄ alkyl group),
      g. a nitro group, and
      h. a phenyl group,
   (6) a C₃₋₈ cycloalkyl group,
   (7) an adamantyl group,
   (8) -S-R¹⁴
      (R¹⁴ represents a phenyl group),
   (9) -CONR^{15a}R^{15b}
      (R^{15a} represents a hydrogen atom, and R^{15b} represents a benzyl group, or R^{15a} and R^{15b}, together with an adjacent nitrogen atom, may form a piperidine ring),
   (10) a C₁₋₄ alkyl group substituted with 1 to 3 substituents that are the same or different and selected from the following group C:
      [group C]
      a. -COOR¹⁶
         (R¹⁶ represents a hydrogen atom or a C₁₋₄ alkyl group),
      b. -CONR^{17a}R^{17b}
         (R^{17a} and R^{17b} represent a hydrogen atom),
      c. a thiazolyl group which may be substituted with 1 or 2 C₁₋₄ alkyl groups that are the same or different,
      d. a phenyl group which may be substituted with 1 to 5 substituents that are the same or different and selected from halogen atoms and C₁₋₄ alkyl groups,
      e_{.} -S-R¹⁸
         (R¹⁸ represents a C₁₋₄ alkyl group),
      f. -O-R¹⁹
         (R¹⁹ represents a hydrogen atom, a phenyl group, a benzyl group, a C₁₋₄ alkyl group, or a C₁₋₄ alkyl group substituted with 1 to 3 substituents that are the same or different and selected from the following group D)
         [group D]

      a'_{.} a halogen atom,
      b'. -COOR²⁰
         (R²⁰ represents a hydrogen atom or a C₁₋₄ alkyl group), c'. -CONR^{21a}R^{21b}
         (R^{21a} and R^{22b} are the same or different and each represent a hydrogen atom, a C₁₋₄ alkyl group, or a benzyl group, or R^{21a} and R^{21b}, together with an adjacent nitrogen atom, may form a morpholine ring),
      g. -O-CO-R²²
         (R²² represents a C₁₋₄ alkyl group, a phenyl group, a C₃₋₈ cycloalkyl group, or a thienyl group),
      h. -NR²³-CO-R²⁴
         (R²³ represents a hydrogen atom, and R²⁴ represents a C₁₋₄ alkyl group or a phenyl group [the phenyl group may be substituted with 1 to 5 halogen atoms that are the same or different]), and
      i. NR²⁵-S(O)ₚ-R²⁶
         (R²⁵ represents a hydrogen atom, p is an integer of 2, and R²⁶ represents a phenyl group which may be substituted with 1 to 5 C₁₋₄ alkyl groups that are the same or different),
   (11) a polycyclic aromatic heterocyclic ring group obtained by condensation of a 5- or 6-membered monocyclic aromatic heterocyclic ring and 1 benzene ring, or R⁴ and R⁷, together with an adjacent nitrogen atom and ring Q2, may form a substituent represented by the following general formula (VII): 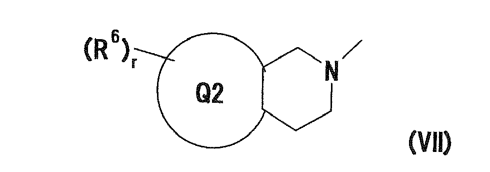 (wherein
      ring Q2, r, and R⁶ have the same meanings as defined above).
5. The pyrazole compound or a salt thereof or a solvate thereof according to the above 1 selected from the group consisting of the following (1-1) to (1-88), (2-1), and (3-1) to (3-25):
   (1-1)
      5-(2-chloro-4,5-difluoro-benzoylamino)-1H-pyrazole-3-carboxylic acid (5-ethoxycarbonyl-furan-2-ylmethyl)-amide:
   (1-2)
      5-(2-chloro-4,5-difluoro-benzoylamino)-1H-pyrazole-3-carboxylic acid (3-diethylsulfamoyl-thiophen-2-ylmethyl)-amide:
   (1-3)
      5-(2-chloro-4,5-difluoro-benzoylamino)-1H-pyrazole-3-carboxylic acid (3-benzyloxy-thiophen-2-ylmethyl)-amide:
   (1-4)
      5-(2-chloro-4,5-difluoro-benzoylamino)-1H-pyrazole-3-carboxylic acid [5-(2-methyl-thiazol-4-yl)-thiophen-2-ylmethyl]-amide:
   (1-5)
      5-(2-chloro-4,5-difluoro-benzoylamino)-1H-pyrazole-3-carboxylic acid [2-(4-chloro-phenyl)-thiazol-4-ylmethyl]-amide:
   (1-6)
      5-(2-chloro-4,5-difluoro-benzoylamino)-1H-pyrazole-3-carboxylic acid [4-methyl-2-(4-trifluoromethyl-phenyl)-thiazol-5-ylmethyl]-amide:
   (1-7)
      5-(2-chloro-4,5-difluoro-benzoylamino)-1H-pyrazole-3-carboxylic acid [4-methyl-2-(5-methyl-thiophen-2-yl)-thiazol-5-ylmethyl]-amide:
   (1-8)
      5-(2-chloro-4,5-difluoro-benzoylamino)-1H-pyrazole-3-carboxylic acid [2-(5-chlorothiophen-2-yl)-4-methyl-thiazol-5-ylmethyl]-amide:
   (1-9)
      5-(2-chloro-4,5-difluoro-benzoylamino)-1H-pyrazole-3-carboxylic acid (2-benzo[b]thiophen-2-yl-4-methyl-thiazol-5-ylmethyl)-amide:
   (1-10)
      5-(2-chloro-4,5-difluoro-benzoylamino)-1H-pyrazole-3-carboxylic acid (2-ethoxycarbonyl-4-methyl-thiazol-5-ylmethyl)-amide:
   (1-11)
      5-(2-chloro-4,5-difluoro-benzoylamino)-1H-pyrazole-3-carboxylic acid (4-adamantan-1-yl-2-methyl-thiazol-5-ylmethyl)-amide:
   (1-12)
      5-(2-chloro-4,5-difluoro-benzoylamino)-1H-pyrazole-3-carboxylic acid (2-methyl-6,7-dihydro-4H-thiazolo[5,4-c]pyridin-5-yl)-amide:
   (1-13)
      5-(2-chloro-4,5-difluoro-benzoylamino)-1H-pyrazole-3-carboxylic acid (5-ethoxycarbonyl-4-methyl-1H-imidazol-2-ylmethyl)-amide:
   (1-14)
      5-(2-chloro-4,5-difluoro-benzoylamino)-1H-pyrazole-3-carboxylic acid [3-(4-cyano-phenyl)-5-methylisoxazol-4-ylmethyl]-amide:
   (1-15)
      5-(2-chloro-4,5-difluoro-benzoylamino)-1H-pyrazole-3-carboxylic acid [3-(4-dimethylamino-phenyl)-5-methylisoxazol-4-ylmethyl]-amide:
   (1-16)
      5-(2-chloro-4,5-difluoro-benzoylamino)-1H-pyrazale-3-carboxylic acid [3-(4-methanesulfonyl-phenyl)-5-methylisoxazol-4-ylmethyl]-amide:
   (1-17)
      5-(2-chloro-4,5-difluoro-benzoylamino)-1H-pyrazole-3-carboxylic acid [3-(2-fluoro-phenyl)-5-methylisoxazol-4-ylmethyl]-amide:
   (1-18)
      5-(2-chloro-4,5-difluoro-benzoylamino)-1H-pyrazole-3-carboxylic acid (5,3'-dimethyl-[3,5']biisoxazolyl-4'-ylmethyl)-amide:
   (1-19)
      5-(2-chloro-4,5-difluoro-benzoylamino)-1H-pyrazole-3-carboxylic acid [3-methyl-5-(6-methyl-pyridin-2-yl)-isoxazol-4-ylmethyl]-amide:
   (1-20)
      5-(2-chloro-4,5-difluoro-benzoylamino)-1H-pyrazole-3-carboxylic acid [5-(2-chloro-pyridin-4-yl)-3-methylisoxazol-4-ylmethyl]-amide:
   (1-21)
      5-(2-chloro-4,5-difluoro-benzoylamino)-1H-pyrazole-3-carboxylic acid [3-methyl-5-(6-methyl-pyridin-3-yl)-isoxazol-4-ylmethyl]-amide:
   (1-22)
      5-(2-chloro-4,5-difluoro-benzoylamino)-1H-pyrazole-3-carboxylic acid [5-(2,4-dimethyl-thiazol-5-yl)-3-methylisoxazol-4-ylmethyl]-amide:
   (1-23)
      5-(2-chloro-4,5-difluoro-benzoylamino)-1H-pyrazole-3-carboxylic acid (3-ethoxycarbonylisoxazol-5-ylmethyl)-amide:
   (1-24)
      5-(2-chloro-4,5-difluoro-benzoylamino)-1H-pyrazole-3-carboxylic acid [2-methyl-5-(2-methylsulfanyl-ethyl)-oxazol-4-ylmethyl]-amide:
   (1-25)
      5-(2-chloro-4,5-difluoro-benzoylamino)-1H-pyrazole-3-carboxylic acid [4-(4-fluoro-phenyl)-2-methyl-oxazol-5-ylmethyl]-amide:
   (1-26)
      5-(2-chloro-4,5-difluoro-benzoylamino)-1H-pyrazole-3-carboxylic acid [4-(3-fluoro-phenyl)-2-methyl-oxazol-5-ylmethyl] -amide:
   (1-27)
      5-(2-chloro-4,5-difluoro-benzoylamino)-1H-pyrazole-3-carboxylic acid [4-(2-fluoro-phenyl)-2-methyl-oxazol-5-ylmethyl]-amide:
   (1-28)
      5-(2-chloro-4,5-difluoro-benzoylamino)-1H-pyrazole-3-carboxylic acid [4-(4-chloro-phenyl)-2-methyl-oxazol-5-ylmethyl]-amide:
   (1-29)
      5-(2-chloro-4,5-difluoro-benzoylamino)-1H-pyrazole-3-carboxylic acid [4-(4-methyl-phenyl)-2-methyl-oxazol-5-ylmethyl]-amide:
   (1-30)
      5-(2-chloro-4,5-difluoro-benzoylamino)-1H-pyrazole-3-carboxylic acid [4-(3-methyl-phenyl)-2-methyl-oxazol-5-ylmethyl]-amide:
   (1-31)
      5-(2-chloro-4,5-difluoro-benzoylamino)-1H-pyrazole-3-carboxylic acid [4-(3-methoxy-phenyl)-2-methyl-oxa.zol-5-ylmethyl]-amide:
   (1-32)
      5-(2-chloro-4,5-difluoro-benzoylamino)-1H-pyrazole-3-carboxylic acid (4-biphenyl-4-yl-2-methyl-oxazol-5-ylmethyl)-amide:
   (1-33)
      5-(2-chloro-4,5-difluoro-benzoylamino)-1H-pyrazole-3-carboxylic acid (2-methyl-4-phenethyl-oxazol-5-ylmethyl)-amide:
   (1-34)
      5-(2-chloro-4,5-difluoro-benzoylamino)-1H-pyrazole-3-carboxylic acid (4-ethoxycarbonyl-2-methyl-oxazol-5-ylmethyl)-amide:
   (1-35)
      5-(2-chloro-4,5-difluoro-benzoylamino)-1H-pyrazole-3-carboxylic acid (4-hydroxymethyl-2-methyl-oxazol-5-ylmethyl)-amide:
   (1-36)
      5-(2-chloro-4,5-difluoro-benzoylamino)-1H-pyrazole-3-carboxylic acid (4-benzyl-2-methyl-oxazol-5-ylmethyl)-amide p-toluenesulfonate:
   (1-37)
      5-(2-chloro-4,5-difluoro-benzoylamino)-1H-pyrazole-3-carboxylic acid (4-cyclopropyl-2-methyl-oxazol-5-ylmethyl)-amide:
   (1-38)
      5-(2-chloro-4,5-difluoro-benzoylamino)-1H-pyrazole-3-carboxylic acid (4-cyclohexyl-2-methyl-oxazol-5-ylmethyl)-amide:
   (1-39)
      5-(2-chloro-4,5-difluoro-benzoylamino)-1H-pyrazole-3-carboxylic acid (2-cyclopropyl-4-methyl-oxazol-5-ylmethyl)-amide:
   (1-40)
      5-(2-chloro-4,5-difluoro-benzoylamino)-1H-pyrazole-3-carboxylic acid (2-cyclohexyl-4-methyl-oxazol-5-ylmethyl)-amide:
   (1-41)
      5-(2-chloro-4,5-difluoro-benzoylamino)-1H-pyrazole-3-carboxylic acid (2-benzyl-4-methyl-oxazol-5-ylmethyl)-amide:
   (1-42)
      5-(2-chloro-4,5-difluoro-benzoylamino)-1H-pyrazole-3-carboxylic acid (4-methyl-2-phenethyl-oxazol-5-ylmethyl)-amide:
   (1-43)
      5-(2-chloro-4,5-difluoro-benzoylamino)-1H-pyrazole-3-carboxylic acid (4-methyl-2-trityloxymethyl-oxazol-5-ylmethyl)-amide:
   (1-44)
      5-(2-chloro-4,5-difluoro-benzoylamino)-1H-pyrazole-3-carboxylic acid (4-methyl-2-phenoxymethyl-oxazol-5-ylmethyl)-amide:
   (1-45)
      5-(2-chloro-4,5-difluoro-benzoylamino)-1H-pyrazole-3-carboxylic acid (2-methoxymethyl-4-methyl-oxazol-5-ylmethyl)-amide:
   (1-46)
      5-(2-chloro-4,5-difluoro-benzoylamino)-1H-pyrazole-3-carboxylic acid [4-methyl-2-(2,2,2-trifluoro-ethoxymethyl)-oxazol-5-ylmethyl)]-amide:
   (1-47)
      5-(2-chloro-4,5-difluoro-benzoylamino)-1H-pyrazole-3-carboxylic acid (2-benzyloxymethyl-4-methyl-oxazol-5-ylmethyl)-amide:
   (1-48)
      5-(2-chloro-4,5-difluoro-benzoylamino)-1H-pyrazole-3-carboxylic acid (2-ethoxycarbonylmethoxymethyl-4-methyl-oxazol-5-ylmethyl)-amide:
   (1-49)
      5-(2-chloro-4,5-difluoro-benzoylamino)-1H-pyrazole-3-carboxylic acid (4-methyl-2-phenylsulfanyl-oxazol-5-ylmethyl)-amide:
   (1-50)
      5-(2-chloro-4,5-difluoro-benzoylamino)-1H-pyrazole-3-carboxylic acid (2-methoxycarbonyl-4-methyl-oxazol-5-ylmethyl)-amide:
   (1-51)
      5-(2-chloro-4,5-difluoro-benzoylamino)-1H-pyrazole-3-carboxylic acid (5-ethoxycarbonyl-4-methyl-oxazol-2-ylmethyl)-amide:
   (1-52)
      5-(2-chloro-4,5-difluoro-benzoylamino)-1H-pyrazole-3-carboxylic acid [5-(4-chloro-phenyl)-[1,3,4]oxadiazol-2-ylmethyl]-amide:
   (1-53)
      5-(2-chloro-4,5-difluoro-benzoylamino)-1H-pyrazole-3-carboxylic acid [5-(2-methyl-phenyl)-[1,3,4]oxadiazol-2-ylmethyl]-amide:
   (1-54)
      5-(2-chloro-4,5-difluoro-benzoylamino)-1H-pyrazole-3-carboxylic acid [5-(4-dimethylamino-phenyl)-[1,3,4]oxadiazol-2-ylmethyl]-amide:
   (1-55)
      5-(2-chloro-4,5-difluoro-benzoylamino)-1H-pyrazole-3-carboxylic acid [5-(4-trifluoromethyl-phenyl)-[1,3,4]oxadiazol-2-ylmethyl]-amide:
   (1-56)
      5-(2-chloro-4,5-difluoro-benzoylamino)-1H-pyrazole-3-carboxylic acid [5-(3-nitro-phenyl)-[1,3,4]oxadiazol-2-ylmethyl]-amide:
   (1-57)
      5-(2-chloro-4,5-difluoro-benzoylamino)-1H-pyrazole-3-carboxylic acid [5-(3-hydroxy-phenyl)-[1,3,4]oxadiazol-2-ylmethyl]-amide:
   (1-58)
      5-(2-chloro-4,5-difluoro-benzoylamino)-1H-pyrazole-3-carboxylic acid [5-(2-hydroxy-phenyl)-[1,3,4]oxadiazol-2-ylmethyl]-amide:
   (1-59)
      5-(2-chloro-4,5-difluoro-benzoylamino)-1H-pyrazole-3-carboxylic acid [5-(3-chloro-phenyl)-[1,3,4]oxadiazol-2-ylmethyl]-amide:
   (1-60)
      5-(2-chloro-4,5-difluoro-benzoylamino)-1H-pyrazole-3-carboxylic acid [5-(3-methoxy-phenyl)-[1,3,4]oxadiazol-2-ylmethyl]-amide:
   (1-61)
      5-(2-chloro-4,5-difluoro-benzoylamino)-1H-pyrazole-3-carboxylic acid [5-(2-methoxy-phenyl)-[2,3,4]oxadiazol-2-ylmethyl]-amide:
   (1-62)
      5-(2-chloro-4,5-difluoro-benzoylamino)-1H-pyrazole-3-carboxylic acid (5-ethoxycarbonylmethyl-[1,3,4]oxadiazol-2-ylmethyl)-amide:
   (1-63)
      5-(2-chloro-4,5-difluoro-benzoylamino)-1H-pyrazole-3-carboxylic acid [1-(4-chloro-benzyl)-5-methyl-1H-[1,2,3]triazol-4-ylmethyl]-amide:
   (1-64)
      5-(2-chloro-4,5-difluoro-benzoylamino)-1H-pyrazole-3-carboxylic acid [5-methyl-1-(4-methyl-benzyl)-1H-[1,2,3]triazol-4-ylmethyl]-amide:
   (1-65)
      5-(2-chloro-4,5-difluoro-benzoylamino)-1H-pyrazole-3-carboxylic acid [5-methyl-1-(2-methyl-thiazol-4-ylmethyl)-1H-[1,2,3]triazol-4-ylmethyl]-amide:
   (1-66)
      5-(2-chloro-4,5-difluoro-benzoylamino)-1H-pyrazole-3-carboxylic acid [3,5-dimethyl-1-(3-methyl-phenyl)-1H-pyrazol-4-ylmethyl]-amide:
   (1-67)
      5-(2-chloro-4,5-difluoro-benzoylamino)-1H-pyrazole-3-carboxylic acid [5-cyclopropyl-1-(4-fluoro-phenyl)-1H-pyrazol-4-ylmethyl]-amide:
   (1-68)
      5-(2-chloro-4,5-difluoro-benzoylamino)-1H-pyrazole-3-carboxylic acid (4-cyclopropyl-2-methyl-pyrimidin-5-ylmethyl)-amide:
   (1-69)
      5-(2-chloro-4,5-difluoro-benzoylamino)-1H-pyrazole-3-carboxylic acid [4-(4-methoxy-phenyl)-[1,2,3]thiadiazol-5-ylmethyl]-amide:
   (1-70)
      5-(2-chloro-4,5-difluoro-benzoylamino)-1H-pyrazole-3-carboxylic acid [5-methyl-3-(3-methyl-[1,2,4]oxadiazol-5-yl)-isoxazol-4-ylmethyl]-amide:
   (1-71)
      5-(2-chloro-4,5-difluoro-benzoylamino)-1H-pyrazole-3-carboxylic acid [2-methyl-4-(3-methyl-[1,2,4]oxadiazol-5-yl)-oxazol-5-ylmethyl]-amide:
   (1-72)
      5-(2-chloro-4,5-difluoro-benzoylamino)-2H-pyrazole-3-carboxylic acid [5-(3-fluoro-phenyl)-[2,3,4]oxadiazol-2-ylmethyl]-amide:
   (1-73)
      5-(2-chloro-4,5-difluoro-benzoylamino)-1H-pyrazole-3-carboxylic acid [5-(2-methyl-phenyl)-[1,3,4]oxadiazol-2-ylmethyl]-amide:
   (1-74)
      5-(2-chloro-4,5-difluoro-benzoylamino)-1H-pyrazole-3-carboxylic acid (5-benzyl-[1,3,4]oxadiazol-2-ylmethyl)-amide:
   (1-75)
      5-(2-chloro-4,5-difluoro-benzoylamino)-1H-pyrazole-3-carboxylic acid [5-(1-methyl-1H-pyrrol-2-yl)-[1,3,4]oxadiazol-2-ylmethyl]-amide:
   (1-76)
      5-(2-chloro-4,5-difluoro-benzoylamino)-1H-pyrazole-3-carboxylic acid [5-(5-methyl-2H-pyrazol-3-yl)-[1,3,4]oxadiazol-2-ylmethyl]-amide:
   (1-77)
      5-(2-chloro-4,5-difluoro-benzoylamino)-1H-pyrazole-3-carboxylic acid (5-benzo[b]thiophen-2-yl-[1,3,4]oxadiazol-2-ylmethyl)-amide:
   (1-78)
      5-(2-chloro-4,5-difluoro-benzoylamino)-1H-pyrazole-3-carboxylic acid [5-(4-methyl-[1,2,3]thiadiazol-5-yl)-[1,3,4]oxadiazol-2-ylmethyl]-amide:
   (1-79)
      5-(2-chloro-4,5-difluoro-benzoylamino)-1H-pyrazole-3-carboxylic acid [4-(3-fluoro-phenyl)-2-methyl-pyrimidin-5-ylmethyl]-amide:
   (1-80)
      5-(2-chloro-4,5-difluoro-benzoylamino)-1H-pyrazole-3-carboxylic acid (2-cyclopropyl-4-methyl-pyrimidin-5-ylmethyl)-amide:
   (1-81)
      5-(2-chloro-4,5-difluoro-benzoylamino)-1H-pyrazole-3-carboxylic acid (4-cyclopropyl-pyrimidin-5-ylmethyl)-amide:
   (1-82)
      5-[2-chloro-4,5-difluoro-benzoylamino)-1H-pyrazole-3-carboxylic acid (2,4-dicyclopropyl-pyrimidin-5-ylmethyl)-amide hydrochloride:
   (1-83)
      5-(2-chloro-4,5-difluoro-benzoylamino)-1H-pyrazole-3-carboxylic acid [4-(2,4-dimethyl-oxazol-5-yl)-2-methyl-pyrimidin-5-ylmethyl]-amide:
   (1-84)
      5-(2-chloro-4,5-difluoro-benzoylamino)-1H-pyrazole-3-carboxylic acid [4-(2,5-dimethyl-furan-3-yl)-[1,2,3]thiadiazol-5-ylmethyl]-amide:
   (1-85)
      5-(2-chloro-4,5-difluoro-benzoylamino)-2H-pyrazole-3-carboxylic acid [5-(3-chloro-phenyl)-[1,3,4]triazol-2-ylmethyl]-amide:
   (1-86)
      5-(2-ehloro-4,5-difluoro-benzoylamino)-1H-pyrazole-3-carboxylic acid [2-(5-ethyl-1H-[1,2,4]thiazol-3-ylmethyl)-4-methyl-oxazol-5-ylmethyl]-amide hydrochloride:
   (1-87)
      5-(2-chloro-4,5-difluoro-benzoylamino)-1H-pyrazole-3-carboxylic acid [5-(4-chloro-phenyl)-1H-[1,2,4]triazol-3-ylmethyl]-amide:
   (1-88)
      5-(2-chloro-4,5-difluoro-benzoylamino)-1H-pyrazole-3-carboxylic acid [5-(3,4-dimethoxy-benzyl)-1H-[1,2,4]triazol-3-ylmethyl]-amide:
   (2-1)
      5-(4,5-difluoro-2-methyl-benzoylamino)-1H-pyrazole-3-carboxylic acid (4-ethoxycarbonyl-2-methyl-oxazol-5-ylmethyl)-amide:
   (3-1)
      5-(2-chloro-4,5-difluoro-benzoylamino)-1H-pyrazole-3-carboxylic acid (5-carboxy-furan-2-ylmethyl)-amide: (3-2)
   sodium 5-({[5-(2-chloro-4,5-difluoro-benzoylamino)-1H-pyrazole-3-carbonyl]-amino}-methyl)-4-methyl-thiazole-2-carboxylate:
   (3-3)
      5-(2-chloro-4,5-difluoro-benzoylamino)-1H-pyrazole-3-carboxylic acid (3-carboxy-isoxazol-5-ylmethyl)-amide:
   (3-4)
      5-(2-chloro-4,5-difluoro-benzoylamino)-1H-pyrazole-3-carboxylic acid (4-acetoxymethyl-2-methyl-oxazol-5-ylmethyl)-amide:
   (3-5)
      5-(2-chloro-4,5-difluoro-benzoylamino)-1H-pyrazole-3-carboxylic acid (4-benzoyloxymethyl-2-methyl-oxazol-5-ylmethyl)-amide:
   (3-6)
      5-(2-chloro-4,5-difluoro-benzoylamino)-1H-pyrazole-3-carboxylic acid (4-carboxy-2-methyl-oxazol-5-ylmethyl)-amide:
   (3-7)
      5-(2-chloro-4,5-difluoro-benzoylamino)-1H-pyrazole-3-carboxylic acid (2-carboxymethoxymethyl-4-methyl-oxazol-5-ylmethyl)-amide:
   (3-8)
      5-(2-chloro-4,5-difluoro-benzoylamino)-1H-pyrazole-3-carboxylic acid (2-diethylcarbamoylmethoxymethyl-4-methyl-oxazol-5-ylmethyl)-amide:
   (3-9)
      5-(2-chloro-4,5-difluoro-benzoylamino)-1H-pyrazole-3-carboxylic acid [2-(benzylcarbamoyl-methoxymethyl)-4-methyl-oxazol-5-ylmethyl]-amide:
   (3-10)
      5-(2-chloro-4,5-difluoro-benzoylamino)-1H-pyrazole-3-carboxylic acid [4-methyl-2-(2-morpholin-4-yl-2-oxo-ethoxymethyl)-oxazol-5-ylmethyl]-amide:
   (3-11)
      5-(2-chloro-4,5-difluoro-benzoylamino)-1H-pyrazole-3-carboxylic acid {4-methyl-2-[(toluene-4-sulfonylamino)-methyl]-oxazol-5-ylmethyl}-amide:
   (3-12)
      5-(2-chloro-4,5-difluoro-benzoylamino)-1H-pyrazole-3-carboxylic acid [2-(acetylamino-methyl)-4-methyl-oxazol-5-ylmethyl]-amide dihydrochloride:
   (3-13)
      5-(2-chloro-4,5-difluoro-benzoylamino)-1H-pyrazole-3-carboxylic acid {2-[(4-chloro-benzoylamino)-methyl]-4-methyl-oxazol-5-ylmethyl}-amide:
   (3-14) sodium 5-({[5-(2-chloro-4,5-difluoro-benzoylamino)-1H-pyrazole-3-carbonyl]-amino}-methyl)-4-methyl-oxazole-2-carboxylate:
   (3-15)
      5-(2-chloro-4,5-difluoro-benzoylamino)-1H-pyrazole-3-carboxylic acid [4-methyl-2-(piperidine-1-carbonyl)-oxazol-5-ylmethyl]-amide:
   (3-16)
      5-(2-chloro-4,5-difluoro-benzoylamino)-1H-pyrazole-3-carboxylic acid (2-benzylcarbamoyl-4-methyl-oxazol-5-ylmethyl)-amide:
   (3-17)
      5-(2-chloro-4,5-difluoro-benzoylamino)-1H-pyrazole-3-carboxylic acid (2-benzyloxycarbonyl-4-methyl-oxazol-5-ylmethyl)-amide:
   (3-18)
      5-(2-chloro-4,5-difluoro-benzoylamino)-1H-pyrazole-3-carboxylic acid (5-carboxy-4-methyl-oxazol-2-ylmethyl)-amide:
   (3-19)
      5-(2-chloro-4,5-difluoro-benzoylamino)-1H-pyrazole-3-carboxylic acid (5-carboxymethyl-[1,3,4]oxadiazol-2-ylmethyl)-amide:
   (3-20)
      5-(2-chloro-4,5-difluoro-benzoylamino)-1H-pyrazole-3-carboxylic acid (5-carbamoylmethyl-[1,3,4]oxadiazol-2-ylmethyl)-amide:
   (3-21)
      5-(4,5-difluoro-2-methyl-benzoylamino)-1H-pyrazole-3-carboxylic acid (4-carboxy-2-methyl-oxazol-5-ylmethyl)-amide:
   (3-22)
      5-(2-chloro-4,5-difluoro-benzoylamino)-1H-pyrazole-3-carboxylic acid [2-methyl-4-(thiophene-2-carbonyloxymethyl)-oxazol-5-ylmethyl]-amide:
   (3-23)
      5-(2-chloro-4,5-difluoro-benzoylamino)-1H-pyrazole-3-carboxylic acid (4-cyclohexanecarbonyloxymethyl-2-methyl-oxazol-5-ylmethyl)-amide:
   (3-24)
      5-(2-chloro-4,5-difluoro-benzoylamino)-1H-pyrazole-3-carboxylic acid (2-hydroxymethyl-4-methyl-oxazol-5-ylmethyl)-amide hydrochloride:
   (3-25)
      5-(2-chloro-4,5-difluoro-benzoylamino)-1H-pyrazole-3-carboxylic acid [2-(benzyl-methyl-carbamoyl)-4-methyl-oxazol-5-ylmethyl]-amide:
6. A pharmaceutical composition comprising the pyrazole compound or a salt thereof or a solvate thereof according to any one of the above 1 to 5.
7. A liver glycogen phosphorylase inhibitor comprising the pyrazole compound or a salt thereof or a solvate thereof according to any one of the above 1 to 5.
8. A blood sugar level lowering agent comprising the pyrazole compound or a salt thereof or a solvate thereof according to any one of the above 1 to 5.
9. A therapeutic or prophylactic agent for diabetes comprising the pyrazole compound or a salt thereof or a solvate thereof according to any one of the above 1 to 5.
10. A method for inhibiting liver glycogen phosphorylase comprising administration of a pharmaceutically effective amount of the pyrazole compound or a salt thereof or a solvate thereof according to any one of the above 1 to 5.
11. A method for lowering a blood sugar level comprising administration of a pharmaceutically effective amount of the pyrazole compound or a salt thereof or a solvate thereof according to any one of the above 1 to 5.
12. A method for therapeutic or prophylactic treatment of diabetes comprising administration of a pharmaceutically effective amount of the pyrazole compound or a salt thereof or a solvate thereof according to any one of the above 1 to 5.
13. Use of the pyrazole compound or a salt thereof or a solvate thereof according to any one of the above 1 to 5 for production of a liver glycogen phosphorylase inhibitor.
14. Use of the pyrazole compound according to any one of the above 1 to 5 or a salt thereof or a solvate thereof for production of a blood sugar level lowering agent.
15. Use of the pyrazole compound according to any one of the above 1 to 5 or a salt thereof or a solvate thereof for production of a therapeutic or prophylactic agent for diabetes.

### Advantages of the Invention

The present invention has a potent liver glycogen phosphorylase inhibitory effect that is superior in selectivity. Therefore, the present invention has an excellent blood sugar level lowering effect and is very useful as a therapeutic or prophylactic agent for diabetes with minimal adverse drug reactions.

### Best Mode for Carrying Out the Invention

The pyrazole skeleton of the pyrazole compound of present invention not only means the following structure: 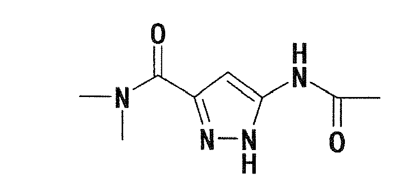 but also encompasses the following isomers.

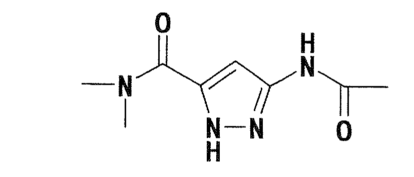

Therefore, the pyrazole compound of the present invention is not limited to pyrazole compounds represented by the above general formula (I) but encompasses the following pyrazole compounds (I').

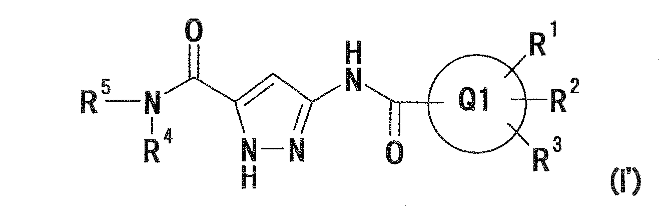

Furthermore, substituents used in this specification and sites thereof are defined as follows.

The "aryl group" means a monocyclic or polycyclic aromatic hydrocarbon group having 6 to 14 carbon atoms (the polycyclic aromatic hydrocarbon group means a condensed ring group, which has a benzene nucleus therein), and examples thereof include a phenyl group; polycyclic aromatic hydrocarbon groups having 8 carbon atoms such as a pentalenyl group; polycyclic aromatic hydrocarbon groups having 10 carbon atoms such as a naphthyl group and an azulenyl group; polycyclic aromatic hydrocarbon groups having 14 carbon atoms such as an anthryl group and a phenanthryl group; polycyclic aromatic hydrocarbon groups having 9 carbon atoms that include a saturated bond partially, such as an indenyl group and an indanyl group; polycyclic aromatic hydrocarbon groups having 10 carbon atoms that include a saturated bond partially, such as a 1,4-dihydronaphthyl group and a tetrahydronaphthyl group (for example, 1,2,3,4-tetrahydronaphthyl group); polycyclic aromatic hydrocarbon groups having 13 carbon atoms that included a saturated bond partially, such as a fluorenyl group, and so forth. Preferred examples thereof are monocyclic or polycyclic aromatic hydrocarbon groups having 6 to 10 carbon atoms, and more preferred examples thereof are a phenyl group and a naphthyl group. A phenyl group is yet more preferred.

The "halogen atom" means a fluorine atom, a chlorine atom, a bromine atom or an iodine atom, preferably a fluorine atom or a chlorine atom.

The "C₁₋₆ alkyl group" means a straight or branched alkyl group having 1 to 6 carbon atoms, and examples thereof include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1,2-dimethyl-propyl group, a hexyl group, a 1,3-dimethyl-butyl group, and so forth. A "C₁₋₄ alkyl group" is preferred, and a methyl group and an ethyl group are more preferred.

The "C₁₋₄ alkyl group" means a straight or branched alkyl group having 1 to 4 carbon atoms, and examples thereof include a methyl group, an ethyl group, a propyl group, an isopropyl group, a butyl group, an isobutyl group, a sec-butyl group, and a tert-butyl group. A methyl group and an ethyl group are preferred.

The "-CₙH₂ₙ-" means a straight or branched saturated hydrocarbon chain having n carbon atoms. n is an integer of 1 to 4, preferably an integer of 1 or 2, more preferably an integer of 1.

The "C₃₋₈ cycloalkyl group" means a monocyclic saturated hydrocarbon group having 3 to 8 carbon atoms, and examples thereof include a cyclopropyl group, a cyclobutyl group, a cyclopentyl group, a cyclohexyl group, a cycloheptyl group, and a cyclooctyl group. A "C₃₋₆ cycloalkyl group" is preferred, and a cyclopropyl group and a cyclohexyl group are more preferred.

The "C₃₋₆ cycloalkyl group" means a monocyclic saturated hydrocarbon group having 3 to 6 carbon atoms, and examples thereof include a cyclopropyl group, a cyclobutyl group, a cyclopentyl group, and a cyclohexyl group. A cyclopropyl group is preferred.

The "C₃₋₈ cycloalkenyl group" means a group comprising a cycloalkene having 3 to 8 carbon atoms, and examples thereof include a cyclopropenyl group, a cyclobutenyl group, a cyclopentenyl group, a cyclopentadienyl group, a cyclohexenyl group, a cyclohexadienyl group (for example, a 2,4-cyclohexadienyl group, a 2,5-cyclohexadienyl group), a cycloheptenyl group, a cyclooctenyl group, and so forth. A C₃-₆ cycloalkenyl group comprising a cycloalkene having 3 to 6 carbon atoms is preferred. A cyclohexenyl group is more preferred.

The "aralkyl group" means a "C₁₋₆ alkyl group" substituted with 1 to 3 "aryl groups" that are the same or different, and examples thereof include a benzyl group, a phenylethyl group, a phenylpropyl group, a phenylbutyl group, a phenylpentyl group, a phenylhexyl group, a diphenylmethyl group, a trityl group, a naphthylmethyl group, a naphthylethyl group, a naphthylpropyl group, a naphthylbutyl group, a naphthylpentyl group, and so forth. A "C₁₋₆ alkyl group" substituted with 1 to 3 phenyl groups or naphthyl groups is preferred, a "C₁₋₄ alkyl group" substituted with 1 to 3 phenyl groups or naphthyl groups is more preferred, and a benzyl group is yet more preferred.

The expression "C₁₋₆ alkyl group which may be substituted with 1 to 3 substituents that are the same or different" means an unsubstituted C₁₋₆ alkyl group or a C₁₋₆ alkyl group substituted with 1 to 3 substituents that are the same or different.

Preferred examples of the "C₁₋₆ alkyl group substituted with 1 to 3 halogen atoms that are the same or different" include "C₁₋₄ alkyl groups substituted with 1 to 3 halogen atoms that are the same or different", more preferred examples thereof include a trifluoromethyl group, a trichloromethyl group, a 2,2,2-trifluoroethyl group, and a 2,2,2-trichloromethyl group, and a trifluoromethyl group and a 2,2,2-trifluoroethyl group are yet more preferred.

The "aryl group which may be substituted with 1 to 5 substituents that are the same or different" means an unsubstituted aryl group or an aryl group substituted with 1 to 5 substituents that are the same or different.

Preferred examples of the "aryl group substituted with 1 to 5 substituents that are the same or different" include an "aryl group substituted with 1 to 3 substituents that are the same or different", and an aryl group substituted with 1 substituent is more preferred.

The "heterocyclic ring group" means a single ring group or a polycyclic group that comprises at least one heteroatom selected from a nitrogen atom, an oxygen atom, and a sulfur atom as atoms constituting a ring in addition to carbon atoms, and consists of 5 to 14 atoms constituting a ring. Examples thereof include a "monocyclic heterocyclic ring group", a "polycyclic heterocyclic ring group", a "aromatic heterocyclic ring group", or a "nonaromatic heterocyclic ring group". Preferred examples thereof include a "5- or 6-membered monocyclic aromatic heterocyclic ring group", a "5- to 7-membered monocyclic nonaromatic heterocyclic ring group", a "8- to 10-membered polycyclic aromatic heterocyclic ring group", and a "8- to 10-membered polycyclic nonaromatic heterocyclic ring group".

The "monocyclic heterocyclic ring group" means a "heterocyclic ring group", a single ring that consists of 5 to 7 atoms constituting a ring, and examples thereof include a "monocyclic aromatic heterocyclic ring group" and a "monocyclic nonaromatic heterocyclic ring group".

The "monocyclic aromatic heterocyclic ring group" means an aromatic ring group which is a single ring that comprises 1 to 4 heteroatoms selected from a nitrogen atom, an oxygen atom, and a sulfur atom that are the same or different as atoms constituting a ring in addition to carbon atoms, and consists of 5 to 7 atoms constituting a ring. Examples thereof include "5-membered monocyclic aromatic heterocyclic ring groups" such as pyrrolyl (for example, 1H-pyrrolyl, 2H-pyrrolyl, 3H-pyrrolyl), furyl, thienyl, imidazolyl (for example, 1H-imidazolyl, 2H-imidazolyl, 4H-imidazolyl), pyrazolyl (for example, 1H-pyrazolyl, 3H-pyrazolyl, 4H-pyrazolyl), oxazolyl, isoxazolyl, thiazolyl, isothiazolyl, triazolyl (e.g., 1H-[1,2,3]triazolyl, 2H-[1,2,3]triazolyl, 4H-[1,2,3]triazolyl, 1H-[1,2,4]triazolyl, 3H-[1,2,4]triazolyl, 4H-[1,2,4]triazolyl), tetrazolyl (for example, 1H-tetrazolyl, 2H-tetrazolyl, 5H-tetrazolyl), oxadiazolyl (for example, 1,2,3-oxadiazolyl, 1,2,4-oxadiazolyl, 1,2,5-oxadiazolyl, 1,3,4-oxadiazolyl), and thiadiazolyl (for example, [1,2,3]thiadiazolyl, [1,2,4]thiadiazolyl, [1,2,5]thiadiazolyl, [1,3,4]thiadiazolyl) groups; "6-membered monocyclic aromatic heterocyclic ring groups" such as pyridyl, pyrimidinyl, pyridazinyl, pyrazinyl, and triazinyl (for example, [1,2,3]triazinyl, [1,2,4]triazinyl, [1,3,5]triazinyl) groups; "7-membered monocyclic aromatic heterocyclic ring groups" such as an oxepinyl group, and so forth. Preferred examples thereof are "5- or 6-membered monocyclic aromatic heterocyclic ring groups", and more preferred examples thereof include furyl, thienyl, imidazolyl, pyrazolyl, oxazolyl, isoxazolyl, thiazolyl, triazolyl, oxadiazolyl, thiadiazolyl groups, pyridyl, and pyrimidinyl groups.

The "monocyclic nonaromatic heterocyclic ring group" means a nonaromatic ring group, which is a single ring that comprises 1 to 4 heteroatoms selected from a nitrogen atom, an oxygen atom, and a sulfur atom that are the same or different as atoms constituting a ring in addition to carbon atoms, and consists of 5 to 7 atoms constituting a ring. Examples thereof include "monocyclic nonaromatic saturated heterocyclic ring groups" and "monocyclic nonaromatic unsaturated heterocyclic ring groups".

The "monocyclic nonaromatic saturated heterocyclic ring group" means a "monocyclic nonaromatic heterocyclic ring group" that consists of saturated bonds constituting a ring, and specific examples thereof include "5-membered monocyclic nonaromatic saturated heterocyclic ring groups" such as pyrrolidinyl, tetrahydrofuryl, tetrahydrothienyl, imidazolidinyl, pyrazolidinyl, 1,3-dioxolanyl, 1,3-oxathiolanyl, oxazolidinyl, 2-oxopyrrolidinyl, thiazolidinyl and isothiazolidinyl groups; "6-membered monocyclic nonaromatic saturated heterocyclic ring groups" such as piperidyl, piperazinyl, tetrahydropyranyl, tetrahydrothiopyranyl, 1,4-dioxanyl, 1,4-dithianyl, morpholinyl, and thiomorpholinyl groups; "7-membered monocyclic nonaromatic saturated heterocyclic ring groups" such as an azepanyl group, and so forth. Preferred examples of the "monocyclic nonaromatic saturated heterocyclic ring group" include "5- or 6-membered monocyclic nonaromatic saturated heterocyclic ring groups", and a piperidyl group and a morpholinyl group are more preferred.

The "monocyclic nonaromatic unsaturated heterocyclic ring group" means a "monocyclic nonaromatic heterocyclic ring group" which includes at least one unsaturated bond constituting a ring, and specific examples thereof include "5-membered monocyclic nonaromatic unsaturated heterocyclic ring groups" such as imidazolinyl (for example, 2-imidazolinyl, 3-imidazolinyl, 4-imidazolinyl), pyrazolinyl (for example, 1-pyrazolinyl, 2-pyrazolinyl, 3-pyrazolinyl), oxazolinyl (for example, 2-oxazolinyl, 3-oxazolinyl, 4-oxazolinyl), isoxazolinyl (for example, 2-isoxazolinyl, 3-isoxazolinyl, 4-isoxazolinyl), thiazolinyl (for example, 2-thiazolinyl, 3-thiazolinyl, 4-thiazolinyl), and isothiazolinyl (for example, 2-isothiazolinyl, 3-isothiazolinyl, 4-isothiazolinyl) groups; "6-membered monocyclic nonaromatic unsaturated heterocyclic ring groups" such as pyranyl (for example, 2H-pyranyl, 4H-pyranyl) groups, and so forth.

The "monocyclic nonaromatic heterocyclic ring formed together with an adjacent nitrogen atom" has the same meaning as a ring constituting a "monocyclic nonaromatic heterocyclic ring group" comprising at least one nitrogen atom as atoms constituting a ring. A "monocyclic nonaromatic saturated heterocyclic ring" comprising at least one nitrogen atom as atoms constituting a ring is preferred, and more preferred examples thereof include "5-membered monocyclic nonaromatic saturated heterocyclic rings" such as pyrrolidine, imidazolidine, pyrazolidine, oxazolidine, 2-oxopyrrolidine, thiazolidine, and isothiazolidine; and "6-membered monocyclic nonaromatic saturated heterocyclic rings" such as piperidine, piperazine, morpholine, and thiomorpholine. Piperidine is yet more preferred.

The "polycyclic heterocyclic ring group" means a "heterocyclic ring group" which is a condensed ring that comprises 8 to 14 atoms constituting a ring, and examples thereof include "polycyclic aromatic heterocyclic ring groups" and "polycyclic nonaromatic heterocyclic ring groups".

The "polycyclic aromatic heterocyclic ring group" means an aromatic ring group, a condensed ring that comprises 1 to 4 heteroatoms selected from a nitrogen atom, an oxygen atom, and a sulfur atom that are the same or different as atoms constituting a ring in addition to carbon atoms, and consists of 8 to 14 atoms constituting a ring. Examples thereof include a "polycyclic aromatic heterocyclic ring group" obtained by condensation of a "monocyclic aromatic heterocyclic ring" and 1 or 2 rings selected from a benzene ring, a "monocyclic aromatic heterocyclic ring", a "monocyclic nonaromatic heterocyclic ring", a "C₃-₈ cycloalkane", and a "C₃-₈ cycloalkene" that are the same or different, a "polycyclic aromatic heterocyclic ring group" obtained by condensation of a "monocyclic nonaromatic heterocyclic ring" and 1 or 2 benzene rings, and so forth. Here, the "monocyclic aromatic heterocyclic ring", "monocyclic nonaromatic heterocyclic ring", "C₃₋₈ cycloalkane", and "C₃₋₈ cycloalkene" have the same meanings as a ring constituting a "monocyclic aromatic heterocyclic ring group", a "monocyclic nonaromatic heterocyclic ring group", a "C₃₋₈ cycloalkyl group", and a "C₃₋₈ cycloalkenyl group" described above, respectively. Preferred examples of the "polycyclic aromatic heterocyclic ring group" include a "polycyclic aromatic heterocyclic ring group" obtained by condensation of a "monocyclic aromatic heterocyclic ring" and 1 or 2 rings selected from a benzene ring, a "monocyclic aromatic heterocyclic ring", a "monocyclic nonaromatic heterocyclic ring", a "C₃₋₈ cycloalkane", and "C₃₋₈ cycloalkene" that are the same or different or a "polycyclic aromatic heterocyclic ring group" obtained by condensation of a "monocyclic nonaromatic heterocyclic ring" and 1 or 2 benzene rings. More preferred examples of the "polycyclic aromatic heterocyclic ring group" include a "polycyclic aromatic heterocyclic ring group" obtained by condensation of a "monocyclic aromatic heterocyclic ring" and 1 or 2 "monocyclic nonaromatic heterocyclic rings" and a "polycyclic aromatic heterocyclic ring group" obtained by condensation of a "monocyclic aromatic heterocyclic ring" and 1 or 2 benzene rings.

Examples of the "polycyclic aromatic heterocyclic ring group" obtained by condensation of a "5-membered monocyclic aromatic heterocyclic ring" and 1 or 2 benzene rings include benzofuranyl, isobenzofuranyl, benzothienyl, benzooxazolyl, benzoisoxazolyl, benzothiazolyl, benzoimidazolyl (for example, 1H-benzoimidazolyl, 2H-benzoimidazolyl), indazolyl (for example, 1H-indazolyl, 2H-indazolyl, 3H-indazolyl), benzotriazolyl (e.g., 1H-benzotriazolyl, 2H-benzotriazolyl), indolyl (for example, 1H-indolyl, 2H-indolyl, 3H-indolyl), isoindolyl (for example, 1H-isoindolyl, 2H-isoindolyl), and dibenzofuranyl groups, and so forth.

Examples of the "polycyclic aromatic heterocyclic ring group" obtained by condensation of a "6-membered monocyclic aromatic heterocyclic ring" and 1 or 2 benzene rings include quinolyl, isoquinolyl, cinnolinyl, quinazolinyl, quinoxalinyl, and phthalazinyl groups, and so forth.

Examples of the "polycyclic aromatic heterocyclic ring group" obtained by condensation of a "7-membered monocyclic aromatic heterocyclic ring" and 1 or 2 benzene rings include groups constituted by a ring such as 7-oxabenzocycloheptane.

Specific examples of the "polycyclic aromatic heterocyclic ring group" obtained by condensation of a "monocyclic aromatic heterocyclic ring" and 1 or 2 "monocyclic aromatic heterocyclic rings" include indolizinyl, purinyl (for example, 7H-purinyl, 8H-purinyl, 9H-purinyl), thienopyrazolyl (for example, 1H-thieno[2,3-c]pyrazolyl, 2H-thieno[2,3-c]pyrazolyl, 3H-thieno[2,3-c]pyrazolyl, 5H-thieno[2,3-c]pyrazolyl), naphthyridinyl (for example, [1,8]naphthyridinyl, [2,7]naphthyridinyl, [2,6]naphthyridinyl), quinolizinyl, and pteridinyl groups, and so forth.

Examples of the "polycyclic aromatic heterocyclic ring group" obtained by condensation of a "monocyclic aromatic heterocyclic ring" and 1 or 2 "monocyclic nonaromatic heterocyclic rings" include 2,3-dihydrofuro[2,3-b]pyridyl, 4,5,6,7-tetrahydrofuro[2,3-b]pyridyl, 5,6-dihydro-4H-thieno[2,3-b]pyrrolyl, and 1,2,3,4-tetrahydro[1,8]naphthyridinyl groups, and so forth. A "polycyclic aromatic heterocyclic ring group" obtained by condensation of a "5-membered monocyclic aromatic heterocyclic ring" and a "6-membered monocyclic nonaromatic heterocyclic ring" is preferred, and 4,5,6,7-tetrahydrothiazolo[5,4-c]pyridyl group is more preferred.

Examples of the "polycyclic aromatic heterocyclic ring group" obtained by condensation of a "monocyclic aromatic heterocyclic ring" and 1 or 2 rings selected from "C₃₋₈ cycloalkane" and "C₃₋₈ cycloalkene" that are the same or different include 5,6,7,8-tetrahydroquinolyl, 4,5,6,7-tetrahydro-1H-indolyl, 5,8-dihydroquinolyl, and 4,7-dihydro-1H-indolyl groups, and so forth.

Examples of the "polycyclic aromatic heterocyclic ring group" obtained by condensation of a "monocyclic nonaromatic heterocyclic ring" and 1 or 2 benzene rings include 1,2,3,4-tetrahydroquinolyl, chromanyl, isochromanyl, chromenyl (for example, 2H-chromenyl, 4H-chromenyl), 2,3-dihydro-1H-indolyl, 2,3-dihydro-1H-isoindolyl, 1,3-dihydro-benzo[c]thienyl, and 1,3-dihydro-isobenzofuranyl groups, and so forth.

The "polycyclic nonaromatic heterocyclic ring group" means a nonaromatic ring group, which is a condensed ring that comprises 1 to 4 heteroatoms selected from a nitrogen atom, an oxygen atom, and a sulfur atom that are the same or different as atoms constituting a ring in addition to carbon atoms, and consists of 8 to 14 atoms constituting a ring. Examples thereof include a "polycyclic nonaromatic heterocyclic ring group" obtained by condensation of a "5- to 7-membered monocyclic nonaromatic heterocyclic ring" and a 1 or 2 rings selected from "5 to 7-membered monocyclic nonaromatic heterocyclic rings", "C₃-₈ cycloalkane", and "C₃-₈ cycloalkene" that are the same or different, and so forth. Specific examples thereof include decahydroquinolyl, octahydroindolyl, and 2,3,4,7-tetrahydro-1H-indolyl groups, and so forth.

The "aromatic heterocyclic ring group" means a "heterocyclic ring group" having an aromatic ring, and examples thereof include the above "monocyclic aromatic heterocyclic ring groups" and "polycyclic aromatic heterocyclic ring groups".

The "nonaromatic heterocyclic ring group" means a "heterocyclic ring group" having no aromatic ring, and examples thereof include the above "monocyclic nonaromatic heterocyclic ring groups" and "polycyclic nonaromatic heterocyclic ring groups".

Preferred examples of each group are as follows.

Ring Q1 preferably represents a phenyl group.

R¹, R² and R³ preferably each represent a halogen atom or a C₁₋₄ alkyl group that may be the same or different, more preferably a halogen atom or a methyl group, and it is particularly preferred that R¹ and R² represent a fluorine atom, and R³ represents a chlorine atom or a methyl group.

R⁴ preferably represents a hydrogen atom or a C₁₋₄ alkyl group, more preferably a hydrogen atom or a methyl group, particularly preferably, a hydrogen atom.

Ring Q2 preferably represents a "5- or 6-membered monocyclic aromatic heterocyclic ring group", and specific examples thereof include a furyl group, a thienyl group, a thiazolyl group, an imidazolyl group, an isoxazolyl group, an oxadiazolyl group, a triazolyl group, a pyrazolyl group, a pyrimidinyl group, a thiadiazolyl group, an oxazolyl group, and so forth. More preferred examples thereof include 5-membered monocyclic aromatic heterocyclic ring groups, and specific examples thereof include a furyl group, a thienyl group, a thiazolyl group, an imidazolyl group, an isoxazolyl group, an oxadiazolyl group, a triazolyl group, a pyrazolyl group, a thiadiazolyl group, an oxazolyl group, and so forth.

n is an integer of 1 to 4, more preferably an integer of 1 or 2, yet more preferably an integer of 1.

When ring Q2 is a 5-membered monocyclic aromatic heterocyclic ring group comprising 1 heteroatom as an atom constituting a ring, r is preferably 0 to 2. When ring Q2 is a 5-membered monocyclic aromatic heterocyclic ring group comprising 2 heteroatoms, r is preferably 0 to 1. When ring Q2 is a 5-membered monocyclic aromatic heterocyclic ring group comprising 3 heteroatoms, r is preferably 0. Furthermore, when ring Q2 is a 6-membered monocyclic aromatic heterocyclic ring group comprising 1 or 2 heteroatoms as atoms constituting a ring, r is preferably 0 to 2. When ring Q2 is a 6-membered monocyclic aromatic heterocyclic ring group comprising 3 heteroatoms, r is preferably 0 or 1.

R⁶ preferably represents a C₁₋₄ alkyl group or a C₃₋₈ cycloalkyl group, more preferably a methyl group or a cyclopropyl group.

R⁸ preferably represents a hydrogen atom, a C₁₋₄ alkyl group, or a benzyl group.

R^{9a} and R^{9b} preferably each represent a C₁₋₄ alkyl group that may be the same or different, more preferably a methyl group or an ethyl group.

R¹⁰ preferably represents a benzyl group.

Preferred examples of the "monocyclic aromatic heterocyclic ring group" as R⁷ include a "5- or 6-membered monocyclic aromatic heterocyclic ring group", and specific examples thereof include a thiazolyl group, a thienyl group, an isoxazolyl group, a pyridyl group, and so forth.

The "halogen atom" in "group A" is preferably a fluorine atom or a chlorine atom.

The "C₁₋₆ alkyl group which may be substituted with 1 to 3 halogen atoms that are the same or different" in "group A" is preferably a C₁₋₄ alkyl group which may be substituted with 1 to 3 halogen atoms that are the same or different, and specific examples thereof include, in addition to an unsubstituted C₁₋₄ alkyl group, a trifluoromethyl group, a trichloromethyl group, a difluoromethyl group, a dichloromethyl group, a dibromomethyl group, a fluoromethyl group, a 2,2,2-trifluoroethyl group, a 2,2,2-trichloroethyl group, a 2-bromoethyl group, a 2-chloroethyl group, a 2-fluoroethyl group, a 2-iodoethyl group, a 3-chloropropyl group, a 4-fluorobutyl group, a 2,2-dibromoethyl group, and so forth.

The "aryl group" preferred as of R⁷ is a phenyl group or a naphthyl group, more preferably a phenyl group.

The "C₁₋₆ alkyl group which may be substituted with 1 to 3 halogen atoms that are the same or different" in "group B" is preferably a C₁₋₄ alkyl group which may be substituted with 1 to 3 halogen atoms that are the same or different, and specific examples thereof include, in addition to an unsubstituted C₁₋₄ alkyl group, a trifluoromethyl group, a trichloromethyl group, a difluoromethyl group, a dichloromethyl group, a dibromomethyl group, a fluoromethyl group, a 2,2,2-trifluoroethyl group, a 2,2,2-trichloroethyl group, a 2-bromoethyl group, a 2-chloroethyl group, a 2-fluoroethyl group, a 2-iodoethyl group, a 3-chloropropyl group, a 4-fluorobutyl group, a 2,2-dibromoethyl group, and so forth.

R^{11a} and R^{11b} preferably each represent a C₁₋₄ alkyl group that may be the same or different, more preferably a methyl group or an ethyl group.

R¹² preferably represents a C₁₋₄ alkyl group, more preferably a methyl group or an ethyl group.

_{R}¹³ preferably represents a hydrogen atom or a C₁₋₄ alkyl group.

R¹⁴ preferably represents a phenyl group.

R^{15a} preferably represents a hydrogen atom, and R^{15b} preferably represents a benzyl group.

The "C₁₋₆ alkyl group" preferred as R⁷ is a C₁₋₄ alkyl group, more preferably a methyl group or an ethyl group.

R¹⁶ preferably represents a hydrogen atom, a C₁₋₄ alkyl group, or an aralkyl group, more preferably a hydrogen atom, a methyl group, an ethyl group, or a benzyl group.

R^{17a} and R^{17b} preferably represent a hydrogen atom.

The monocyclic nonaromatic heterocyclic ring formed by R^{17a} and R^{17b} together with an adjacent nitrogen atom are preferably a monocyclic nonaromatic saturated heterocyclic ring, more preferably piperidine.

The "monocyclic aromatic heterocyclic ring group which may be substituted with 1 to 5 substituents that are the same or different and selected from group A" in "group C" is preferably a thiazolyl group which may be substituted with 1 to 3 C₁₋₄ alkyl groups that are the same or different.

The "aryl group which may be substituted with 1 to 5 substituents that are the same or different and selected from group B" in "group C" is preferably a phenyl group which may be substituted with 1 to 5 substituents selected from halogen atoms and C₁₋₄ alkyl groups that are the same or different.

R¹⁸ preferably represents a C₁₋₄ alkyl group.

R¹⁹ preferably represents a hydrogen atom, a phenyl group, a benzyl group, a C₁₋₄ alkyl group, or a C₁₋₄ alkyl group substituted with 1 to 3 substituents that are the same or different and selected from group D.

The "halogen atom" in "group D" is preferably a fluorine atom.

R²⁰ preferably represents a hydrogen atom or a C₁₋₄ alkyl group.

R^{21a} and R^{21b} preferably represent a hydrogen atom, a C₁₋₄ alkyl group, or a benzyl group that may be the same or different.

The monocyclic nonaromatic heterocyclic ring formed by R^{21a} and R^{21b} together with an adjacent nitrogen atom is preferably a monocyclic nonaromatic saturated heterocyclic ring, more preferably morpholine.

R²² preferably represents a C₁₋₄ alkyl group, a phenyl group, a C₃₋₈ cycloalkyl group, or a thienyl group.

R²³ preferably represents a hydrogen atom.

R²⁴ preferably represents a C₁₋₄ alkyl group or a phenyl group. Here, the phenyl group may be substituted with 1 to 5 halogen atoms that are the same or different.

R²⁵ preferably represents a hydrogen atom.

R²⁶ preferably represents a phenyl group which may be substituted with 1 to 5 C₁₋₄ alkyl groups that are the same or different.

l, m, and p each represent an integer of 1 or 2, preferably an integer of 2.

Specific examples of a "polycyclic aromatic heterocyclic ring group" preferred as R⁷ include benzofuranyl, isobenzofuranyl, benzothienyl, benzooxazolyl, benzoisoxazolyl, benzothiazolyl, benzoimidazolyl (for example, 1H-benzoimidazolyl , 2H-benzoimidazolyl), indazolyl (for example, 1H-indazolyl, 2H-indazolyl, 3H-indazolyl), benzotriazolyl (for example, 1H-benzotriazolyl , 2H-benzotriazolyl), indolyl (for example, 1H-indolyl, 2H-indolyl, 3H-indolyl), isoindolyl (for example, 1H-isoindolyl, 2H-isoindolyl), and dibenzofuranyl groups, and so forth. Preferred examples thereof include a "polycyclic aromatic heterocyclic ring group" obtained by condensation of a "5-membered monocyclic aromatic heterocyclic ring" and 1 benzene ring, and a benzothienyl group is more preferred.

Ring Q3 preferably represents a 5- or 6-membered monocyclic nonaromatic heterocyclic ring, more preferably piperidine.

As salts of the compound of the present invention, pharmaceutically acceptable salts are preferred, and examples thereof include salts with inorganic bases, salts with organic bases, salts with inorganic acids, salts with organic acids, salts with basic or acidic amino acids, and so forth. Examples of salts with inorganic bases include alkali metal salts such as sodium salts and potassium salts; alkaline earth metal salts such as calcium salts and magnesium salts; aluminum salts; ammonium salts, and so forth. Examples of salts with organic bases include salts with trimethylamine, triethylamine, pyridine, picoline, ethanolamine, diethanolamine, triethanolamine, dicyclohexylamine, N,N-dibenzyl ethylenediamine, and the like. Examples of salts with inorganic acids include salts with hydrochloric acid, hydrobromic acid, nitric acid, sulfuric acid, phosphoric acid, and the like. Examples of salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, and p-toluenesulfonic acid. Examples of salts with basic amino acids include salts with arginine, lysine, ornithine, and the like. Examples of salts with acidic amino acids include aspartic acid, glutamic acid, and the like. Each salt can be obtained by reacting with an inorganic base, organic base, inorganic acid, organic acids, or basic or acidic amino acid according to a known method.

Furthermore, various solvates (for example, hemihydrates, monohydrates, dihydrates) can exist for the compound of the present invention or salts thereof. These solvates can be obtained according to known methods.

Furthermore, various isomers, for example, optical isomers, stereoisomers, geometrical isomers, tautomers, and the like can exist for the compound of the present invention. All these isomers and mixtures thereof fall within the scope of the present invention.

Furthermore, the compound of the present invention may be labeled with isotopes (for example, ³H, ¹⁴C, ³⁵S, etc.).

The compound of the present invention or salts thereof or solvates thereof are preferably substantially purified compounds of the present invention or salts thereof or solvates thereof. Here, the expression "substantially purified" means that the compound of the present invention or salts thereof or solvates thereof is purified to have purity of not lower than 80 % by weight, preferably not lower than 90 % by weight, more preferably not lower than 95 % by weight.

Further, various prodrugs can exist for the compound of the present invention. The prodrug of the compound of the present invention means a compound that is converted to the compound of the present invention by reactions by enzymes, gastric acid, or the like under physiological conditions in an organism. That is, the prodrug of the compound of the present invention is a compound that is converted to the compound of the present invention by enzymatically induced oxidation, reduction, hydrolysis, or the like or a compound that is changed to the compound of the present invention by hydrolysis or the like induced by gastric acid or the like.

Examples of prodrugs of the compound of the present invention include amide derivatives, alkyl derivatives, ester derivatives, and the like of the compound of the present invention.

Specific examples thereof include compounds in which an amino group of the compound of the present invention is acylated or alkylated (for example, amide derivatives in which an amino group is docosanoylated, amidated by alanine, hexylaminocarbonylated, or the like; alkyl derivatives in which an amino group is tetrahydrofuranylfuranylated, pyrrolidylmethylated, pivaloyloxymethylated, tert-butylated, or the like, etc.); compounds in which a hydroxyl group of the compound of the present invention is acylated, alkylated, succinated, or phosphorylated (for example, derivatives in which a hydroxyl group is acetylated, palmitoylated, propanoylated, pivaloylated, succinylated, phthalylated, alanylated, dimethylaminomethylcarbonylated, succinated, phosphorylated, or benzylated, etc.); compounds in which a carboxyl group of the compound of the present invention esterified or amidated (for example, ester derivatives in which in which a carboxyl group is ethyl-esterified, phenyl-esterified, carboxymethyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolene-4-yl)methyl-esterified, cyclohexyloxycarbonylethyl-esterified, or the like; amide derivatives in which a carboxyl group is methylamidated, amidated with phenylalanine, or the like, etc.); and so forth.

These prodrugs can be obtained from the compound of the present invention by known methods.

Examples of pharmaceutical compositions comprising the compound of the present invention or a salt thereof or a solvate thereof include oral agents such as a tablet, capsule, granule, powder, lozenge, syrup, emulsion, suspension, and so forth and parenteral agents such as a topical agent, suppository, injection, eye drop, nasal agent, pulmonary agent, and so forth.

These pharmaceutical compositions are produced by suitably mixing the compound of the present invention or a salt thereof or a solvate thereof and at least one type of pharmaceutically acceptable carriers or the like in suitable amounts or the like according to methods known in the technical field of pharmaceutical preparations. The content of the compound of the present invention or a salt thereof in a pharmaceutical composition varies depending on the dosage form, the dose of the compound of the present invention or a salt thereof, and the like, but is, for example, 0.1 to 100% by weight of the total amount of the composition.

Examples of "pharmaceutically acceptable carriers" include various organic or inorganic carrier substances commonly used as preparation materials, and examples thereof include excipients, disintegrating agents, binders, fluidizers, lubricants, and so forth in solid preparations and solvents, dissolving aids, suspending agents, isotonizing agents, buffers, soothing agents, and so forth in liquid preparations. Furthermore, if necessary, additives such as preservatives, antioxidants, coloring materials, and sweeteners are used.

Examples of "excipients" include lactose, sucrose, D-mannitol, D-sorbitol, maize starch, dextrin, microcrystalline cellulose, crystalline cellulose, carmellose, carmellose calcium, sodium carboxymethyl starch, low-substituted hydroxypropylcellulose, gum arabic, and so forth.

Examples of "disintegrating agents" include carmellose, carmellose calcium, carmellose sodium, sodium carboxymethyl starch, croscarmellose sodium, crospovidone, low-substituted hydroxypropylcellulose, hydroxypropylmethylcellulose, crystalline cellulose, and so forth.

Examples of "binders" include hydroxypropylcellulose, hydroxypropylmethylcellulose, povidone, crystalline cellulose, sucrose, dextrin, starch, gelatin, carmellose sodium, gum arabic, and so forth.

Examples of "fluidizers" include light anhydrous silicic acid, magnesium stearate, and so forth.

Examples of "lubricants" include magnesium stearate, calcium stearate, talc, and so forth.

Examples of "solvents" include purified water, ethanol, propylene glycol, macrogol, sesame oil, corn oil, olive oil, and so forth.

Examples of "dissolving aids" include propylene glycol, D-mannitol, benzyl benzoate, ethanol, triethanolamine, sodium carbonate, sodium citrate, and so forth.

Examples of "suspending agents" include benzalkonium chloride, carmellose, hydroxypropylcellulose, propylene glycol, povidone, methylcellulose, glycerine monostearate, and so forth.

Examples of "isotonizing agents" include glucose, D-sorbitol, sodium chloride, D-mannitol, and so forth.

Examples of "buffers" include sodium hydrogenphosphate, sodium acetate, sodium carbonate, sodium citrate, and so forth.

Examples of "soothing agents" include benzyl alcohol, and so forth.

Examples of "preservatives" include ethyl paraoxybenzoate, chlorobutanol, benzyl alcohol, sodium dihydroacetate, sorbic acid, and so forth.

Examples of "antioxidants" include sodium sulfite, ascorbic acid, and so forth.

Examples of "coloring materials" include food dyes (for example, Food Red No. 2 or 3, Food Yellow No. 4 or 5, and the like), β-carotene, and so forth.

Examples of "sweeteners" include saccharin sodium, dipotassium glycyrrhizinate, aspartame, and so forth.

Preparation examples of the present invention include the following preparations. However, the scope of the present invention is not limited to these preparation examples.

### Preparation Example 1 (production of capsule)

| | |
|---|---|
| 1) Compound of Example 1 | 30 mg |
| 2) Microcrystalline cellulose | 10 mg |
| 3) Lactose | 19 mg |
| 4) Magnesium stearate | 1 mg |

| | |
|---|---|
| 1), 2), 3), and 4) are mixed and filled in a gelatin capsule. | |

### Preparation Example 2 (production of tablet)

| | |
|---|---|
| 1) Compound of Example 1 | 10 g |
| 2) Lactose | 50 g |
| 3) Maize starch | 15 g |
| 4) Carmellose calcium | 44 g |
| 5) Magnesium stearate | 1 g |

All the amounts of 1), 2), and 3) and 30 g of 4) are kneaded together with water, vacuum dried, and then sized. To this sized powder are added 14 g of 4) and 1 g of 5), and tablets are made by a tableting machine. Thus, 1000 tablets each containing 10 mg of the compound of Example 1 are obtained.

The pharmaceutical composition of the present invention can be administered to not only humans but also mammals other than humans (for example, mice, rats, hamsters, guinea pigs, rabbits, cats, dogs, pigs, cows, horses, sheep, monkeys, etc.) orally or parenterally (for example, local, rectal, venous administrations, etc.). The dose varies depending on the intended patient, disease, symptom, dosage form, administration route, and the like, but, for example, the dose for oral administration to an adult diabetic patient (body weight: about 60 kg) is usually in the range of about 1 mg to 1 g daily of the compound of the present invention as an active ingredient. These doses can be administered at once or divided into several doses.

The compound of the present invention or a salt thereof or a solvate thereof can be used as an active ingredient of a therapeutic or prophylactic agent for diabetes.

The compound of the present invention or a salt thereof or a solvate thereof can also be used, for example, as a therapeutic agent for insulin resistance diseases, diabetic neuropathy, diabetic nephropathy, diabetic retinopathy, cataract, hypercholesterolemia, hypertension, hyperinsulinism, hyperlipemia, atherosclerosis, tissue ischemia, myocardial ischemia; an appetite regulating agent and a therapeutic agent for obesity; a therapeutic agent for infections such as bacterial, fungal, parasitic and viral infections, and so forth.

The "liver glycogen phosphorylase inhibitor" means an agent that specifically inhibits the function of liver glycogen phosphorylase as an enzyme and thereby eliminates or attenuates the activity thereof, for example, an agent that inhibits the function of liver glycogen phosphorylase based on the conditions of Test Example 1 described later. The "liver glycogen phosphorylase inhibitors" are preferably human liver glycogen phosphorylase inhibitors.

The compound of the present invention or a salt thereof or a solvate thereof can be used in combination with another drug or other several drugs (hereinafter, may be referred to combination drugs) by common methods employed in the pharmaceutical field (hereinafter, may be referred to combination use).

The administration timings of the compound of the present invention or a salt thereof or a solvate thereof and a combination drug are not limited, and these drugs may be administered to an intended patient as a combination, or both the preparations may be administered simultaneously or at a certain interval. Furthermore, the compound of the present invention or a salt thereof or a solvate thereof may be used as a medicament characterized by a kit comprising the pharmaceutical composition of the present invention and a combination drug. It is sufficient that the dose of a combination drug conforms to clinically used doses, and can be suitably selected depending on the intended patient, disease, symptom, dosage form, administration route, administration time, combination, and the like. The administration form of the combination drug is not particularly limited, and it is sufficient that the compound of the present invention or a salt thereof or a solvate thereof and a combination drug are combined.

Examples of the combination drug include
(1) a therapeutic agent for diabetes,
(2) a therapeutic agent for diabetic complications,
(3) an antihyperlipidemic drug,
(4) an antihypertensive drug,
(5) an anti-obesity agent,
(6) a diuretic, or
(7) an anti-thrombogenic agent
and the like, and 1 to 3 agents thereof and the compound of the present invention or a salt thereof or a solvate thereof can be used in combination.

### Production method

Methods for production of the compound of the present invention will be described below, but the production method of the present invention is not limited to these methods. Types, doses, and the like of solvents used in each step are not particularly limited so long as the progression of reactions is not affected. Furthermore, it is sufficient to perform reactions in each step by usual methods, and isolation and purification are performed by usual methods, for example, recrystallization, reprecipitation, or methods usually used for isolation and purification of organic compounds, for example, by suitably performing adsorption column chromatography, partition column chromatography, ion exchange chromatography, gel filtration, and the like in combination.

The compound of the present invention may be produced according to the following Production Method A (A-1 to A-2) or Production Method B, but may also be produced according to the examples described later or based on these production methods. In production of the compound of the present invention, the reaction orders can be suitably changed. Furthermore, if necessary, protection and deprotection can be suitably performed. If necessary, reactions other than the steps may be suitably performed. To regulate progression of reactions, reagents other than reagents mentioned as examples can be suitably used.

The following production step diagrams show examples of methods for producing the compound represented by the general formula (I), and production of the compound of the present invention is not particularly limited to the following methods. All the compounds obtained in each step can be isolated and purified by usual methods, but a reaction can be preceded to a subsequent step without isolation or purification in some cases.

### Production Method A (Production Methods A-1 to A-2):

Production Method A will be shown below. 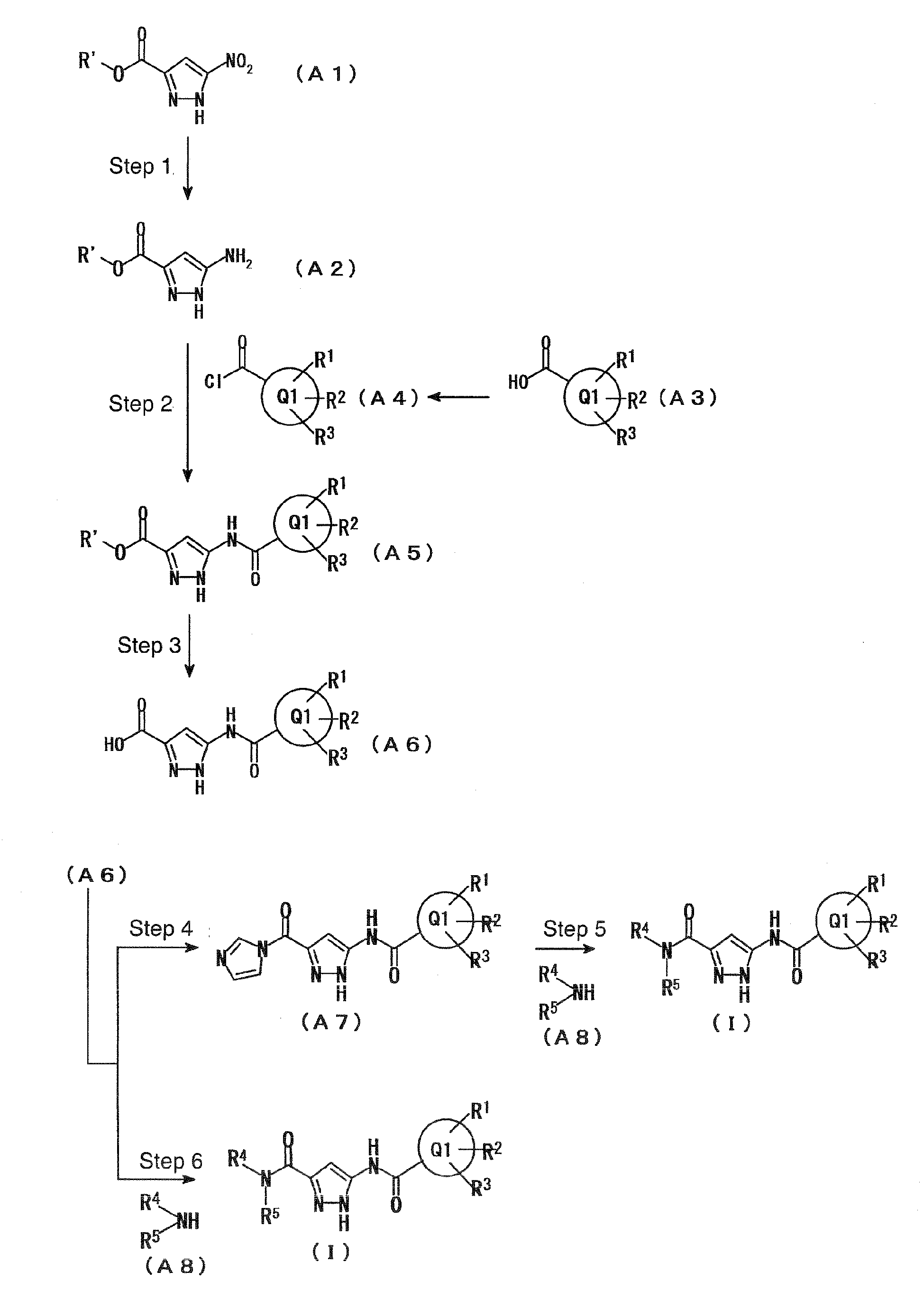 [wherein R' represents a carboxy protection group (here, the carboxy protection group means a carboxy protection group commonly used in the field of organic synthetic chemistry, and examples thereof include a methyl group, an ethyl group, a propyl group, a tert-butyl group, a benzyl group, a p-methoxybenzyl group, and so forth), and forms an ester that is easily converted to carboxylic acid by hydrolysis or the like. R' is preferably an ethyl group. Other symbols have the same meanings as defined above. Definitions are the same in the formulas shown below].

### Step 1: Production of compound (A2)

Compound (A2) can be obtained by reducing a nitro group of compound (A1) to an amino group with hydrogen in a solvent in the presence of a catalyst.

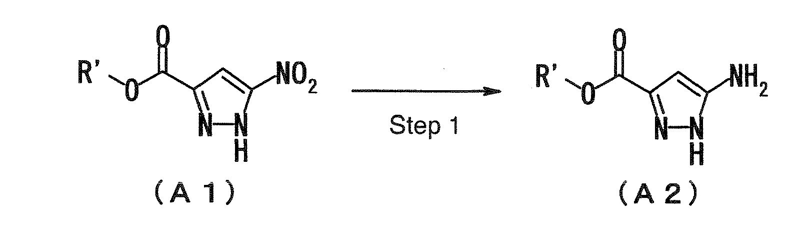

Examples of solvents used for the reaction include alcoholic solvents such as methanol and ethanol; ether solvents such as dioxane and tetrahydrofuran; ester solvents such as ethyl acetate; polar solvents such as N,N-dimethylformamide; water, and so forth, and these solvents can be used solely or in combination.

Examples of catalysts used in the reaction include palladium catalysts such as palladium black and palladium-carbon; nickel catalysts such as Raney nickel; platinum catalysts such as platinum oxide and platinum oxide/carbon, and so forth (refer to M. Hudlicky, Reductions in Organic Chemistry, Wiley-Interscience, New York, 1984.).

The reaction temperature is usually about 0 to 100°C, preferably about 0°C to room temperature.

The reaction time is usually about 10 minutes to 48 hours, preferably about 30 minutes to 24 hours.

Examples of hydrogen donors used instead of hydrogen include formic acid, ammonium formate, cyclohexene, hydrazine, and so forth (refer to M. Hudlicky, Reductions in Organic Chemistry, Wiley-Interscience, New York, 1984.).

Furthermore, examples of other methods of reducing a nitro group include reactions using zinc/hydrochloric acid, stannous chloride, sodium hydrosulfite, titanium trichloride, and the like (refer to M. Hudlicky, Reductions in Organic Chemistry, Wiley-Interscience, New York, 1984).

### Step 2: Production of compound (A5)

Compound (A5) can be obtained by converting compound (A3) to compound (A4) by a usual acid chloride synthesis method and acylating an amino group of compound (A2) in a solvent in the presence of a base using the compound (A4).

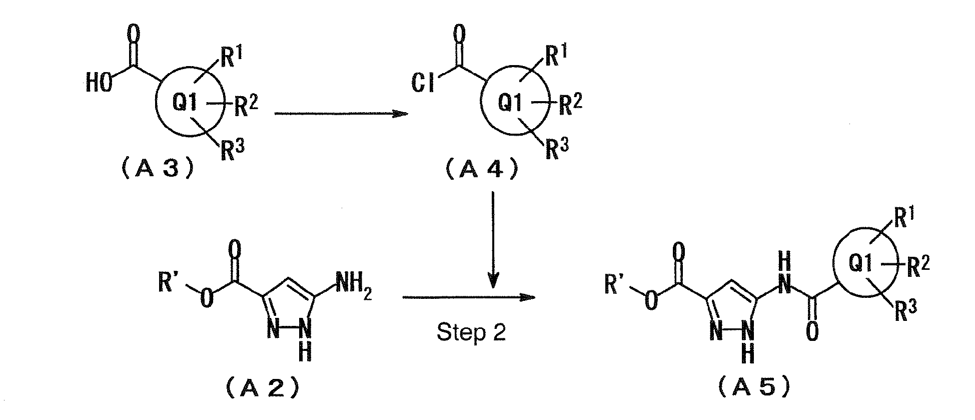

Examples of reagents used in the acid chloride synthesis reaction include oxalyl chloride, thionyl chloride, phosphorus pentachloride, and so forth.

Furthermore, if necessary, N,N-dimethylformamide can be added to promote the reaction.

Furthermore, if necessary, as reaction solvents, for example, ether solvents such as diethyl ether, tetrahydrofuran, dioxane, 1,2-dimethoxyethane, and diglyme; hydrocarbon solvents such as benzene, toluene, hexane, and xylene; halogenated hydrocarbon solvents such as methylene chloride, chloroform , carbon tetrachloride, and 1,2-dichloroethane; ester solvents such as ethyl acetate, methyl acetate, and butyl acetate, and so forth can be used solely or in combination.

The reaction temperature is usually about -20 to 120°C, preferably about 0 to 80°C.

The reaction time is usually about 10 minutes to 48 hours, preferably about 30 minutes to 24 hours.

Examples of solvents used for the acylation reaction include ether solvents such as diethyl ether, tetrahydrofuran, dioxane, 1,2-dimethoxyethane, and diglyme; hydrocarbon solvents such as benzene, toluene, hexane, and xylene; halogenated hydrocarbon solvents such as methylene chloride, chloroform, carbon tetrachloride, and 1,2-dichloroethane; ester solvents such as ethyl acetate, methyl acetate, and butyl acetate; polar solvents such as acetone, N,N-dimethylacetamide, and dimethyl sulfoxide; water, and so forth, and these solvents can be used solely or in combination.

Examples of bases used in the reaction include organic bases such as triethylamine, pyridine, 4-dimethylaminopyridine, and N-methylmorpholine; and inorganic bases such as lithium hydroxide, sodium hydroxide, potassium hydroxide, sodium carbonate, potassium carbonate, sodium hydrogencarbonate, and potassium hydrogencarbonate.

The reaction temperature is usually about 0 to 120°C, preferably about 0 to 95°C.

The reaction time is usually about 10 minutes to 48 hours, preferably about 30 minutes to 24 hours.

Furthermore, compound (A5) can also be obtained by directly bonding compound (A3) and compound (A2) by amide bond formation reactions other than described here.

Examples of methods that can be used include liquid phase synthesis methods using a mixed anhydrate method, acid azide method, DCC method, EDC method, EDC-HOBt method, EDC-HOSu method, other active ester methods, CDI method, and the like; a peptide solid phase synthesis method, and so forth.
(DCC: 1,3-dicyclohexylcarbodiimide)
(EDC: 1-ethyl-3-(3'-dimethylaminopropyl)carbodiimide)
(HOBt: 1-hydroxybenzotriazole)
(HOSu: 1-hydroxy succinimide)
(CDI: 1,1'-carbonyldiimidazole)

### Step 3: Production of compound (A6)

Compound (A6) can be obtained by converting an ester group of compound (A5) to carboxylic acid in a solvent under usual hydrolysis conditions.

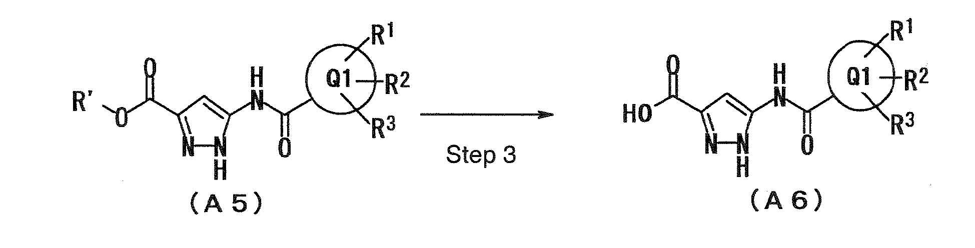

Examples of solvents used for the reaction include alcoholic solvents such as methanol and ethanol; ether solvents such as dioxane and tetrahydrofuran; ketone solvents such as acetone; polar solvents such as N,N-dimethylformamide and dimethyl sulfoxide; water, and so forth, and these solvents can be used solely or in combination.

Examples of bases used in the reaction include alkali metal hydroxides such as sodium hydroxide and potassium hydroxide; alkaline earth metal hydroxides such as barium hydroxide, and so forth.

The reaction temperature is usually about 0 to 100°C, preferably about 0°C to room temperature. The reaction time is usually about 10 minutes to 48 hours, preferably about 30 minutes to 24 hours.

### Steps 4 to 6: Production of compound represented by the general formula (I)

Outlines of Steps 4 to 6 will be described below.

The compound represented by the general formula (I) can be produced by isolating a compound obtained by activation of a carboxyl group of compound (A6), for example, as an active carbonyl compound of an active amide such as imidazolide (compound [A7]) and then forming an amide bond with compound (A8), an amine (see Steps 4 and 5 of the following Production Method A-1).

Furthermore, without isolating the above active carbonyl compound, the compound represented by the general formula (I) can be obtained directly from compound (A6) by forming an amide bond with compound (A8), an amine, using usual amide bond formation reactions, for example, a liquid phase synthesis method using a mixed anhydrate method, acid azide method, DCC method, EDC method, EDC-HOBt method, EDC-HOSu method, other active ester methods, CDI method, or the like; peptide solid phase synthesis method, and the like (see Step 6 of the following Production Method A-2).
(refer to E. Gross, The Peptides, Academic Press, 1981.)

### Production Method A-1: Method for producing compound represented by general formula (I) via compound (A7)

### Step 4: Production of compound (A7)

Compound (A7) can be obtained by reacting compound (A6) with CDI in a solvent.

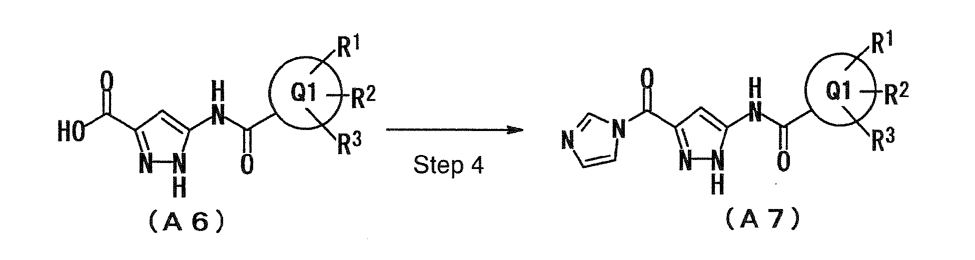

Examples of solvents used for the reaction include ether solvents such as tetrahydrofuran; halogenated hydrocarbon solvents such as chloroform; hydrocarbon solvents such as toluene; polar solvents such as N,N-dimethylformamide, and so forth, and these solvents can be used solely or in combination.

The reaction temperature is usually about 0 to 100°C, preferably room temperature to about 80°C.

The reaction time is usually about 10 minutes to 48 hours, preferably about 30 minutes to 24 hours.

When, instead of CDI, compounds related thereto, for example, 1,1'-carbonyl bis(2-methylimidazole) and the like are used, corresponding active amides can be obtained.

Furthermore, compound (A7) or corresponding active amides can be obtained by other active amide synthesis reactions.

### Step 5: Production of compound represented by general formula (I) from compound (A7)

The compound represented by the general formula (I) can be obtained by reacting compound (A7) with compound (A8), an amine, in a solvent.

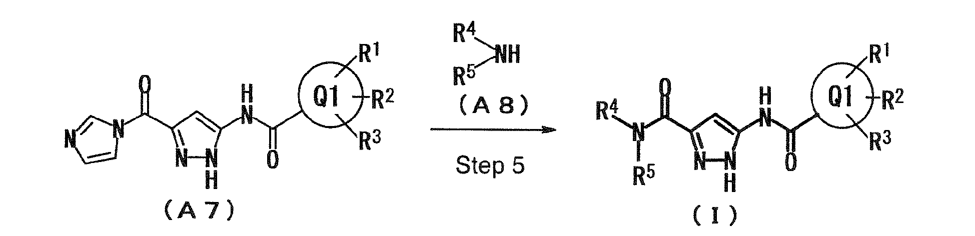

Examples of solvents used for the reaction include ether solvents such as tetrahydrofuran; ester solvents such as ethyl acetate; halogenated hydrocarbon solvents such as chloroform; hydrocarbon solvents such as toluene; polar solvents such as N,N-dimethylformamide, and so forth, and these solvents can be used solely or in combination.

The reaction temperature is usually about 0 to 100°C, preferably about 0°C to room temperature.

The reaction time is usually about 10 minutes to 48 hours, preferably about 30 minutes to 24 hours.

### Production Method A-2: Method for directly producing compound represented by general formula (I) from compound (A6)

### Step 6: Production of compound represented by general formula (I) from compound (A6)

The compound represented by the general formula (I) can be obtained from compound (A6) by usual amide bond formation reactions, for example, by forming an amide bond with compound (A8), an amine, by a liquid phase synthesis method such as a mixed anhydrate method, acid azide method, DCC method, EDC method, EDC-HOBt method, EDC-HOSu method, other active ester methods, or CDI method; a peptide solid phase synthesis method, and the like.

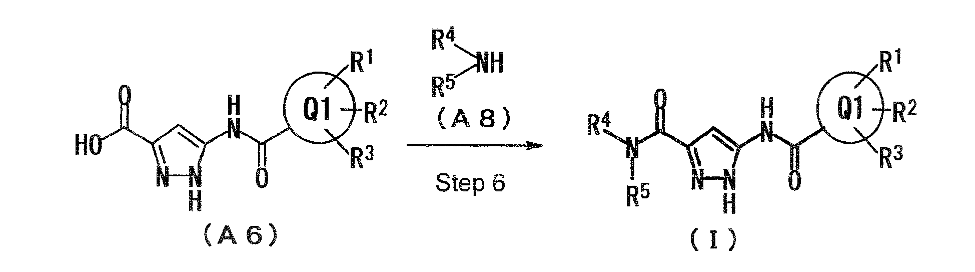

Examples of solvents used for the reaction include ether solvents such as tetrahydrofuran; ester solvents such as ethyl acetate; halogenated hydrocarbon solvents such as chloroform; hydrocarbon solvents such as toluene; polar solvents such as N,N-dimethylformamide; water, and so forth, and these solvents can be used solely or in combination.

The reaction temperature is usually about 0 to 100°C, preferably about 0°C to room temperature.

The reaction time is usually about 10 minutes to 48 hours, preferably about 30 minutes to 24 hours.

### Production Method B

Production Method B is shown in the following figure. Symbols in the formulas have the same meanings as defined above. Definitions are the same in the formulas shown below.

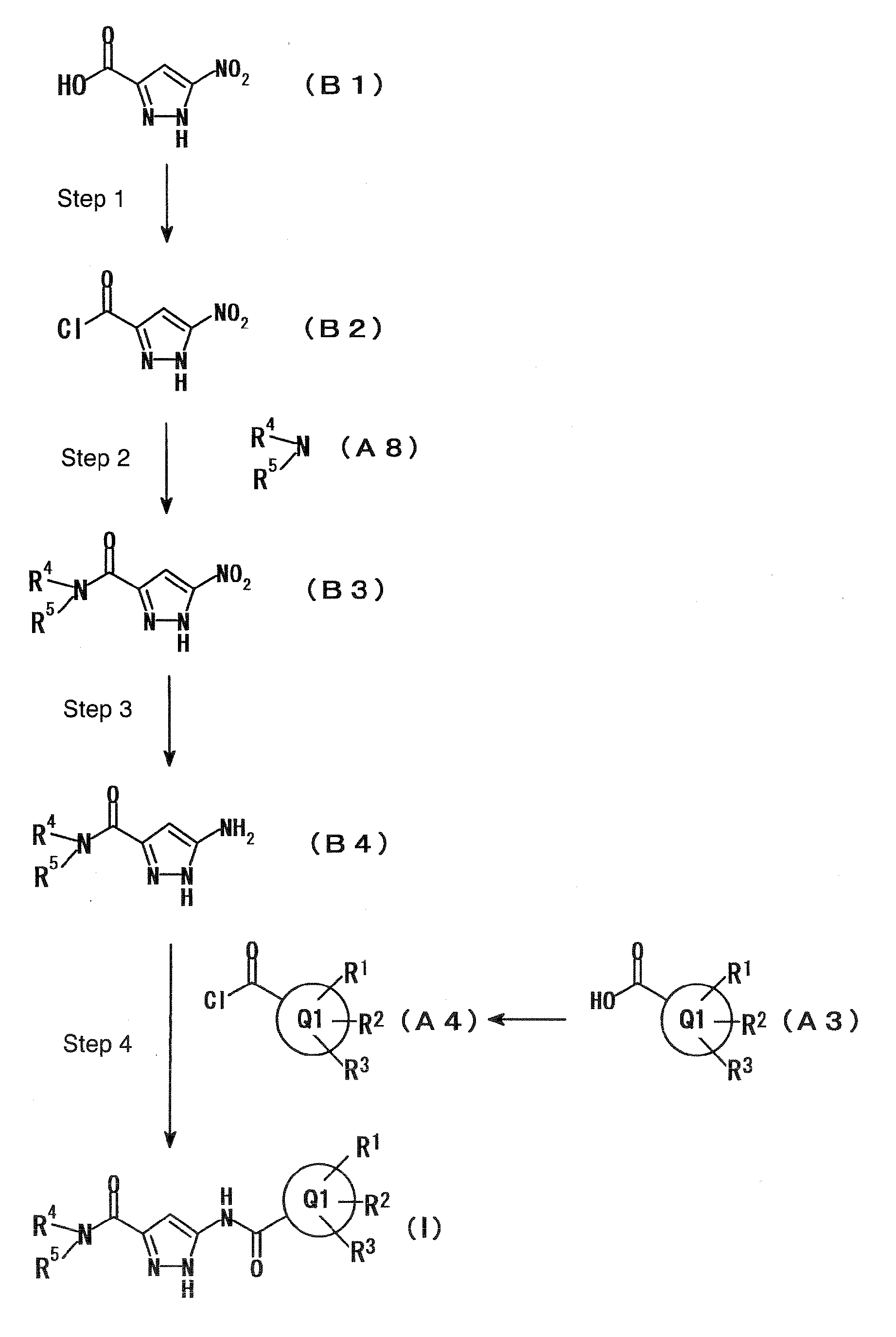

### Step 1: Production of compound (B2)

In the same manner as in the acid chloride synthesis reaction in Step 2 of production method A, compound (B2) can be obtained from compound (B1).

### Step 2: Production of compound (B3)

Compound (B3) can be obtained by reacting compound (B2) with compound (A8) in a solvent, if necessary, in the presence of a base.

Examples of solvents used for the reaction include ether solvents such as diethyl ether, tetrahydrofuran, dioxane, 1,2-dimethoxyethane, and diglyme; hydrocarbon solvents such as benzene, toluene, hexane, and xylene; halogenated hydrocarbon solvents such as methylene chloride, chloroform, carbon tetrachloride, and 1,2-dichloroethane; ester solvents such as ethyl acetate, methyl acetate, and butyl acetate; polar solvents such as acetone and N,N-dimethylformamide; water, and so forth, and these solvents can be used solely or in combination.

Examples of bases used include organic bases such as triethylamine, pyridine, 4-dimethylaminopyridine, and N-methylmorpholine; inorganic bases such as lithium hydroxide, sodium hydroxide, potassium hydroxide, sodium carbonate, potassium carbonate, sodium hydrogencarbonate, and potassium hydrogencarbonate, and so forth.

The reaction temperature is usually about 0 to 120°C, preferably about 0 to 60°C.

The reaction time is usually about 10 minutes to 48 hours, preferably about 30 minutes to 24 hours.

### Step 3: Production of compound (B4)

In the same manner as in Step 1 of Production Method A, compound (B4) can be obtained by reducing a nitro group of compound (B3) with hydrogen in a solvent in the presence of a catalyst.

### Step 4: Production of compound represented by general formula (I) from compound (B4)

A compound represented by the general formula (I) can be obtained by acylating an amino group of compound (B4) in a solvent in the same manner as in Step 2 of Production Method A using compound (A4) in the presence of a base.

### Examples

The present invention will be explained more specifically with reference to the following examples. However, the scope of the present invention is not limited to these examples. Unless otherwise specified, reactions were performed with stirring, and concentration was performed under reduced pressure.

### Reference Example 1

### Production of 5-(2-chloro-4,5-difluoro-benzoylamino)-1H-pyrazole-3-carboxylic acid imidazolide:

### (Step 1)

### Production of 2-chloro-4,5-difluoro-benzoyl chloride:

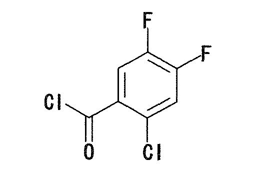

To a solution of 2-chloro-4,5-difluoro-benzoic acid (508.20 g) in toluene (250 mL) was added N,N-dimethylformamide (0.2 mL), the mixture was heated to 65°C, and then thionyl chloride (230 mL) was added dropwise. After stirring at 120°C for 3 hours, the reaction mixture was concentrated, and the resulting residue was distilled under reduced pressure (bp 51 to 64°C (130 to 140 Pa)) to obtain the title compound (534.45 g) as a light yellow oily matter.

### (Step 2)

### Production of ethyl 5-nitro-3-pyrazolecarboxylate:

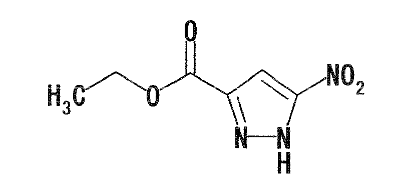

To a solution of 5-nitro-3-pyrazolecarboxylic acid (310.24 g) in ethanol (3 L) was added methanesulfonic acid (143 mL). After stirring overnight at 94°C, the reaction mixture was concentrated followed by addition of water (1.5 L) to the resulting residue, and then aqueous potassium carbonate (prepared by dissolving 153.07 g of potassium carbonate in 750 mL of water) was added with ice cooling. The precipitated solid was collected by filtration to obtain the title compound (343.09 g) as white crystals.

### (Step 3)

### Production of ethyl 5-amino-3-pyrazolecarboxylate:

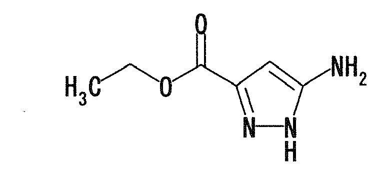

To a solution of ethyl 5-nitro-3-pyrazolecarboxylate (331.48 g) in tetrahydrofuran (1.5 L) and ethyl acetate (1.5 L) was added 7.5% palladium carbon (30.036 g), and the mixture was stirred overnight at room temperature under a hydrogen atmosphere. The catalyst was filtered off, the filtrate was concentrated, and the resulting solid was recrystallized with ethyl acetate/hexane to obtain the title compound (265.38 g) as white crystals.

### (Step 4)

### Production of ethyl 5-(2-chloro-4,5-difluoro-benzoylamino)-1H-pyrazole-3-carboxylate:

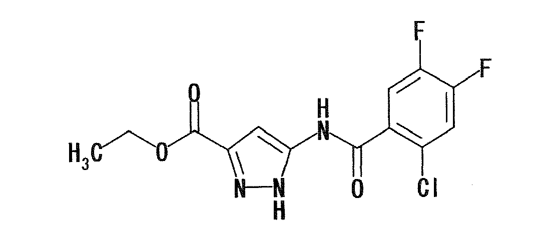

To a solution of ethyl 5-amino-3-pyrazolecarboxylate (265.38 g) in tetrahydrofuran (1.33 L) was added pyridine (153 mL). To this solution, 2-chloro-4,5-difluorobenzoyl chloride (362.00 g) produced in Step 1 of Reference Example 1 was added dropwise with ice cooling, and then the mixture was stirred at room temperature for 2 hours. The reaction mixture was concentrated and recrystallized with 2-propanol/water to obtain the title compound (538.85 g) as white crystals.

### (Step 5)

### Production of 5-(2-chloro-4,5-difluoro-benzoylamino)-1H-pyrazole-3-carboxylic acid:

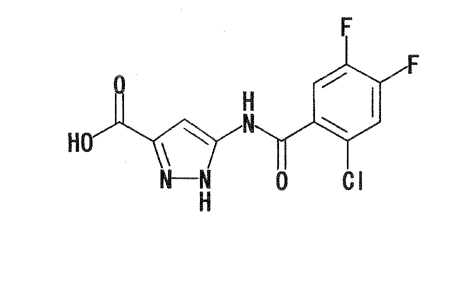

To a suspension of ethyl 5-(2-chloro-4,5-difluoro-benzoylamino)-1H-pyrazole-3-carboxylate (568.22 g) in ethanol (1 L) was added water (1 L), and then 4 N sodium hydroxide aqueous solution (500 mL) was added dropwise with ice cooling. After stirring at room temperature for 3 days, the reaction mixture was added dropwise to 3 N hydrochloric acid (1.2 L) with ice cooling. Water (2 L) was added to the resulting suspension, the mixture was stirred at room temperature, and then the precipitated solid was collected by filtration and dried under reduced pressure at 110°C to obtain the title compound (504.71 g) as white crystals.

### (Step 6)

### Production of 5-(2-chloro-4,5-difluoro-benzoylamino)-1H-pyrazole-3-carboxylic acid imidazolide:

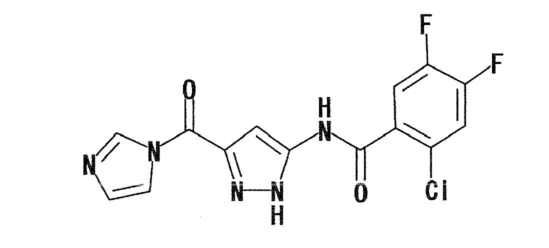

To a suspension of 1,1'-carbonyldiimidazole (64.65 g) in tetrahydrofuran (1.3 L), a solution of 5-(2-chloro-4,5-difluoro-benzoylamino)-1H-pyrazole-3-carboxylic acid (100.05 g) in tetrahydrofuran (700 mL) was added dropwise at 55°C. After stirring at 73°C for 1 hour, ethyl acetate (500 mL) was added, and the reaction mixture was concentrated. To a suspension obtained by adding ethyl acetate (1 L) and hexane (2 L) to the residue, cold water (1 L) was added with ice cooling. After stirring for 10 minutes, the precipitated solid was collected by filtration to obtain the title compound (110.42 g) as white crystals.
¹H NMR (400 MHz, DMSO-*d*₆)
δ: 13.97 (1H, br s), 11.66 (1H, br s), 8.76 (1H, s), 8.05-7.87 (3H, m), 7.18 (1H, s), 6.90 (1H, s).

The method for producing compound (A8), an amine, will be shown below.

### Reference Example 2

### Production of ethyl 5-aminomethyl-furan-2-carboxylate hydrochloride:

### (Step 1)

### Production of ethyl 5-azidomethyl-furan-2-carboxylate:

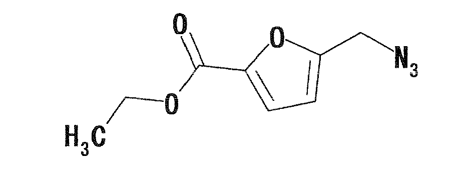

To a solution of ethyl 5-chloromethyl-furan-2-carboxylate (1.021 g) in N,N-dimethylformamide (4 mL) was added sodium azide (433 mg). After stirring at 70°C for 7 hours, water was added to the reaction mixture, and the mixture was extracted with ethyl acetate. The organic layer was successively washed with water and saturated brine, dried over anhydrous sodium sulfate, and then concentrated to obtain the title compound (998 mg) as a brown oily matter.

### (Step 2)

### Production of ethyl 5-aminomethyl-furan-2-carboxylate hydrochloride:

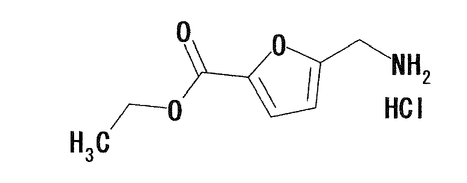

To a solution of ethyl 5-azidomethyl-furan-2-carboxylate (988 mg) in tetrahydrofuran (7 mL) was added a solution of triphenylphosphine (1.411 g) in tetrahydrofuran (4 mL) at 50°C. After stirring for,2 hours, water (0.2 mL) was added to the reaction mixture, and the mixture was stirred for 1 hour. The reaction mixture was cooled to room temperature, stirred overnight, and then concentrated. The residue was dissolved in ethyl acetate (30 mL) followed by addition of 4 N hydrochloric acid/ethyl acetate (2 mL), and the precipitated solid was collected by filtration to obtain the title compound (949 mg) as a white solid.

### Reference Example 3

### Production of 2-aminomethyl-3-diethylsulfamoyl-thiophene hydrochloride:

### (Step 1)

### Production of methyl 3-diethylsulfamoyl-thiophene-2-carboxylate:

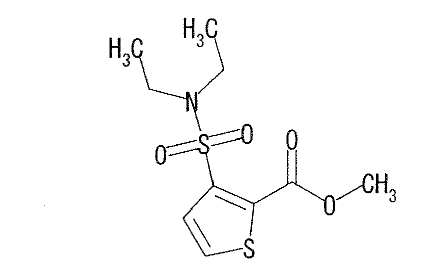

To a solution of methyl 3-chlorosulfonyl-thiophene-2-carboxylate (4.697 g) in tetrahydrofuran (75 mL) was added triethylamine (2.71 mL) followed by addition of diethylamine (1.02 mL) with ice cooling. After stirring overnight at room temperature, water was added to the reaction mixture, and the mixture was extracted with ethyl acetate. The organic layer was washed with saturated brine, dried over anhydrous sodium sulfate, and then concentrated to obtain the title compound (3.94 g) as a brown oily matter.

### (Step 2)

### Production of 3-diethylsulfamoyl-thiophene-2-carboxylic acid

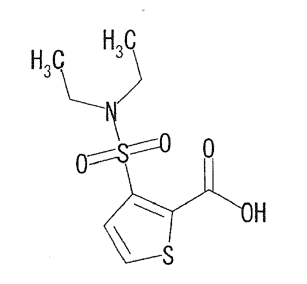

A solution of methyl 3-diethylsulfamoyl-thiophene-2-carboxylate (907 mg) in methanol (10 mL) was added 1 N sodium hydroxide aqueous solution (3.6 mL). After stirring overnight at room temperature, 1 N hydrochloric acid (5 mL) was added to the reaction mixture, and the mixture was extracted with ethyl acetate. The organic layer was washed with saturated brine, dried over anhydrous sodium sulfate, and then concentrated to obtain the title compound (836 mg) as a brown oily matter.

### (Step 3)

### Production of 3-diethylsulfamoyl-2-hydroxymethyl-thiophene:

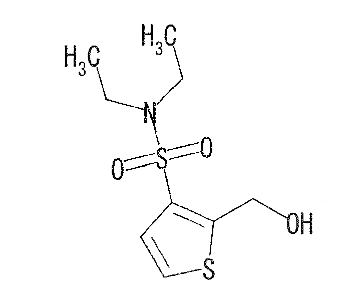

To a solution of 3-diethylsulfamoyl-thiophene-2-carboxylic acid (836 mg) in tetrahydrofuran (8 mL) was added triethylamine (0.486 mL) followed by addition of ethyl chloroformate (0.332 mL) with ice cooling. After stirring for 1 hour with ice cooling, the insoluble matters were filtered off, and an aqueous solution of sodium borohydride (360 mg) was added dropwise to the filtrate with ice cooling. After stirring for 1 hour and 30 minutes with ice cooling, acetone (2.8 mL) was added to the reaction mixture. After stirring at room temperature for 30 minutes, water was added, and the mixture was extracted with ethyl acetate. The organic layer was washed with saturated brine, dried over anhydrous sodium sulfate, and concentrated. The residue was purified by silica gel column chromatography (hexane/ethyl acetate, 4:1 → 2:1) to obtain the title compound (705 mg) as a yellow oily matter.

### (Step 4)

### Production of 2-azidomethyl-3-diethylsulfamoyl-thiophene:

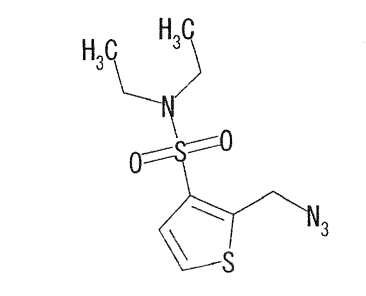

To a solution of 3-diethylsulfamoyl-2-hydroxymethyl-thiophene (705 mg) in chloroform (7 mL) was added triethylamine (0.434 mL) followed by addition of methanesulfonyl chloride (0.23 mL) with ice cooling. After stirring overnight at room temperature, water was added to the reaction mixture, the organic layer was separated, and then the aqueous layer was extracted with chloroform. The combined organic layers were washed with saturated brine, dried over anhydrous sodium sulfate, and then concentrated to obtain 2-chloromethyl-3-diethylsulfamoyl-thiophene.

Subsequently, to a solution of 2-chloromethyl-3-diethylsulfamoyl-thiophene in N,N-dimethylformamide (3 mL) was added sodium azide (220 mg). After stirring at 70°C for 6 hours and 30 minutes, water was added to the reaction mixture, and the mixture was extracted with ethyl acetate. The organic layer was successively washed with water and saturated brine, dried over anhydrous sodium sulfate, and then concentrated to obtain the title compound (792 mg) as a brown oily matter.

### (Step 5)

### Production of 2-aminomethyl-3-diethylsulfamoyl-thiophene hydrochloride:

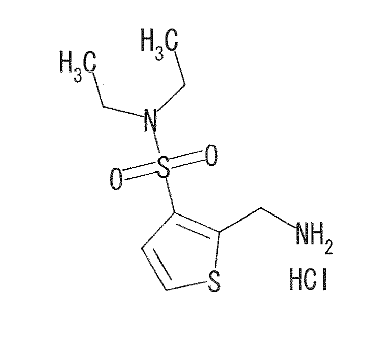

To a solution of 2-azidomethyl-3-diethylsulfamoyl-thiophene (792 mg) in tetrahydrofuran (5 mL) was added a solution of triphenylphosphine (862 mg) in tetrahydrofuran (2.5 mL) at 50°C. After stirring for 2 hours, water (0.1 mL) was added to the reaction mixture, and the mixture was stirred for 30 minutes. The reaction mixture was cooled to room temperature, stirred overnight, and then the reaction mixture was concentrated. The residue was dissolved in ethyl acetate (10 mL) followed by addition of 4 N hydrochloric acid/ethyl acetate (3 mL), and the precipitated solid was collected by filtration to obtain the title compound (484 mg) as a white solid.

### Reference Example 4

### Production of 2-aminomethyl-3-benzyloxy-thiophene hydrochloride:

### (Step 1)

### Production of methyl 3-benzyloxy-thiophene-2-carboxylate:

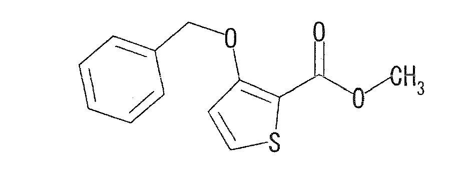

To a solution of methyl 3-hydroxy-thiophene-2-carboxylate (4.90 g) in N,N-dimethylformamide (35 mL) was added benzyl bromide (4.79 mL) followed by addition of sodium hydride (1.49 g) with ice cooling. After stirring overnight at room temperature, 10% aqueous citric acid was added to the reaction mixture, and the mixture was extracted with ethyl acetate. The organic layer was successively washed with water and saturated brine, dried over anhydrous sodium sulfate, and then concentrated. The residue was purified by silica gel column chromatography (hexane/ethyl acetate, 6:1) to obtain the title compound (7.29 g) as a yellow oily matter.

### (Step 2)

### Production of 3-benzyloxy-2-hydroxymethylthiophene:

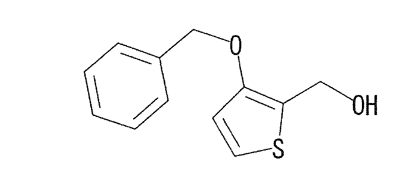

To a suspension of lithium aluminum hydride (622 mg) in tetrahydrofuran (36 mL), a solution (5 mL) of methyl 3-benzyloxy-thiophene-2-carboxylate (3.60 g) in tetrahydrofuran was added dropwise with ice cooling. After stirring at room temperature for 1 hour, water (0.622 mL), 4 N sodium hydroxide aqueous solution (1.81 mL), and water (1.87 mL) were successively added to the reaction mixture, and the mixture was stirred at room temperature. The organic layer was separated, dried over anhydrous sodium sulfate, and then concentrated. The residue was purified by silica gel column chromatography (hexane/ethyl acetate, 3:1) to obtain the title compound (3.02 g) as a yellow oily matter.

### (Step 3)

### Production of 2-(3-benzyloxy-thiophen-2-ylmethyl)-isoindole-1,3-dione:

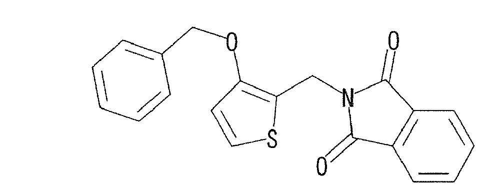

To a solution of 3-benzyloxy-2-hydroxymethylthiophene (2.01 g) in tetrahydrofuran (20 mL) were added phthalimide (1.48 g) and triphenylphosphine (2.62 g) followed by addition of diisopropyl azodicarboxylate (2.07 mL) with ice cooling. After stirring 1 hour, the reaction mixture was returned to room temperature and stirred overnight. Water was added to the reaction mixture, and the mixture was extracted with ethyl acetate. The organic layer was successively washed with water and saturated brine, dried over anhydrous sodium sulfate, and then concentrated. The residue was purified by silica gel column chromatography (hexane/ethyl acetate, 4:1) to obtain the title compound (1.85 g) as a yellow solid.

### (Step 4)

### Production of 2-aminomethyl-3-benzyloxy-thiophene hydrochloride:

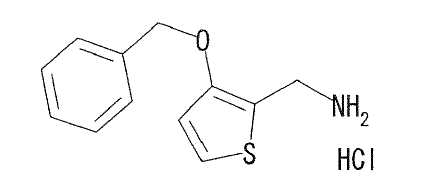

To a suspension of 2-(3-benzyloxy-thiophen-2-ylmethyl)-isoindole-1,3-dione (1.84 g) in ethanol (50 mL) was added hydrazine monohydrate (2.55 mL). After stirring at 85°C for 3 hours and 30 minutes, the mixture was cooled to room temperature, and the insoluble matters were filtered off. The filtrate was concentrated, 2-propanol was added to the residue, the mixture was stirred at room temperature for 10 minutes, and the precipitates were filtered off. The filtrate was concentrated, saturated aqueous sodium hydrogencarbonate was added to the residue, and the mixture was extracted with ethyl acetate. The organic layer was washed with saturated brine, dried over anhydrous sodium sulfate, and concentrated to about a half volume followed by addition of 4 N hydrochloric acid/ethyl acetate with ice cooling. After stirring at room temperature for 1 hour, the precipitated solid was collected by filtration to obtain the title compound (631 mg) as a brown solid.

### Reference Example 5

### Production of 2-aminomethyl-5-(2-methyl-thiazol-4-yl)-thiophene dihydrochloride:

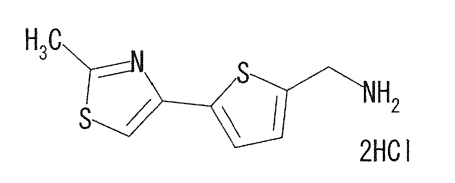

In the same manner as in Steps 3 and 4 of Reference Example 4, the title compound (394 mg) was obtained from 2-hydroxymethyl-5-(2-methyl-thiazol-4-yl)-thiophene as an orange color solid.

### Reference Example 6

### Production of 5-aminomethyl-4-methyl-2-(5-methyl-thiophen-2-yl)-thiazole:

### (Step 1)

### Production of 5-methyl-thiophene-2-carboxylic acid amide:

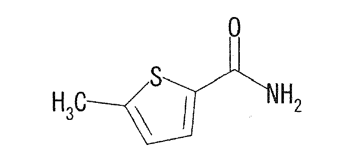

To a solution of 5-methyl-thiophene-2-carboxylic acid (15.5 g) in dimethoxyethane (60 mL) was added a catalytic amount of N,N-dimethylformamide followed by addition of oxalyl chloride (11.2 mL) with ice cooling. After stirring overnight at room temperature, the reaction mixture was added dropwise to 28% aqueous ammonia (200 mL) with ice cooling. After stirring for 2 hours, water (100 mL) was added to the reaction mixture, and the precipitated solid was collected by filtration to obtain the title compound (14.9 g) as a white solid.

### (Step 2)

### Production of 5-methyl-thiophene-2-thiocarboxylic acid amide:

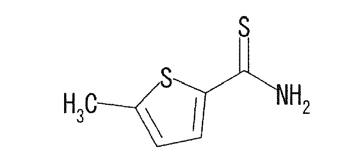

To a suspension of 5-methyl-thiophene-2-carboxylic acid amide (9.23 g) in tetrahydrofuran (200 mL) was added Lawesson's reagent (15.8 g). After stirring at 75°C for 3 hours, saturated aqueous sodium hydrogencarbonate was added to the reaction mixture. The insoluble matters were filtered off, and the mixture was extracted with ethyl acetate. The organic layer was washed with saturated brine, dried over anhydrous sodium sulfate, and then concentrated. The residue purified by silica gel column chromatography (hexane/ethyl acetate, 1:1) to obtain the title compound (8.34 g) as an orange color solid.

### (Step 3)

### Production of ethyl 4-methyl-2-(5-methyl-thiophen-2-yl)-thiazole-5-carboxylate:

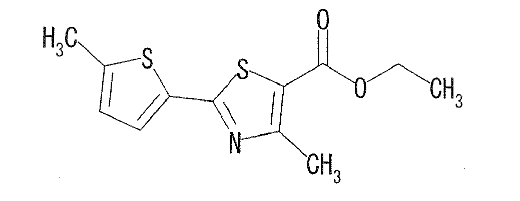

To a suspension of 5-methyl-thiophene-2-thiocarboxylic acid amide (8.34 g) in 1-propanol (70 mL) was added 2-ethyl chloroacetoacetate (8.73 g). After stirring at 105°C for 4 hours, the reaction mixture was concentrated. The residue was purified by silica gel column chromatography (hexane/ethyl acetate, 9:1) to obtain the title compound (10.3 g) as a yellow solid.

### (Step 4)

### Production of 5-hydroxymethyl-4-methyl-2-(5-methyl-thiophen-2-yl)-thiazole:

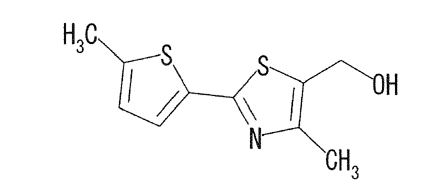

To a suspension of lithium aluminum hydride (510 mg) in tetrahydrofuran (30 mL), a solution (5 mL) of ethyl 4-methyl-2-(5-methyl-thiophen-2-yl)-thiazole-5-carboxylate (3.00 g) in tetrahydrofuran was added dropwise with ice cooling. After stirring at room temperature for 5 hours, water (0.510 mL), 4 N sodium hydroxide aqueous solution (1.49 mL), and water (1.53 mL) were successively added to the reaction mixture, and the mixture was stirred at room temperature. The organic layer was separated, dried over anhydrous sodium sulfate, and then concentrated. The residue was purified by silica gel column chromatography (hexane/ethyl acetate, 1:1) to obtain the title compound (2.52 g) as a yellow oily matter.

### (Step 5)

### Production of 5-chloromethyl-4-methyl-2-(5-methyl-thiophen-2-yl)-thiazole hydrochloride:

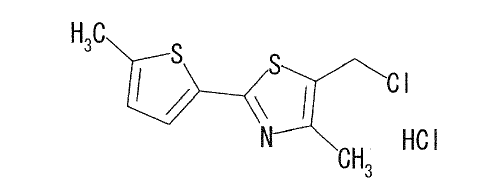

To a solution of 5-hydroxymethyl-4-methyl-2-(5-methyl-thiophen-2-yl)-thiazole (1.10 g) in chloroform (12 mL) was added thionyl chloride (0.605 mL) with ice cooling. After stirring at room temperature for 2 hours, the reaction mixture was concentrated to obtain the title compound as a yellow solid.

### (Step 6)

### Production of 5-aminomethyl-4-methyl-2-(5-methyl-thiophen-2-yl)-thiazole:

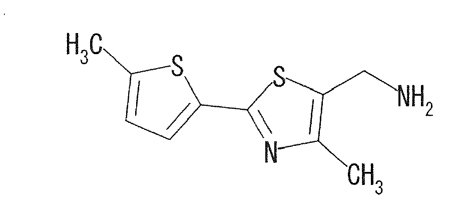

To a solution of 5-chloromethyl-4-methyl-2-(5-methyl-thiophen-2-yl)-thiazole hydrochloride in N,N-dimethylformamide (8 mL) were added potassium carbonate (673 mg) and sodium azide (381 mg). After stirring at 70°C for 6 hours and 30 minutes, water was added to the reaction mixture, and the mixture was extracted with ethyl acetate. The organic layer was successively washed with water and saturated brine, dried over anhydrous sodium sulfate, and then concentrated to obtain 5-azidomethyl-4-methyl-2-(5-methyl-thiophen-2-yl)-thiazole.

Subsequently, to a solution of 5-azidomethyl-4-methyl-2-(5-methyl-thiophen-2-yl)-thiazole in ethyl acetate (10 mL), a solution of triphenylphosphine (1.28 g) in tetrahydrofuran (5 mL) was added dropwise at 50°C. After stirring for 4 hours, water (0.097 mL) was added to the reaction mixture, and the mixture was further stirred for 4 hours. The reaction mixture was cooled to room temperature followed by addition of 4 N hydrochloric acid/ethyl acetate, and the precipitated solids were collected by filtration. Saturated aqueous sodium hydrogencarbonate was gradually added to the resulting solids, and the mixture was extracted with ethyl acetate. The organic layer was washed with saturated brine, dried over anhydrous sodium sulfate, and then concentrated. The residue was purified by silica gel column chromatography (chloroform/methanol, 10:1) to obtain the title compound (178 mg) as a yellow solid.

### Reference Example 7

### Production of 5-aminomethyl-2-(5-chlorothiophen-2-yl)-4-methyl-thiazole:

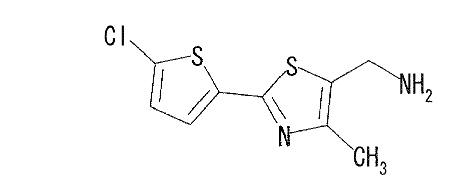

In the same manner as in Steps 1 to 6 of Reference Example 6, the title compound (1.41 g) was obtained from 5-chlorothiophene-2-carboxylic acid as a yellow solid.

### Reference Example 8

### Production of 5-aminomethyl-2-(benzo[b]thiophen-2-yl)-4-methyl-thiazole:

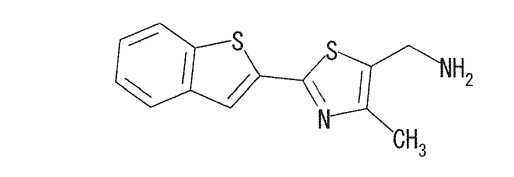

In the same manner as in Steps 2 to 6 of Reference Example 6, the title compound (1.43 g) was obtained from benzo[b]thiophene-2-carboxylic acid amide as an ocher solid.

### Reference Example 9

### Production of 5-aminomethyl-2-ethoxycarbonyl-4-methylthiazole:

### (Step 1)

### Production of tert-butyl 2-chloro-acetoacetate:

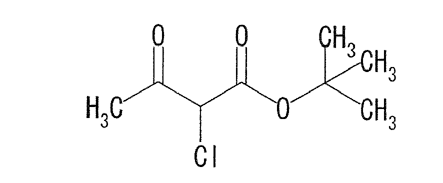

According to a reference (Tetrahedron Lett., 46, 623, 2005), the title compound (15.5 g) was obtained from tert-butyl acetoacetate (25.0 g) as a light yellow oily matter.

### (Step 2)

### Production of tert-butyl 2-ethoxycarbonyl-4-methylthiazole-5-carboxylate:

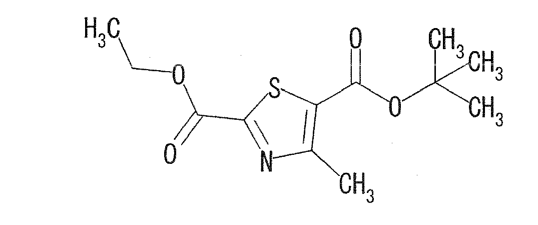

To a solution of tert-butyl 2-chloro-acetoacetate (7.28 g) in N,N-dimethylformamide (40 mL) was added ethyl thiooxamate (5.03 g). After stirring at 90°C for 21 hours, water was added to the reaction mixture, and the mixture was extracted with ethyl acetate. The organic layer was successively washed with water and saturated brine, dried over anhydrous sodium sulfate, and then concentrated. The residue was purified by silica gel column chromatography (hexane/ethyl acetate, 6:1) to obtain the title compound (2.77 g) as a brown solid.

### (Step 3)

### Production of 2-ethoxycarbonyl-4-methyl-thiazole-5-carboxylic acid:

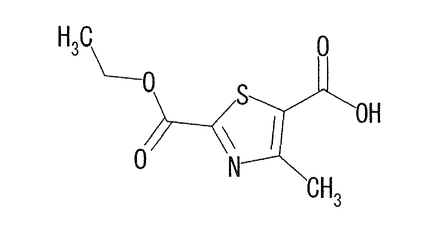

To a solution of tert-butyl 2-ethoxycarbonyl-4-methyl-thiazole-5-carboxylate (2.37 g) in toluene (10 mL) was added trifluoroacetic acid (20 mL). After stirring overnight at room temperature, the reaction mixture was concentrated, and hexane was added to the resulting residue. After stirring at room temperature for 1 hour, the precipitated solid was collected by filtration to obtain the title compound (1.79 g) as an ocher solid.

### (Step 4)

### Production of 2-ethoxycarbonyl-5-hydroxymethyl-4-methylthiazole:

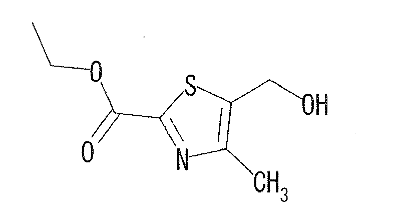

To a solution of 2-ethoxycarbonyl-4-methyl-thiazole-5-carboxylic acid (1.74 g) in tetrahydrofuran (10 mL), a solution (12.1 mL) of 1 M borane-tetrahydrofuran complex in tetrahydrofuran was added dropwise with ice cooling. After stirring overnight at room temperature, water was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 1:2) to obtain the title compound (1.02 g) as a yellow solid.

### (Step 5)

### Production of 5-chloromethyl-2-ethoxycarbonyl-4-methylthiazole:

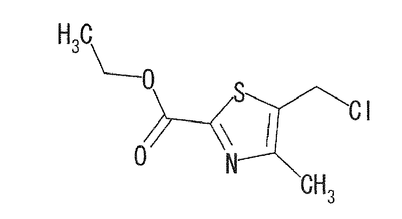

To a solution of 2-ethoxycarbonyl-5-hydroxymethyl-4-methyl-thiazole (1.02 g) in chloroform (10 mL) was added thionyl chloride (0.629 mL) with ice cooling. After stirring at room temperature for 3 hours, the reaction mixture was concentrated. The residue was purified by silica gel column chromatography (hexane/ethyl acetate, 2:1) to obtain the title compound (1.09 g) as a yellow oily matter.

### (Step 6)

### Production of 5-aminomethyl-2-ethoxycarbonyl-4-methylthiazole:

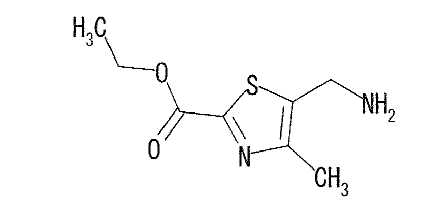

To a solution of 5-chloromethyl-2-ethoxycarbonyl-4-methyl-thiazole (1.19 g) in N,N-dimethylformamide (10 mL) was added sodium azide (424 mg). After stirring at 70°C for 3 hours, water was added to the reaction mixture, and the mixture was extracted with ethyl acetate. The organic layer was successively washed with water and saturated brine, dried over anhydrous sodium sulfate, and then concentrated to obtain 5-azidomethyl-2-ethoxycarbonyl-4-methyl-thiazole.

Subsequently, to a solution of 5-azidomethyl-2-ethoxycarbonyl-4-methyl-thiazole in ethyl acetate (10 mL), a solution of triphenylphosphine (1.42 g) in tetrahydrofuran (5 mL) was added dropwise at 50°C. After stirring for 2 hours, water (0.108 mL) was added to the reaction mixture. After further stirring for 1 hour and 30 minutes, the reaction mixture was cooled to room temperature followed by addition of 4 N hydrochloric acid/ethyl acetate. The precipitates were washed with ethyl acetate and made basic with saturated aqueous sodium hydrogencarbonate, and then the mixture was extracted with tetrahydrofuran. The organic layer was dried over anhydrous sodium sulfate, and then concentrated. The residue was purified by silica gel column chromatography (chloroform/methanol, 9: 1) to obtain the title compound (190 mg) as a yellow oily matter.

### Reference Example 10

### Production of 4-(1-adamantyl)-5-aminomethyl-2-methylthiazole:

### (Step 1)

### Production of ethyl 3-(1-adamantyl)-2-chloro-3-oxo-propionate:

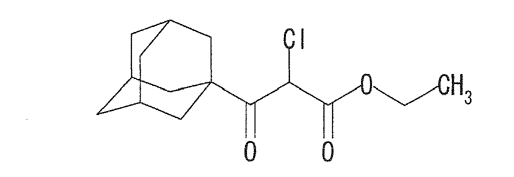

In the same manner as in Step 1 of Reference Example 9, the title compound (9.41 g) was obtained from ethyl 3-(1-adamantyl)-3-oxo-propionate as a colorless oily matter.

### (Step 2)

### Production of ethyl 4-(1-adamantyl)-2-methyl-thiazole-5-carboxylate:

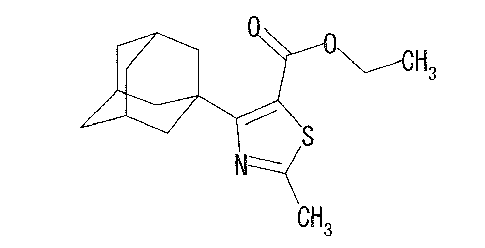

To a solution of ethyl 3-(1-adamantyl)-2-chloro-3-oxo-propionate (5.65 g) in ethanol (45 mL) was added thioacetamide (1.64 g). After stirring at 90°C for 2 hours, the reaction mixture was concentrated. Saturated aqueous sodium hydrogencarbonate was added to the residue, and the mixture was extracted with ethyl acetate. The organic layer was washed with saturated brine, dried over anhydrous sodium sulfate, and then concentrated. The residue was purified by silica gel column chromatography (hexane/ethyl acetate, 20:1) to obtain the title compound (3.79 g) as a brown solid.

### (Step 3)

### Production of 4-(1-adamantyl)-5-aminomethyl-2-methylthiazole:

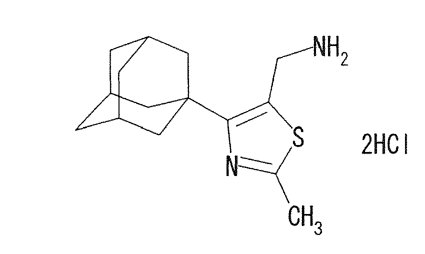

In the same manner as in Step 4 of Reference Example 6, 4-(1-adamantyl)-5-hydroxymethyl-2-methyl-thiazole was obtained from ethyl 4-(1-adamantyl)-2-methyl-thiazole-5-carboxylate (2.0 g).

Subsequently, to a solution of 4-(1-adamantyl)-5-hydroxymethyl-2-methyl-thiazole in chloroform were added pyridine (1.3 mL) and 4-dimethylaminopyridine (catalytic amount) followed by addition of methanesulfonyl chloride (1.2 mL). After stirring at 60°C for 5 hours, water was added to the reaction mixture, and the mixture was extracted with chloroform. The organic layer was successively washed with 10% aqueous citric acid, water, saturated aqueous sodium hydrogencarbonate, water, and saturated brine, dried over anhydrous sodium sulfate, and then concentrated to obtain 4-(1-adamantyl)-5-chloromethyl-2-methyl-thiazole.

To a solution of 4-(1-adamantyl)-5-chloromethyl-2-methyl-thiazole in N,N-dimethylformamide (10 mL) was added sodium azide (494 mg). After stirring at 70°C for 8 hours, water was added to the reaction mixture, and the mixture was extracted with ethyl acetate. The organic layer was successively washed with water and saturated brine, dried over anhydrous sodium sulfate, and then concentrated to obtain 4-(1-adamantyl)-5-azidomethyl-2-methyl-thiazole.

To a solution of 4-(1-adamantyl)-5-azidomethyl-2-methyl-thiazole in ethyl acetate (10 mL), a solution of triphenylphosphine (839 mg) in tetrahydrofuran (10 mL) was added dropwise at 50°C. After 1 hour and 30 minutes, water (0.058 mL) was added to the reaction mixture, the mixture was further stirred for 2 hours, and then the reaction mixture was cooled to room temperature followed by addition of 4 N hydrochloric acid/ethyl acetate. After stirring at room temperature for 1 hour, the precipitated solid was collected by filtration to obtain the title compound (309 mg) as an ocher solid.

### Reference Example 11

### Production of 2-methyl-4,5,6,7-tetrahydrothiazolo[5,4-c]pyridine hydrobromide:

### (Step 1)

### Production of benzyl 3-bromo-4-oxo-piperidine-1-carboxylate:

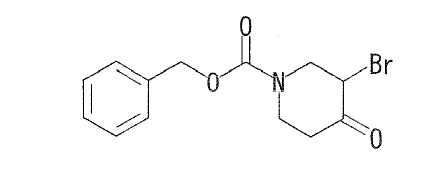

To a solution of benzyl 4-oxo-piperidine-l-carboxylate (3.00 g) in chloroform (30 mL) was added bromine (0.7 mL) with ice cooling. After stirring overnight at room temperature, water was added to the reaction mixture. The organic layer was separated, and the aqueous layer was extracted with chloroform. The combined organic layers were successively washed with water and saturated brine, dried over anhydrous sodium sulfate, and then concentrated to obtain the title compound (3.16 g) as a colorless oily matter.

### (Step 2)

### Production of benzyl 2-methyl-6,7-dihydro-4H-thiazolo[5,4-c]pyridine-5-carboxylate:

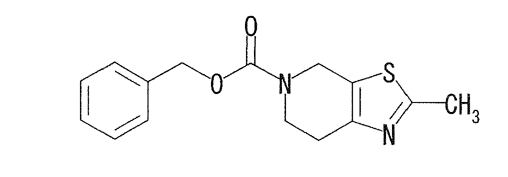

To a solution of benzyl 3-bromo-4-oxo-piperidine-l-carboxylate (3.16 g) in N,N-dimethylformamide (15 mL) was added thioacetamide (2.95 g). After stirring at 100°C for 1 hour and 30 minutes, water was added, and the mixture was extracted 5 times with ethyl acetate. The organic layer was washed with saturated brine, dried over anhydrous sodium sulfate, and then concentrated. The residue was purified by silica gel column chromatography (chloroform/methanol, 97:3) to obtain the title compound (1.65 g) as a colorless oily matter.

### (Step 3)

### Production of 2-methyl-4,5,6,7-tetrahydrothiazolo[5,4-c]pyridine hydrobromide:

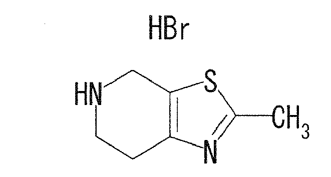

To benzyl 2-methyl-6,7-dihydro-4H-thiazolo[5,4-c]pyridine-5-carboxylate (1.65 g) was added 25% hydrogen bromide/acetic acid (5.0 mL) with ice cooling. After stirring at room temperature for 30 minutes, the precipitated solid was collected by filtration and washed with methanol to obtain the title compound (1.02 g) as a light yellow solid.

### Reference Example 12

### Production of ethyl 2-aminomethyl-5-methylimidazole-4-carboxylate hydrobromide:

### (Step 1)

### Production of ethyl 2-(benzyloxycarbonylamino-methyl)-5-methylimidazole-4-carboxylate:

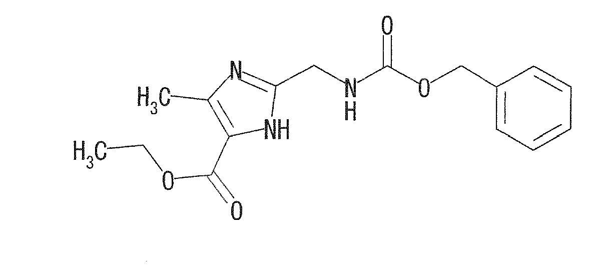

To a suspension of N-benzyloxycarbonyl-glycine (7.749 g) in ethyl acetate (100 mL) were added ethyl 2-chloroacetoacetate (5.38 mL) and triethylamine (15.4 mL) with ice cooling. After stirring overnight at 70°C, water was added to the reaction mixture, and the organic layer was separated. The organic layer was successively washed with 10% aqueous citric acid and saturated brine, dried over anhydrous sodium sulfate, and then concentrated to obtain ethyl 2-(2-benzyloxycarbonylamino-acetoxy)-3-oxo-butyrate (7.709 g) as a brown oily matter.

To a solution of ethyl 2-(2-benzyloxycarbonylamino-acetoxy)-3-oxo-butyrate (7.709 g) in acetic acid (14 mL) was added ammonium acetate (9.45 g). After stirring overnight at 135°C, the reaction mixture was concentrated. Saturated aqueous sodium hydrogencarbonate was added to the residue, and the mixture was extracted with ethyl acetate, dried over anhydrous sodium sulfate, and then concentrated. The residue was purified by silica gel column chromatography (hexane/ethyl acetate, 1:1 → 1:3 → 1:5) to obtain the title compound (2.245 g) as an ocher solid.

### (Step 2)

### Production of ethyl 2-aminomethyl-5-methylimidazole-4-carboxylate hydrobromide:

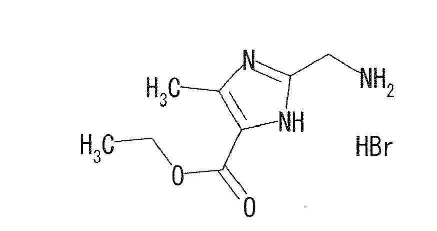

To ethyl 2-(benzyloxycarbonylamino-methyl)-5-methylimidazole-4-carboxylate (720 mg) was added 25% hydrogen bromide/acetic acid (7 mL) with ice cooling. After stirring for 3 hours, ethyl acetate was added to the reaction mixture, and the precipitated solid was collected by filtration to obtain the title compound (751 mg) as a white solid.

### Reference Example 13

### Production of 4-aminomethyl-3-(4-cyano-phenyl)-5-methylisoxazole:

### (Step 1)

### Production of 4-cyanobenzaldehyde oxime:

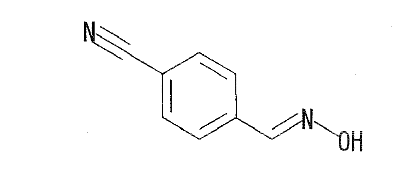

To a solution of 4-cyanobenzaldehyde (1.97 g) in chloroform (10 mL) was added hydroxylamine hydrochloride (1.10 g), and N-methylmorpholine (1.75 mL) was added dropwise to the resulting suspension with ice cooling. After stirring for 2 hours, water was added to the reaction mixture, and the organic layer was separated. Hexane (20 mL) was added to the organic layer with stirring, and the produced solid was collected by filtration, washed with water, and dried to obtain the title compound (2.10 g) as a white solid.

### (Step 2)

### Production of 4-cyanobenzohydroxymoyl chloride:

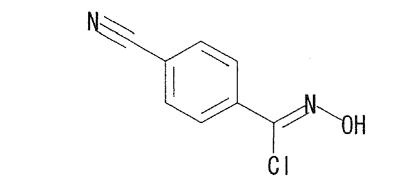

To a solution of 4-cyanobenzaldehyde oxime (2.08 g) in ethyl acetate (20 mL) was added pyridine (0.58 mL) followed by addition of N-chlorosuccinimide (2.00 g) at room temperature. After stirring at 60°C for 2 hours, the reaction mixture was cooled to room temperature, water (20 mL) and heptane (20 mL) were added with stirring, and the produced solid was collected by filtration. The organic layer was separated, washed with water, then concentrated, and combined with the resulting solid to obtain the title compound (2.03 g) as a light yellow solid.

### (Step 3)

### Production of methyl 3-(4-cyano-phenyl)-5-methylisoxazole-4-carboxylate:

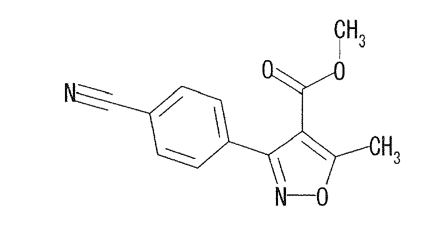

To a solution of 4-cyanobenzohydroxymoyl chloride (2.03 g) and methyl acetoacetate (1.85 mL) in methanol (20 mL) was added potassium carbonate (2.40 g) at room temperature with stirring. After stirring overnight at room temperature, the insoluble matters were filtered off, and the filtrate was concentrated. The residue was dissolved in ethyl acetate washed with water, and then the mixture was concentrated. The crude product was purified by silica gel column chromatography (hexane/chloroform, 1:1 → chloroform) to obtain the title compound (761 mg) as a white solid.

### (Step 4)

### Production of 3-(4-cyano-phenyl)-5-methyl-isoxazole-4-carboxylic acid:

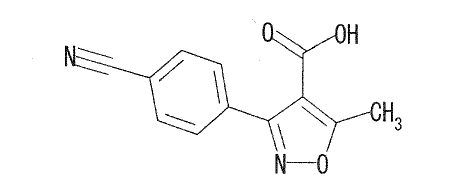

To a solution of methyl 3-(4-cyano-phenyl)-5-methylisoxazole-4-carboxylate (500 mg) in tetrahydrofuran (5 mL)/ethanol (2 mL) were added 2 N sodium hydroxide aqueous solution (1.05 mL) and water (4 mL) at room temperature. After stirring for 3 hours, the reaction mixture was concentrated, and most of the organic solvent was distilled off. The resulting residue was washed 3 times with toluene followed by addition of 1 N hydrochloric acid (2.10 mL) with stirring, and the produced solid was collected by filtration, washed with water, and dried to obtain the title compound (413 mg) as a white solid.

### (Step 5)

### Production of 3-(4-cyano-phenyl)-4-hydroxymethyl-5-methyl-isoxazole:

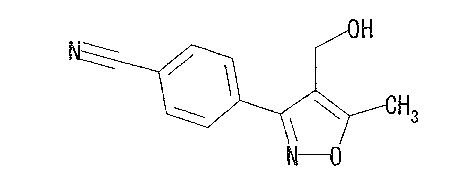

To a suspension of CDI (300 mg) in tetrahydrofuran (4 mL), 3-(4-cyano-phenyl)-5-methyl-isoxazole-4-carboxylic acid (1.00 g) was gradually added at room temperature. After heating at 65°C for 1 hour, the reaction mixture was cooled to room temperature and gradually added to a solution of sodium borohydride (110 mg) in tetrahydrofuran (2 mL)/water (5 mL) with ice cooling. After stirring at room temperature for 1 hour, 6 N hydrochloric acid (1.1 mL) was added dropwise with ice cooling. After stirring at 65°C for 1 hour, the reaction mixture was concentrated, and the organic solvent was distilled off. The produced solid was collected by filtration, washed with water, and dried to obtain the title compound (287 mg) as a white solid.

### (Step 6)

### Production of 4-chloromethyl-3-(4-cyano-phenyl)-5-methylisoxazole:

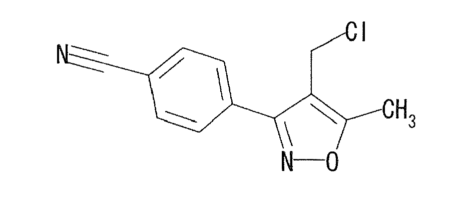

To a suspension of 3-(4-cyano-phenyl)-4-hydroxymethyl-5-methyl-isoxazole (287 mg) in chloroform (5 mL) was added N-methylmorpholine (0.370 mL) followed by addition of methanesulfonyl chloride (0.207 mL) with ice cooling. After stirring at 65°C for 15 minutes, the reaction mixture was cooled to room temperature and successively washed with water, saturated aqueous sodium hydrogencarbonate, and saturated brine. The organic layer was concentrated, and the resulting residue was purified by silica gel column chromatography (hexane/chloroform, 1:1 → chloroform) to obtain the title compound (231 mg) as a white solid.

### (Step 7)

### Production of 4-aminomethyl-3-(4-cyano-phenyl)-5-methylisoxazole:

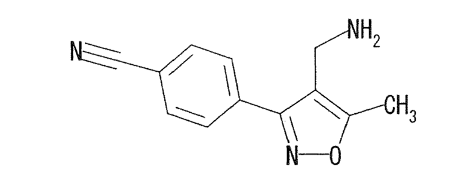

To a solution of 4-chloromethyl-3-(4-cyano-phenyl)-5-methyl-isoxazole (231 mg) in dioxane (10 mL) was added 28% aqueous ammonia (10 mL) at room temperature. After stirring for 18 hours, the reaction mixture was concentrated. The residue was dissolved in 2 N sodium hydroxide aqueous solution (1.0 mL) followed by addition of saturated brine, and the mixture was extracted 3 times with chloroform. The combined organic layers were concentrated and purified by silica gel column chromatography (chloroform/methanol, 9:1) to obtain the title compound (137 mg) as a white solid.

### Reference Example 14

### Production of 4-aminomethyl-3-(4-dimethylamino-phenyl)-5-methyl-isoxazole:

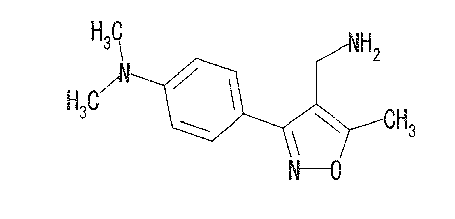

In the same manner as in Steps 1 to 7 of Reference Example 13, the title compound (669 mg) was obtained from 4-dimethylaminobenzaldehyde as a yellow oily matter.

### Reference Example 15

### Production of 4-aminomethyl-3-(4-methanesulfonyl-phenyl)-5-methyl-isoxazole:

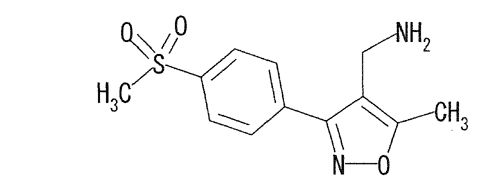

In the same manner as in Steps 1 to 7 of Reference Example 13, the title compound (20 mg) was obtained from 4-methanesulfonylbenzaldehyde as a white solid.

### Reference Example 16

### Production of 4-aminomethyl-3-(2-fluoro-phenyl)-5-methylisoxazole:

### (Step 1)

### Production of 2-fluoro-benzaldehyde oxime:

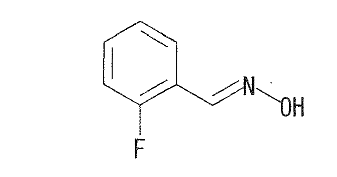

In the same manner as in Step 1 of Reference Example 13, the title compound (2.17 g) was obtained from 2-fluoro-benzaldehyde (2.00 g) as a white solid.

### (Step 2)

### Production of 2-fluoro-benzohydroxymoyl chloride:

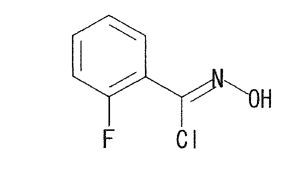

To a solution of 2-fluoro-benzaldehyde oxime (2.17 g) in ethyl acetate (20 mL) was added pyridine (0.63 mL) followed by addition of N-chlorosuccinimide (2.18 g) at room temperature. After stirring for 2 hours, hexane (10 mL) was added to the reaction mixture, and the mixture was washed 3 times with water. The organic layer was concentrated, and then the crude product was purified by silica gel column chromatography (hexane/chloroform, 1:2 → chloroform) to obtain the title compound (1.79 g) as a light yellow solid.

### (Step 3)

### Production of 3-(2-fluoro-phenyl)-5-methyl-isoxazole-4-carboxylic acid:

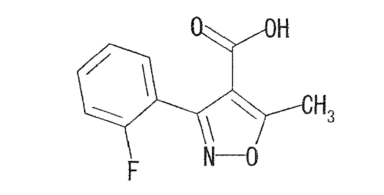

To a solution of 2-fluoro-benzohydroxymoyl chloride (1.79 g) in methanol (10 mL) were added a solution of sodium methyl acetoacetate in methanol (prepared with methyl acetoacetate [1.85 mL] and a solution of sodium methoxide in methanol [about 1 equivalent]) at room temperature. After stirring at 60°C for 1 hour, the reaction mixture was cooled to room temperature followed by addition of tetrahydrofuran (10 mL) and 2 N sodium hydroxide aqueous solution. After stirring at room temperature for 2 hours, the reaction mixture was concentrated to remove the organic solvent. The reaction mixture was washed twice with toluene and made acidic with 2 N hydrochloric acid. The produced solid was collected by filtration, washed with water, and dried to obtain the title compound (1.47 g) as a light brown solid.

### (Step 4)

### Production of 4-aminomethyl-3-(2-fluoro-phenyl)-5-methylisoxazole:

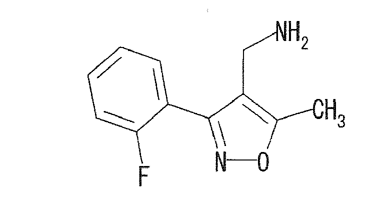

In the same manner as in Steps 5 to 7 of Reference Example 13, the title compound (200 mg) was obtained from 3-(2-fluoro-phenyl)-5-methyl-isoxazole-4-carboxylic acid as a yellow oily matter.

### Reference Example 17

### Production of 4'-aminomethyl-5,3'-dimethyl-[3,5']biisoxazolyl:

### (Step 1)

### Production of ethyl 3-(5-methyl-isoxazol-3-yl)-3-oxo-propionate:

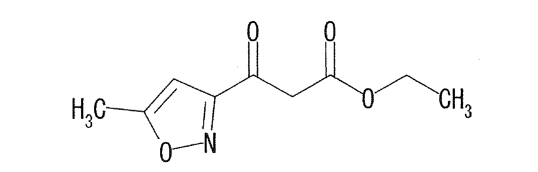

To a suspension of 5-methyl-isoxazole-3-carboxylic acid (2.78 g) in tetrahydrofuran (20 mL) was gradually added CDI (3.60 g). After stirring at room temperature for 30 minutes, the reaction mixture was heated on an oil bath to 60°C. After 1 hour, the reaction mixture was cooled to room temperature, magnesium chloride (2.30 g) was added followed by addition of ethyl potassium malonate (4.50 g). After stirring at room temperature for 3 hours, water (15 mL) was added to the reaction mixture followed by addition of 6 N hydrochloric acid (5.0 mL). The reaction mixture was concentrated, and the produced solid was collected by filtration, washed with water, and dried to obtain the title compound (3.99 g) as a white solid.

### (Step 2)

### Production of acetohydroxymoyl chloride:

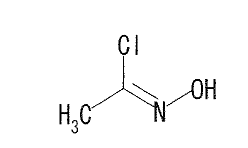

To a solution of acetoaldoxime (1.00 g) in tetrahydrofuran (15 mL)/chloroform (7.5 mL) was added N-chlorosuccinimide, and then pyridine (0.700 mL) was added dropwise. During this procedure, the reaction temperature was regulated to maintain at 20 to 40°C. After stirring at room temperature for 4 hours, the insoluble matters were filtered off, and the filtrate was concentrated. The crude product was dissolved in ethanol (5 mL) without performing purification and used in the following step.

### (Step 3)

### Production of ethyl 5,3'-dimethyl-[3,5°]biisoxazolyl-4'-carboxylate:

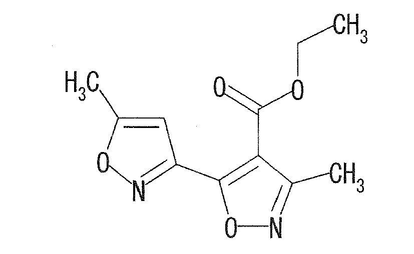

To a solution of acetohydroxymoyl chloride produced in Step 2 in ethanol were added ethyl 3-(5-methylisoxazol-3-yl)-3-oxo-propionate (1.50 g) produced in Step 1, tetrahydrofuran (15 mL), and potassium carbonate (1.16 g), and the mixture was stirred at room temperature. Progression of the reaction was monitored by thin layer chromatography every about 30 minutes, and a suitable volume of acetohydroxymoyl chloride solution and a required amount of potassium carbonate were added until ethyl 3-(5-methylisoxazol-3-yl)-3-oxo-propionate was eliminated. After completion of the reaction, the reaction mixture was concentrated followed by addition of ethyl acetate and concentrated again. The produced solid was collected by filtration and suspended in chloroform, and the solution portion was purified by silica gel column chromatography (chloroform) to obtain the title compound (942 mg) as a white solid.

### (Step 4)

### Production of 4'-aminomethyl-5,3'-dimethyl-[3,5']biisoxazolyl:

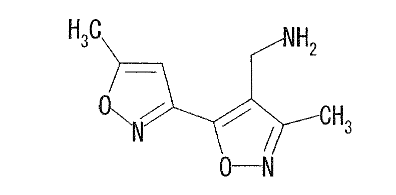

In the same manner as in Steps 4 to 7 of Reference Example 13, a crude product of the title compound was obtained from ethyl 5,3'-dimethyl-[3,5']biisoxazolyl-4'-carboxylate.

### Reference Example 18:

### Production of 4-aminomethyl-3-methyl-5-(6-methyl-pyridin-2-yl)-isoxazole:

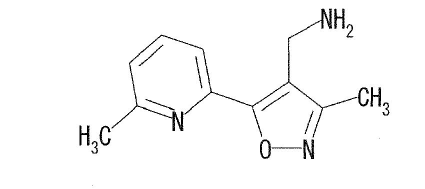

In the same manner as in Steps 1 to 4 of Reference Example 17, a crude product of the title compound was obtained from 6-methyl-pyridine-2-carboxylic acid.

### Reference Example 19

### Production of 4-aminomethyl-S-(2-chloro-pyridin-4-yl)-3-methyl-isoxazole:

### (Step 1)

### Production of ethyl 5-(2-chloro-pyridin-4-yl)-3-methylisoxazole-4-carboxylate:

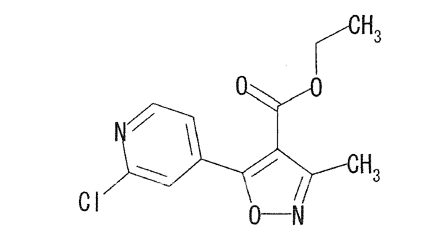

In the same manner as in Steps 1 to 3 of Reference Example 17, the title compound (1.17 g) was obtained from 2-chloro-pyridine-4-carboxylic acid as a white solid.

### (Step 2)

### Production of 5-(2-chloro-pyridin-4-yl)-3-methylisoxazole-4-carboxylic acid:

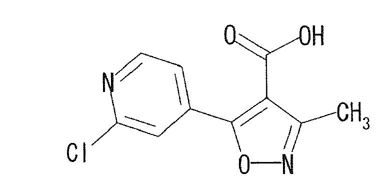

To a solution of ethyl 5-(2-chloro-pyridin-4-yl)-3-methyl-isoxazole-4-carboxylate (1.15 g) in tetrahydrofuran (4 mL)/ethanol (2 mL) were added 2 N sodium hydroxide aqueous solution (3.00 mL) and water (3 mL). After stirring at room temperature for 2 hours, the reaction mixture was concentrated, and the organic solvent was distilled off. Water was added to the resulting residue followed by addition of 6 N hydrochloric acid (1.00 mL) with stirring. The produced solid was collected by filtration, washed with water, and dried to obtain the title compound (994 mg) as a light yellow solid.

### (Step 3)

### Production of 5-(2-chloro-pyridin-4-yl)-4-hydroxymethyl-3-methyl-isoxazole:

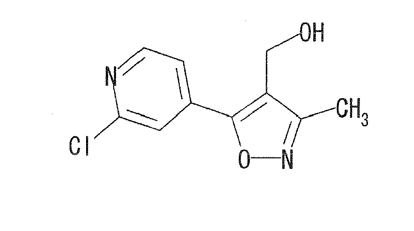

To a suspension of CDI (707 mg) in tetrahydrofuran (4 mL), 5-(2-chloro-pyridin-4-yl)-3-methyl-isoxazole-4-carboxylic acid (1.00 g) was gradually added at room temperature. After heating at 60°C for 1 hour, the reaction mixture was cooled to room temperature and gradually added to a solution of sodium borohydride (235 mg) in tetrahydrofuran (1 mL)/water (6 mL) with ice cooling. After stirring at room temperature for 1 hour, 6 N hydrochloric acid (2.5 mL) was added dropwise to the reaction mixture with ice cooling. After stirring at 70°C for 1 hour, the reaction mixture was concentrated, and the organic solvent was distilled off. Saturated brine was added to the residue, and the mixture was extracted with ethyl acetate. The organic layer was successively washed with water and saturated aqueous sodium hydrogencarbonate and then concentrated. The crude product was suspended in 2-propanol/water (1:2) and collected by filtration, washed with water, and dried to obtain the title compound (730 mg) as a white solid.

### (Step 4)

### Production of 5-(2-chloro-pyridin-4-yl)-4-chloromethyl-3-methyl-isoxazole:

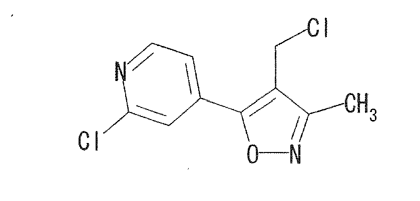

To a suspension of 5-(2-chloro-pyridin-4-yl)-4-hydroxymethyl-3-methyl-isoxazole (200 mg) in chloroform (2 mL) was added thionyl chloride (0.137 mL) followed by addition of pyridine (0.100 mL). After stirring at room temperature for 20 minutes, the reaction mixture was concentrated. Ethyl acetate was added to the residue, and the mixture was successively washed with water and saturated aqueous sodium hydrogencarbonate. The organic layer was concentrated and purified by silica gel column chromatography (hexane/chloroform, 1:1 → chloroform) to obtain the title compound (219 mg) as a light yellow solid.

### (Step 5)

### Production of 4-aminomethyl-5-(2-chloro-pyridin-4-yl)-3-methyl-isoxazole:

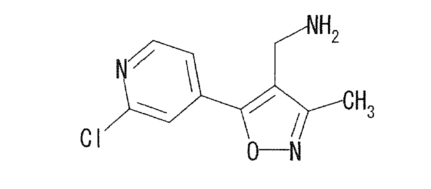

To a solution of 5-(2-chloro-pyridin-4-yl)-4-chloromethyl-3-methyl-isoxazole (231 mg) in tetrahydrofuran (3 mL)/ethanol (3 mL) was added 28% aqueous ammonia (10 mL). After stirring overnight at room temperature, 4 N sodium hydroxide aqueous solution (0.25 mL) was added to the reaction mixture, the mixture was concentrated, and most of the organic solvent was distilled off. The produced solid was collected by filtration, washed with water, and dried to obtain the title compound (146 mg) as a light yellow solid.

### Reference Example 20

### Production of 4-aminomethyl-3-methyl-5-(6-methyl-pyridin-3-yl)-isoxazole:

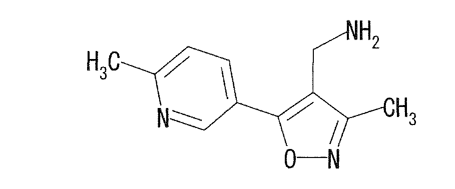

In the same manner as in Steps 1 to 5 of Reference Example 19, the title compound (72 mg) was obtained from 6-methyl-pyridine-3-carboxylic acid as a white solid.

### Reference Example 21

### Production of 4-aminomethyl-5-(2,4-dimethyl-thiazol-5-yl)-3-methyl-isoxazole:

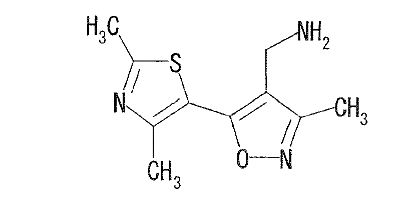

In the same manner as in Steps 1 to 5 of Reference Example 19, the title compound (54 mg) was obtained from 2,4-dimethyl-thiazole-5-carboxylic acid as a white solid.

### Reference Example 22

### Production of ethyl 5-amino-methyl-isoxazole-3-carboxylate trifluoroacetate:

### (Step 1)

### Production of N-(tert-butoxycarbonyl)propargylamine:

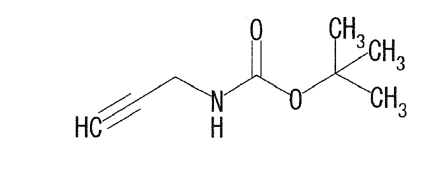

To a solution of propargylamine (2.51 g) in diethyl ether (35 mL) was added triethylamine (6.4 mL) followed by addition of di-tert-butyl dicarbonate (11.4 g) with ice cooling. After stirring overnight at room temperature, the reaction mixture was successively washed with saturated aqueous ammonium chloride, water and saturated brine, dried over anhydrous sodium sulfate, and then concentrated. The residue was purified by silica gel column chromatography (hexane/ethyl acetate, 3:1) to obtain the title compound (7.32 g) as a colorless oily matter.

### (Step 2)

### Production of ethyl 5-(tert-butoxycarbonylamino-methyl)-isoxazole-3-carboxylate:

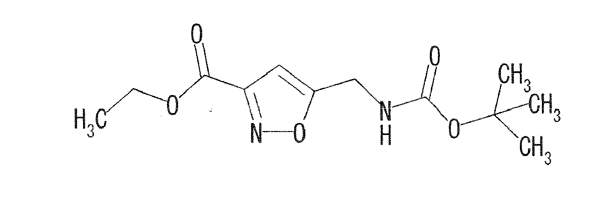

To a solution of ethyl 2-chloro-2-hydroxyiminoacetate (4.77 g) in tetrahydrofuran (35 mL), a solution of N-(tert-butoxycarbonyl)propargylamine (5.62 g) and triethylamine (5.0 mL) in diethyl ether (120 mL) was added dropwise. After stirring overnight at room temperature, 5% aqueous ammonium chloride was added to the reaction mixture. The organic layer was separated, successively washed with water and saturated brine, dried over anhydrous sodium sulfate, and then concentrated. The residue was purified by silica gel column chromatography (hexane/ethyl acetate, 3:1) to obtain the title compound (1.89 g) as a light yellow oily matter.

### (Step 3)

### Production of ethyl 5-aminomethyl-isoxazole-3-carboxylate trifluoroacetate:

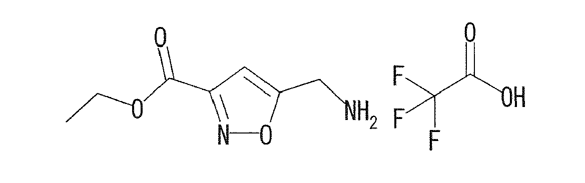

To ethyl 5-(tert-butoxycarbonylamino-methyl)-isoxazole-3-carboxylate (1.86 g) was added trifluoroacetic acid (3.7 mL) with ice cooling. After stirring at room temperature for 1 hour, the mixture was concentrated, and diethyl ether was added to the resulting residue. After 1 hour, the precipitated solid was collected by filtration to obtain the title compound (1.55 g) as a white solid.

### (Refer to J. Chem. Soc., Perkin Trans. 1, 1999, 2713-2723)

### Reference Example 23

### Production of 4-aminomethyl-2-methyl-5-(2-methylsulfanyl-ethyl)-oxazole dihydrochloride:

### (Step 1)

### Production of diethyl 2-acetylamino-2-(4-methylsulfanyl-2-oxo-butyl)-malonate:

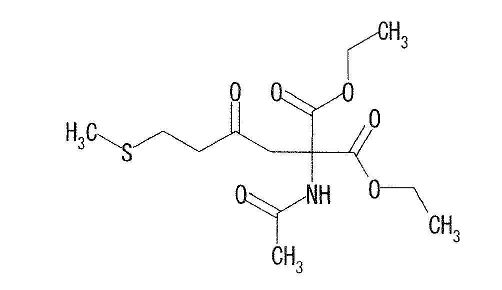

To a solution of potassium tert-butoxide (2.834 g) in tetrahydrofuran (100 mL), a solution of diethyl acetamide malonate (5.481 g) in tetrahydrofuran (30 mL) was added dropwise. After stirring at room temperature for 30 minutes, a solution of 3-methylthiopropionyl chloride (2.92 mL) in tetrahydrofuran (10 mL) was added dropwise. After 4 hours and 30 minutes, water was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 3:1 → 2:1) to obtain the title compound (3.560 g) as a yellow oily matter.

### (Step 2) Production of ethyl 2-methyl-5-(2-methylsulfanyl-ethyl)-oxazole-4-carboxylate:

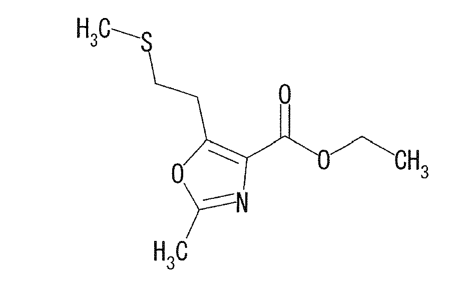

To a solution of diethyl 2-acetylamino-2-(4-methylsulfanyl-2-oxo-butyl)-malonate (510 mg) in N-methylpyrrolidone (3 mL) was added water (0.064 mL). After stirring at 200°C for 2 hours, water was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 4:1→ 3:1) to obtain the title compound (243 mg) as a yellow oily matter.

### (Step 3)

### Production of 4-hydroxymethyl-2-methyl-5-(2-methylsulfanyl-ethyl)-oxazole:

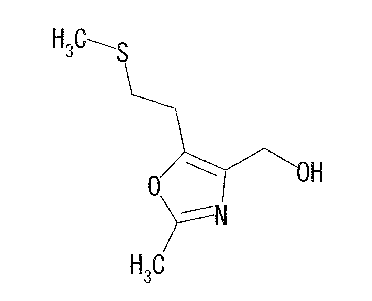

To a suspension of lithium aluminum hydride (60 mg) in tetrahydrofuran (10 mL), a solution of ethyl 2-methyl-5-(2-methylsulfanyl-ethyl)-oxazole-4-carboxylate (243 mg) in tetrahydrofuran (2 mL) was added dropwise with ice cooling. After stirring for 50 minutes, water (0.06 mL), 15% sodium hydroxide aqueous solution (0.06 mL), and water (0.06 mL) were successively added. After stirring for 45 minutes, the organic layer was separated, dried over anhydrous sodium sulfate, and then concentrated. The residue was purified by silica gel column chromatography (chloroform/ethyl acetate, 5:1 → 1:1 → 1:3) to obtain the title compound (108 mg) as a yellow oily matter.

### (Step 4)

### Production of 4-azidomethyl-2-methyl-5-(2-methylsulfanyl-ethyl)-oxazole:

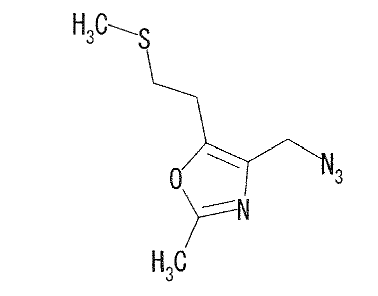

To a solution of 4-hydroxymethyl-2-methyl-5-(2-methylsulfanyl-ethyl)-oxazole (108 mg) in chloroform (2 mL) was added thionyl chloride (0.5 mL). After stirring at room temperature for 2 hours, the reaction mixture was concentrated to obtain 4-chloromethyl-2-methyl-5-(2-methylsulfanyl-ethyl)-oxazole hydrochloride.

Subsequently, in the same manner as in Step 1 of Reference Example 2, the title compound (89 mg) was obtain from 4-chloromethyl-2-methyl-5-(2-methylsulfanyl-ethyl)-oxazole hydrochloride as a brown oily matter.

### (Step 5)

### Production of 4-aminomethyl-2-methyl-5-(2-methylsulfanyl-ethyl)-oxazole dihydrochloride:

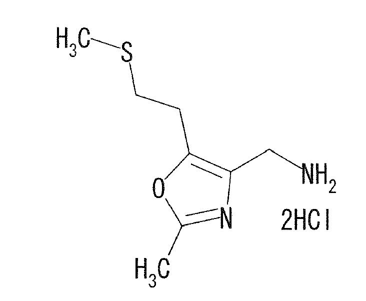

In the same manner as in Step 2 of Reference Example 2, the title compound (84 mg) was obtained from 4-azidomethyl-2-methyl-5-(2-methylsulfanyl-ethyl)-oxazole (89 mg) as a yellow solid.

### Reference Example 24

### Production of 5-aminomethyl-4-(4-fluoro-phenyl)-2-methyl-oxazole dihydrochloride:

### (Step 1)

### Production of ethyl 2-chloro-3-(4-fluoro-phenyl)-3-oxo-propionate:

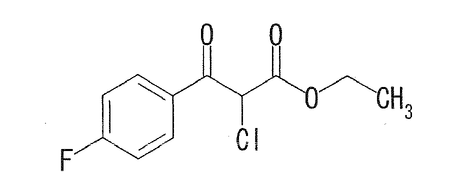

To a solution of ethyl 3-(4-fluoro-phenyl)-3-oxo-propionate (9.97 g) in chloroform (100 mL), sulfuryl chloride (3.85 mL) was added dropwise with ice cooling. After stirring overnight at room temperature, water was added to the reaction mixture, the organic layer was separated, and the aqueous layer was extracted with chloroform. The combined organic layers were successively washed with water and saturated brine, dried over anhydrous sodium sulfate, and then concentrated. The residue was purified by silica gel column chromatography (chloroform) to obtain the title compound (11.9 g) as a light yellow oily matter.

### (Step 2)

### Production of ethyl 2-acetoxy-3-(4-fluoro-phenyl)-3-oxo-propionate:

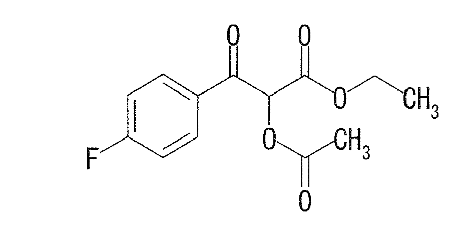

To a solution of ethyl 2-chloro-3-(4-fluoro-phenyl)-3-oxo-propionate (11.8 g) in acetic acid (60 mL) was added sodium acetate (11.7 g). After stirring overnight at 120°C under an argon atmosphere, the reaction mixture was concentrated, water was added to the residue, and the mixture was extracted with ethyl acetate. The organic layer was successively washed with water, saturated aqueous sodium hydrogencarbonate, and saturated brine, dried over anhydrous sodium sulfate, and then concentrated. The residue was purified by silica gel column chromatography (hexane/ethyl acetate, 3:1) to obtain the title compound (11.0 g) as a light yellow oily matter.

### (Step 3)

### Production of ethyl 4-(4-fluoro-phenyl)-2-methyl-oxazole-5-carboxylate:

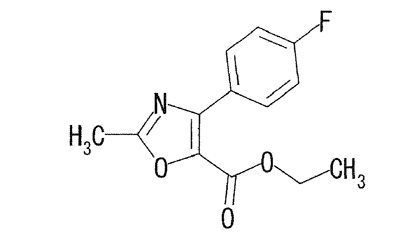

To a solution of ethyl 2-acetoxy-3-(4-fluorophenyl)-3-oxo-propionate (11.0 g) in acetic acid (55 mL) was added ammonium acetate (14.2 g). After stirring at 120°C for 4 hours and 30 minutes, the reaction mixture was concentrated, water was added to the residue, and the mixture was extracted with ethyl acetate. The organic layer was successively washed with water, saturated aqueous sodium hydrogencarbonate, and saturated brine, dried over anhydrous sodium sulfate, and then concentrated. The residue was purified by silica gel column chromatography (hexane/ethyl acetate, 3:1) to obtain the title compound (6.55 g) as a light yellow solid.

### (Step 4)

### Production of 4-(4-fluoro-phenyl)-2-methyl-oxazole-5-carboxylic acid amide:

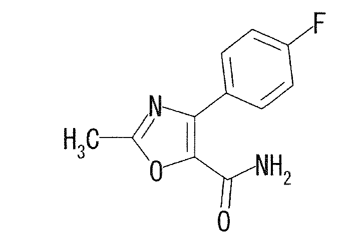

To a solution of ethyl 4-(4-fluoro-phenyl)-2-methyl-oxazole-5-carboxylate (6.52 g) in 7 N ammonia/methanol (65 mL) was added a solution (5.05 g) of 28% sodium methoxide in methanol. After stirring overnight at room temperature, the reaction mixture was concentrated, water was added to the residue, and the mixture was extracted with ethyl acetate. The organic layer was washed with saturated brine, dried over anhydrous sodium sulfate, and then concentrated to obtain the title compound (4.34 g) as a white solid.

### (Step 5)

### Production of 5-cyano-4-(4-fluoro-phenyl)-2-methyl-oxazole:

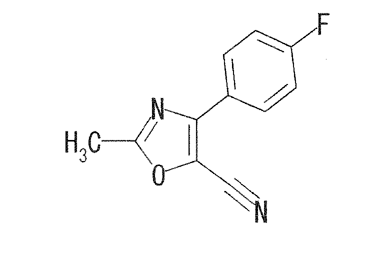

To a solution of 4-(4-fluoro-phenyl)-2-methyl-oxazole-5-carboxylic acid amide (4.34 g) in ethyl acetate (35 mL) was added N-methylmorpholine (4.75 mL), and then trichloroacetyl chloride (2.43 mL) was added dropwise with ice cooling. After stirring at room temperature for 1 hour, water was added to the reaction mixture, and the mixture was extracted with ethyl acetate. The combined organic layers were successively washed with 10% aqueous potassium hydrogen sulfate, saturated aqueous sodium hydrogencarbonate, and saturated brine, dried over anhydrous sodium sulfate, and then concentrated. The residue was purified by silica gel column chromatography (chloroform/methanol, 95:5) to obtain the title compound (3.90 g) as a light yellow solid.

### (Step 6)

### Production of 5-aminomethyl-4-(4-fluoro-phenyl)-2-methyl-oxazole dihydrochloride:

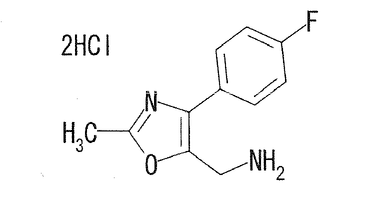

To a solution of 5-cyano-4-(4-fluoro-phenyl)-2-methyl-oxazole (907 mg) in methanol (10 mL)/ethyl acetate (10 mL) were added concentrated hydrochloric acid (1.18 mL) and 7.5% palladium-carbon (96 mg), and the mixture was stirred at room temperature for 5 hours under a hydrogen atmosphere. The catalyst was filtered off, the filtrate was concentrated, ethanol was added to the residue, and the mixture was stirred at room temperature. After 2 hours, the precipitated solid was collected by filtration to obtain the title compound (467 mg) as a light yellow solid.

### Reference Example 25

### Production of 5-aminomethyl-4-(3-fluoro-phenyl)-2-methyl-oxazole dihydrochloride:

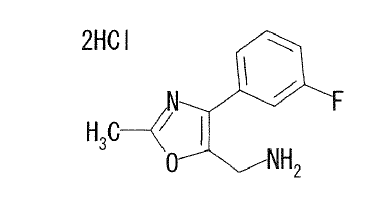

In the same manner as in Steps 1 to 6 of Reference Example 24, the title compound (641 mg) was obtained from ethyl 3-(3-fluoro-phenyl)-3-oxo-propionate as a light yellow solid.

### Reference Example 26

### Production of 5-aminomethyl-4-(2-fluoro-phenyl)-2-methyl-oxazole dihydrochloride:

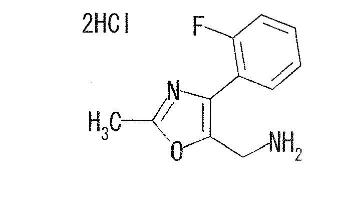

In the same manner as in Steps 1 to 6 of Reference Example 24, the title compound (1.22 g) was obtained from 3-(2-fluoro-phenyl)-3-oxo-ethyl propionate as a white solid.

### Reference Example 27

### Production of 5-aminomethyl-4-(4-chloro-phenyl)-2-methyl-oxazole dihydrochloride:

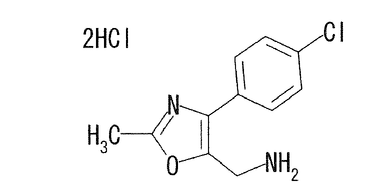

In the same manner as in Steps 1 to 6 of Reference Example 24, the title compound (871 mg) was obtained from ethyl 3-(4-chloro-phenyl)-3-oxo-propionate as a white solid.

### Reference Example 28

### Production of 5-aminomethyl-2-methyl-4-(4-methyl-phenyl)-oxazole dihydrochloride:

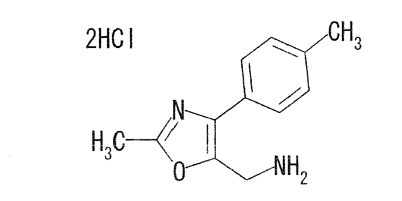

In the same manner as in Steps 1 to 6 of Reference Example 24, the title compound (713 mg) was obtained from ethyl 3-oxo-3-(4-methyl-phenyl)propionate as a white solid.

### Reference Example 29

### Production of 5-aminomethyl-2-methyl-4-(3-methyl-phenyl)-oxazole dihydrochloride:

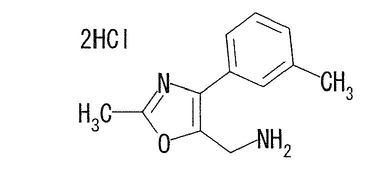

In the same manner as in Steps 1 to 6 of Reference Example 24, the title compound (1.02 g) was obtained from ethyl 3-oxo-3-(3-methyl-phenyl)propionate as a white solid.

### Reference Example 30

### Production of 5-aminomethyl-4-(3-methoxy-phenyl)-2-methyl-oxazole dihydrochloride:

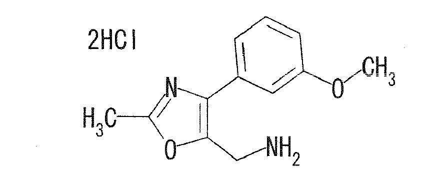

In the same manner as in Steps 1 to 6 of Reference Example 24, the title compound (1.14 g) was obtained from ethyl 3-oxo-3-(3-methoxy-phenyl)propionate as a white solid.

### Reference Example 31

### Production of 5-aminomethyl-4-biphenyl-4-yl-2-methyl-oxazole dihydrochloride:

### (Step 1)

### Production of methyl 3-biphenyl-4-yl-3-oxo-propionate:

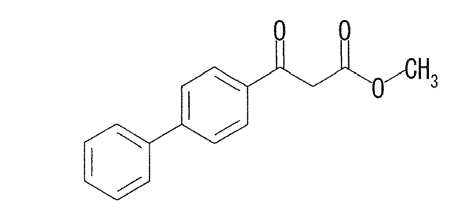

To a suspension of sodium hydride (4.53 g) in N,N-dimethylformamide (30 mL), a solution of 4-acetylbiphenyl (10.0 g) in N,N-dimethylformamide (50 mL) was added over 15 minutes. After stirring at room temperature for 30 minutes, the mixture was ice cooled, and dimethyl carbonate (27.0 mL) was added dropwise. After stirring at room temperature for 3 hours, the reaction mixture was poured over saturated aqueous sodium hydrogencarbonate, and the mixture was extracted with diethyl ether. The organic layer was washed with saturated brine, dried over anhydrous sodium sulfate, and then concentrated. Ethanol was added to the residue, the mixture was stirred at 70°C for 1 hour, and the precipitated solid was collected by filtration to obtain the title compound (9.42 g) as a yellow solid.

### (Step 2)

### Production of 5-aminomethyl-4-biphenyl-4-yl-2-methyl-oxazole dihydrochloride:

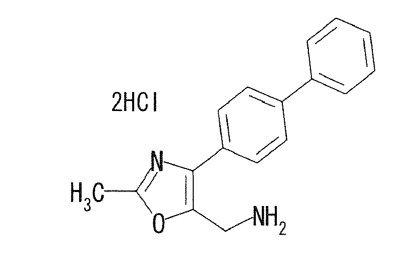

In the same manner as in Steps 1 to 6 of Reference Example 24, the title compound (370 mg) was obtained from methyl 3-biphenyl-4-yl-3-oxo-propionate as a yellow solid.

### Reference Example 32

### Production of 5-aminomethyl-2-methyl-4-phenethyl-oxazole dihydrochloride:

### (Step 1)

### Production of ethyl 3-oxo-5-phenyl-valerate:

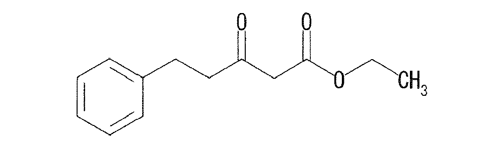

According to a reference (J. Org. Chem., 58, 7932, 1993.), the title compound (21.1 g) was obtained from 3-phenylpropionyl chloride as a yellow oily matter.

### (Step 2)

### Production of ethyl 2-chloro-3-oxo-5-phenyl-valerate:

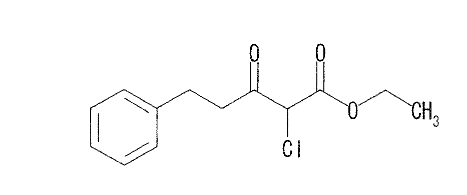

According to a reference (Tetrahedron Lett., 46, 623, 2005.), the title compound (16.6 g) was obtained from ethyl 3-oxo-5-phenyl-valerate as a pale pink oily matter.

### (Step 3)

### Production of ethyl 2-methyl-4-phenethyl-oxazole-5-carboxylate:

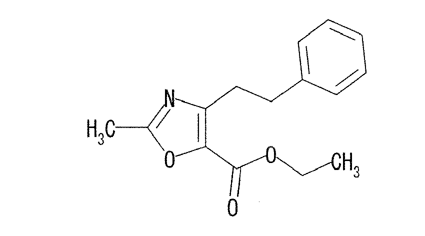

To ethyl 2-chloro-3-oxo-5-phenyl-valerate (8.0 g) was added acetamide (5.57 g). After stirring at 120°C for 25 hours, water was added to the reaction mixture, and the mixture was extracted with ethyl acetate. The organic layer was successively washed with water and saturated brine, dried over anhydrous sodium sulfate, and then concentrated. The residue was purified by silica gel column chromatography (hexane/ethyl acetate, 9:1→ 6:1) to obtain the title compound (4.93 g) as a yellow oily matter.

### (Step 4)

### Production of 5-cyano-2-methyl-4-phenethyl-oxazole:

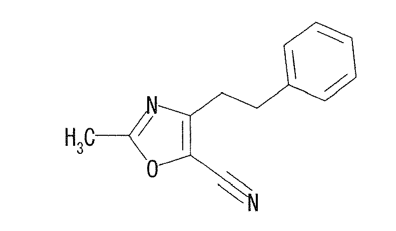

To ethyl 2-methyl-4-phenethyl-oxazole-5-carboxylate (4.93 g) was added 7 N ammonia/methanol (190 mL). After stirring at room temperature for 6 days, the reaction mixture was concentrated to obtain 2-methyl-4-phenethyl-oxazole-5-carboxylic acid amide as a mixture of a yellow oily matter and a solid.

Subsequently, to a solution of 2-methyl-4-phenethyl-oxazole-5-carboxylic acid amide in ethyl acetate (50 mL) was added followed by addition of N-methylmorpholine (5.64 mL), and then trichloroacetyl chloride (2.76 mL) was added dropwise with ice cooling. After stirring overnight at room temperature, water was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 4:1) to obtain the title compound (3.17 g) as a yellow oily matter.

### (Step 5)

### Production of 5-aminomethyl-2-methyl-4-phenethyl-oxazole dihydrochloride:

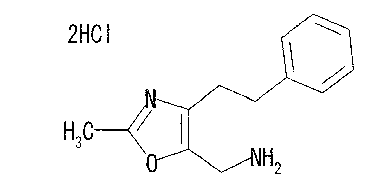

To a solution of 5-cyano-2-methyl-4-phenethyl-oxazole (1.6 g) in methanol (32 mL) were added concentrated hydrochloric acid (2 mL) and 10% palladium-carbon (160 mg), and the mixture was stirred at room temperature for 6 hours under 3 to 4 atm of a hydrogen atmosphere. The catalyst was filtered off, the filtrate was concentrated, ethanol was added to the residue, and the mixture was concentrated again. 2-Propanol was added to the residue, and the mixture was stirred at room temperature for 1 hour, and then the precipitated solid was collected by filtration to obtain the title compound (685 mg) as beige crystals.

### Reference Example 33

### Production of 5-aminomethyl-4-ethoxycarbonyl-2-methyl-oxazole dihydrobromide:

### (Step 1)

### Production of ethyl 4-benzyloxycarbonylamino-3-oxo-butyrate:

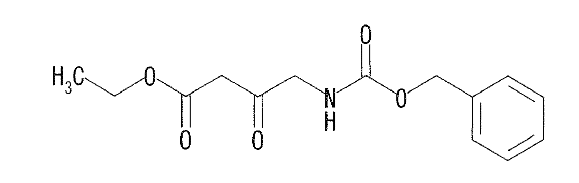

To a solution of N-benzyloxycarbonyl-glycine (7.749 g) in tetrahydrofuran (100 mL) was added CDI (3.414 g). After stirring at room temperature for 2 hours, magnesium chloride (1.822 g) was added to the reaction mixture followed by addition of ethyl potassium malonate (3.265 g). After stirring overnight at 35°C, the insoluble matters were filtered off, and ethyl acetate was added to the filtrate. The organic layer was successively washed with 1 N hydrochloric acid, water, and saturated brine, dried over anhydrous sodium sulfate, and then concentrated. The residue was purified by silica gel column chromatography (hexane/ethyl acetate, 5:1 → 2:1) to obtain the title compound (4.021 g) as a yellow oily matter.

### (Step 2)

### Production of ethyl 4-benzyloxycarbonylamino-2-hydroxyimino-3-oxo-butyrate:

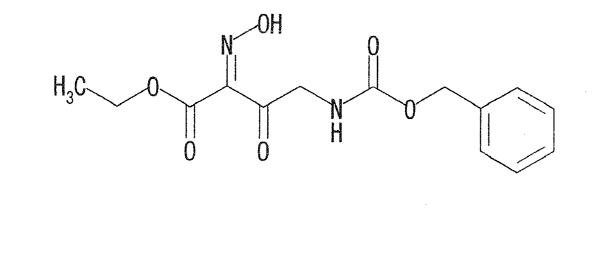

To a solution of ethyl 4-benzyloxycarbonylamino-3-oxo-butyrate (4.021 g) in acetic acid (15 mL), aqueous sodium nitrite (1.318 g) was added dropwise. After stirring at room temperature for 3 hours, water was added to the reaction mixture, and the mixture was extracted with ethyl acetate. The organic layer was successively washed with water and saturated brine, dried over anhydrous sodium sulfate, and then concentrated to obtain the title compound (4.824 g) as a brown solid.

### (Step 3)

### Production of ethyl 2-acetylamino-4-benzyloxycarbonylamino-3-oxo-butyrate:

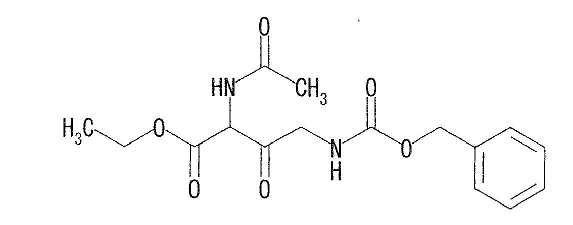

To a solution of ethyl 4-benzyloxycarbonylamino-2-hydroxy imino-3-oxo-butyrate (4.824 g) in acetic acid (40 mL) was added acetic anhydride (8.6 mL), and then zinc (7.954 g) was gradually added. After stirring overnight at room temperature, the insoluble matters were filtered off, and the filtrate was concentrated. Ethyl acetate was added to the residue, and the mixture was successively washed with water and saturated brine, dried over anhydrous sodium sulfate, and then concentrated. The residue was purified by silica gel column chromatography (hexane/ethyl acetate, 2:1 → 1:2) to obtain the title compound (3.299 g) as a white solid.

### (Step 4)

### Production of 5-(benzyloxycarbonylamino-methyl)-4-ethoxycarbonyl-2-methyl-oxazole:

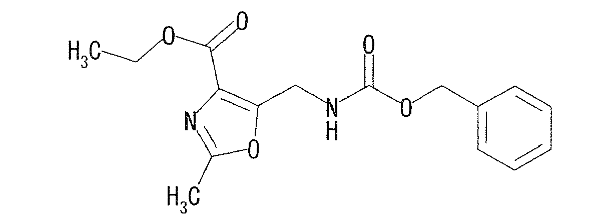

To a suspension of ethyl 2-acetylamino-4-benzyloxycarbonylamino-3-oxo-butyrate (497 mg) in toluene (5 mL) was added thionyl chloride (0.6 mL). After stirring at 100°C for 5 hours and 30 minutes, the reaction mixture was concentrated. Water was added to the residue, and the mixture was extracted with ethyl acetate. The organic layer was successively washed with water and saturated brine, dried over anhydrous sodium sulfate, and then concentrated. The residue was purified by silica gel column chromatography (hexane/ethyl acetate, 2:1 → 1:1) to obtain the title compound (249 mg) as a white solid.

### (Step 5)

### Production of 5-aminomethyl-4-ethoxycarbonyl-2-methyl-oxazole dihydrobromide:

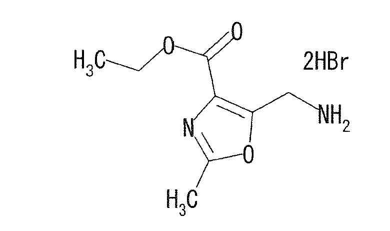

To 5-(benzyloxycarbonylamino-methyl)-4-ethoxycarbonyl-2-methyl-oxazole (4.065 g) was added 25% hydrogen bromide/acetic acid (25 mL) with ice cooling. After stirring for 4 hours with ice cooling, ethyl acetate was added to the reaction mixture, and the precipitated solid was collected by filtration to obtain the title compound (3.314 g) as a white solid.

### Reference Example 34

### Production of 5-aminomethyl-4-hydroxymethyl-2-methyl-oxazole dihydrochloride:

### (Step 1)

### Production of 5-benzyloxycarbonylamino-methyl-4-hydroxymethyl-2-methyl-oxazole:

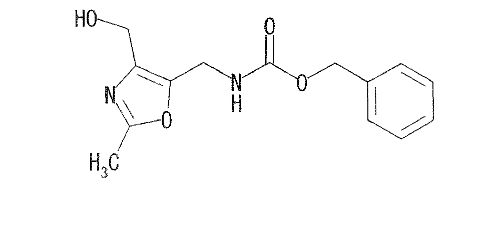

To a solution of 5-(benzyloxycarbonylamino-methyl)-4-ethoxycarbonyl-2-methyl-oxazole (4.66 g) produced in Step 4 of Reference Example 33 in dichloromethane (100 mL), a solution (51.6 mL) of 1 M diisobutylaluminum hydride in toluene was added dropwise at -78°C. After stirring at -78°C for 2 hours, the mixture was stirred at 0°C for 2 hours. Saturated aqueous ammonium chloride was added to the reaction mixture, the insoluble matters were filtered off, and the mixture was extracted with ethyl acetate. The organic layer was washed with saturated brine, dried over anhydrous sodium sulfate, and then concentrated. The residue was purified by silica gel column chromatography (ethyl acetate) to obtain the title compound (2.01 g) as a pale yellow solid.

### (Step 2)

### Production of 5-aminomethyl-4-hydroxymethyl-2-methyl-oxazole dihydrochloride:

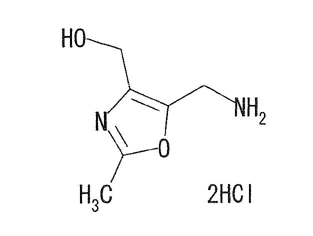

To a solution of 5-benzyloxycarbonylamino-methyl-4-hydroxymethyl-2-methyl-oxazole (1.0 g) in methanol (10 mL) were added concentrated hydrochloric acid (0.96 mL) and 7.5% palladium-carbon (150 mg). After stirring at room temperature for 12 hours under a hydrogen atmosphere, the catalyst was filtered off. The filtrate was concentrated to obtain the title compound (782 mg) as a beige crystals.

### Reference Example 35

### Production of 5-aminomethyl-4-benzyl-2-methyl-oxazole dihydrochloride:

### (Step 1)

### Production of methyl 4-benzyl-2-methyl-oxazole-5-carboxylate:

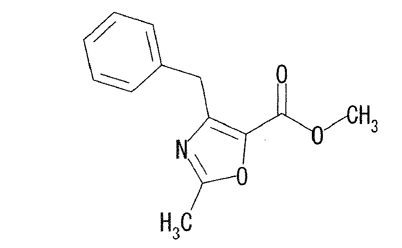

In same manner as in Steps 1 to 3 of Reference Example 32, the title compound (240 mg) was synthesized from benzylacetyl chloride as a colorless oily matter.

### (Step 2)

### Production of 5-aminomethyl-4-benzyl-2-methyl-oxazole dihydrochloride:

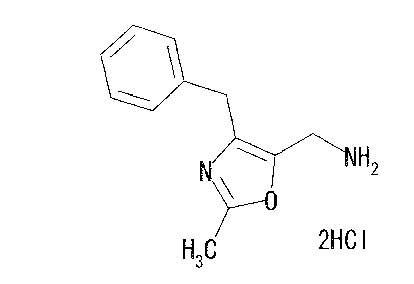

In the same manner as in Steps 4 and 5 of Reference Example 32, the title compound (202 mg) was obtained from methyl 4-benzyl-2-methyl-oxazole-5-carboxylate as a yellow solid.

### Reference Example 36

### Production of 5-aminomethyl-4-cyclopropyl-2-methyl-oxazole dihydrochloride:

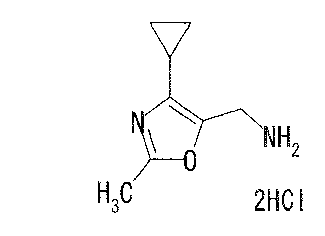

In the same manner as in Steps 1 to 6 of Reference Example 24, the title compound (2.90 g) was obtained from methyl 3-cyclopropyl-3-oxo-propionate as a white solid.

### Reference Example 37

### Production of 5-aminomethyl-4-cyclohexyl-2-methyl-oxazole dihydrochloride:

### (Step 1)

### Production of methyl 3-cyclohexyl-3-oxo-propionate:

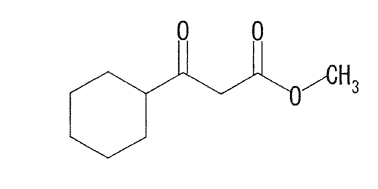

According to a reference (J. Org. Chem., 58, 7932, 1993.), the title compound (9.11 g) was obtained from cyclohexane carbonyl chloride (10.7 mL) and Meldrum's acid (11.88 g) as a light yellow oily matter.

### (Step 2)

### Production of methyl 2-chloro-3-cyclohexyl-3-oxo-propionate:

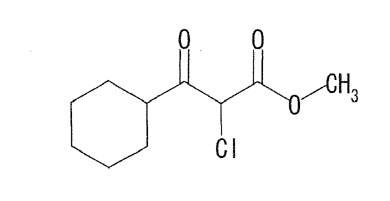

According to a reference (Tetrahedron Lett., 46, 623, 2005.), the title compound (10.3 g) was obtained from methyl 3-cyclohexyl-3-oxo-propionate (9.10 g) as a colorless oily matter.

### (Step 3)

### Production of 5-aminomethyl-4-cyclohexyl-2-methyl-oxazole dihydrochloride:

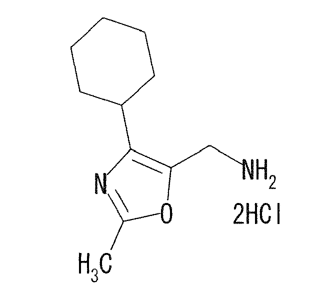

In the same manner as in Steps 2 to 6 of Reference Example 24, the title compound (654 mg) was obtained from methyl 2-chloro-3-cyclohexyl-3-oxo-propionate (10.1 g) as a yellow solid.

### Reference Example 38

### Production of 5-aminomethyl-2-cyclopropyl-4-methyl-oxazole dihydrochloride:

### (Step 1)

### Production of ethyl 2-cyclopropyl-4-methyl-oxazole-5-carboxylate:

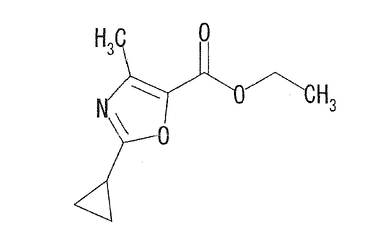

To 2-chloroethyl acetoacetate (10.0 mL) was added cyclopropanecarboxylic acid amide (12.4 g), and the mixture was stirred at 120°C for 20 hours. The mixture was cooled to room temperature, ethyl acetate was added to the reaction mixture, and the insoluble matters were filtered off. Water was added to the filtrate, the organic layer was separated, and the aqueous layer was extracted with ethyl acetate. The combined organic layers were washed with saturated brine, dried over anhydrous sodium sulfate, and then concentrated. The residue was purified by silica gel column chromatography (hexane/ethyl acetate, 3: 1) to obtain the title compound (12.9 g) as a colorless oily matter.

### (Step 2)

### Production of 5-aminomethyl-2-cyclopropyl-4-methyl-oxazole dihydrochloride:

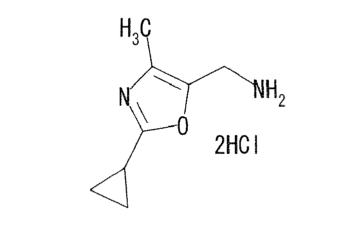

In the same manner as in Steps 4 to 6 of Reference Example 24, the title compound (1.39 g) was obtained from ethyl 2-cyclopropyl-4-methyl-oxazole-5-carboxylate as a light yellow solid.

### Reference Example 39

### Production of 5-aminomethyl-2-cyclohexyl-4-methyl-oxazole dihydrochloride:

### (Step 1)

### Production of ethyl 2-cyclohexyl-4-methyl-oxazole-5-carboxylate:

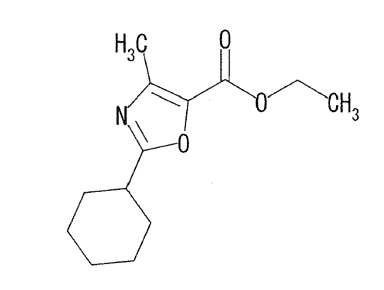

In the same manner as in Step 1 of Reference Example 38, the title compound (2.84 g) was obtained from 2-chloroethyl acetoacetate (6.03 g) and cyclohexane carboxamide (11.0 g) as a colorless oily matter.

### (Step 2)

### Production of 5-aminomethyl-2-cyclohexyl-4-methyl-oxazole dihydrochloride:

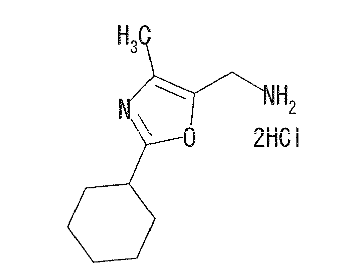

In the same manner as in Steps 4 to 6 of Reference Example 24, the title compound (2.18 g) was obtained from ethyl 2-cyclohexyl-4-methyl-oxazole-5-carboxylate (2.84 g) as a light yellow solid.

### Reference Example 40

### Production of 5-aminomethyl-2-benzyl-4-methyl-oxazole dihydrochloride:

### (Step 1)

### Production of ethyl 2-benzyl-4-methyl-oxazole-5-carboxylate:

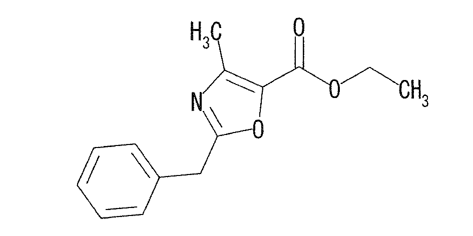

In the same manner as in Step 1 of Reference Example 38, the title compound (2.10 g) was obtained from phenyl acetamide and 2-chloroethyl acetoacetate as a white solid.

### (Step 2)

### Production of 5-aminomethyl-2-benzyl-4-methyl-oxazole dihydrochloride:

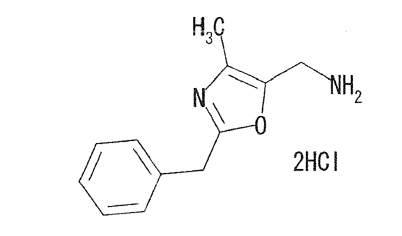

In the same manner as in Steps 4 and 5 of Reference Example 32, the title compound (483 mg) was obtained from ethyl 2-benzyl-4-methyl-oxazole-5-carboxylate as a yellow solid.

### Reference Example 41

### Production of 5-aminomethyl-4-methyl-2-phenethyl-oxazole dihydrochloride:

### (Step 1)

### Production of 3-phenyl-propionamide:

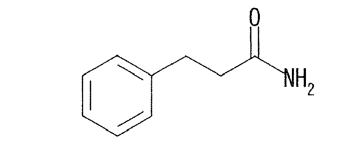

To 28% aqueous ammonia (250 mL), a solution of 3-phenylpropionyl chloride (21.3 g) in dimethoxyethane (20 mL) was added dropwise with ice cooling. After stirring for 2 hours with ice cooling, the precipitated solid was collected by filtration to obtain the title compound (9.15 g) as a white solid.

### (Step 2)

### Production of ethyl 4-methyl-2-phenethyl-oxazole-5-carboxylate:

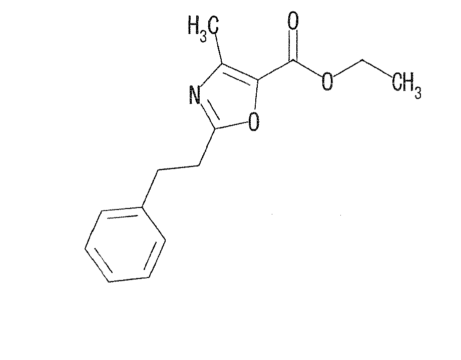

To 3-phenyl-propionamide (9.13 g) was added 2-chloroethyl acetoacetate (5.04 g). After stirring at 120°C for 24 hours, water was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 4:1) to obtain the title compound (5.23 g) as a yellow oily matter.

### (Step 3)

### Production of 4-methyl-2-phenethyl-oxazole-5-carboxylic acid amide:

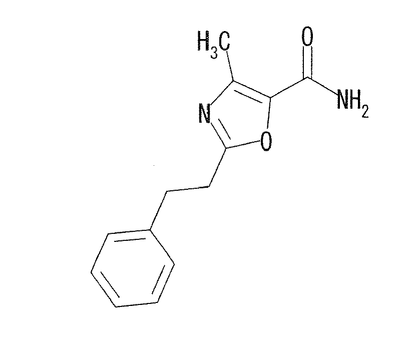

To a solution (100 mL) of ethyl 4-methyl-2-phenethyl-oxazole-5-carboxylate (5.22 g) in 7 N ammonia/methanol was added a solution of 28% sodium methoxide in methanol. After stirring at room temperature for 7 days, ammonium chloride (150 mg) was added to the reaction mixture, and the mixture was concentrated. The residue was dissolved in methanol (20 mL) followed by addition of water, and the precipitated solid was collected by filtration to obtain the title compound (3.15 g) as a white solid.

### (Step 4)

### Production of 5-cyano-4-methyl-2-phenethyl-oxazole:

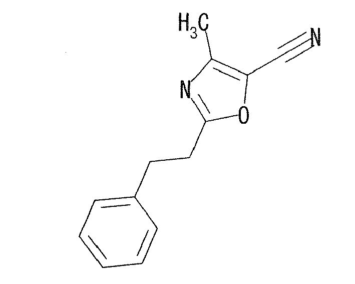

To a solution of 4-methyl-2-phenethyl-oxazole-5-carboxylic acid amide (3.07 g) in ethyl acetate (40 mL) was added N-methylmorpholine (3.95 mL), and then trichloroacetyl chloride (1.93 mL) was added dropwise with ice cooling. After stirring at room temperature for 5 hours, water was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 4:1) to obtain the title compound (2.79 g) as a yellow oily matter.

### (Step 5)

### Production of 5-aminomethyl-4-methyl-2-phenethyl-oxazole dihydrochloride:

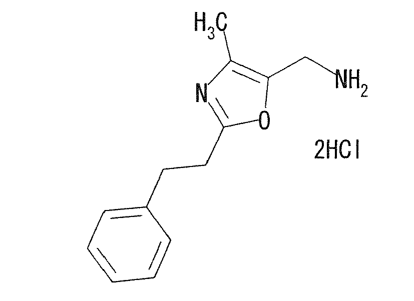

To a solution of 5-cyano-4-methyl-2-phenethyl-oxazole (1.6 g) in methanol (32 mL) were added concentrated hydrochloric acid (2 mL) and 10% palladium-carbon (160 mg). After stirring at room temperature for 5 hours under 3 to 4 atm of a hydrogen atmosphere, the catalyst was filtered off. The filtrate was concentrated, ethanol was added to the residue, and the mixture was concentrated again. 2-Propanol was added to the residue, the mixture was stirred at room temperature for 1 hour, and the precipitated solid was collected by filtration to obtain the title compound (893 mg) as a beige solid.

### Reference Example 42

### Production of 5-aminomethyl-4-methyl-2-trityloxymethyl-oxazole:

### (Step 1)

### Production of ethyl 4-methyl-oxazole-5-carboxylate:

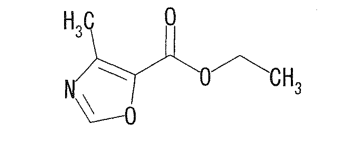

To 2-chloroethyl acetoacetate (132.545 g) was added formamide (90 mL), and the mixture was stirred overnight at 120°C. Water was added to the reaction mixture, and the mixture was extracted with ethyl acetate, washed with saturated brine, dried over anhydrous sodium sulfate, and then concentrated. The residue was distilled under reduced pressure (bp 66 to 69°C (100 to 150 Pa)) to obtain the title compound (68.976 g) as colorless crystals.

### (Step 2)

### Production of 5-hydroxymethyl-4-methyl-oxazole:

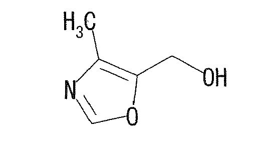

To a suspension of lithium aluminum hydride (23 g) in tetrahydrofuran (700 mL), a solution of ethyl 4-methyl-oxazole-5-carboxylate (101.87 g) in tetrahydrofuran (100 mL) was added dropwise at -50 to - 60°C. After stirring for 10 minutes, water (23 mL), 15% sodium hydroxide aqueous solution (23 mL), and water (69 mL) were successively added to the reaction mixture. After stirring at room temperature for 40 minutes, the organic layer was separated, dried over anhydrous sodium sulfate, and then concentrated to obtain the title compound (54.487 g) as a yellow solid.

### (Step 3)

### Production of 5-(tert-butyl-dimethyl-silanyloxymethyl)-4-methyl-oxazole:

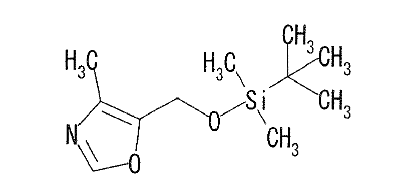

To a solution of 5-hydroxymethyl-4-methyl-oxazole (7.89 g) in chloroform (70 mL) were added triethylamine (11.6 mL) and 4-dimethylaminopyridine (451 mg) followed by addition of tert-butyldimethylsilyl chloride (11.0 g) with ice cooling. After stirring overnight at room temperature, water was added to the reaction mixture. After stirring for 2 hours, the organic layer was separated, and the aqueous layer was extracted with chloroform. The combined organic layers were washed with saturated brine, dried over anhydrous sodium sulfate, and then concentrated. The residue was purified by silica gel column chromatography (hexane/ethyl acetate, 4:1) to obtain the title compound (12.862 g) as a colorless oily matter.

### (Step 4)

### Production of 5-(tert-butyl-dimethyl-silanyloxymethyl)-2-hydroxymethyl-4-methyl-oxazole:

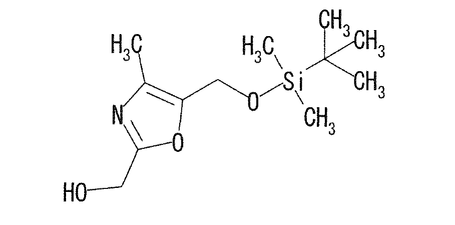

To a solution of 5-(tert-butyl-dimethyl-silanyloxymethyl)-4-methyl-oxazole (2.000 g) in tetrahydrofuran (20 mL), 1.59 M *n*-butyllithium/hexane solution (5.55 mL) was added dropwise at -78°C. After stirring at -78°C for 1 hour, N,N-dimethylformamide (0.82 mL) was added. After stirring at room temperature for 15 hours, water was added to the reaction mixture, and the mixture was extracted with ethyl acetate. The organic layer was washed with saturated brine, dried over anhydrous sodium sulfate, and then concentrated to obtain 5-(tert-butyl-dimethyl-silanyloxymethyl)-4-methyl-oxazole-2-carboaldehyde (2.168 g).

Subsequently, to a solution of 5-(tert-butyldimethyl-silanyloxymethyl)-4-methyl-oxazole-2-carboaldehyde (2.168 g) in methanol (22 mL), sodium borohydride (384 mg) was gradually added with ice cooling. After stirring at room temperature for 30 minutes, saturated aqueous ammonium chloride was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 2:1 → 1:1) to obtain the title compound (1.069 g) as a white solid.

### (Step 5)

### Production of 5-(tert-butyl-dimethyl-silanyloxymethyl)-4-methyl-2-trityloxymethyl-oxazole:

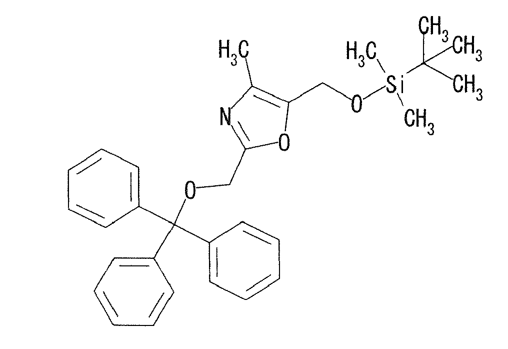

To a solution of 5-(tert-butyl-dimethyl-silanyloxymethyl)-2-hydroxymethyl-4-methyl-oxazole (1.069 g) in chloroform (10 mL) were added triethylamine (1.15 mL) and 4-dimethylaminopyridine (36 mg) followed by addition of trityl chloride (1.50 g) with ice cooling. After stirring overnight at room temperature, water was added to the reaction mixture, the organic layer was separated, and the aqueous layer was extracted with chloroform. The combined organic layers were concentrated, and the residue was purified by silica gel column chromatography (hexane/ethyl acetate, 6:1 → 4:1) to obtain the title compound (2.48 g) as a yellow oily matter.

### (Step 6)

### Production of 5-hydroxymethyl-4-methyl-2-trityloxymethyl-oxazole:

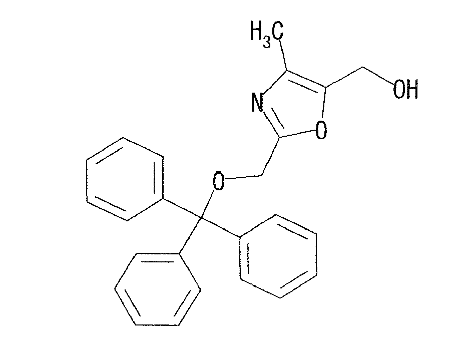

To a solution of 5-(tert-butyl-dimethyl-silanyloxymethyl)-4-methyl-2-trityloxymethyl-oxazole (2.48 g) in tetrahydrofuran (25 mL) was added a solution of 1 M tetrabutylammonium fluoride in tetrahydrofuran (8.5 mL) with ice cooling. After stirring at room temperature for 1 hour, saturated aqueous ammonium chloride was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 3:1 → 1:1) to obtain the title compound (1.59 g) as a colorless oily matter.

### (Step 7)

### Production of 5-chloromethyl-4-methyl-2-trityloxymethyl-oxazole:

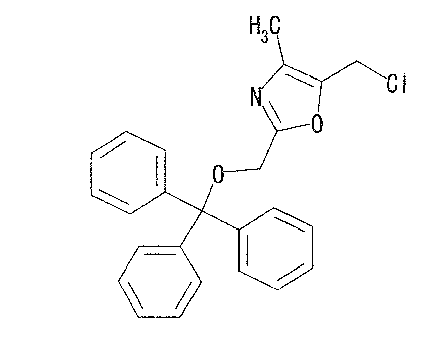

To a solution of 5-hydroxymethyl-4-methyl-2-trityloxymethyl-oxazole (727 mg) in chloroform (10 mL) was added triethylamine (0.29 mL) followed by addition of methanesulfonyl chloride (0.155 mL) with ice cooling. After stirring overnight at room temperature, water was added to the reaction mixture, the organic layer was separated, and the aqueous layer was extracted with chloroform. The combined organic layers were washed with saturated brine, dried over anhydrous sodium sulfate, and then concentrated to obtain the title compound (753 mg) as a yellow oily matter.

### (Step 8)

### Production of 5-(1,3-dioxo-1,3-dihydro-isoindol-2-ylmethyl)-4-methyl-2-trityloxymethyl-oxazole:

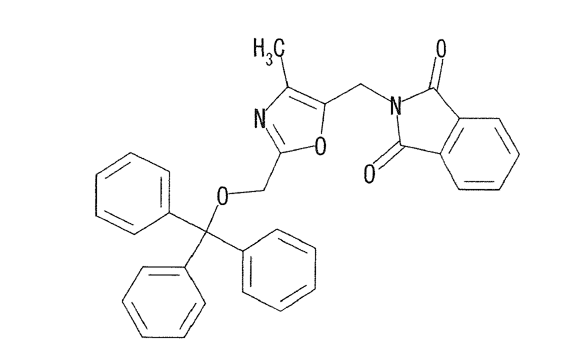

To a solution of 5-chloromethyl-4-methyl-2-trityloxymethyl-oxazole (753 mg) in N,N-dimethylformamide (5 mL) was added potassium phthalimide (350 mg). After stirring at 70°C for 6 hours and 30 minutes, water was added to the reaction mixture, and the mixture was extracted with a mixed solvent of ethyl acetate/tetrahydrofuran. The organic layer was successively washed with water and saturated brine, dried over anhydrous sodium sulfate, and then concentrated to obtain the title compound (942 mg) as a colorless oily matter.

### (Step 9)

### Production of 5-aminomethyl-4-methyl-2-trityloxymethyl-oxazole:

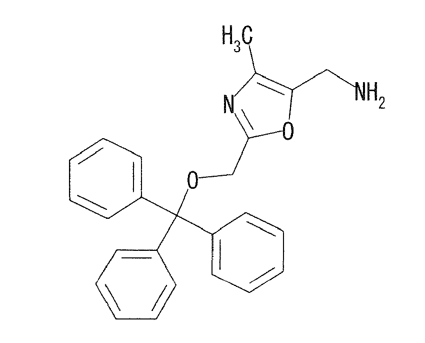

To a solution of 5-(1,3-dioxo-1,3-dihydro-isoindol-2-ylmethyl)-4-methyl-2-trityloxymethyl-oxazole (942 mg) in ethanol (20 mL) was added hydrazine monohydrate (0.3 mL). After stirring at 90°C for 5 hours, the precipitated insoluble matters were filtered off, and the filtrate was concentrated. Chloroform was added to the residue, the precipitated insoluble matters were filtered off, and the filtrate was concentrated to obtain the title compound (654 mg) as a yellow oily matter.

### Reference Example 43

### Production of 5-aminomethyl-4-methyl-2-phenoxymethyl-oxazole dihydrochloride:

### (Step 1)

### Production of 5-(tert-butyl-dimethyl-silanyloxymethyl)-4-methyl-2-phenoxymethyl-oxazole:

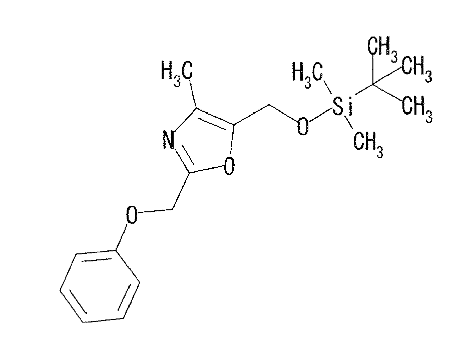

To a solution of 5-(tert-butyl-dimethyl-silanyloxymethyl)-2-hydroxymethyl-4-methyl-oxazole (1.098 g) produced in Step 4 of Reference Example 42 in chloroform (10 mL) was added triethylamine (0.65 mL) followed by addition of methanesulfonyl chloride (0.35 mL) with ice cooling. After stirring overnight at room temperature, water was added to the reaction mixture, the organic layer was separated, and the aqueous layer was extracted with chloroform. The combined organic layers were washed with saturated brine, dried over anhydrous sodium sulfate, and then concentrated to obtain a mixture (1.323 g) of 5-(tert-butyl-dimethyl-silanyloxymethyl)-2-methanesulfonyloxymethyl-4-methyl-oxazole and 5-(tert-butyl-dimethyl-silanyloxymethyl)-2-chloromethyl-4-methyl-oxazole as a yellow oily matter.

Subsequently, to a solution of the mixture (1.323 g) of 5-(tert-butyl-dimethyl-silanyloxymethyl)-2-methanesulfonyloxymethyl-4-methyl-oxazole and 5-(tert-butyl-dimethyl-silanyloxymethyl)-2-chloromethyl-4-methyl-oxazole in N,N-dimethylformamide (7 mL) were added phenol (425 mg) and potassium carbonate (663 mg). After stirring at 70°C for 4 hours, water was added to the reaction mixture, and the mixture was extracted with ethyl acetate. The organic layer was successively washed with water and saturated brine, dried over anhydrous sodium sulfate, and then concentrated to obtain the title compound (1.439 g) as a yellow oily matter.

### (Step 2)

### Production of 5-hydroxymethyl-4-methyl-2-phenoxymethyl-oxazole:

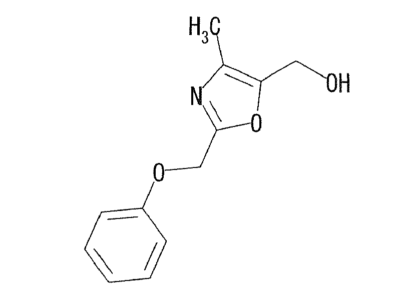

In the same manner as in Step 6 of Reference Example 42, the title compound (867 mg) was obtained from 5-(tert-butyl-dimethyl-silanyloxymethyl)-4-methyl-2-phenoxymethyl-oxazole (1.439 g) as a colorless oily matter.

### (Step 3)

### Production of 5-aminomethyl-4-methyl-2-phenoxymethyl-oxazole dihydrochloride:

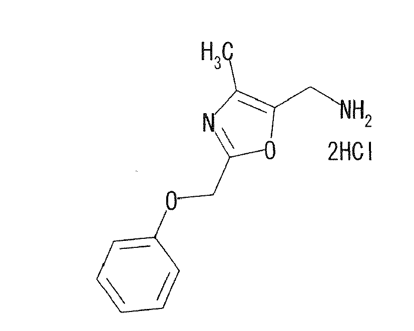

In the same manner as in Steps 4 and 5 of Reference Example 3, the title compound (1.045 g) was obtained from 5-hydroxymethyl-4-methyl-2-phenoxymethyl-oxazole (867 mg) as a white solid.

### Reference Example 44

### Production of 5-aminomethyl-2-methoxymethyl-4-methyl-oxazole dihydrochloride:

### (Step 1)

### Production of 5-hydroxymethyl-2-methoxymethyl-4-methyl-oxazole:

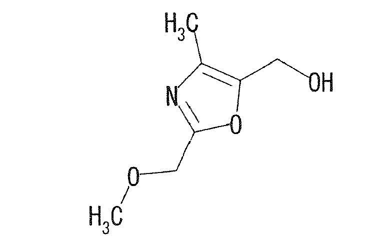

To a solution of 5-(tert-butyl-dimethyl-silanyloxymethyl)-2-hydroxymethyl-4-methyl-oxazole (1.105 g) produced in Step 4 of Reference Example 42 in N,N-dimethylformamide (8 mL) was added methyl iodide (0.54 mL) followed by addition of 60% sodium hydride (214 mg) with ice cooling. After stirring overnight at room temperature, water was added to the reaction mixture, and the mixture was extracted with ethyl acetate. The organic layer was successively washed with water and saturated brine, dried over anhydrous sodium sulfate, and then concentrated to obtain 5-(tert-butyl-dimethyl-silanyloxymethyl)-2-methoxymethyl-4-methyl-oxazole (1.222 g) as a yellow oily matter.

Subsequently, in the same manner as in Step 6 of Reference Example 42, the title compound (619 mg) was obtained from 5-(tert-butyl-dimethyl-silanyloxymethyl)-2-methoxymethyl-4-methyl-oxazole (1.222 g) as a colorless oily matter.

### (Step 2)

### Production of 5-aminomethyl-2-methoxymethyl-4-methyl-oxazole dihydrochloride:

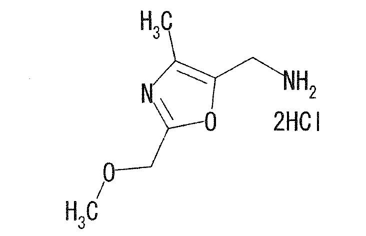

In the same manner as in Steps 4 and 5 of Reference Example 3, the title compound (851 mg) was obtained from 5-hydroxymethyl-2-methoxymethyl-4-methyl-oxazole (619 mg) as a yellow oily matter.

### Reference Example 45

### Production of 5-aminomethyl-4-methyl-2-(2,2,2-trifluoro-ethoxymethyl)-oxazole:

### (Step 1)

### production of 5-hydroxymethyl-4-methyl-2-(2,2,2-trifluoro-ethoxymethyl)-oxazole:

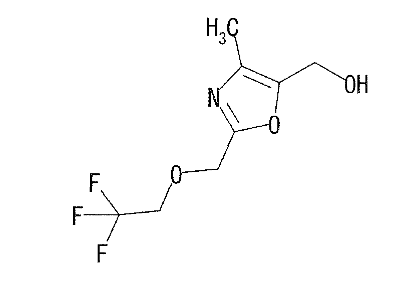

To a solution of 5-(tert-butyl-dimethyl-silanyloxymethyl)-2-hydroxymethyl-4-methyl-oxazole (1.127 g) produced in Step 4 of Reference Example 42 in chloroform (10 mL) was added triethylamine (0.668 mL) followed by methanesulfonyl chloride (0.372 mL) with ice cooling. After stirring overnight at room temperature, water was added to the reaction mixture, the organic layer was separated, and the aqueous layer was extracted with chloroform. The combined organic layers were washed with saturated brine, dried over anhydrous sodium sulfate, and then concentrated to obtain 5-(tert-butyl-dimethyl-silanyloxymethyl)-2-chloromethyl-4-methyl-oxazole as a yellow oily matter.

Subsequently, to a solution of 5-(tert-butyldimethyl-silanyloxymethyl)-2-chloromethyl-4-methyl-oxazole in N,N-dimethylformamide (5 mL) was added 2,2,2-trifluoroethanol (0.4 mL) followed by addition of 60% sodium hydride (202 mg) with ice cooling. After stirring overnight at room temperature, water was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 8:1 → 3:1) to obtain 5-(tert-butyl-dimethyl-silanyloxymethyl)-4-methyl-2-(2,2,2-trifluoro-ethoxymethyl)-oxazole (671 mg) as a colorless oily matter.

Subsequently, in the same manner as in Step 6 of Reference Example 42, the title compound (432 mg) was obtained from 5-(tert-butyl-dimethyl-silanyloxymethyl)-4-methyl-2-(2,2,2-trifluoro-ethoxymethyl)-oxazole (671 mg) as a colorless oily matter.

### (Step 2)

### synthesis of 5-aminomethyl-4-methyl-2-(2,2,2-trifluoro-ethoxymethyl)-oxazole:

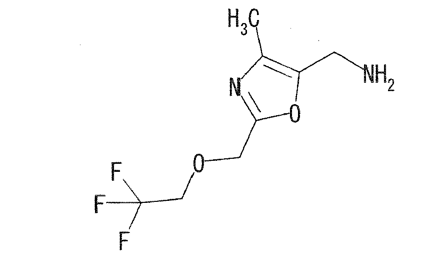

In the same manner as in Steps 7 to 9 of Reference Example 42, the title compound (251 mg) was obtained from 5-hydroxymethyl-4-methyl-2-(2,2,2-trifluoro-ethoxymethyl)-oxazole (649 mg) as a yellow oily matter.

### Reference Example 46

### Production of 5-aminomethyl-2-benzyloxymethyl-4-methyl-oxazole:

### (Step 1)

### Production of 2-benzyloxymethyl-5-hydroxymethyl-4-methyl-oxazole:

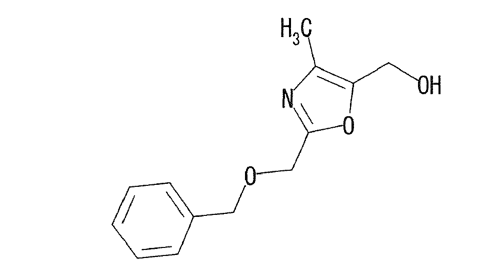

In the same manner as in Step 1 of Reference Example 44, the title compound (649 mg) was obtained from 5-(tert-butyl-dimethyl-silanyloxymethyl)-2-hydroxymethyl-4-methyl-oxazole (801 mg) produced in Step 4 of Reference Example 42 and benzyl bromide (0.407 mL) as a colorless oily matter.

### (Step 2)

### Production of 5-aminomethyl-2-benzyloxymethyl-4-methyl-oxazole:

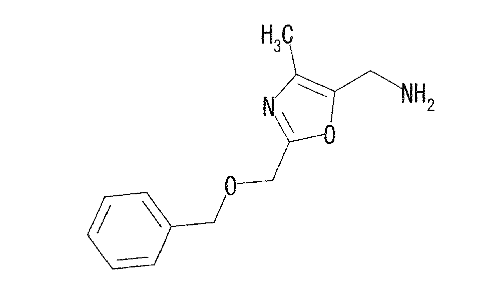

In the same manner as in Steps 7 to 9 of Reference Example 42, the title compound (669 mg) was obtained from 2-benzyloxymethyl-5-hydroxymethyl-4-methyl-oxazole (649 mg) as a yellow oily matter.

### Reference Example 47

### Production of 5-aminomethyl-2-ethoxycarbonylmethoxymethyl-4-methyl-oxazole dihydrochloride:

### (Step 1)

### Production of 2-ethoxycarbonylmethoxymethyl-5-hydroxymethyl-4-methyl-oxazole:

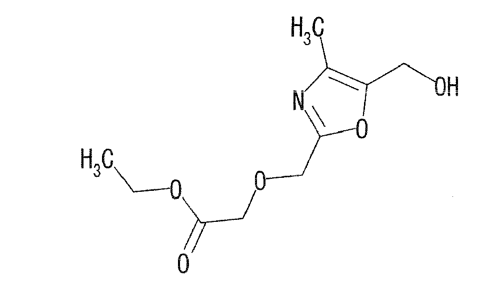

In the same manner as in Step 1 of Reference Example 44, the title compound (504 mg) was obtained from 5-(tert-butyl-dimethyl-silanyloxymethyl)-2-hydroxymethyl-4-methyl-oxazole (1.285 g) produced in Step 4 of Reference Example 42 and ethyl chloroacetate (0.558 mL) as a colorless oily matter.

### (Step 2)

### Production of 5-aminomethyl-2-ethoxycarbonylmethoxymethyl-4-methyl-oxazole dihydrochloride:

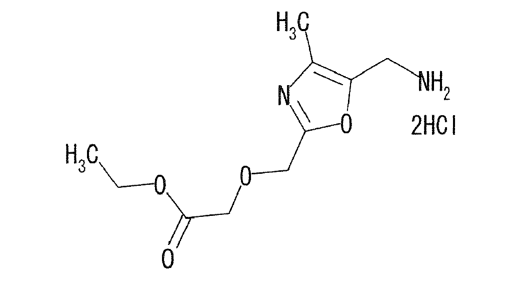

In the same manner as in Steps 4 and 5 of Reference Example 3, the title compound (535 mg) was obtained from 2-ethoxycarbonylmethoxymethyl-5-hydroxymethyl-4-methyl-oxazole (504 mg) as a colorless oily matter.

### Reference Example 48

### Production of 5-aminomethyl-4-methyl-2-phenylsulfanyl-oxazole:

### (Step 1)

### Production of 5-(tert-butyl-dimethyl-silanyloxymethyl)-4-methyl-2-phenylsulfanyl-oxazole:

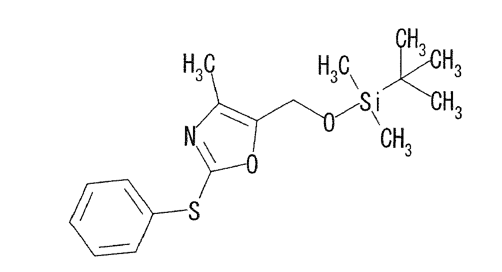

To a solution of 5-(tert-butyl-dimethyl-silanyloxymethyl)-4-methyl-oxazole (3.020 g) produced in Step 3 of Reference Example 42 in tetrahydrofuran (30 mL), 1.6 M *n*-butyllithium/hexane solution (9.1 mL) was added dropwise at -78°C. After stirring for 30 minutes, diphenyl disulfide (3.519 g) was added to the reaction mixture, and the mixture was heated to room temperature. After stirring for 4 hours and 30 minutes, saturated aqueous ammonium chloride was added to the reaction mixture, and the mixture was extracted with ethyl acetate. The organic layer was washed with saturated brine, dried over anhydrous sodium sulfate, and then concentrated. The residue was purified by silica gel column chromatography (hexane/ethyl acetate, 20:1 → 10:1) to obtain the title compound (3.932 g) as a yellow oily matter.

### (Step 2)

### Production of 5-hydroxymethyl-4-methyl-2-phenylsulfanyl-oxazole:

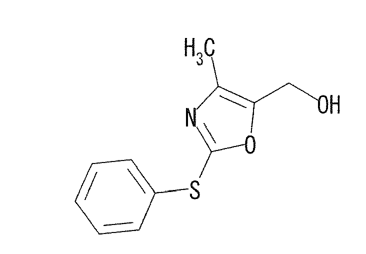

In the same manner as in Step 6 of Reference Example 42, the title compound (885 mg) was obtained from 5-(tert-butyl-dimethyl-silanyloxymethyl)-4-methyl-2-phenylsulfanyl-oxazole (1.323 g) as a yellow oily matter.

### (Step 3)

### Production of 5-aminomethyl-4-methyl-2-phenylsulfanyl-oxazole:

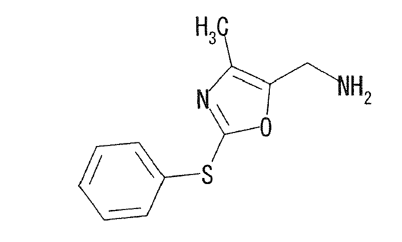

To a solution of 5-hydroxymethyl-4-methyl-2-phenylsulfanyl-oxazole (855 mg) in chloroform (10 mL) was added triethylamine (0.6 mL) followed by addition of methanesulfonyl chloride (0.33 mL) with ice cooling. After stirring overnight at room temperature, water was added to the reaction mixture, the organic layer was separated, and the aqueous layer was extracted with chloroform. The combined organic layers were washed with saturated brine, dried over anhydrous sodium sulfate, and then concentrated to obtain 5-chloromethyl-4-methyl-2-phenylsulfanyl-oxazole as a yellow oily matter.

Subsequently, to a solution of 5-chloromethyl-4-methyl-2-phenylsulfanyl-oxazole in dioxane (20 mL) was added 28% aqueous ammonia (20 mL). After stirring overnight at room temperature, the reaction mixture was concentrated. 8 N Aqueous potassium hydroxide was added to the residue, and the mixture was extracted with chloroform. The organic layer was washed with saturated brine, dried over anhydrous sodium sulfate, and then concentrated to obtain the title compound (410 mg) as a brown oily matter.

### Reference Example 49

### Production of methyl 5-aminomethyl-4-methyl-oxazole-2-carboxylate:

### (Step 1)

### Production of methyl 5-(tert-butyl-dimethyl-silanyloxymethyl)-4-methyl-oxazole-2-carboxylate:

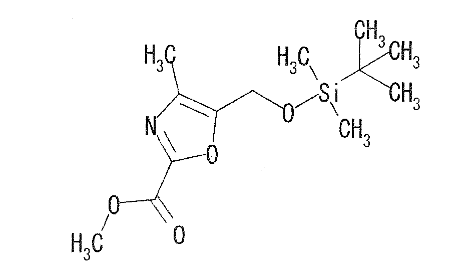

To a solution of 5-(tert-butyl-dimethyl-silanyloxymethyl)-4-methyl-oxazole (1.218 g) produced in Step 3 of Reference Example 42 in tetrahydrofuran (7.5 mL), 1.59 M *n*-butyllithium/hexane solution (5.55 mL) was added dropwise at -78°C. After stirring for 1 hour, dry ice (2 g) was added to the reaction mixture to heat the mixture to room temperature. After 1 hour, methyl iodide (0.5 mL) was added to the reaction mixture followed by further addition of acetic acid (0.3 mL) after 1 hour. After stirring for 1 hour, ethyl acetate and ice water were added to the reaction mixture. The organic layer was washed with saturated brine, dried over anhydrous sodium sulfate, and then concentrated. The residue was purified by silica gel column chromatography (hexane/ethyl acetate, 5:1) to obtain the title compound (1.20 g) as a colorless oily matter.

### (Step 2)

### Production of methyl 5-chloromethyl-4-methyl-oxazole-2-carboxylate:

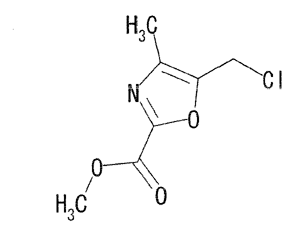

In the same manner as in Steps 6 and 7 of Reference Example 42, the title compound (1.20 g) was obtained from methyl 5-(tert-butyl-dimethyl-silanyloxymethyl)-4-methyl-oxazole-2-carboxylate as a light yellow oily matter.

### (Step 3)

### Production of methyl 5-aminomethyl-4-methyl-oxazole-2-carboxylate:

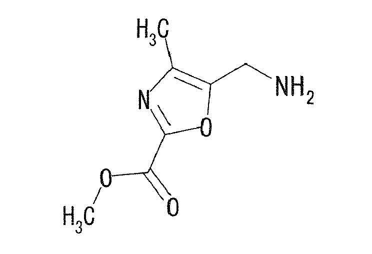

To a solution of methyl 5-chloromethyl-4-methyl-oxazole-2-carboxylate (1.20 g) in N,N-dimethylformamide (12 mL) was added sodium azide (0.56 g). After stirring at room temperature for 2 hours, ethyl acetate and water were added to the reaction mixture, and the organic layer was separated. The organic layer was washed 3 times with water, dried over anhydrous sodium sulfate, and then concentrated. The residue was dissolved in ethanol (20 mL) followed by addition of platinum oxide (100 mg), and the mixture was stirred at room temperature for 2 hours under hydrogen pressure (3.5 kgf/cm²). The catalyst was filtered off, and the filtrate was concentrated to obtain the title compound (614 mg) as a light yellow solid.

### Reference Example 50

### Production of ethyl 2-aminomethyl-4-methyl-oxazole-5-carboxylate:

### (Step 1)

### Production of ethyl 2-(benzyloxycarbonylamino-methyl)-4-methyl-oxazole-5-carboxylate:

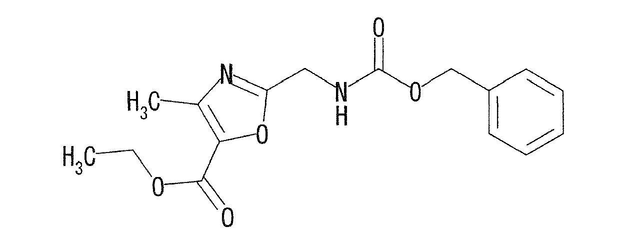

To a suspension of N-benzyloxycarbonyl-glycine (6.908 g) in ethyl acetate (100 mL) were added 2-chloroethyl acetoacetate (4.8 mL) and triethylamine (13.7 mL) with ice cooling. After stirring overnight at 70°C, 1 N hydrochloric acid (65 mL) was added to the reaction mixture. The organic layer was separated, washed with saturated brine, dried over anhydrous sodium sulfate, and then concentrated. The residue was purified by silica gel column chromatography (hexane/ethyl acetate, 4:1) to obtain ethyl 2-(2-benzyloxycarbonylamino-acetoxy)-3-oxo-butyrate (5.809 g) as a yellow oily matter.

To a solution of ethyl 2-(2-benzyloxycarbonylamino-acetoxy)-3-oxo-butyrate (4.724 g) in acetic acid (20 mL) was added ammonium acetate (5.519 g). After stirring at 140°C for 3 hours, water was added to the reaction mixture, and the mixture was extracted with ethyl acetate. The organic layer was successively washed with water and saturated brine, dried over anhydrous sodium sulfate, and then concentrated. The residue was purified by silica gel column chromatography (hexane/ethyl acetate, 4:1 → 2:1) to obtain the title compound (1.806 g) as a yellow oily matter.

### (Step 2)

### Production of ethyl 2-aminomethyl-4-methyl-oxazole-5-carboxylate:

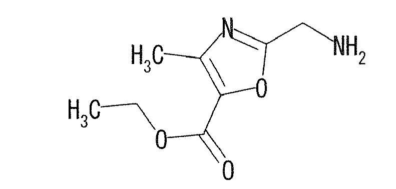

To a solution of ethyl 2-(benzyloxycarbonylaminomethyl)-4-methyl-oxazole-5-carboxylate (453 mg) in ethanol (10 mL) was added 10% palladium-carbon (75 mg), and the mixture was stirred at room temperature for 5 hours and 30 minutes under a hydrogen atmosphere. The catalyst was filtered off, and the filtrate was concentrated to obtain the title compound (252 mg) as a yellow solid.

### Reference Example 51:

### Production of 2-aminomethyl-5-(4-chloro-phenyl)-[1,3,4]oxadiazole trifluoroacetate:

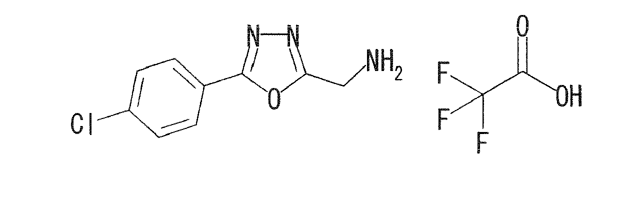

Based on a reference (Tetrahedron Lett., 47, 2006, 4827-4830), a crude product (4.89 g) of tert-butyl [5-(4-chloro-phenyl)-[1,3,4]oxadiazol-2-ylmethyl]-carbamate was obtained from N-(tert-butoxycarbonyl)-glycine (2.01 g) and 4-chloro-benzoic acid hydrazide (0.99 g) as a light yellow solid. To this compound was added trifluoroacetic acid (10 mL), and the mixture was stirred at room temperature for 1 hour and concentrated. Ethyl acetate was added to the residue, the mixture was stirred at room temperature, and the precipitated solid was collected by filtration to obtain the title compound (1.41 g) as a white solid.

### Reference Example 52

### Production of 2-aminomethyl-5-(2-methyl-phenyl)-[1,3,4]oxadiazole trifluoroacetate:

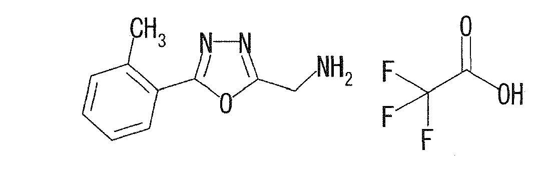

In the same manner as in Reference Example 51, the title compound (0.74 g) was obtained from N-(tert-butoxycarbonyl)-glycine and 2-methyl-benzoic acid hydrazide as a white solid.

### Reference Example 53

### Production of 2-aminomethyl-5-(4-dimethylamino-phenyl)-[1,3,4]oxadiazole trifluoroacetate:

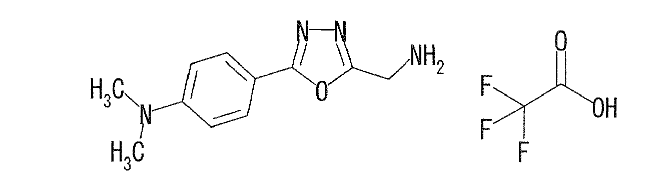

In the same manner as in Reference Example 51, the title compound (1.68 g) was obtained from N-(tert-butoxycarbonyl)-glycine and 4-dimethylamino-benzoic acid hydrazide as a yellow solid.

### Reference Example 54

### Production of 2-aminomethyl-5-(4-trifluoromethyl-phenyl)-[1,3,4]oxadiazole trifluoroacetate:

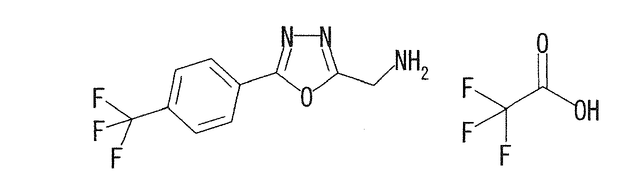

In the same manner as in Reference Example 51, the title compound (1.66 g) was obtained from N-(tert-butoxycarbonyl)-glycine and 4-trifluoromethyl-benzoic acid hydrazide as a light yellow solid.

### Reference Example 55

### Production of 2-aminomethyl-5-(3-nitro-phenyl)-[1,3,4]oxadiazole dihydrochloride:

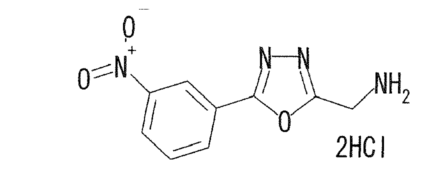

In the same manner as in Reference Example 51, a crude product of 2-aminomethyl-5-(3-nitro-phenol)-[1,3,4]oxadiazole trifluoroacetate was obtained from N-(tert-butoxycarbonyl)-glycine and 3-nitrobenzoic acid hydrazide. To this compound was added 1 N sodium hydroxide aqueous solution to make it basic, and the mixture was extracted several times with ethyl acetate. The combined organic layers were washed with saturated brine, dried over anhydrous sodium sulfate, and then concentrated. The residue (1.0 g) was dissolved in ethyl acetate (12 mL), and 4 N hydrochloric acid/ethyl acetate (3.0 mL) was added dropwise. After stirring at room temperature for 1 hour, the precipitated solid was collected by filtration to obtain the title compound (1.08 g) as a brown solid.

### Reference Example 56

### Production of 2-aminomethyl-5-(3-hydroxy-phenyl)-[1,3,4]oxadiazole trifluoroacetate:

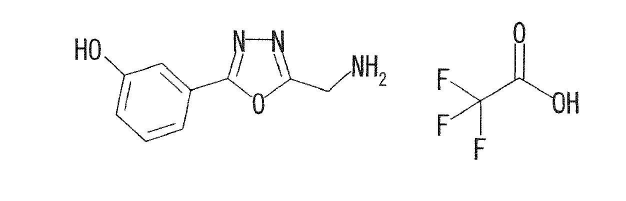

In the same manner as in Reference Example 51, the title compound (1.05 g) was obtained from N-(tert-butoxycarbonyl)-glycine and 3-hydroxy-benzoic acid hydrazide as a light brown solid.

### Reference Example 57

### Production of 2-aminomethyl-5-(2-hydroxy-phenyl)-[1,3,4]oxadiazole trifluoroacetate:

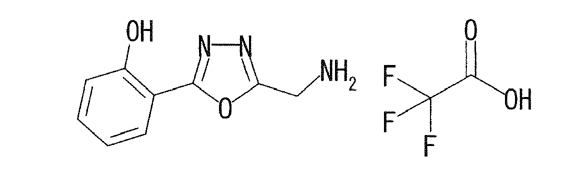

In the same manner as in Reference Example 51, the title compound (998 mg) was obtained from N-(tert-butoxycarbonyl)-glycine and salicyl hydrazide as a light yellow solid.

### Reference Example 58

### Production of 2-aminomethyl-5-(3-chloro-phenyl)-[1,3,4]oxadiazole trifluoroacetate:

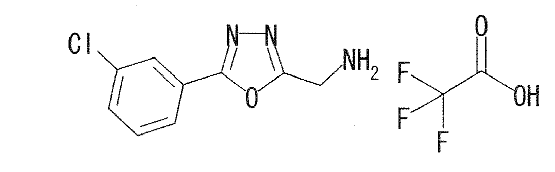

In the same manner as in Reference Example 51, the title compound (1.23 g) was obtained from N-(tert-butoxycarbonyl)-glycine and 3-chloro-benzoic acid hydrazide as a white solid.

### Reference Example 59

### Production of 2-aminomethyl-5-(3-methoxy-phenyl)-[1,3,4]oxadiazole trifluoroacetate:

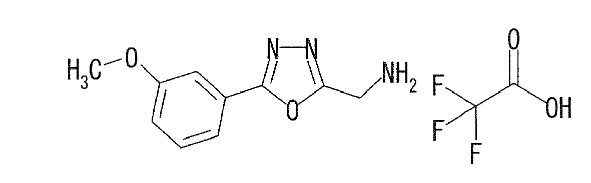

In the same manner as in Reference Example 51, the title compound (1.04 g) was obtained from N-(tert-butoxycarbonyl)-glycine and 3-methoxy-benzoic acid hydrazide as a white solid.

### Reference Example 60

### Production of 2-aminomethyl-5-(2-methoxy-phenyl)-[1,3,4]oxadiazole trifluoroacetate:

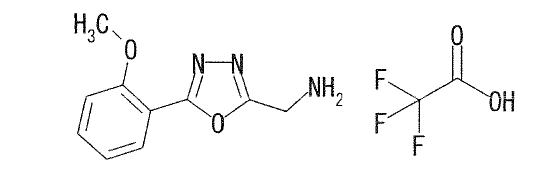

In the same manner as in Reference Example 51, the title compound (1.27 g) was obtained from N-(tert-butoxycarbonyl)-glycine and 2-methoxy-benzoic acid hydrazide as a light yellow brown solid.

### Reference Example 61

### Production of 2-aminomethyl-5-ethoxycarbonylmethyl-[1,3,4]oxadiazole:

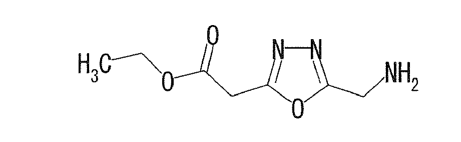

In the same manner as in Reference Example 51, ethyl (5-aminomethyl-[1,3,4]oxadiazol-2-yl)-acetate trifluoroacetate was produced from N-(tert-butoxycarbonyl)-glycine (1.00 g) and ethyl hydrazinocarbonyl/acetate (0.85 g). To this compound was added 4 N sodium hydroxide aqueous solution, and the mixture was extracted with ethyl acetate. The organic layer was dried over anhydrous sodium sulfate and concentrated, and the resulting residue was purified by silica gel chromatography (chloroform/methanol, 95:5) to obtain the title compound (0.41 g) as an orange color oily matter.

### Reference Example 62

### Production of 4-aminomethyl-1-(4-chloro-benzyl)-5-methyl-1H-[1,2,3]triazole dihydrochloride:

### (Step 1)

### Production of ethyl 1-(4-chloro-benzyl)-5-methyl-1H-[1,2,3]triazole-4-carboxylate:

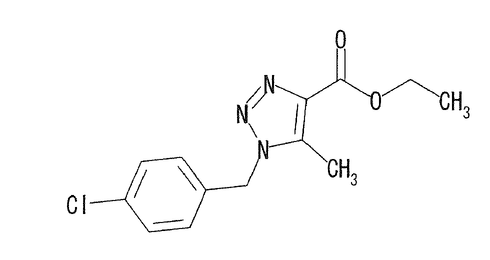

Based on a reference (J. Heterocycl. Chem., 2, 301, 1991), the title compound (9.817 g) was obtained from 4-chlorobenzyl chloride (6.675 g) as colorless crystals.

### (Step 2)

### Production of 4-aminomethyl-1-(4-chloro-benzyl)-5-methyl-1H-[1,2,3]triazole dihydrochloride (00):

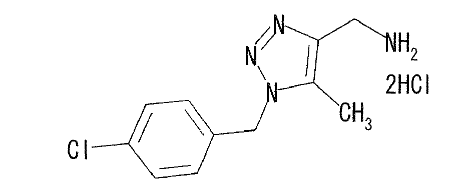

In the same manner as in Steps 2 to 5 of Reference Example 3, the title compound (1.013 g) was obtained from ethyl 1-(4-chloro-benzyl)-5-methyl-1H-[1,2,3]triazole-4-carboxylate as a white solid.

### Reference Example 63

### Production of 4-aminomethyl-5-methyl-1-(4-methyl-benzyl)-1H-[1,2,3]triazole dihydrochloride:

### (Step 1)

### Production of ethyl 5-methyl-1-(4-methyl-benzyl)-1H-[1,2.3]triazole-4-carboxylate:

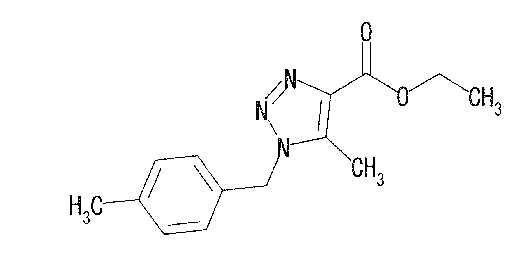

Based on a reference (J. Heterocycl. Chem., 2, 301, 1991), the title compound (3.455 g) was obtained from α-chloro-p-xylene (3.016 g) as a yellow solid.

### (Step 2)

### Production of 4-aminomethyl-5-methyl-1-(4-methyl-benzyl)-1H-[1,2,3]triazole dihydrochloride:

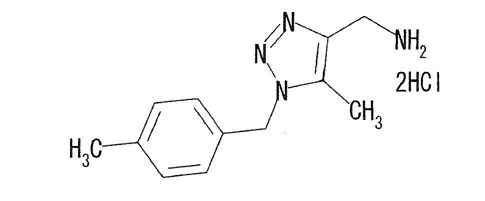

In the same manner as in Steps 2 to 5 of Reference Example 3, the title compound (542 mg) was obtained from ethyl 5-methyl-1-(4-methyl-benzyl)-1H-[1,2,3]triazole-4-carboxylate as a white solid.

### Reference Example 64

### Production of 4-aminomethyl-5-methyl-1-(2-methyl-thiazol-4-ylmethyl)-1H-[1,2,3]triazole dihydrochloride:

### (Step 1)

### Production of 4-chloromethyl-2-methyl-thiazole:

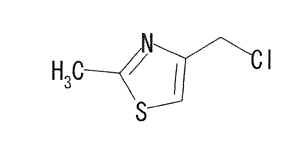

To a solution of 1,3-dichloro-2-propanone (7.561 g) in acetone (70 mL) was added thioacetamide (4.473 g). After stirring overnight at room temperature, the precipitated solid was collected by filtration to obtain 2-(3-chloro-2-oxo-propyl)-isothiourea (11.335 g) as a white solid.

Subsequently, to a solution of 2-(3-chloro-2-oxopropyl)-isothiourea (11.335 g) in methanol (110 mL) was added zinc chloride (2.298 g). After stirring at 80°C for 9 hours, the reaction mixture was concentrated, and ethyl acetate (100 mL) and an aqueous solution (100 mL) of potassium carbonate (10.032 g) were added to the residue. The separated organic layer was successively washed with water and saturated brine, dried over anhydrous sodium sulfate, and then concentrated to obtain the title compound (8.133 g) as a brown oily matter.

### (Step 2)

### Production of ethyl 5-methyl-1-(2-methyl-thiazol-4-ylmethyl)-1H-[1,2,3]triazole-4-carboxylate:

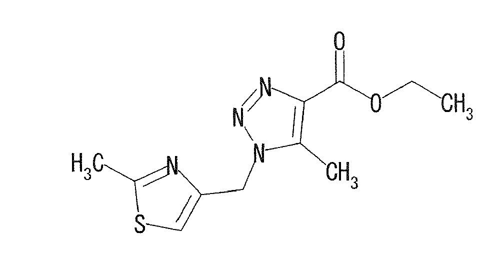

Based on a reference (J. Heterocycl. Chem., 2, 301, 1991), the title compound (4.913 g) was obtained from 4-chloromethyl-2-methyl-thiazole (3.515 g) as colorless crystals.

### (Step 3)

### Production of 4-aminomethyl-5-methyl-1-(2-methyl-thiazol-4-ylmethyl)-1H-[1,2,3]triazole dihydrochloride:

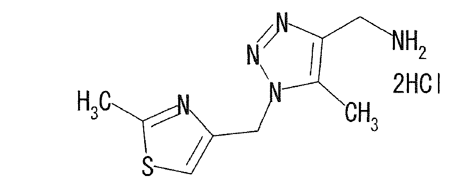

In the same manner as in Steps 2 to 5 of Reference Example 3, the title compound (387 mg) was obtained from ethyl 5-methyl-1-(2-methyl-thiazol-4-ylmethyl)-1H-[1,2,3]triazole-4-carboxylate as a yellow solid.

### Reference Example 65

### Production of 4-aminomethyl-3,5-dimethyl-1-(3-methylphenyl)-1H-pyrazole hydrochloride:

### (Step 1)

### Production of ethyl 3-acetyl-4-oxo-valerate:

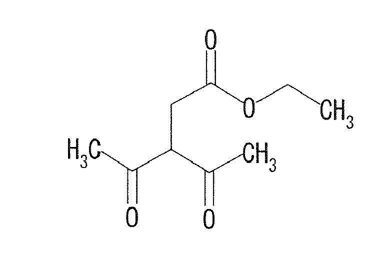

To a solution of 2,4-pentanedione (6.00 g) in chloroform (30 mL) was added ethyl bromoacetate (10.0 g) followed by addition of potassium carbonate (7.50 g) and cesium carbonate (2.00 g). After heating at 65°C for 27 hours, the reaction mixture was cooled, and the insoluble matters were filtered off. The filtrate was concentrated and purified by silica gel column chromatography (hexane/chloroform, 4:1 → 2:1 → 1:1) to obtain the title compound (8.89 g) as a yellow oily matter.

### (Step 2)

### Production of [3,5-dimethyl-1-(3-methyl-phenyl)-1H-pyrazol-4-yl]-acetic acid:

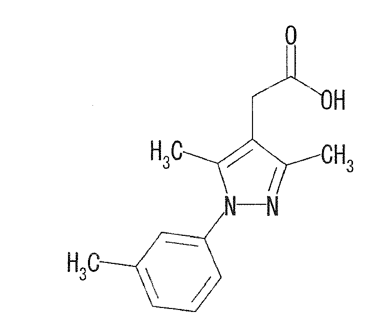

To a solution of ethyl 3-acetyl-4-oxo-valerate (1.53 g) in ethanol (6 mL) was added m-tolyl hydrazine (1.00 g). After stirring at room temperature for 1 hour and 30 minutes, 4 N sodium hydroxide aqueous solution was added to the reaction mixture. After stirring at room temperature for 1 hour, the reaction mixture was concentrated, and most of the organic solvent was distilled off. The residue was diluted with water and washed 3 times with diethyl ether followed by addition of 6 N hydrochloric acid (1.4 mL). The liberated oily matter was extracted with ethyl acetate and washed with water, and the organic layer was concentrated to obtain the title compound (1.31 g) as a brown oily matter.

### (Step 3)

### Production of 4-tert-butoxycarbonylaminomethyl-3,5-dimethyl-1-(3-methyl-phenyl)-1H-pyrazole:

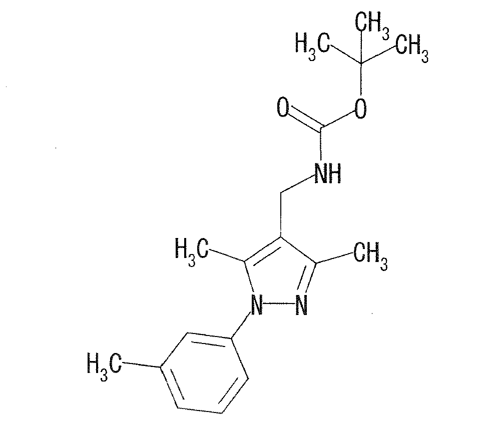

To a solution of [3,5-dimethyl-1-(3-methyl-phenyl)-1H-pyrazol-4-yl]-acetic acid (1.31 g) in toluene (15 mL) was added N-methylmorpholine (0.620 mL), then diphenyl phosphoryl azide (1.20 mL) was added dropwise with ice cooling over 10 minutes, the mixture was removed from the ice bath, and the reaction mixture was heated to room temperature. After 1 hour, the reaction mixture was added dropwise to tert-butyl alcohol (10 mL) at 110°C. After 5 hours, the reaction mixture was cooled to room temperature and concentrated. The residue was diluted with toluene and successively washed with water and aqueous sodium hydrogencarbonate, and the organic layer was purified by silica gel column chromatography (chloroform → chloroform/ethyl acetate, 20:1) to obtain the title compound (238 mg) as a yellow oily matter.

### (Step 4)

### Production of 4-aminomethyl-3,5-dimethyl-1-(3-methylphenyl)-1H-pyrazole hydrochloride:

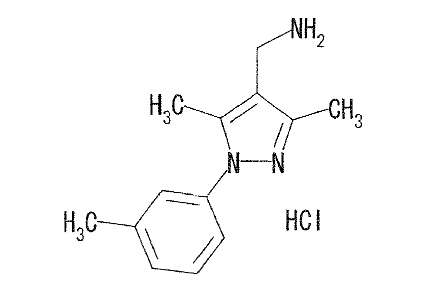

To a solution of 4-tert-butoxycarbonyl-aminomethyl-3,5-dimethyl-2-(3-methyl-phenyl)-1H-pyrazole (228 mg) in ethanol (1 mL) was added 6 N hydrochloric acid (0.500 mL). After 8 hours, the reaction mixture was concentrated, and the resulting crude product was crystallized from acetonitrile to obtain the title compound (128 mg) as a light brown solid.

### Reference Example 66

### Production of 4-aminomethyl-5-cyclopropyl-l-(4-fluorophenyl)-1H-pyrazole dihydrochloride:

### (Step 1)

### Production of methyl 5-cyclopropyl-l-(4-fluoro-phenyl)-1H-pyrazole-4-carboxylate:

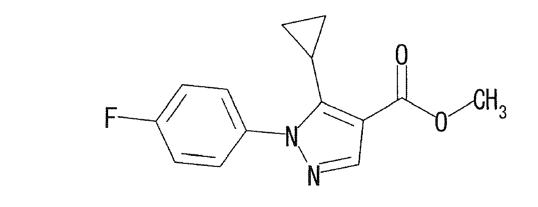

To a solution of methyl 2-cyclopropanecarbonyl-3-dimethylamino-acrylate (3.079 g) and 4-fluorophenyl hydrazine (2.744 g) in ethanol (30 mL) was added triethylamine (2.75 mL). After stirring overnight at 95°C, the reaction mixture was concentrated. The residue was purified by silica gel column chromatography (hexane/ethyl acetate, 8:1 → 3:1) to obtain the title compound (4.728 g) as a brown oily matter.

### (Step 2)

### Production of 4-aminomethyl-5-cyclopropyl-1-(4-fluorophenyl)-1H-pyrazole dihydrochloride:

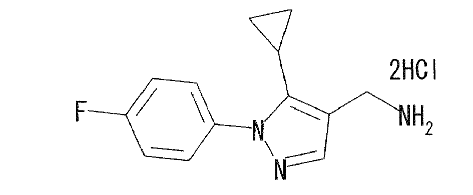

In the same manner as in Steps 2 to 5 of Reference Example 3, the title compound (542 mg) was obtained from ethyl 5-cyclopropyl-1-(4-fluoro-phenyl)-1H-pyrazole-4-carboxylate as a yellow solid.

### Reference Example 67

### Production of 5-aminomethyl-4-cyclopropyl-2-methylpyrimidine hydrochloride:

### (Step 1)

### Production of 4-cyclopropyl-5-hydroxymethyl-2-methylpyrimidine:

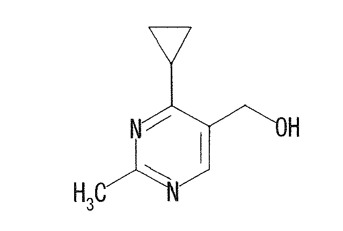

To 20% sodium ethoxide/ethanol solution (3.476 g) were added ethanol (10 mL) and acetamidine hydrochloride (1.01 g) followed by a solution of methyl 2-cyclopropane carbonyl-3-dimethylamino-acrylate (2.2 g) in ethanol (5 mL). After stirring at 100°C for 4 hours, the reaction mixture was concentrated, water was added to the residue, and the aqueous layer was extracted with ethyl acetate. The organic layer was washed with saturated brine, dried over anhydrous sodium sulfate, and then concentrated. The residue was purified by silica gel column chromatography (hexane/ethyl acetate, 6:1 → 4:1) to obtain a mixture (1.897 g) of methyl 4-cyclopropyl-2-methyl-.pyrimidine-5-carboxylate and ethyl 4-cyclopropyl-2-methyl-pyrimidine-5-carboxylate as a yellow oily matter.

Subsequently, to a suspension of lithium aluminum hydride (234 mg) in tetrahydrofuran (5 mL), a solution of the mixture (896 mg) of methyl 4-cyclopropyl-2-methylpyrimidine-5-carboxylate and ethyl 4-cyclopropyl-2-methyl-pyrimidine-5-carboxylate in tetrahydrofuran (3 mL) was added dropwise with ice cooling. After stirring for 1 hour and 30 minutes, water (0.25 mL), 15% sodium hydroxide aqueous solution (0.25 mL), and water (0.75 mL) were successively added to the reaction mixture. After stirring at room temperature for 45 minutes, the organic layer was separated, dried over anhydrous sodium sulfate, and then concentrated. The residue was purified by silica gel column chromatography (hexane/ethyl acetate, 2:1 → 0:1) to obtain the title compound (202 mg) as a yellow oily matter.

### (Step 2)

### Production of 5-aminomethyl-4-cyclopropyl-2-methylpyrimidine hydrochloride:

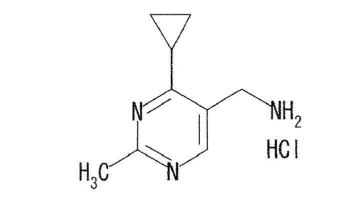

In the same manner as in Steps 4 and 5 of Reference Example 3, the title compound (240 mg) was obtained from 4-cyclopropyl-5-hydroxymethyl-2-methyl-pyrimidine (202 mg) as a light yellow solid.

### Reference Example 68

### Production of 5-aminomethyl-4-(4-methoxyphenyl)-[1,2,3]thiadiazole:

### (Step 1)

### Production of ethyl 3-(4-methoxy-phenyl)-3-(4-toluenesulfonyl-hydrazono)-propionate:

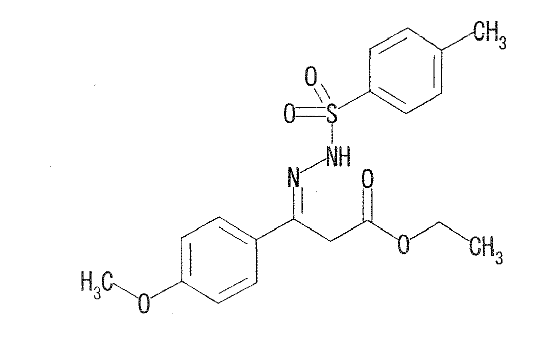

To a solution of ethyl p-methoxybenzoylacetate (2.00 g) in toluene (20 mL) was added p-toluenesulfonyl hydrazide (1.68 g). After stirring at 100°C for 3 hours, the reaction mixture was cooled to room temperature and washed with water. The organic layer was purified by silica gel column chromatography (hexane/chloroform, 1:1 → 1:2) to obtain the title compound (2.55 g) as a yellow solid.

### (Step 2)

### Production of ethyl 4-(4-methoxyphenyl)-[1,2,3]thiadiazole-5-carboxylate:

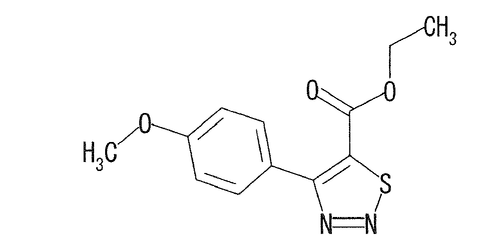

To a solution of ethyl 3-(4-methoxy-phenyl)-3-(4-toluenesulfonyl-hydrazono)-propionate (600 mg) in chloroform (3 mL), thionyl chloride (1.0 mL) was added dropwise. After stirring for 3 hours at room temperature, the reaction mixture was concentrated. Ethyl acetate was added to the residue, the mixture was successively washed with water, saturated sodium hydrogencarbonate, and water, and morpholine (0.500 mL) was added to the organic layer. After stirring overnight at room temperature, the reaction mixture was washed with water and concentrated. The residue was purified by silica gel column chromatography (chloroform) to obtain the title compound (284 mg) as a light yellow solid.

### (Step 3)

### Production of 5-aminomethyl-4-(4-methoxyphenyl)-[1,2,3)thiadiazole:

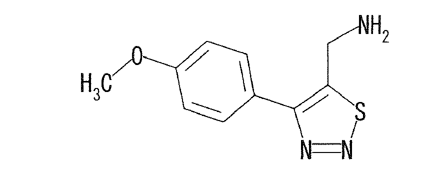

In the same manner as in Steps 2 to 5 of Reference Example 19, the title compound (38 mg) was obtained from ethyl 4-(4-methoxyphenyl)-[1,2,3]thiadiazole-5-carboxylate as a white solid.

### Reference Example 69

### Production of 4-aminomethyl-5-methyl-3-(3-methyl-[1,2,4]oxadiazol-5-yl)-isoxazole:

### (Step 1)

### Production of 4-tert-butyl 3-ethyl 5-methyl-isoxazole-3,4-dicarboxylate:

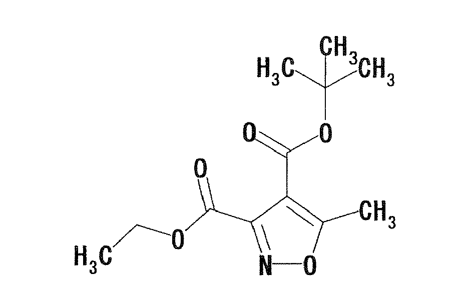

To a solution of tert-butyl acetoacetate (6.00 mL) and potassium tert-butoxide (4.10 g) in tetrahydrofuran (60 mL), a solution of ethyl 2-chloro-2-hydroxyiminoacetate (5.00 g) in tetrahydrofuran (25 mL) was added dropwise at 60°C. After stirring at 60°C for 5 hours, the reaction mixture was concentrated, and heptane was added to the residue. The precipitated insoluble matters were removed, and the filtrate was washed with water and concentrated. Tetrahydrofuran (20 mL), heptane (20 mL), and hydrazine monohydrate (0.6 mL) were added to the resulting residue. After stirring at room temperature for 10 minutes, the reaction mixture was concentrated, and hexane was added to the residue. The organic layer was successively washed with water and saturated brine, dried over anhydrous sodium sulfate, and then concentrated. The resulting residue was purified by silica gel column chromatography (hexane/ethyl acetate, 2:1 → 1:1) to obtain the title compound (4.886 g) as a yellow oily matter.

### (Step 2)

### Production of 4-tert-butyl 5-methyl-isoxazole-3,4-dicarboxylate:

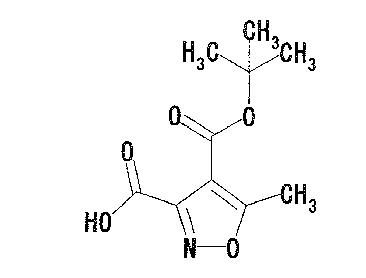

In the same manner as in Step 2 of Reference Example 3, the title compound (0.93 g) was obtained from 4-tert-butyl 3-ethyl 5-methyl-isoxazole-3,4-dicarboxylate (1.20 g) as a yellow oily matter.

### (Step 3)

### Production of tert-butyl 5-methyl-3-(3-methyl-[1,2,4]oxadiazol-5-yl)-isoxazole-4-carboxylate:

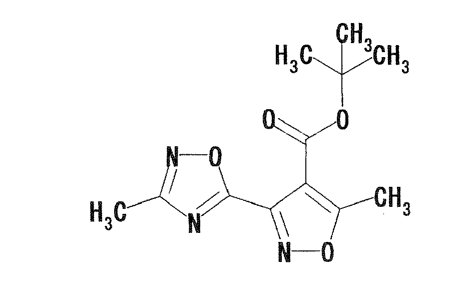

To a solution of 4-tert-butyl 5-methyl-isoxazole-3,4-dicarboxylate (920 mg) in tetrahydrofuran (7 mL) was added CDI (700 mg). After stirring at 60°C for 30 minutes, acetamide oxime (600 mg) was added. After stirring at 60°C for 1 hour and 30 minutes, the reaction mixture was concentrated. Ethyl acetate was added to the residue, and the mixture was washed with water and then concentrated. Dioxane (5 mL) and acetamide oxime (500 mg) was added to the residue. After stirring at 100°C for 2 hours, the reaction mixture was concentrated. Ethyl acetate was added to the residue, and the organic layer was washed with water, dried over anhydrous sodium sulfate, and concentrated to obtain 4-tert-butyl 3-(1-hydroxyimino-ethylcarbamoyl)-5-methyl-isoxazole-4-carboxylate.

To a solution of 4-tert-butyl 3-(1-hydroxyimino-ethylcarbamoyl)-5-methyl-isoxazole-4-carboxylate in ethanol (5 mL) were added sodium acetate (330 mg) and water (1.5 mL). After stirring at 100°C for 1 hour, the reaction mixture was concentrated. Ethyl acetate was added to the residue, and the organic layer was washed with water, dried over anhydrous sodium sulfate, and concentrated. The resulting residue was purified by silica gel column chromatography (hexane/chloroform, 1:1 → chloroform) to obtain the title compound (538 mg) as a yellow oily matter.

### (Step 4)

### Production of 5-methyl-3-(3-methyl-[1,2,4]oxadiazol-5-yl)-isoxazole-4-carboxylic acid:

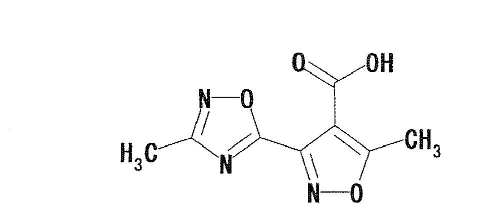

To a solution of tert-butyl 5-methyl-3-(3-methyl-[1,2,4]oxadiazol-5-yl)-isoxazole-4-carboxylate (525 mg) in chloroform (1 mL) was added trifluoroacetic acid (1 mL). After stirring for 2 hours, the reaction mixture was concentrated. Sodium hydroxide aqueous solution was added to the residue, and the aqueous layer was washed with toluene. The aqueous layer was made acidic with hydrochloric acid, and the precipitated solid was collected by filtration to obtain the title compound (198 mg) as a pale yellow solid.

### (Step 5)

### Production of 4-aminomethyl-5-methyl-3-(3-methyl-[1,2,4]oxadiazol-5-yl)-isoxazole:

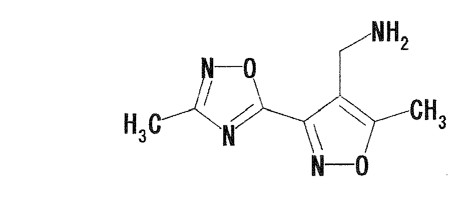

In the same manner as in Steps 5 to 7 of Reference Example 13, a crude product of the title compound was obtained from 5-methyl-3-(3-methyl-[1,2,4]oxadiazol-5-yl)-isoxazole-4-carboxylic acid (190 mg).

### Reference Example 70

### Production of 5-aminomethyl-2-methyl-4-(3-methyl-[1,2,4]oxadiazol-5-yl)-oxazole hydrochloride:

### (Step 1)

### Production of 5-(tert-butyloxycarbonylamino-methyl)-4-carbonyl-2-methyl-oxazole:

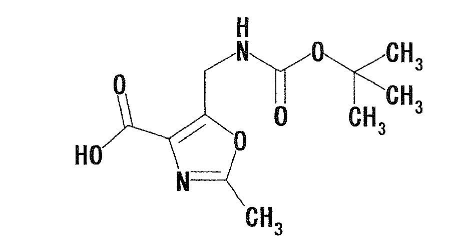

In the same manner as in Step 2 of Reference Example 3, the title compound (1.435 g) was obtained from 5-(tert-butyloxycarbonylamino-methyl)-4-ethoxycarbonyl-2-methyl-oxazole (2.00 g) produced in Step 3 of Example 2-1 as pale orange color solid.

### (Step 2)

### Production of 5-(tert-butyloxycarbonylamino-methyl)-2-methyl-4-(3-methyl-[1,2,4]oxadiazol-5-yl)-oxazole:

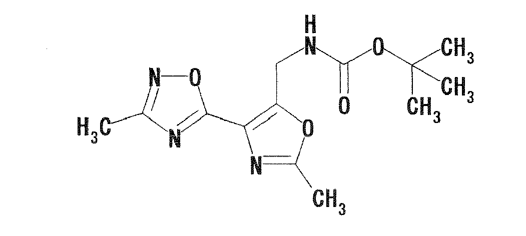

In the same manner as in Step 3 of Reference Example 69, the title compound (179 mg) was obtained from 5-(tert-butyloxycarbonylamino-methyl)-4-carbonyl-2-methyl-oxazole (300 mg) as a pale yellow oily matter.

### (Step 3)

### Production of 5-aminomethyl-2-methyl-4-(3-methyl-[1,2,4]oxadiazol-5-yl)-oxazole hydrochloride:

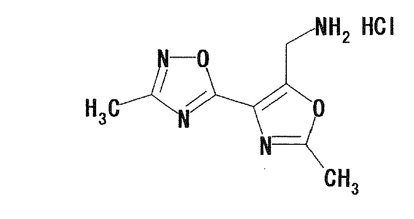

To a solution of 5-(tert-butyloxycarbonylamino-methyl)-2-methyl-4-(3-methyl-[1,2,4]oxadiazol-5-yl)-oxazole (170 mg) in chloroform (2 mL) was added trifluoroacetic acid (1 mL). After stirring for 30 minutes, the reaction mixture was concentrated. The residue was dissolved in chloroform followed by addition of 4 N hydrochloric acid/dioxane, and the precipitated solid was collected by filtration to obtain the title compound (139 mg) as a white solid.

### Reference Example 71

### Production of 2-aminomethyl-5-(3-fluoro-phenyl)-[1,3,4]oxadiazole trifluoroacetate:

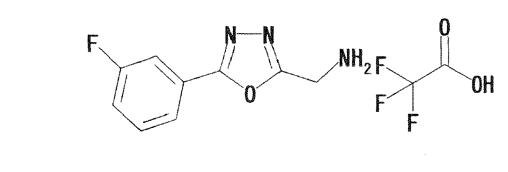

In the same manner as in Reference Example 51, the title compound (1.383 g) was obtained from N-(tert-butoxycarbonyl)-glycine (1.004 g) and 3-fluoro-benzoic acid hydrazide (0.915 g) as a white solid.

### Reference Example 72

### Production of 2-aminomethyl-5-(3-methyl-phenyl)-[1,3,4]oxadiazole trifluoroacetate:

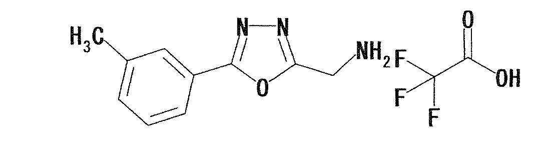

In the same manner as in Reference Example 51, the title compound (1.203 g) was obtained from N-(tert-butoxycarbonyl)-glycine (1.000 g) and 3-methyl-benzoic acid hydrazide (0.888 g) as a white solid.

### Reference Example 73

### Production of 2-aminomethyl-5-benzyl-[1,3,4]oxadiazole:

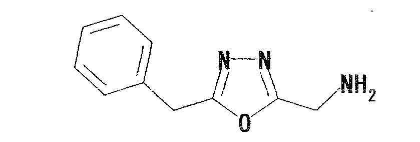

In the same manner as in Reference Example 51, 2-aminomethyl-5-benzyl-[1,3,4]oxadiazole trifluoroacetate was produced from N-(tert-butoxycarbonyl)-glycine (1.004 g) and phenylacetic acid hydrazide (0.879 g). To this compound was added 4 N sodium hydroxide aqueous solution, and the mixture was extracted with chloroform. The organic layer was dried over anhydrous sodium sulfate, and then concentrated to obtain the title compound (0.740 g) as an orange color oily matter.

### Reference Example 74

### Production of 2-aminomethyl-5-(1-methyl-1H-pyrrol-2-yl)-[1,3,4]oxadiazole trifluoroacetate:

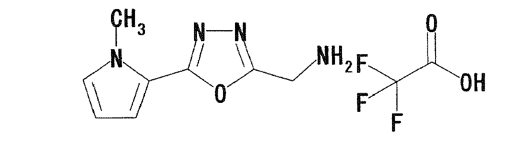

In the same manner as in Reference Example 51, the title compound (0.893 g) was obtained from N-(tert-butoxycarbonyl)-glycine (1.006 g) and 1-methylpyrrole-2-carboxylic acid hydrazide (0.800 g) as a white solid.

### Reference Example 75

### Production of 2-aminomethyl-5-(5-methyl-2H-pyrrol-3-yl)-[1,3,4]oxadiazole trifluoroacetate:

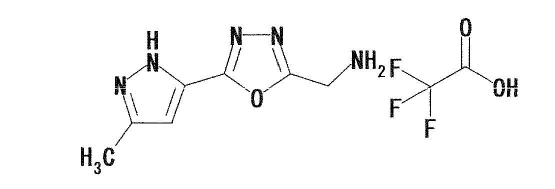

In the same manner as in Reference Example 51, the title compound (1.070 g) was obtained from N-(tert-butoxycarbonyl)-glycine (1.004 g) and 3-methylpyrazole-5-carboxylic acid hydrazide (0.807 g) as a white solid.

### Reference Example 76

### Production of 2-aminomethyl-5-benzo[b]thiophen-2-yl-[1,3,4]oxadiazole trifluoroacetate:

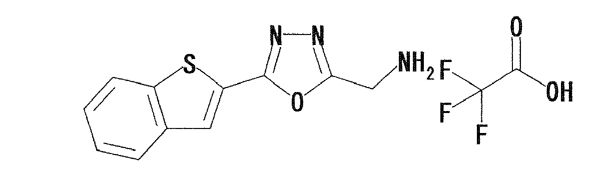

In the same manner as in Reference Example 51, the title compound (1.605 g) was obtained from N-(tert-butoxycarbonyl)-glycine (1.001 g) and benzo[b]thiophene-2-carboxylic acid hydrazide (1.263 g) as a white solid.

### Reference Example 77

### Production of 2-aminomethyl-5-(4-methyl-[1,2,3]thiadiazol-5-yl)-[1,3,4]oxadiazole trifluoroacetate:

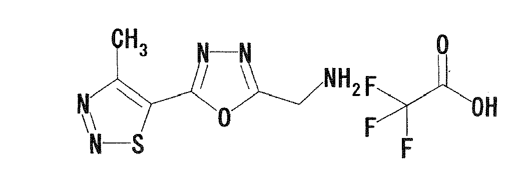

In the same manner as in Reference Example 51, the title compound (0.744 g) was obtained from N-(tert-butoxycarbonyl)-glycine (1.003 g) and 4-methyl-[1,2,3]thiadiazole-5-carboxylic acid hydrazide (0.904 g) as a white solid.

### Reference Example 78

### Production of 4-aminomethyl-5-methyl-1-(2-methyl-thiazol-4-ylmethyl)-1H-[1,2,3]triazole:

### (Step 1)

### Production of ethyl 3-(3-fluoro-phenyl)-3-oxo-propionate:

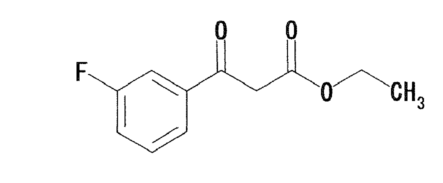

In the same manner as in Step 1 of Reference Example 17, the title compound (7.256 g) was obtained from 3-fluorobenzoic acid (5.004 g) as a pale yellow oily matter.

### (Step 2)

### Production of ethyl 4-(3-fluoro-phenyl)-2-methylpyrimidine-5-carboxylate:

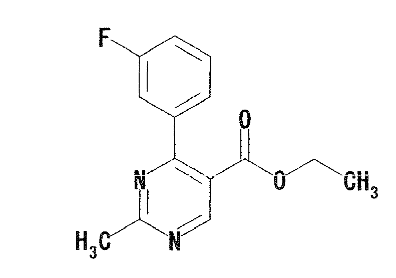

To a solution of ethyl 3-(3-fluoro-phenyl)-3-oxo-propionate (7.256 g) in toluene (50 mL) was added N,N-dimethylformamide dimethylacetal (9.3 mL). After stirring at 125°C for 10 hours, the reaction mixture was concentrated to obtain ethyl 3-dimethylamino-2-(3-fluorobenzoyl)-acrylate.

Subsequently, to a solution of ethyl 3-dimethylamino-2-(3-fluoro-benzoyl)-acrylate in ethanol (50 mL) were added acetamidine hydrochloride (3.666 g) and 20% sodium ethoxide/ethanol solution (13.25 g). After stirring at 100°C for 4 hours, the reaction mixture was concentrated, water was added to the residue, and the mixture was extracted with ethyl acetate. The organic layer was washed with saturated brine, dried over anhydrous sodium sulfate, and then concentrated. The resulting residue was purified by silica gel column chromatography (hexane/ethyl acetate, 6:1 → 3:1) to obtain the title compound (6.954 g) as a yellow oily matter.

### (Step 3)

### Production of 4-(3-fluoro-phenyl)-2-methyl-pyrimidine-5-carboxylic acid:

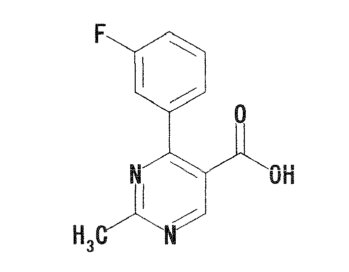

In the same manner as in Step 2 of Reference Example 3, the title compound (4.497 g) was obtained from ethyl 4-(3-fluoro-phenyl)-2-methyl-pyrimidine-5-carboxylate (5.673 g) as a white solid.

### (Step 4)

### Production of 4-(3-fluoro-phenyl)-5-hydroxymethyl-2-methyl-pyrimidine:

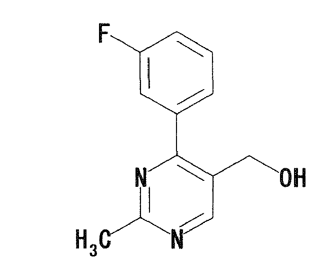

In the same manner as in Step 3 of Reference Example 3, the title compound (600 mg) was obtained from 4-(3-fluoro-phenyl)-2-methyl-pyrimidine-5-carboxylic acid (1.026 g) as colorless crystals.

### (Step 5)

### Production of 5-aminomethyl-4-(3-fluoro-phenyl)-2-methylpyrimidine:

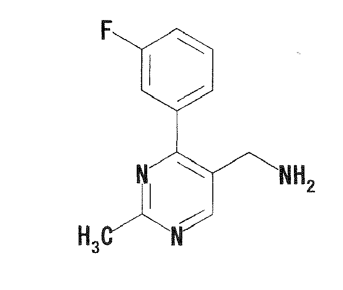

To a solution of 4-(3-fluoro-phenyl)-5-hydroxymethyl-2-methyl-pyrimidine (590 mg) in chloroform (10 mL) was added triethylamine (0.414 mL) followed by addition of methanesulfonyl chloride (0.23 mL) with ice cooling, and the mixture was stirred overnight at room temperature. The reaction mixture was successively washed with water and saturated brine, and the organic layer was dried over anhydrous sodium sulfate and then concentrated to obtain 5-chloromethyl-4-(3-fluorophenyl)-2-methyl-pyrimidine.

Subsequently, to 5-chloromethyl-4-(3-fluoro-phenyl)-2-methyl-pyrimidine was added 7 N ammonia/methanol solution. After stirring at room temperature for 3 days, the reaction mixture was concentrated. The residue was dissolved in 8 N sodium hydroxide aqueous solution, and the mixture was extracted with tetrahydrofuran. The organic layer was successively washed with water and saturated brine, dried over anhydrous sodium sulfate, and then concentrated to obtain the title compound (557 mg) as a brown oily matter.

### Reference Example 79

### Production of 5-aminomethyl-2-cyclopropyl-4-methyl-pyrimidine:

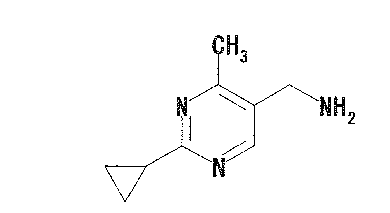

In the same manner as in Steps 2 to 5 of Reference Example 78, the title compound (604 mg) was obtained using ethyl acetoacetate (1.8 mL) and cyclopropanecarboxyamidine hydrochloride (1.79 g) as a brown oily matter.

### Reference Example 80

### Production of 5-aminomethyl-4-cyclopropyl-pyrimidine:

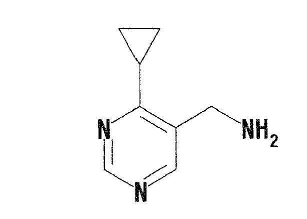

In the same manner as in Steps 2 to 5 of Reference Example 78, the title compound (106 mg) was obtained from methyl 3-cyclopropyl-3-oxo-propionate as a brown oily matter.

### Reference Example 81

### Production of 4-aminomethyl-5-cyclopropyl-1-(4-fluorophenyl)-1H-pyrazole:

### (Step 1)

### Production of 2,4-dicyclopropyl-pyrimidine-5-carboxylic acid:

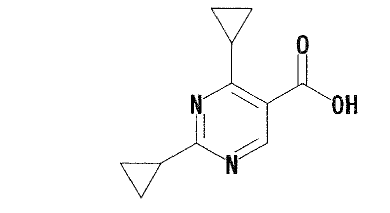

To a solution of methyl 3-cyclopropyl-3-oxo-propionate (2.23 g) in toluene (20 mL) was added N,N-dimethylformamide dimethylacetal (4.2 mL). After stirring at 125°C for 10 hours, the reaction mixture was concentrated to obtain methyl 2-cyclopropanecarbonyl-3-dimethylamino-acrylate.

Subsequently, to a solution of methyl 2-cyclopropanecarbonyl-3-dimethylamino-acrylate in ethanol (20 mL) were added cyclopropanecarboxyamidine hydrochloride (2.039 g) and 20% sodium ethoxide/ethanol solution (5.605 g). After stirring at 95°C for 10 hours, the reaction mixture was concentrated, water was added to the residue, and the mixture was extracted with ethyl acetate. The organic layer was washed with saturated brine, dried over anhydrous sodium sulfate, and concentrated under reduced pressure. The resulting residue was purified by silica gel column chromatography (hexane/ethyl acetate, 6:1 → 3:1) to obtain a mixture (2.892 g) of methyl 2,4-dicyclopropyl-pyrimidine-5-carboxylate and ethyl 2,4-dicyclopropyl-pyrimidine-5-carboxylate as a yellow oily matter.

Subsequently, in the same manner as in Step 2 of Reference Example 3, the title compound (2.276 g) was obtained from the mixture (2.892 g) of methyl 2,4-dicyclopropyl-pyrimidine-5-carboxylate and ethyl 2,4-dicyclopropyl-pyrimidine-5-carboxylate as a white solid.

### (Step 2)

### Production of 2,4-dicyclopropyl-5-hydroxymethyl-pyrimidine:

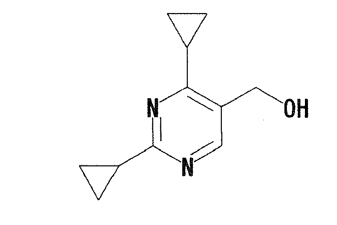

In the same manner as in Step 3 of Reference Example 3, the title compound (760 mg) was obtained from 2,4-dicyclopropyl-pyrimidine-5-carboxylic acid (1.017 g) as colorless crystals.

### (Step 3)

### Production of 5-aminomethyl-2,4-dicyclopropyl-pyrimidine:

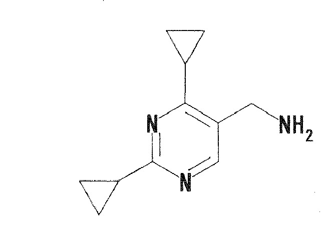

In the same manner as in Step 5 of Reference Example 78, the title compound (536 mg) was obtained from 2,4-dicyclopropyl-5-hydroxymethyl-pyrimidine (760 mg) as a pale yellow solid.

### Reference Example 82

### Production of 5-aminomethyl-4-(2,4-dimethyl-oxazol-5-yl)-2-methyl-4-pyrimidine:

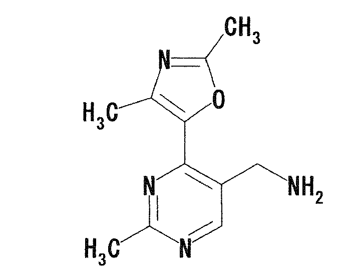

In the same manner as in Steps 1 to 5 of Reference Example 78, a crude product of the title compound was obtained from 2,4-dimethyl-oxazole-5-carboxylic acid (3.506 g) as a brown oily matter.

### Reference Example 83

### Production of 5-aminomethyl-4-(2,5-dimethyl-furan-3-yl)-[1,2,3]thiadiazole:

### (Step 1)

### Production of ethyl 3-(2,5-dimethyl-furan-3-yl)-3-oxo-propionate:

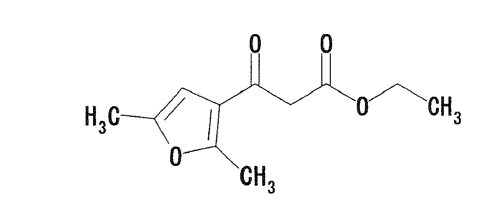

In the same manner as in Step 1 of Reference Example 17, the title compound (1.58 g) was obtained from 2,5-dimethyl-furan-3-carboxylic acid (2.90 g) as a yellow oily matter.

### (Step 2)

### Production of ethyl 4-(2,5-dimethyl-furan-3-yl)-[1,2,3]thiadiazole-5-carboxylate:

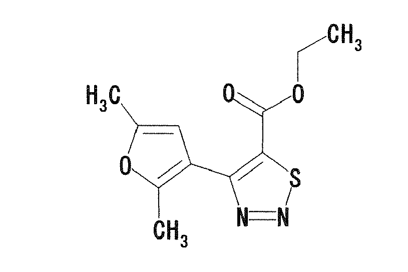

To a solution of ethyl 3-(2,5-dimethyl-furan-3-yl)-3-oxo-propionate (1.58 g) in ethanol (25 mL) was added p-toluenesulfonyl hydrazide (1.68 g). After stirring at 60°C for 1 hour and at 80°C for 7 hours, the reaction mixture was concentrated. Ethyl acetate was added to the residue, and the organic layer was washed with water, dried over anhydrous sodium sulfate, and concentrated. The resulting residue was purified by silica gel column chromatography (hexane/chloroform, 2:1 → 1:1 → chloroform) to obtain a hydrazone intermediate (2.306 g) as a yellow oily matter.

Subsequently, to a solution of thionyl chloride (4.00 mL) in chloroform (20 mL), a solution of a hydrazone intermediate (2.306 g) in chloroform (5 mL) was added dropwise. After stirring at room temperature for 1 hour and 30 minutes, the reaction mixture was concentrated. Ethyl acetate was added to the residue, and the organic layer was washed with saturated aqueous sodium hydrogencarbonate, and morpholine (0.95 mL) was added to the organic layer. After stirring for 1 hour and 30 minutes, the reaction mixture was washed with water, dried over anhydrous sodium sulfate, and then concentrated. The resulting residue was purified by silica gel column chromatography (hexane/chloroform, 2:1 → 1:1 → chloroform) to obtain the title compound (835 mg) as a yellow oily matter.

### (Step 3)

### Production of 4-(2,5-dimethyl-furan-3-yl)-[1,2,3]thiadiazole-5-carboxylic acid:

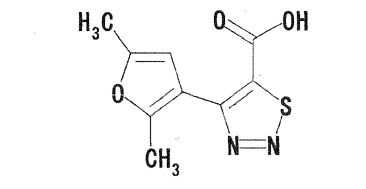

In the same manner as in Step 2 of Reference Example 3, the title compound (615 mg) was obtained from ethyl 4-(2,5-dimethyl-furan-3-yl)-[1,2,3]thiadiazole-5-carboxylate (765 mg) as a yellow solid.

### (Step 4)

### Production of 5-aminomethyl-4-(2,5-dimethyl-furan-3-yl)-[1,2,3]thiadiazole:

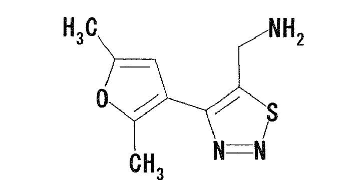

In the same manner as in Steps 5 to 7 of Reference Example 13, the title compound (207 mg) was obtained from 4-(2,5-dimethyl-furan-3-yl)-[1,2,3]thiadiazole-5-carboxylic acid (610 mg) as a brown solid.

### Reference Example 84

### Production of 2-aminomethyl-5-(3-chloro-phenyl)-[1,3,4]thiadiazole trifluoroacetate:

### (Step 1)

### Production of tert-butyl {2-[N'-(3-chloro-benzoyl)-hydrazino]-2-oxo-ethyl}-carbamate:

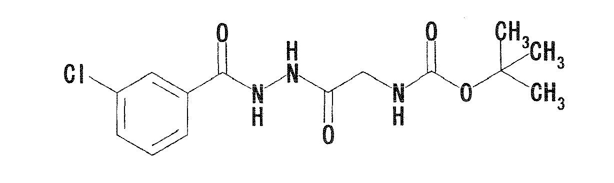

To a solution of N-(tert-butoxycarbonyl)-glycine (1.004 g) in chloroform (15 mL) was gradually added CDI (0.978 g). After stirring for 1 hour and 30 minutes, 3-chlorobenzoic acid hydrazide (0.999 g) was added. After stirring for 8 days, water was added, and the precipitated solid was collected by filtration to obtain the title compound (0.992 g) as a white solid.

### (Step 2)

### Production of 2-(tert-butyloxycarbonylamino-methyl)-5-(3-chloro-phenyl)-[1,3,4]thiadiazole:

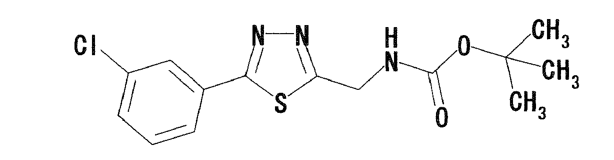

To a solution of tert-butyl {2-[N°-(3-chlorobenzoyl)-hydrazino]-2-oxo-ethyl}-carbamate (1.002 g) in tetrahydrofuran (40 mL) was added Lawesson's reagent (1.36 g). After stirring overnight at 70°C, the reaction mixture was concentrated, water was added to the residue, and the mixture was extracted with ethyl acetate. The organic layer was successively washed with water and saturated brine, dried over anhydrous sodium sulfate, and then concentrated. The resulting residue was purified by silica gel column chromatography (hexane/ethyl acetate, 2:1) to obtain the title compound (0.866 g) as white crystals.

### (Step 3)

### Production of 2-aminomethyl-5-(3-chloro-phenyl)-[1,3,4]thiadiazole trifluoroacetate:

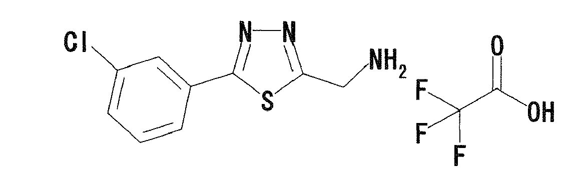

To 2-(tert-butyloxycarbonylamino-methyl)-5-(3-chloro-phenyl)-[1,3,4]thiadiazole (0.866 g) was added trifluoroacetic acid (2 mL) at room temperature. After stirring for 1 hour, the reaction mixture was concentrated. Ethyl acetate and diethyl ether were added to the residue, and the precipitated solid was collected by filtration to obtain the title compound (0.861 g) as a white solid.

### Reference Example 85

### Production of C-[2-(5-ethyl-1H-[1,2,4]triazol-3-ylmethyl)-4-methyl-oxazol-5-yl]-methylamine hydrochloride:

### (Step 1)

### Production of ethyl 2-methoxycarbonylmethyl-4-methyl-oxazole-5-carboxylate:

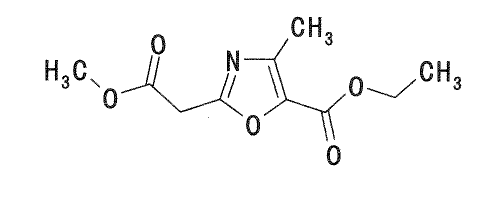

In the same manner as in Step 3 of Reference Example 32, the title compound (3.62 g) was synthesized from 2-chloroethyl acetoacetate (13.64 mL) and methylmalonic acid monoamide (19 mL).

### (Step 2)

### Production of ethyl 2-hydrazinocarbonylmethyl-4-methyl-oxazole-5-carboxylate:

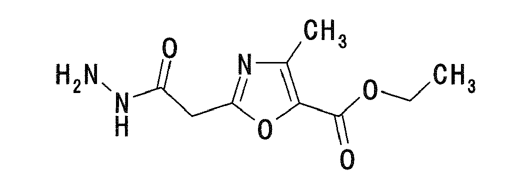

To a solution of ethyl 2-methoxycarbonylmethyl-5-methyl-oxazole-4-carboxylate (3.29 g) in ethyl alcohol (33 mL) was added hydrazine monohydrate (0.72 mL) with stirring. This mixture was stirred at room temperature for 3 days. This solution was concentrated to obtain the title compound (0.72 g).

### (Step 3)

### Production of methyl thiopropionimidate hydroiodide;

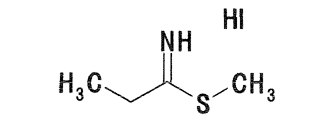

To a solution (10 mL) of thiopropionamide (0.968 g) in acetone was added methyl iodide (0.68 mL). The reaction mixture was stirred at room temperature for 1 day. This solution was concentrated to obtain the title compound (1.6 g) as a yellow oily matter.

### (Step 4)

### Production of ethyl 2-(5-ethyl-1H-[1,2,4]triazol-3-ylmethyl)-4-methyl-oxazole-5-carboxylate:

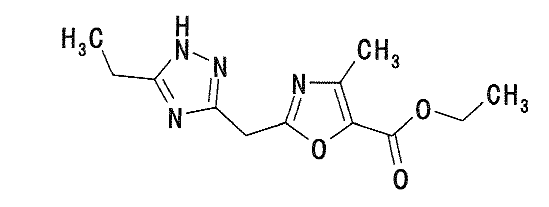

Ethyl 2-hydrazinocarbonylmethyl-5-methyl-oxazole-4-carboxylate (367 mg) and methyl thiopropionimidate hydroiodide (750 mg) were suspended in ethyl alcohol (1 mL). This mixture was heated at 80°C for 1 hour. This solution was vacuum concentrated, and diphenyl ether (2 mL) was added to the residue. This mixture was heated at 160°C for 6 hours. The reaction mixture was cooled to room temperature, and the residue was extracted with 1 N hydrochloric acid. This aqueous solution was made alkaline with sodium hydrogencarbonate, and the residue was extracted with THF. This solution was dried, filtered, and vacuum concentrated. The resulting residue was purified by silica gel column chromatography (developing solvent: chloroform containing 5% methanol) to obtain the title compound (120 mg) as a light yellow solid.

### (Step 5)

### Production of 2-(5-ethyl-1H-[1,2,4]triazol-3-ylmethyl)-4-methyl-oxazole-5-carboxylic acid amide:

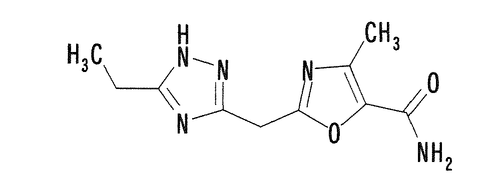

Ethyl 2-(5-ethyl-1H-[1,2,4]triazol-3-ylmethyl)-5-methyl-oxazole-4-carboxylate (120 mg) was dissolved in 7 N ammonia/methanol solution (10 mL) and allowed to stand at room temperature for 1 day. The reaction solution was concentrated, and the resulting residue was purified by silica gel column chromatography (developing solvent: chloroform containing 6% methanol) to obtain the title compound (97 mg) as a light yellow solid.

### (Step 6)

### Production of 2-(5-ethyl-1H-[1,2,4]triazol-3-ylmethyl)-4-methyl-oxazole-5-carbonitryl:

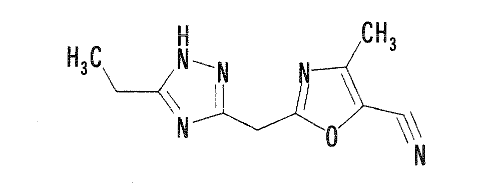

According to the method described in Step 5 of Reference Example 24, the title compound (87 mg) was obtained from 2-(5-ethyl-1H-[1,2,4]triazol-3-ylmethyl)-5-methyl-oxazole-4-carboxylic acid amide (96 mg).

### (Step 7)

### Production of C-[2-(5-ethyl-1H-[1,2,4]triazol-3-ylmethyl)-4-methyl-oxazol-5-yl]-methylamine hydrochloride:

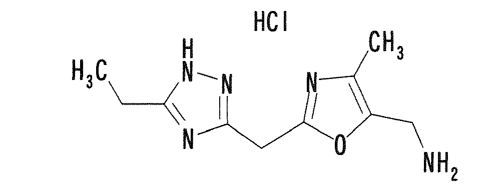

According to the method described in Step 6 of Reference Example 24, the title compound (77 mg) was obtained from 2-(5-ethyl-1H-[1,2,4]triazol-3-ylmethyl)-5-methyl-oxazole-4-carbonitryl (87 mg) as a brown solid.

### Reference Example 86

### Production of C-[5-(4-chloro-phenyl)-2H-[1,2,4]triazol-3-yl]-methylamine:

### (Step 1)

### Production of methyl 4-chlorothiobenzimidate hydroiodide:

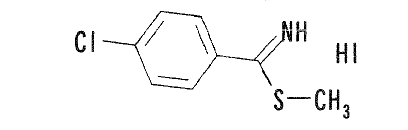

According to the method described in Step 3 of Reference Example 85, the title compound was synthesized from 4-chlorothiobenzamide.

### (Step 2)

### Production of C-[5-(4-chloro-phenyl)-2H-[1,2,4]triazol-3-yl]-methylamine:

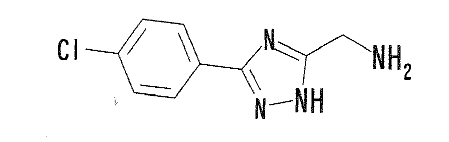

Glycine Boc hydrazide (190 mg) and methyl 4-chlorothiobenzimidate hydroiodide (320 mg) were suspended in ethyl alcohol (3 mL). This mixture was stirred at room temperature for 2 hours and heated at 100°C for 1 hour. Diphenyl ether (1.5 mL) was added to this mixture and heated at 160°C for 1 hour. The reaction mixture was cooled to room temperature to precipitate a white solid. This solid was isolated, washed with diethyl ether, and dried under reduced pressure to obtain the title compound (150 mg).

### Reference Example 87

### Production of C-[5-(3,4-dimethoxy-benzyl)-2H-[1,2,4]triazol-3-yl]-methylamine:

### (Step 1)

### Production of methyl 2-benzyloxycarbonylaminothioacetoimidate hydroiodide:

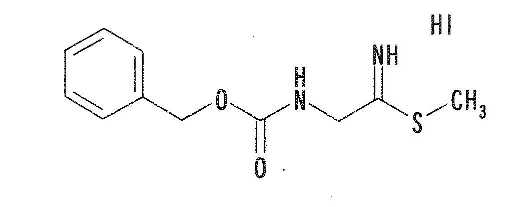

According to the method described in Step 3 of Reference Example 85, the title compound (1.02 g) was obtained from benzyl thiocarbonylmethyl-carbamate (0.672 g) as a yellow solid.

### (Step 2)

### Production of C-[5-(3,4-dimethoxy-benzyl)-2H-[1,2,4]triazol-3-yl]-methylamine:

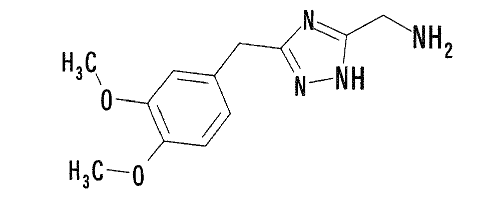

Methyl 2-benzyloxycarbonylaminothioacetoimidate hydroiodide (201 mg) and (3,4-dimethoxy-phenyl)-acetic acid hydrazide (116 mg) were suspended in ethanol (1.5 mL). This mixture was heated at 70°C for 1 hour. Diphenyl ether (1.0 mL) was added to this reaction solution, and the mixture was heated at 160°C for 2 hours. After cooling to room temperature, n-hexane was added to the reaction mixture to separate the resulting oily matter. The residue was purified by silica gel column chromatography (developing solvent: chloroform containing 5% methanol) to obtain the title compound (120 mg) as a light yellow solid. This residue was dissolved in a mixed solvent of dioxane (5 mL) and acetic acid (1 mL). To this solution was added 10% palladium-carbon (50 mg), and the mixture was stirred for 5 hours under a hydrogen pressure (3.5 MPa). The reaction mixture was filtered, and the filtrate was solidified by concentration to obtain the title compound (53 mg) as a light yellow solid.

Subsequently, according to the above Production Method A-1, the following compounds were produced.

### Example 1-1

### Production of 5-(2-chloro-4,5-difluoro-benzoylamino)-1H-pyrazole-3-carboxylic acid (5-ethoxycarbonyl-furan-2-ylmethyl)-amide;

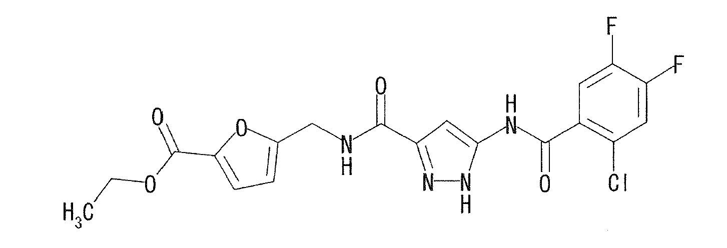

To a suspension of 5-(2-chloro-4,5-difluoro-benzoylamino)-1H-pyrazole-3-carboxylic acid imidazolide (502 mg) produced in Reference Example 1 and ethyl 5-aminomethyl-furan-2-carboxylate hydrochloride (309 mg) produced in Reference Example 2 in N,N-dimethylformamide (3 mL) was added triethylamine (0.22 mL) with ice cooling. After stirring overnight at room temperature, water was added to the reaction mixture, and the precipitated solid was collected by filtration to obtain the title compound (643 mg) as a white solid.

### Example 1-36

### Production of 5-(2-chloro-4,5-difluoro-benzoylamino)-1H-pyrazole-3-carboxylic acid (4-benzyl-2-methyl-oxazol-5-ylmethyl)-amide p-toluenesulfonate:

### (Step 1)

### Production of 5-(2-chloro-4,5-difluoro-benzoylamino)-1H-pyrazole-3-carboxylic acid (4-benzyl-2-methyl-oxazol-5-ylmethyl)-amide:

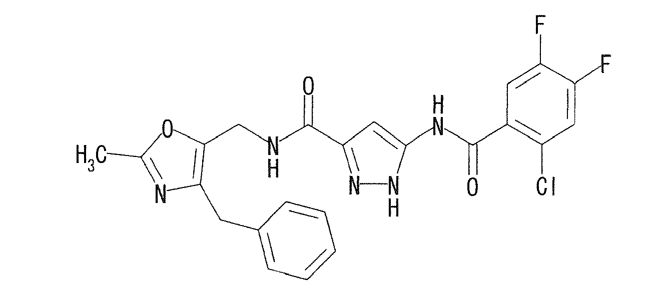

In the same manner as in Example 1-1, the title compound (262 mg) was obtained from 5-aminomethyl-4-benzyl-2-methyl-oxazole dihydrochloride (160 mg) produced in Reference Example 35 as a colorless solid.

### (Step 2)

### Production of 5-(2-chloro-4,5-difluoro-benzoylamino)-1H-pyrazole-3-carboxylic acid (4-benzyl-2-methyl-oxazol--5-ylmethyl)-amide p-toluenesulfonate:

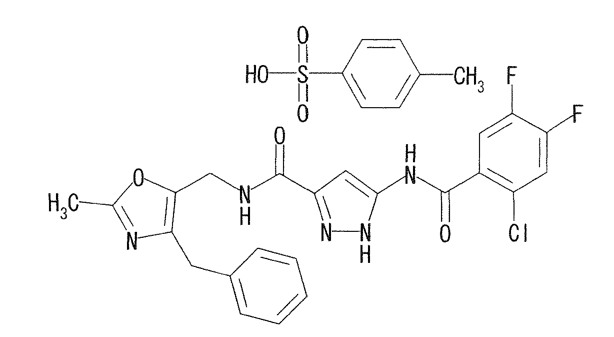

To a solution of 5-(2-chloro-4,5-difluoro-benzoylamino)-1H-pyrazole-3-carboxylic acid (5-benzyl-2-methyl-oxazol-5-ylmethyl)-amide (262 mg) in ethyl acetate (4 mL) was added p-toluenesulfonic acid monohydrate (170 mg), and the mixture was allowed to stand at room temperature for 1 day. The precipitated crystals were collected by filtration and dried to obtain the title compound (102 mg).

In the same manner as in the above Example 1-1, the compounds of Examples 1-2 to 1-35 and Examples 1-37 to 1-88 were produced. In production of the compounds of Examples 1-5 and 1-6, commercially available amines, 4-aminomethyl-2-(4-chloro-phenyl)-thiazole and 5-aminomethyl-4-methyl-2-(4-trifluoromethyl-phenyl)-thiazole, were used as compound (A8) in the above Production Method A-1.

Subsequently, the following compounds were produced according to the above Production Method A-2.

### Example 2-1

### Production of 5-(4,5-difluoro-2-methyl-benzoylamino)-1H-pyrazole-3-carboxylic acid (4-ethoxycarbonyl-2-methyl-oxazol-5-ylmethyl)-amide:

### (Step 1)

### Production of ethyl 5-(4,5-difluoro-2-methyl-benzoylamino)-1H-pyrazole-3-carboxylate:

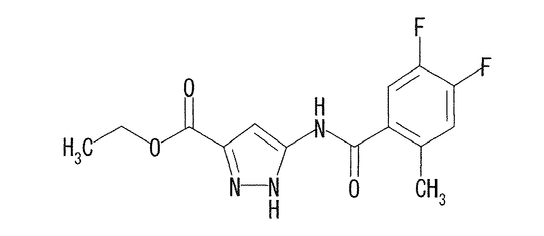

To a suspension of 4,5-difluoro-2-methylbenzoic acid (1.505 g) in chloroform (15 mL) were added oxalyl chloride (1.124 mL) and N,N-dimethylformamide (catalytic amount) with ice cooling. After stirring overnight at room temperature, the reaction mixture was concentrated to obtain 4,5-difluoro-2-methyl-benzoyl chloride as a yellow oily matter.

Subsequently, to a solution of ethyl 5-amino-3-pyrazolecarboxylate (1.337 g) in tetrahydrofuran (15 mL) was added pyridine (1.385 mL), and then a solution of 4,5-difluoro-2-methyl-benzoyl chloride in tetrahydrofuran (2 mL) was added dropwise with ice cooling. After stirring overnight at room temperature, ethanol (5 mL) and water (45 mL) were added to the reaction mixture, and the precipitated solid was collected by filtration to obtain the title compound (2.271 g) as a white solid.

### (Step 2)

### Production of 5-(4,5-difluoro-2-methyl-benzoylamino)-1H-pyrazole-3-carboxylic acid:

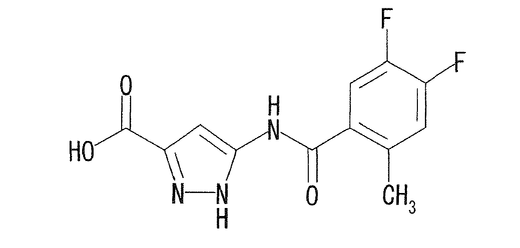

To a suspension of ethyl 5-(4,5-difluoro-2-methyl-benzoylamino)-1H-pyrazole-3-carboxylate (2.271 g) in ethanol (40 mL) was added 2 N sodium hydroxide aqueous solution (11 mL) with ice cooling. After stirring overnight at room temperature, 1 N hydrochloric acid (30 mL) and water (40 mL) were added to the reaction mixture, and the precipitated solid was collected by filtration to obtain the title compound (2.122 g) as a white solid.

### (Step 3)

### Production of 5-(tert-butoxycarbonylamino-methyl)-4-ethoxycarbonyl-2-methyl-oxazole:

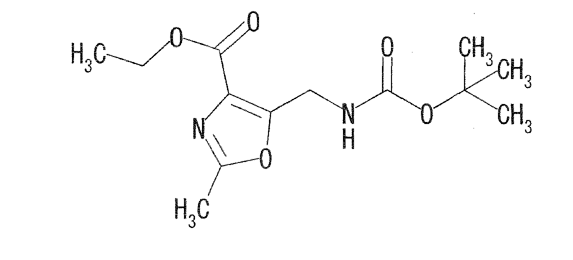

To a solution of 5-aminomethyl-4-ethoxycarbonyl-2-methyl-oxazole dihydrobromide (5.198 g) produced in Reference Example 33 in chloroform (40 mL) was added triethylamine (5.4 mL), and then a solution of di-tert-butyl dicarbonate (4.37 g) in chloroform (10 mL) was added with ice cooling. After stirring overnight at room temperature, the reaction mixture was concentrated. The residue was dissolved in ethyl acetate, and the organic layer was successively washed with water and saturated brine, dried over anhydrous sodium sulfate, and then concentrated. The residue was purified by silica gel column chromatography (hexane/ethyl acetate, 3:1 → 1:1) to obtain the title compound (4.416 g) as a yellow oily matter.

### (Step 4)

### Production of 5-aminomethyl-4-ethoxycarbonyl-2-methyl-oxazole dihydrochloride:

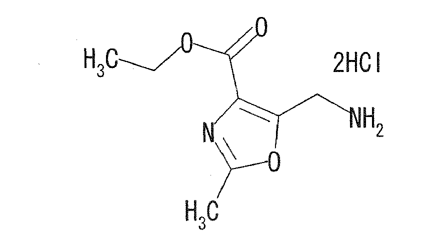

To a solution of 5-(tert-butoxycarbonylaminomethyl)-4-othoxycarbonyl-2-methyl-oxazole (4.416 g) in ethanol (40 mL) was added 4 N hydrochloric acid/ethyl acetate (20 mL). After stirring overnight at room temperature, the reaction mixture was concentrated, ethyl acetate was added to the residue, and the precipitated solid was collected by filtration to obtain the title compound (3.043 g) as a light yellow solid.

### (Step 5)

### Production of 5-(4,5-difluoro-2-methyl-benzoylamino)-1H-pyrazole-3-carboxylic acid (4-ethoxycarbonyl-2-methyl-oxazol-5-ylmethyl)-amide:

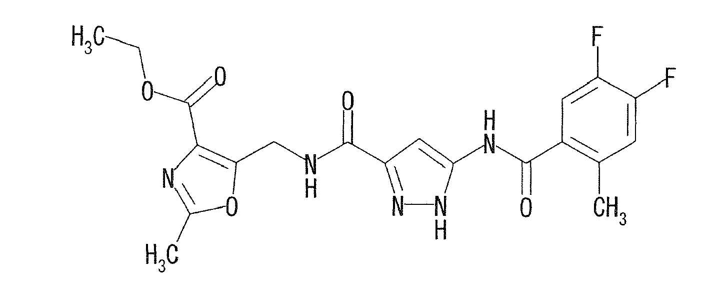

To a solution of 5-aminomethyl-4-ethoxycarbonyl-2-methyl-oxazole dihydrochloride (357 mg), 1-hydroxybenzotriazole monohydrate (226 mg), triethylamine (0.203 mL), and 5-(4,5-difluoro-2-methyl-benzoylamino)-1H-pyrazole-3-carboxylic acid (357 mg) in N,N-dimethylformamide (3 mL) was added EDC hydrochloride (280 mg). After stirring overnight at room temperature, aqueous sodium hydrogencarbonate was added to the reaction mixture, and the precipitated solid was collected by filtration. The resulting solid was purified by silica gel column chromatography (chloroform/ethyl acetate = 1:4) to obtain the title compound (421 mg) as a white solid.

The structural formulas and ¹H-NMR spectrum data of the example compounds are shown in the following Tables 1 to 16.
¹H-NMR spectra were measured using tetramethylsilane in CDC1₃ or DMSO-*d*₆ as the internal standard, and all the δ values are expressed in ppm.

Symbols in the tables denote as follows:
s: Singlet
d: Doublet
t: Triplet
dd: Double doublet
ddd: Double double doublet
brs: Broad singlet
m: Multiplet
J: Coupling constant

**Table 1**

| Example | Structural formula | **NMR** |
|---|---|---|
| 1-1 | 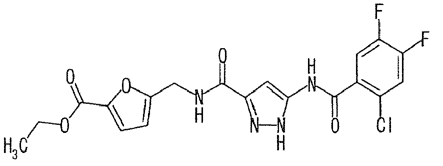 | ¹H-NMR (DMSQ-*d*₆) (δ: 13.28 (1H, br s), 11.16 (1H, br s), 9.21 (1H, t, *J* = 5.6 Hz), 7.86-7.79 (2H, m), 7.36 (1H, s), 7.24 (1H, d, *J* = 3.2 Hz), 6.51 (1H, d, *J* = 3.2 Hz), 4.50 (2H, d, *J* = 5.6 Hz), 4.27 (2H, q, *J* = 7.1 Hz), 1.28 (3H, t, *J* = 7.1 Hz). |
| 1-2 | 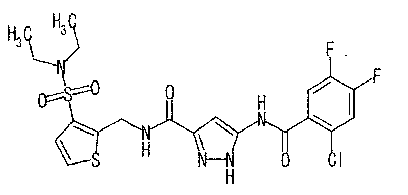 | ¹H-NMR (DMSO-*d*₆) δ: 13.36 (1 H, br s), 11.20 (1H, br s), 9.46 (1H, br s), 7.87-7.81 (2H, m), 7.54 (1H, d, *J* = 5.6 Hz), 7.40 (1H, br s), 7.24 (1H, d, *J* = 5.6 Hz), 4.86 (2H, d, *J* = 5.6 Hz), 3.26 (4H, q, *J* = 7.1 Hz), 1.08 (6H, t, *J* = 7.1 Hz). |
| 1-3 | 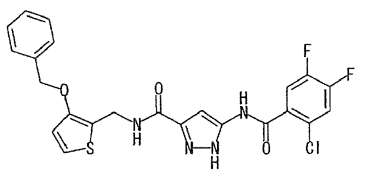 | ¹H-NMR (DMSO-*d*₆) δ: 13.24 (1H, br s), 11.13 (1H, br s), 9.09 (1H, br s), 7.85-7.79 (2H, m), 7.49-7.46 (2H, m), 7.41-7.31 (5H, m), 7.00 (1H, d, *J* = 5.5 Hz), 5.12 (2H, s), 4.48 (2H, d, *J* = 5.7 Hz). |
| 1-4 | 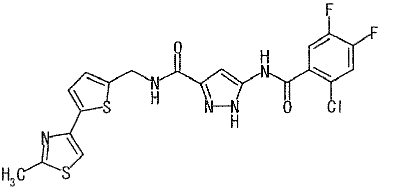 | ¹H-NMR (DMSO-*d*₆) δ: 13.20 (1H, br s), 11.15 (1H, br s), 9.25 (1H, br s), 7.86-7.79 (2H, m), 7.69 (1 H, s), 7.36 (1H, d, *J* = 3.5 Hz), 7.31 (1 H, br s), 6.98 (1 H, d, *J* = 3.5 Hz), 4.59 (2H, d, *J* = 5.7 Hz). 2.66 (3H, s). |
| 1-5 | 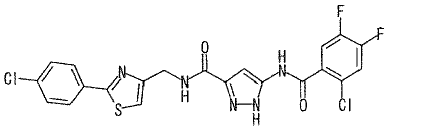 | ¹H-NMR (DMSO-*d*₆) δ: 13.26 (1H, br s), 11.15 (1H, br s), 9.21 (1H, br s), 7.97-7.93 (2H, m), 7.86-7.79 (2H, m), 7.58-7.55 (2H, m), 7.52 (1H, s), 7.39 (1H, br s), 4.60 (2H, d, *J* = 5.7 Hz). |
| 1-6 | 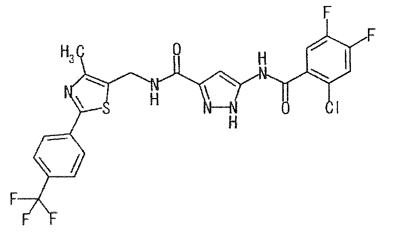 | ¹H-NMR (DMSO-*d*₆) δ: 11.19 (1H, br s), 9.27 (1H, br s), 8.09 (2H, d, *J* = 8.2 Hz), 7.86-7.80 (4H, m). 7.25 (1H, br s), 4.61 (2H, d, *J* = 5.7 Hz), 2.48 (3H, s). |

**Table 2**

| Example | Structural formula | **NMR** |
|---|---|---|
| 1-7 | 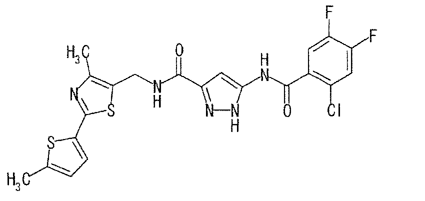 | ¹H-NMR (DMSO-*d₆*) δ 13.29 (1H, br s), 11.15 (1H, s), 9.24 (1H, t. *J* = 5.7 Hz), 7.85-7.78 (2H, m), 7.35 (1H, d, *J* = 3.6 Hz), 7.31 (1H, d, *J* = 2.2 Hz), 6.82 (1 H, d, *J* = 3.6 Hz), 4.54 (2H, d, *J* = 5.7 Hz), 2.46 (3H, s), 2.38 (3H, s). |
| 1-8 | 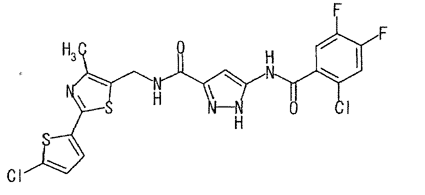 | ¹H-NMR (DMSO-*d*₆) δ: 13.29 (1H, br s), 11.17 (1H, br s), 9.25 (1H, br s), 7.86-7.80 (2H, m), 7.47 (1H, d, *J* = 4.0 Hz), 7.29 (1H, br s), 7.16 (1H, d, *J* = 4.0 Hz), 4.55 (2H, d, *J* = 5.8 Hz), 2.39 (3H, s). |
| 1-9 | 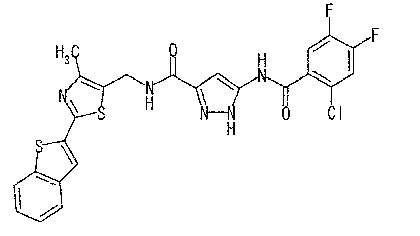 | ¹H-NMR (DMSO-*d*₆) δ: 13.31 (1H, br s), 11.17 (1H, br s), 9.30 (1H, br s), 8.00-7.96 (2H, m), 7.90-7.80 (3H, m), 7.43-7.39 (2H, m), 7.32 (1H, br s), 4.60 (2H, d, *J* = 5.8 Hz), 2.45 (3H, s). |
| 1-10 | 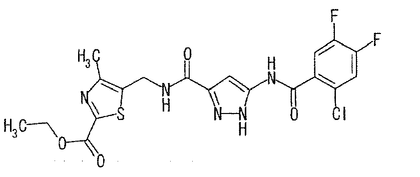 | ¹H-NMR (DMSO-*d*₆) δ: 13.31 (1H, br s), 11.17 (1H, br s), 9.32 (1H, br s), 7.86-7.79 (2H, m), 7.31 (1H, br s), 4.60 (2H, d, *J* = 5.3 Hz), 4.33 (2H, q, *J* = 7.1 Hz), 2.47 (3H, s), 1.30 (3H, t, *J* = 7.1 Hz). |
| 1-11 | 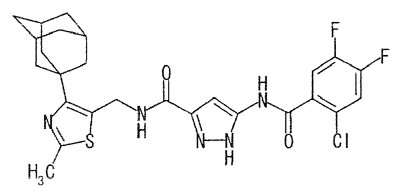 | ¹H-NMR (DMSO-*d*₆) δ: 13.27 (1H, d, *J* = 2.1 Hz), 11.15 (1H, br s), 9.23 (1H, t, *J* = 5.7 Hz), 7.85-7.79 (2H, m), 7.35 (1H, d, *J* = 2.1 Hz), 4.71 (2H, d, *J* = 5.7 Hz), 2.50 (3H, s), 2.07-2.00 (9H, m), 1.77-1.70 (6H, m). |
| 1-12 | 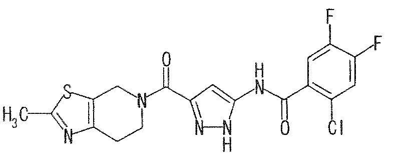 | ¹H-NMR (DMSO-*d*₆) δ: 13.28 (1H, s), 11.22 (1H, s), 7.95-7.79 (2H, m), 6.95 (1H, d, *J* = 1.9 Hz), 4.92-4.80 (2H, m), 3.95 (2H, t, *J* = 5.6 Hz), 2.90-2.80 (2H, m), 2.61 (3H, s). |

**Table 3**

| Example | Structural formula | **NMR** |
|---|---|---|
| 1-13 | 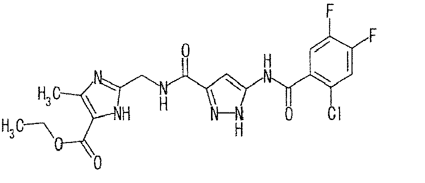 | ¹H-NMR (DMSO-*d*₆) δ: 13.24 (1H, br s), 12.58 (0.3H, br s), 12.30 (0.7H, br s), 11.14 (1H, br s), 9.12 (0.7H, br s), 9.00 (0.3H, br s), 7.85-7.80 (2H, m), 7.37 (1H, br s), 4.44 (0.6H, d, *J* = 5.6 Hz), 4.40 (1.4H, d, *J* = 5.6 Hz), 4.25 (0.6H, q, *J* = 7.1 Hz), 4.18 (1.4H, q, *J* = 7.1 Hz), 2.41 (2.1 H, s), 2.33 (0.9H, s), 1.29 (0.9H, t, *J* = 7.1 Hz), 1.25 (2.1H, t, *J* = 7.1 Hz). |
| 1-14 | 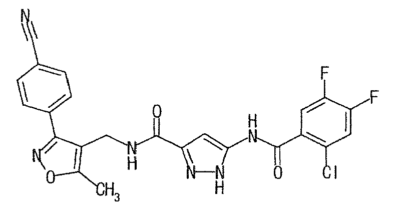 | ¹H-NMR (DMSO-*d*₆) δ: 13.23 (1H, br s), 11.14 (1H, br s), 8.91 (1H, br t, *J* = 5.1 Hz), 7.99 (2H, d, *J* = 8.5 Hz), 7.91 (2H, d, *J* = 8.5 Hz), 7.86-7.76 (2H, m), 7.27 (1H, br s), 4.36 (2H, d, *J* = 5.1 Hz), 2.52 (3H, s). |
| 1-16 | 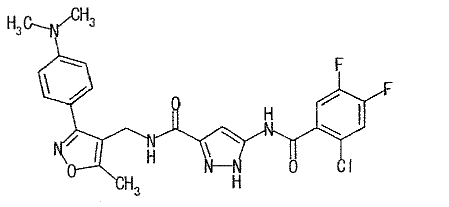 | ¹H-NMR (DMSO-*d*₆) δ: 13.24 (1H, br d, *J* = 1.7 Hz), 11.12 (1H, br s), 8.87 (1H, br t, *J* = 4.6 Hz), 7.85-7.76 (2H, m), 7.52 (2H, d, *J* = 8.8 Hz), 7.34 (1 H, d, *J* = 1.7 Hz), 6.78 (2H, d, *J* = 8.8 Hz), 4.34 (2H, d, *J* = 4.6 Hz), 2.95 (6H, s), 2.45 (3H, s). |
| 1-16 | 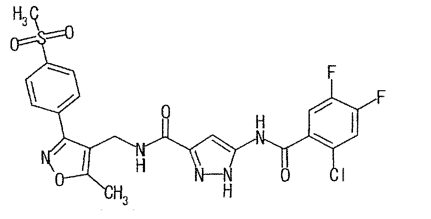 | ¹H-NMR (DMSO-*d*₆) δ: 13.24 (1H, br s), 11.13 (1H, br s), 8.92 (1H, br t, *J* = 4.5 Hz), 8.05 (2H, d, *J* = 8.7 Hz), 7.97 (2H, d, *J* = 8.7 Hz), 7.86-7.76 (2H, m), 7.27 (1H, d, *J* = 1.9 Hz), 4.38 (2H, d, *J* = 4.5 Hz), 3.27 (3H, s), 2.53 (3H, s). |
| 1-17 | 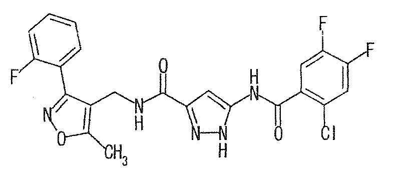 | ¹H-NMR (DMSO-*d*₆) δ: 13.15 (1H. br d, *J* = 1.9 Hz), 11.10 (1H, br s), 8.78 (1 H, br t, *J* = 5.0 Hz), 7.85-7.76 (2H, m), 7.61-7.53 (2H, m), 7.41-7.30 (2H, m), 7.22 (1H, d, *J* = 1.9 Hz), 4.23 (2H, d, *J* = 5.0 Hz), 2.52 (3H, s). |
| 1-18 | 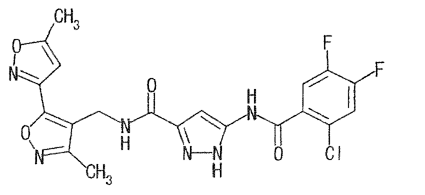 | ¹H-NMR (DMSO-*d*₆) δ: 13.25 (1H, br d, *J* = 1.7 Hz), 11.13 (1H, br s), 8.93 (1 H, br t, *J* = 4.9 Hz), 7.85-7.76 (2H, m), 7.31 (1H. d, *J* = 1.7 Hz), 6.86 (1H, s), 4.55 (2H, d, *J* = 4.9 Hz), 2.53 (3H, s), 2.32 (3H, s). |

**Table 4**

| Example | Structural formula | **NMR** |
|---|---|---|
| 1-19 | 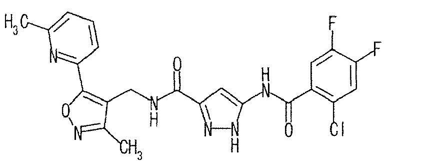 | ¹H-NMR (DMSO-*d*₆) δ 13.25 (1H, br d, *J* = 2.1 Hz), 11.12 (1H, br s), 8.78 (1H, br t, *J* = 5.2 Hz), 7.91-7.72 (4H, m), 7.38 (1H, d, *J* = 7.9 Hz), 7.27 (1H, d, *J* = 2.1 Hz), 4.80 (2H, d, *J* = 5.2 Hz), 2.57 (3H, s), 2.30 (3H, s). |
| 1-20 | 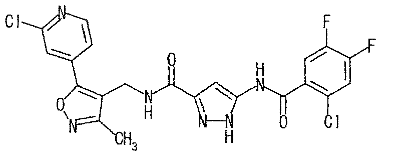 | ¹H-NMR (DMSO-*d*₆) δ: 13.30 (1H, br s), 11.16 (1H, br s), 9.07 (1H, br t, *J* = 5,3 Hz), 8.61 (1H, d, *J* = 5.3 Hz), 7.99 (1H, s), 7.88-7.77 (3H, m), 7.33 (1H, s), 4.50 (2H, d, *J* = 5.3 Hz), 2.35 (3H, s). |
| 1-21 | 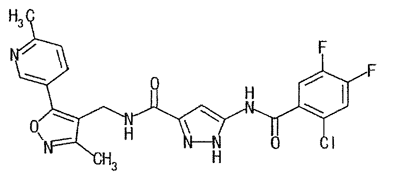 | ¹H-NMR (DMSO-*d*₆) δ: 13.25 (1 H, br s), 11.13 (1H, br s), 8.98 (1H, br s), 8.86 (1H, d, *J* = 2.4 Hz), 8.10 (1H, dd, *J* = 8.0, 2.4 Hz), 7.84-7.75 (2H, m), 7.43 (1H, d, *J* = 8.0 Hz), 7.31 (1H, br s), 4.42 (2H, d, *J* = 4.9 Hz), 2.53 (3H, s), 2.31 (3H, s). |
| 1-22 | 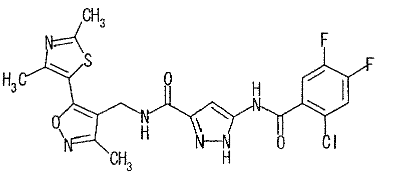 | ¹H-NMR (DMSO-*d*₆) δ: 13.23 (1H, br s), 11.12 (1H, br s), 8.85 (1H, t, *J* = 4.5 Hz), 7.84-7.75 (2H, m), 7.29 (1H, s), 4.29 (2H, d, *J* = 4.5 Hz), 2.65 (3H, s), 2.43 (3H, s), 2.27 (3H, s). |
| 1-23 | 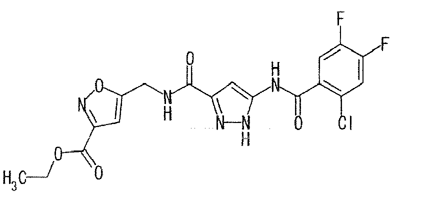 | ¹H-NMR (DMSO-*d*₆) δ: 13.33 (1 H, s), 11.19 (1H, s), 9.32 (1H, t, *J* = 5.4 Hz), 7.86-7.80 (2H, m), 7.36 (1H, s), 6.77 (1 H, s), 4.66 (2H d, *J* = 5.4 Hz), 4.36 (2H, q, *J* = 7.1 Hz), 1.31 (3H, t, *J* = 7.1 Hz). |
| 1-24 | 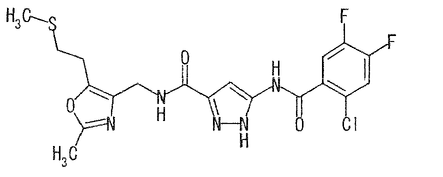 | ¹H-NMR (DMSO-*d*₆) δ: 13.20 (1H, br s), 11.11 (1H, br s), 8.99 (1H, br s), 7.86-7.79 (2H, m), 7.32 (1H, s), 4.23 (2H, d, *J* = 5.6 Hz), 2.96 (2H, t, *J* = 7.5 Hz), 2.70 (2H, t, *J* = 7.5 Hz), 2.33 (3H, s), 2.07 (3H, s). |

**Table 5**

| Example | Structural formula | **NMR** |
|---|---|---|
| 1-25 | 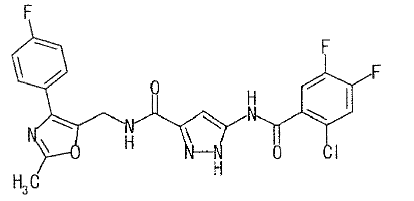 | ¹H-NMR (DMSO-*d*₆) δ: 13.30 (1H, s), 11.16 (1H, s), 9.23 (1H, s), 7.95-7.79 (4H, m), 7.36-7.26 (3H, m), 4.64 (2H, d, *J* = 5.1 Hz), 2.44 (3H, s). |
| 1-26 | 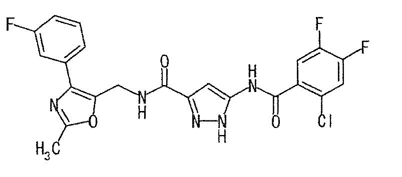 | ¹H-NMR (DMSO-*d*₆) δ: 13.29 (1H, s), 11.16 (1 H, s), 9.26 (1H, t, *J* = 5.4 Hz), 7.86-7.79 (2H, m), 7.66 (2H, m), 7.53-7.47 (1H, m), 7.37 (1H, s), 7.21-7.17 (1H, m), 4.67 (2H, d, *J* = 5.4 Hz), 2.45 (3H, s). |
| 1-27 | 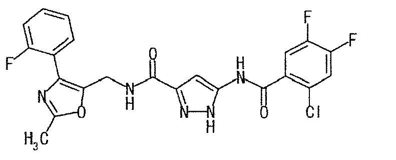 | ¹H-NMR (DMSO-*d*₆) δ: 13.25 (1H, s), 11.13 (1 H, s), 9.09 (1 H, t, *J* = 5.2 Hz), 7.85-7.78 (2H, m), 7.71-7.68 (1H, m), 7.48-7.42 (1H, m), 7.34-7.28 (3H, m), 4.53 (2H, d, *J* = 5.2 Hz), 2.44 (3H, s). |
| 1-28 | 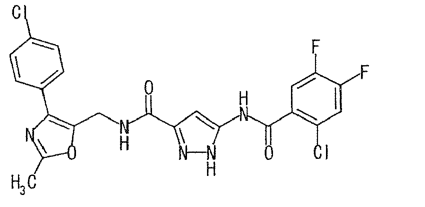 | ¹H-NMR (DMSO-*d*₆) δ: 13.30 (1H, s), 11.16 (1H, s), 9.23 (1H, s), 7.85-7.79 (4H, m), 7.52-7.50 (2H, m), 7.36 (1 H, s), 4.65 (2H, d, *J* = 5.3 Hz), 2.45 (3H, s). |
| 1-29 | 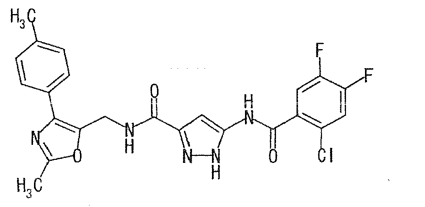 | ¹H-NMR (DMSO-*d*₆) δ: 13.26 (1H, s), 11.15 (1H, s), 9.20 (1H, t, *J* = 4.9 Hz), 7.85-7.79 (2H, m), 7.68 (2H, d, *J* = 7.5 Hz). 7.37 (1H, t, *J* =2.1 Hz), 7.26 (2H, d, *J* = 7.5 Hz), 4.65 (2H, d, *J* = 4.9 Hz), 2.42 (3H, s), 2.34 (3H, s). |
| 1-30 | 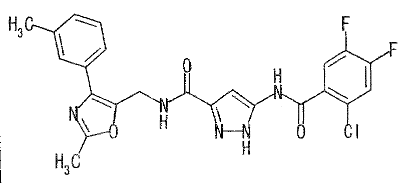 | ¹H-NMR (DMSO-*d*₆) δ: 13.27 (1H, s). 11.15 (1H, s). 9.20 (1H, t, *J* = 5.3 Hz), 7.85-7.79 (2H, m), 7.60-7.58 (2H, m), 7.37-7.31 (2H, m), 7.16 (1H, d. *J* = 7.4 Hz), 4.67 (2H, d *J* = 5.3 Hz), 2.43 (3H, s). 2.36 (3H, s). |

**Table 6**

| Example | Structural formula | **NMR** |
|---|---|---|
| 1-31 | 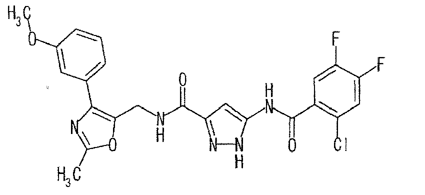 | ¹H-NMR (DMSO-*d*₆) δ: 13.29 (1H, s), 11.15 (1H, s), 9.22 (1H, t, *J* = 5.2 Hz), 7.95-7.79 (2H, m), 7.37-7.34 (4H, m), 6.94-6.91 (1H, m), 4.67 (2H, d, *J* = 5.2 Hz), 3.80 (3H, s), 2.45 (3H, s). |
| 1-32 | 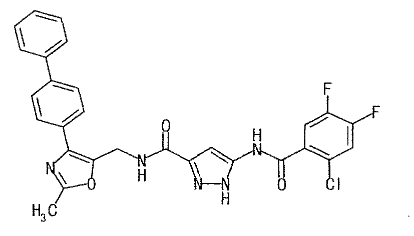 | ¹H-NMR (DMSO-*d*₆) δ: 13.31 (1H, s), 11.15 (1H, s), 9.24 (1 H, t, J = 5.3 Hz), 7.91-7.89 (2H, m), 7.85-7.70 (6H, m), 7.50-7.46 (2H, m), 7.39-7.35 (2H, m), 4.70 (2H, d, *J* = 5.3 Hz), 2.46 (3H, s). |
| 1-33 | 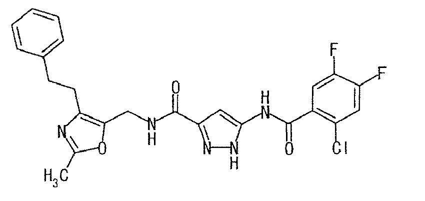 | ¹H-NMR (DMSO-*d*₆) δ: 13.25 (1H, br s), 11.13 (1H, br s), 9.02 (1H, t, *J* = 5.5 Hz), 7.85-7.78 (2H, m), 7.31-7.15 (6H, m), 4.27 (2H, d, *J* = 5.5 Hz), 2.85-2.80 (2H, m), 2.76-2.71 (2H, m), 2.35 (3H, s). |
| 1-34 | 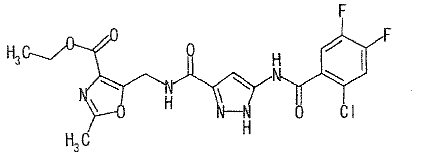 | ¹H-NMR (DMSO-*d*₆) δ: 13.28 (1H, d, *J* = 1.9 Hz), 11.15 (1H, s), 9.11 (1H, t, *J* = 5.3 Hz), 7.85-7.78 (2H, m), 7.33 (1H, d, *J* = 1.9 Hz), 4.79 (2H, d, *J* = 5.3 Hz), 4.28 (2H, q, J =7.1 Hz), 1.31 (3H, t, *J* = 7.1 Hz). |
| 1-35 | 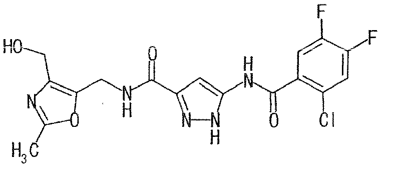 | ¹H-NMR (OMSO-*d*₆) δ: 13.25 (1H, br s), 11.14 (1H, br s), 9.04 (1H, br s), 7.86-7.79 (2H, m), 7.31 (1H, s), 4.98 (1H, br s), 4.47 (2H, d, *J* = 5.6 Hz), 4.36 (2H, s), 2.34 (3H, s). |
| 1-36 | 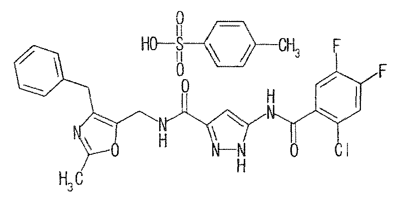 | ¹H-NMR (DMSO-*d*₆) δ: 11.16 (1H, s), 9.09 (1H, t, *J* = 5.3 Hz), 7.88-7.78 (2H, m), 7.48 (2H, d. *J* = 8.2 Hz), 7.27-7.10 (8H, m), 4.49 (2H, d, *J* = 5.3 Hz), 3.83 (2H, s), 2.32 (3H, s), 2.29 (3H, s). |

**Table 7**

| Example | Structural formula | **NMR** |
|---|---|---|
| 1-37 | 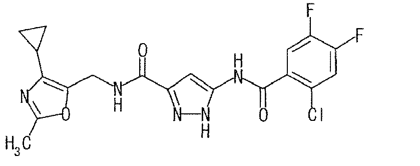 | ¹H-NMR (DMSO-*d*₆) δ: 13.26 (1H, s), 11.15 (1H, s), 9.09 (1H, t, *J* = 5.3 Hz), 7.86-7.80 (2H, m), 7.34 (1 H, s), 4.48 (2H, d, *J* = 5.3 Hz), 2.31 (3H, s), 1.98-1.92 (1H, m), 0.83-0.76 (2H, m), 0.75-0.69 (2H, m). |
| 1-38 | 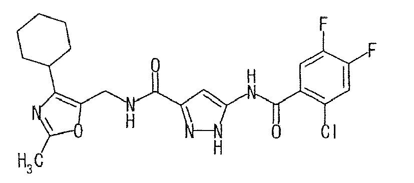 | ¹H-NMR (DMSO-*d*₆) δ: 13.25 (1H, d, *J* = 2.1 Hz), 11.13 (1H, br s), 9.03 (1H, t, *J* = 5.6 Hz), 7.85-7.79 (2H, m), 7.33 (1H, d, *J* = 2.1 Hz), 4.40 (2H, d, *J* = 5.6 Hz), 2.66-2.59 (1H, m), 2.33 (3H, s), 1.77-1.65 (5H, m), 1.51-1.43 (2H, m), 1.35-1.14 (3H, m). |
| 1-39 | 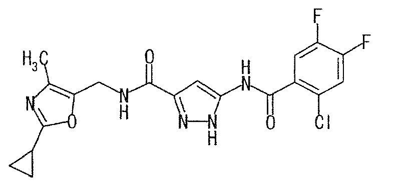 | ¹H-NMR (DMSO-*d*₆) δ: 13.26 (1H, s), 11.15 (1H, s), 9.07-9.02 (1H, m), 7.86-7.80 (2H, m), 7.32 (1H, d, *J* = 1.6 Hz), 4.41-4.37 (2H, m). 3.72 (1H, t, *J* = 6.5 Hz), 2.81 (1 H, t, *J* = 7.3 Hz), 2.13-1.97 (4H, m), 1.00-0.91 (1H, m), 0.88-0.84 (1H, m). |
| 1-40 | 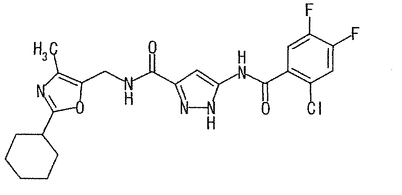 | ¹H-NMR (DMSO-*d*₆) δ: 13.24 (1H, d, *J* = 2.0 Hz), 11.12 (1H, br s), 9.02 (1H, t, *J* = 5.5 Hz), 7.85-7.78 (2H, m), 7.32 (1H, d, *J* = 2.0 Hz), 4.40 (2H, d, *J* = 5.5 Hz), 2.73-2.66 (1H, m), 2.08 (3H, s), 1.94-1.90 (2H, m), 1.73-1.68 (2H, m), 1.65-1.60 (1H, m), 1.50-1.16 (5H, m). |
| 1-41 | 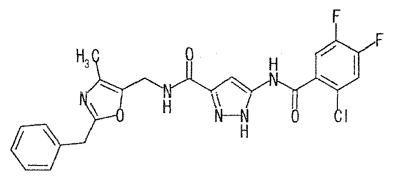 | ¹H-NMR (DMSO-*d*₆) δ: 13.25 (1H, s), 11.13 (1H, s), 9.05 (1H, t *J* = 5.6 Hz), 7.86-7.77 (2H, m), 7.34-7.20 (6H, m), 4.38 (2H, d, *J* = 5.6 Hz), 4.03 (2H, s), 2.08 (3H, s). |
| 1-42 | 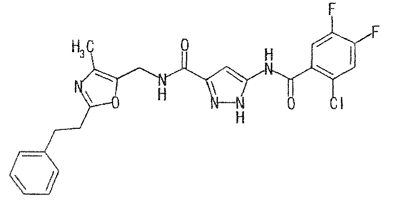 | ¹H-NMR (DMSO-*d*₆) δ: 13.25 (1H, br s), 11.14 (1H, br s), 9.04 (1 H, br s), 7.85-7.79 (2H, m), 7.32 (1H, br s), 7.26-7.13 (5H, m), 4.40 (2H, d, *J* = 5.5 Hz), 2.96 (4H, s), 2.09 (3H, s). |

**Table 8**

| Example | Structural formula | **NMR** |
|---|---|---|
| 1-43 | 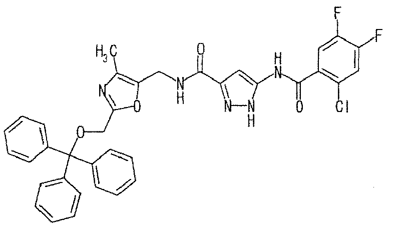 | ¹H-NMR (DMSO-*d*₆) δ: 13.30 (1H, br s), 11.16 (1H, br s), 9.13 (1H, t, *J* = 5.6 Hz), 7.86-7.79 (2H, m), 7.42-7.26 (16H, m). 4.46 (2H, d, *J* = 5.6 Hz), 3.99 (2H, s), 2.13 (3H, s). |
| 1-44 | 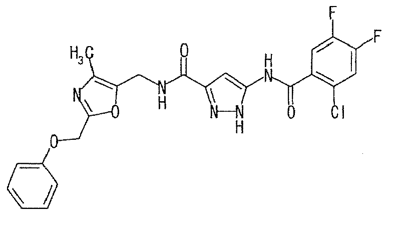 | ¹H-NMR (DMSO-*d*₆) δ: 13.28 (1H, br s), 11.15 (1 H, br s), 9.13 (1H, t, *J* = 5.6 Hz), 7.86-7.79 (2H, m), 7.32-7.27 (3H, m), 7.03-6.95 (3H, m), 5.12 (2H, s), 4.46 (2H, d, *J* = 5.6 Hz), 2.15 (3H, s). |
| 1-45 | 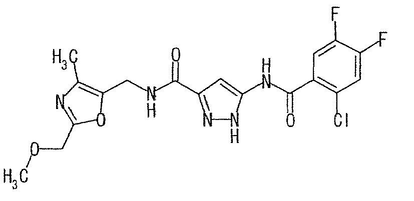 | ¹H-NMR (DMSO-*d*₆) δ: 13.27 (1H, br s), 11.15 (1H, br s), 9.11 (1H, t, *J* = 5.6 Hz), 7.86-7.79 (2H, m), 7.32 (1H, d, *J* = 1.9 Hz), 4.44 (2H, d, *J* = 5.6 Hz), 4.39 (2H, s), 3.29 (3H, s), 2.13 (3H, s). |
| 1-46 | 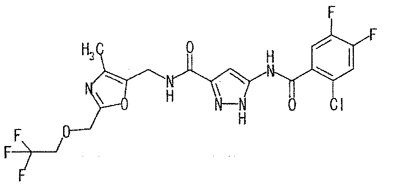 | ¹H-NMR (DMSO-*d*₆) δ: 13.27 (1H, d, *J* = 2.1 Hz), 11.15 (1H, br s), 9.11 (1H, t, *J* = 5.6 Hz), 7.85-7.79 (2H, m), 7.32 (1H, d, *J* = 2.1 Hz), 4.68 (2H, s), 4.45 (2H, d, *J* = 5.6 Hz), 4.18 (2H, q, *J* = 9.2 Hz), 2.15 (3H, s). |
| 1-47 | 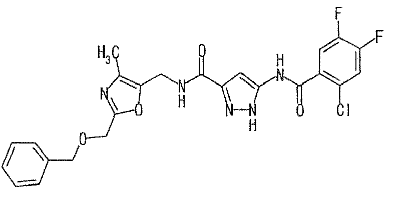 | ¹H-NMR (DMSO-*d*₆) δ: 13.28 (1H, br s), 11.15 (1H, br s), 9.12 (1H, t, *J* = 5.6 Hz), 7.86-7.79 (2H, m), 7.36-7.27 (6H, m), 4.53 (2H, s), 4.51 (2H, s), 4.45 (2H, d, *J* = 5.6 Hz), 2.14 (3H, s). |

**Table 9**

| | | |
|---|---|---|
| Example | Structural formula | **NMR** |
| 1-48 | 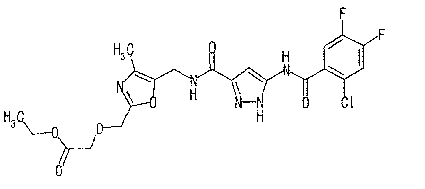 | ¹H-NMR (DMSO-*d*₆) δ: 13.27 (1H, d, *J* = 2.1 Hz), 11.14 (1H, br s), 9.11 (1H, t, *J* = 5.6 Hz). 7.86-7.79 (2H, m), 7.32 (1H, d, *J* = 2.1 Hz), 4.56 (2H, s), 4.44 (2H, d, J = 5.6 Hz), 4.19 (2H, s), 4.11 (2H, q, *J* = 7.1 Hz), 2.13 (3H, s), 1.18 (3H, t, *J* = 7.1 Hz). |
| 1-49 | 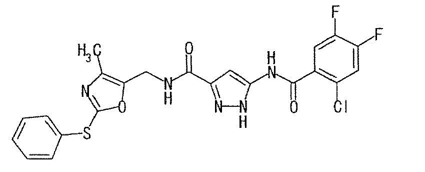 | ¹H-NMR (DMSO-*d*₆) δ: 13.27 (1H, br s), 11.14 (1H, br s), 9.08 (1H, t, *J* = 5.3 Hz), 7.85-7.79 (2H, m), 7.51-7.48 (2H, m), 7.44-7.39 (3H, m), 7.31 (1H, s), 4.42 (2H, d, *J* = 5.3 Hz), 2.13 (3H, s). |
| 1-50 | 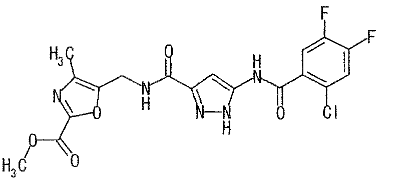 | ¹H-NMR (DMSO-*d*₆) δ: 13.29 (1H, s). 11.15 (1H, s). 9.19 (1H, br s). 7.82 (2.H, m), 7.31 (1H, s), 4.51 (2H, d, *J* = 5.3 Hz), 3.86 (3H, s), 2.21 (3H, s). |
| 1-51 | 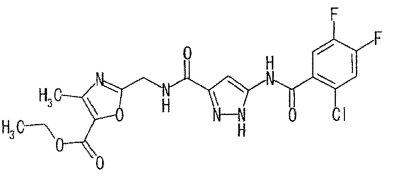 | ¹H-NMR (DMSO-*d*₆) δ: 13.31 (1H, br s), 11.18 (1H, br s), 9.35 (1H, t, *J* = 5.7 Hz), 7.86-7.80 (2H, m), 7.38 (1H, d, *J* = 1.9 Hz), 4.57 (2H, d, *J* = 5.7 Hz), 4.30 (2H, q, *J* = 7.1 Hz), 2.37 (3H, s), 1.29 (3H, t, *J* = 7.1 Hz). |
| 1-52 | 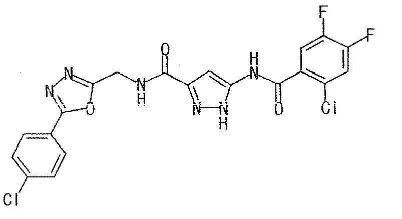 | ¹H-NMR (DMSO-*d*₆) δ: 13.34 (1H, s), 11.19 (1H, s), 9.42 (1H, t, *J* = 5.5 Hz), 8.01-7.98 (2H, m). 7.86-7.80 (2H, m). 7.70-7.67 (2H, m), 7.39 (1H, d, *J* = 2.1 Hz), 4.75 (2H, d, *J* = 5.5 Hz). |
| 1-53 | 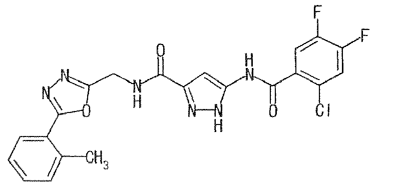 | ¹H-NMR (DMSO-*d*₆) δ: 13.33 (1H, s), 11.18 (1H, s), 9.40 (1H, t, *J* = 5.6 Hz), 7.86-7.80 (3H, m), 7.53-7.39 (4H, m), 4.77 (2H, d, *J* = 5.6 Hz), 2.59 (3.7H, s). |

**Table 10**

| Example | Structural formula | **NMR** |
|---|---|---|
| 1-54 | 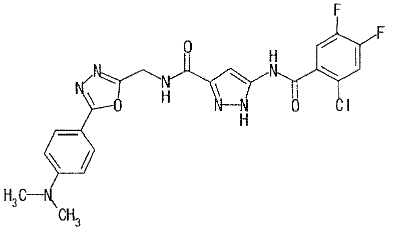 | ¹H-NMR (DMSO-*d*₆) δ: 13.36 (1H, s), 11.19 (1H, s), 9.37 (1H, s), 7.87-7.83 (2H, m), 7.76 (2H, d, *J* = 8.9 Hz), 7.39 (1H, s), 6.83 (2H, d, *J* = 8.9 Hz), 4.72 (2H, d, *J* = 5.6 Hz), 3.01 (6H, s). |
| 1-55 | 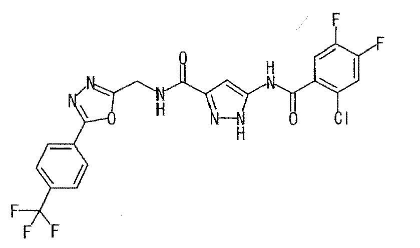 | ¹H-NMR (OMSO-*d*₆) δ: 13.35 (1H, s), 11.19 (1 H, s), 9.44 (1 H, t, *J* = 5.2 Hz), 8.20 (2H, d, *J* = 8.2 Hz), 7.98 (2H, d, *J* = 8.2 Hz), 7.86-7.80 (2H, m), 7.40 (1H, s), 4.81 (2H, d, *J* = 5.2 Hz). |
| 1-56 | 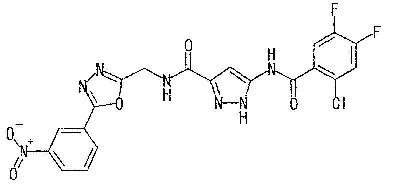 | ¹H-NMR (DMSO-*d*₆) δ: 13.37 (1H, s), 11.19 (1H, s), 9.45 (1H, t, *J* = 5.3 Hz), 8.66 (1H, s), 8.48-8.40 (2H, m), 7.95-7.80 (3H, m), 7.40 (1H, s), 4.82 (2H, d, *J* = 5.3 Hz). |
| 1-57 | 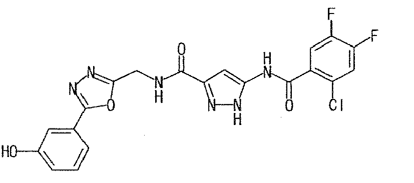 | ¹H-NMR (DMSO-*d*₆) δ: 13.34 (1H, s), 11.19 (1H, s), 9.98 (1H, s), 9.41 (1H, t, *J* = 5.4 Hz), 7.86-7.80 (2H, m), 7.40-7.36 (4H, m), 7.02-6.99 (1H, m), 4.76 (2H, d, *J* = 5.4 Hz). |
| 1-58 | 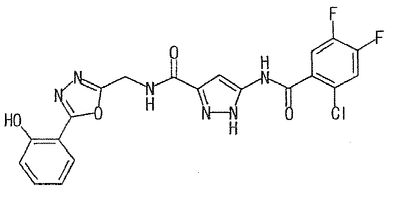 | ¹H-NMR (DMSO-*d*₆) δ: 13.37 (1H, s), 11.20 (1H, s), 10.26 (1H, s), 9.43 (1H, t, *J* = 4.9 Hz), 7.92-7.81 (2H, m). 7.75-7.73 (1H, m), 7.48-7.44 (1H, m), 7.40 (1H, s), 7.09-7.07 (1H, m), 7.03-6.99 (1H, m), 4.78 (2H, d, *J* = 4.9 Hz). |

**Table 11**

| Example | Structural formula | **NMR** |
|---|---|---|
| 1-59 | 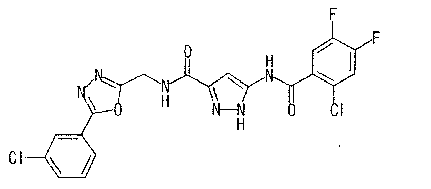 | ¹H-NMR (DMSO-*d*₆) δ: 13.37 (1H, s), 11.20 (1H, s). 9.42 (1H, t, *J* = 5.3 Hz), 7.98-7.94 (2H, m), 7.86-7.80 (2H, m), 7.73-7.71 (1H, m), 7.66-7.63 (1H, m), 7.39 (1H, s), 4.78 (2H, d, *J* = 5.3 Hz). |
| 1-60 | 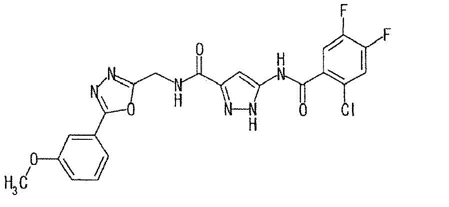 | ¹H-NMR (DMSO-*d*₆) δ: 13.36 (1H, s), 11.19 (1H, s), 9.42 (1H, t, *J* = 4.8 Hz), 7.93-7.80 (2H, m), 7.56-7.41 (3H, m), 7.39 (1H, s), 7.22-7.20 (1H, m), 4.77 (2H, d, *J* = 4.8 Hz), 3.84 (3H, d, *J* = 2.1 Hz). |
| 1-61 | 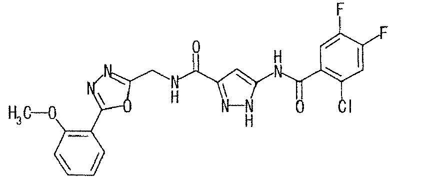 | ¹H-NMR (OMSO-*d*₆) δ: 13.36 (1H, s), 11.19 (1H, s), 9.40 (1H, brs), 7.95-7.77 (3H, m), 7.62-7.58 (1H, m), 7.39 (1H, s), 7.27-7.24 (1 H, m), 7.14-7.11 (1 H, m), 4.75 (2H, d, *J* = 3.1 Hz), 3.85 (3H, d, *J* = 1.2 Hz). |
| 1-62 | 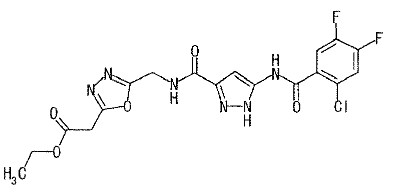 | ¹H-NMR (DMSO-*d*₆) δ: 13.32 (1H, s). 11.18 (1H, s), 9.37 (1H, t, *J* = 5.8 Hz), 7.95-7.80 (2H, m), 7.36 (1H, d, *J* = 1.86 Hz), 4.69 (2H, d, *J* = 5.8 Hz), 4.18 (2H, s), 4.13 (2H, q, *J* = 7.1 Hz), 1.18 (3H, t, *J* = 7.1 Hz). |
| 1-63 | 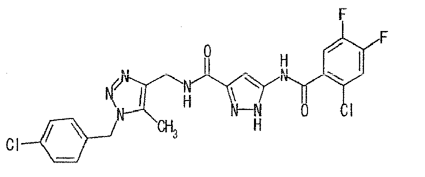 | ¹H-NMR (OMSO-*d*₆) δ: 13.21 (1H, br s), 11.12 (1H, br s). 9.04 (1H, br s), 7.86-7.79 (2H, m), 7.43 (2H, d, *J* = 8.3 Hz), 7.30 (1H, br s), 7.22 (2H, d, *J* = 8.3 Hz), 5.55 (2H, s), 4.43 (2H, d, *J* = 5.3 Hz), 2.24 (3H, s). |
| 1-64 | 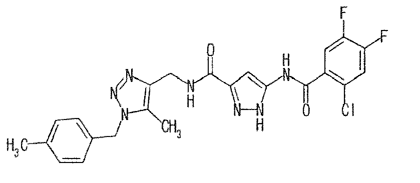 | ¹H-NMR (DMSO-*d*₆) δ: 13.20 (1H, br s), 11.11 (1H, br s), 9.05 (1H, br s), 7.85-7.78 (2H, m), 7.31 (1H, s), 7,16 (2H, d, *J* = 7.9 Hz), 7.10 (2H, d, *J* = 7.9 Hz), 5.48 (2H, s), 4.42 (2H, d, *J* = 5.6 Hz), 2.27 (3H, s), 2.23 (3H, s). |

**Table 12**

| Example | Structural formula | **NMR** |
|---|---|---|
| 1-65 | 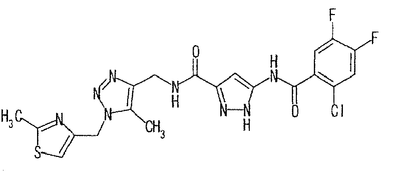 | ¹H-NMR (DMSO-*d*₆) δ: 13.21 (1H, br s), 11.12 (1H, br s), 9.06 (1H, t, *J* = 5.1 Hz), 7.85-7.79 (2H, m), 7.40 (1H, s), 7.31 (1H, s), 5.54 (2H, s), 4.43 (2H, d, *J* = 5.1 Hz), 2.61 (3H, s), 2.35 (3H, s). |
| 1-66 | 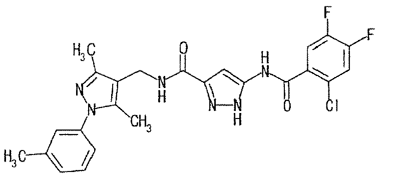 | ¹H-NMR (DMSO-*d*₆) δ: 13.21 (1H, br s), 11.13 (1H, br s), 8.83 (1H, br s), 7.86-7.78 (2H, m), 7.39-7.17 (5H, m), 4.28 (2H, d, *J* = 4.9 Hz), 2.37 (3H, s), 2.32 (3H, s), 2.24 (3H, s). |
| 1-67 | 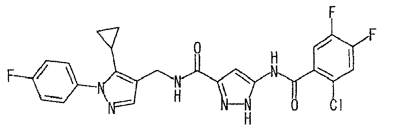 | ¹H-NMR (DMSO-*d*₆) δ: 13.15 (1H, br s), 11.15 (1H, br s), 8.89 (1H, br s), 7.86-7.80 (2H, m), 7.65-7.57 (3H, m), 7.36-7.32 (3H, m), 4.39 (2H, s), 2.00-1.93 (1H, m), 0.85-0.80 (2H, m), 0.50-0.48 (2H, m). |
| 1-68 | 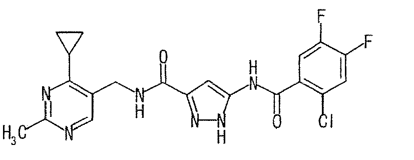 | ¹H-NMR (DMSO-*d*₆) δ: 13.26 (1 H, br s), 11.17 (1H, br s), 9.09 (1H, br s), 8.41 (1H, s), 7.86-7.79 (2H, m), 7.33 (1H, s), 4.55 (2H, d, *J* = 4.9 Hz), 2.34-2.29 (1H, br m), 1.08-0.99 (4H, m). |
| 1-69 | 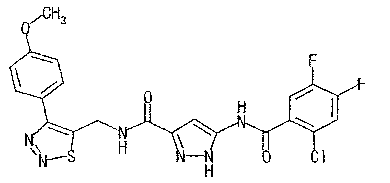 | ¹H-NMR (DMSO-*d*₆) δ: 13.40 (1 H, br s), 11.20 (1H, br s), 9.58 (1 H, t, *J* = 5.7 Hz), 7.87-7.79 (4H, m), 7.34 (1H, br s), 7.16 (2H, d, *J* = 8.7 Hz), 4.83 (2H, d, *J* = 5.7 Hz), 3.86 (3H, s). |
| 1-70 | 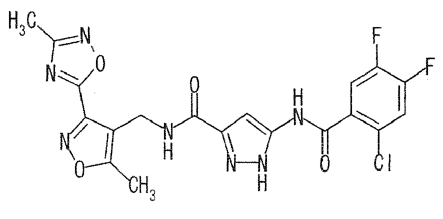 | ¹H-NMR (DMSO-*d*₆) δ: 13.25 (1H, s), 11.14 (1 H, s), 8.84 (1 H, t, *J* = 4.9 Hz), 7.86-7.76 (2H, m), 7.24 (1H, s), 4.57 (2H, d, *J* = 4.9 Hz). 2.57 (3H, s). 2.48 (3H, s). |

**Table 13**

| Example | Structural formula | **NMR** |
|---|---|---|
| 1-71 | 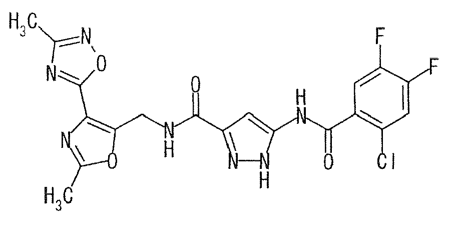 | ¹H-NMR (DMSO-*d*₆) δ: 13.30 (1H, s), 11.16 (1H, s), 9.19 (1H, t, *J* = 5.3 Hz). 7.86-7.78 (2H, m), 7.32 (1H, d, *J* = 1.9 Hz). 4.89 (2H, d, *J* = 5.3 Hz), 2.43 (3H, s). |
| 1-72 | 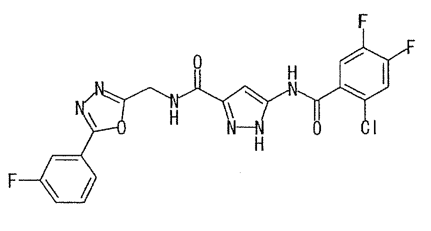 | ¹H-NMR (DMSO-*d*₆) δ: 13.38 (1H, s), 11.21 (1H, s), 9.43-9.42 (1H, br m), 7.88-7.40 (7H, m), 4.80-4.78 (2H, br m). |
| 1-73 | 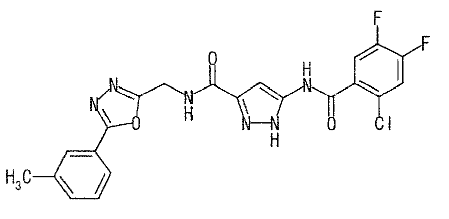 | ¹H-NMR (DMSO-*d*₆) δ: 13.36 (1H, s), 11.19 (1H, s), 9.43-9.40 (1H, m), 7.84-7.77 (4H, m), 7.51-7.38 (3H, m), 4.78-4.76 (2H, m), 2.40 (3H, d, *J* = 3.2 Hz). |
| 1-74 | 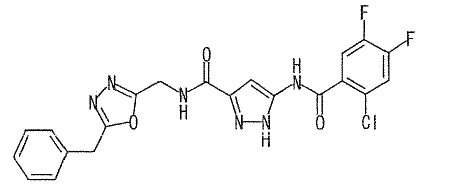 | ¹H-NMR (DMSO-*d*₆) δ: 13.34 (1H, s), 11.18 (1H, s), 9.33 (1H, t. *J* = 5.7 Hz), 7.86-7.80 (2H, m), 7.37-7.26 (6H, m), 4.64 (2H, d, *J* = 5.7 Hz), 4.27 (2H, s). |
| 1-75 | 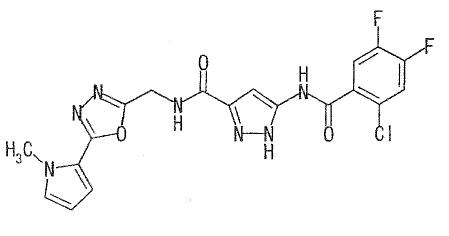 | ¹H-NMR (DMSO-*d*₆) δ: 13.35 (1H, d, *J* = 2.3 Hz), 11.19 (1H, s). 9.39 (1H, t. *J* = 5.6 Hz), 7.86-7.80 (2H, m), 7.38 (1H, d, *J* = 2.3 Hz), 7.15 (1H, dd, *J* = 2.6, 1.9 Hz), 6.77 (1H, dd, *J* = 3.9, 1.9 Hz), 6.20 (1H. dd, *J* = 3.9, 2.6 Hz), 4.72 (2H, d, *J* = 5.6 Hz), 3.95 (3H, s). |

**Table 14**

| Example | Structural formula | ^{NMR} |
|---|---|---|
| 7 -76 | 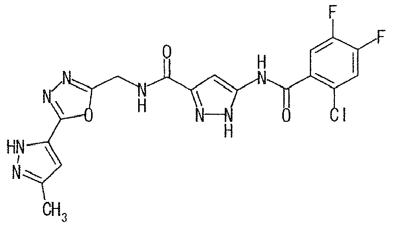 | ¹H-NMR (DMSO-*d*₆) δ: 13.36 (1H, d, *J* = 2.1 Hz), 13.29 (1H, s), 11.19 (1H, s), 9.40 (1H, t, *J* = 5.7 Hz), 7.86-7.80 (2H, m), 7.38 (1H, d, *J* = 2.1 Hz), 6.61 (1H, s), 4.74 (2H, d, *J* = 5.7 Hz), 2.31 (3H, s). |
| 1-77 | 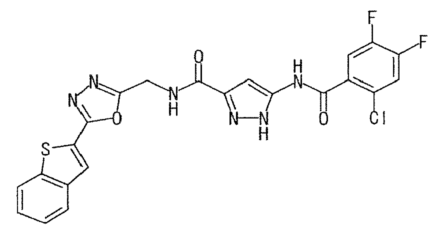 | ¹H-NMR (DMSO-*d*₆) δ: 13.39 (1H, d, *J* = 2.3 Hz), 11.21 (1H, s), 9.45 (1H, t, *J* = 5.6 Hz), 8.20 (1H, s), 8.13-8.11 (1H, m), 8.05-8.03 (1H, m), 7.87-7.81 (2H, m), 7.55-7.47 (2H, m), 7.41 (1H, d, *J* = 2.3 Hz), 4.80 (2H, d, *J* = 5.6 Hz). |
| 1-78 | 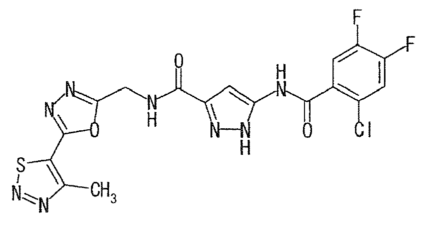 | ¹H-NMR (DMSO-*d*₆) δ: 13.37 (1H, d, *J* = 2.2 Hz), 11.19 (1H, s), 9.45 (1H, t, *J* = 5.7 Hz), 7.86-7.80 (2H, m), 7.39 (1H, d, *J* = 2.2 Hz), 4.81 (2H, d, *J* = 5.7 Hz), 2.98 (3H, s). |
| 1-79 | 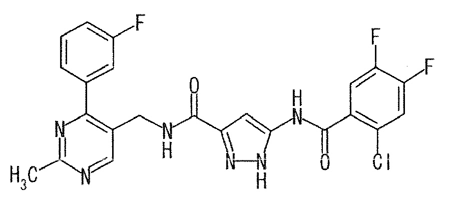 | ¹H-NMR (DMSO-*d*₆) δ: 13.25 (1H, s), 11.16 (1H, s), 9.10 (1H, t, *J* = 5.2 Hz), 8.77 (1H, s), 7.86-7.79 (2H, m), 7.60-7.49 (3H, m), 7.39-7.31 (2H, m), 4.50 (2H, d, *J* = 5.2 Hz), 2.66 (3H, s) |
| 1-80 | 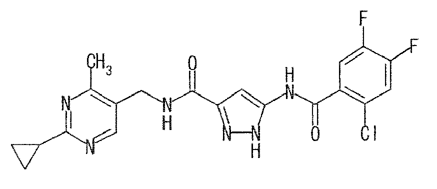 | ¹H-NMR (DMSO-*d₆*) δ: 13,27 (1H, d, *J* = 2.1 Hz), 11.17 (1H, s), 9.01 (1H, t, *J* = 5.3 Hz), 8.42 (1H, s), 7.86-7.79 (2H, m), 7.33 (1H, d, *J* = 2.1 Hz), 4.41 (2H, d, *J* = 5.3 Hz), 2.45 (3H, s). 2.17-2.10 (1H, m), 1.01-0.93 (4H, m). |

**Table 15**

| Example | Structural formula | **NMR** |
|---|---|---|
| 1-81 | 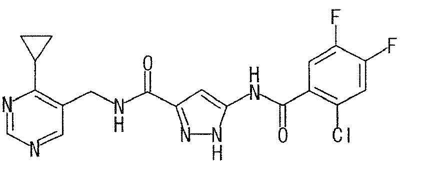 | ¹H-NMR (DMSO-*d₆*) δ: 13.29 (1H, br s), 11.19 (1H, s), 9.15 (1H, t, *J* = 5.6 Hz), 8.88 (1H, s), 8.53 (1H. s), 7.86-7.80 (2H, m), 7.35 (1H, d, *J* = 1.4 Hz), 4.61 (2H, d, *J* = 5.6 Hz), 2.39-2.33 (1H, m), 1.11-1.04 (4H, m). |
| 1-82 | 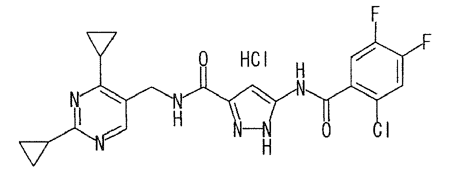 | ¹H-NMR (DMSO*-d₆*) δ: 11.19 (1H, s), 9.10 (1H, t, *J* = 5.3 Hz), 8.50 (1H, s), 7.86-7.79 (2H, m), 7.22 (1H, s), 4.58 (2H, d, *J* = 5.3 Hz), 2.45-2.39 (1H, m), 2.27-2.21 (1H, m), 1.15-1.02 (8H, m). |
| 1-83 | 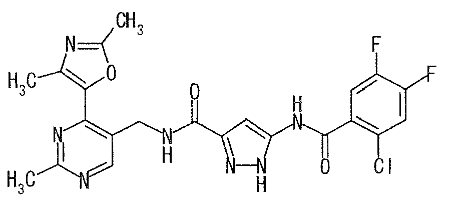 | ¹H-NMR (DMSO-*d₆*) δ: 11.20 (1H, s), 8.95-8.92 (1H, br m), 8.65 (1H, s), 7.86-7.79 (2H, m), 7.22 (1H, s), 4.70 (2H, d, *J* = 5.1 Hz), 2.63 (3H, s), 2.51 (3H, s), 2.45 (3H, s). |
| 1-84 | 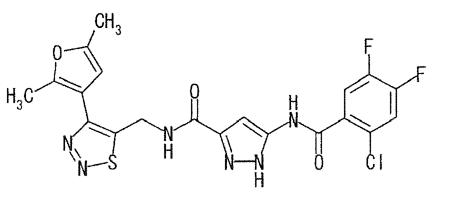 | ¹H-NMR (DMSO-*d*₆) δ: 13.41 (1H, d, *J* = 2.3 Hz), 11.21 (1H, s), 9.53 (1H, t, *J* = 5.7 Hz), 7.87-7.79 (2H, m), 7.34 (1H, d, *J* = 2.3 Hz), 6.54 (1H, s), 4.75 (2H, d, *J* = 5.7 Hz), 2.42 (3H, s), 2.33 (3H, s). |
| 1-85 | 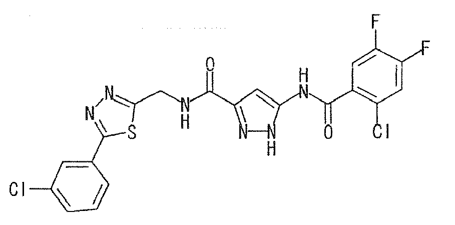 | ¹-NMR (DMSO*-d*₆) δ: 13.40 (1H, s), 11.20 (1H, s), 9.64 (1H, t, *J* = 5.8 Hz), 8.04-8.02 (1H, br m), 7.95-7.80 (3H, m), 7.65-7.55 (2H, m), 7.39 (1H, s), 4.89 (2H, d, *J* = 5.8 Hz). |
| 1-86 | 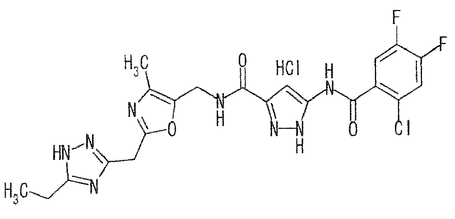 | ¹H-NMR (DMSO-D₆) δ: 11. 18 (1.0H. s), 9. 09 (1.0H, m), 7.87-7.80 (2.0H, m), 7.24 (1.0H, s), 4.41 (2.0H, d, *J* = 5.51 Hz), 4. 26((2. 0H, s), 2. 80 (2. 0H q, *J* = 7. 60 Hz), 2.11 (3.0H, s). |

**Table 16**

| Example | Structural formula | **NMR** |
|---|---|---|
| 1-87 | 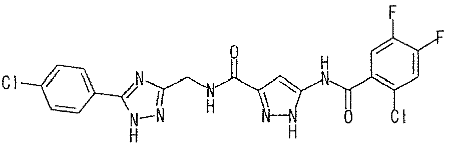 | ¹H-NMR (DMSO-D₆) δ: 14.35(0.3H, s). 14.06 (0.7H, s), 13.30(0.7H, s). 13.24 (0.3H, s,), 11.17 (1.0H, s). 9.29 (0.7H, s), 9.17(0.3H, s), 8.01-7.39 (7.0H, m),, 4.62-4.53 (2.0H, m). |
| 1-88 | 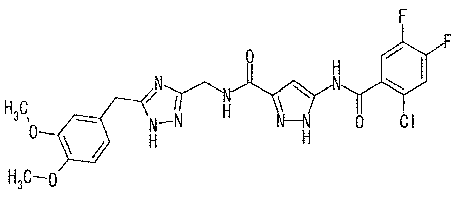 | ¹H-NMR (CDCl₃) δ: 13.48 (1.0H, s), 11.43 (1.0H, s), 9.83(1H,s), 7.40-6.81 (7.0H, m), 4.42(2.0H, m), 4.18 (2.0H, s), 3.87(3.0H, s), 3.85(3.0H, s). |
| 2-1 | 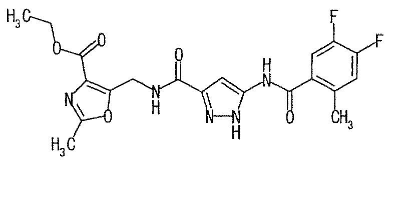 | ¹H-NMR (DMSO-*d*₆) δ: 13.25 (0.9H, br s), 13.19 (0.1H, br s), 11.15 (0.1H, br s), 10.95 (0.9H, br s), 9.10 (0.9H, t, *J* = 5.3 Hz), 8.60 (0.1H, t, *J* = 5.3 Hz), 7.71-7.66 (0.1H, m), 7.60-7.55 (0.9H, m), 7.48-7.43 (0.1H, m), 7.42-7.37 (0.9H, m), 7.35 (0.9H, s), 6.41 (0.1H, s), 4.79 (1.8H, d, *J* = 5.3 Hz), 4.76 (0.2H, d, *J* = 5.3 Hz), 4.29 (2H, q, *J* = 7.1 Hz), 2.42 (3H, s), 2.35 (3H, s), 1.30 (3H, t, J = 7.1 Hz). |

Subsequently, the following compounds were produced.

### Example 3-1

### Production of 5-(2-chloro-4,5-difluoro-benzoylamino)-1H-pyrazole-3-carboxylic acid (2-carboxy-furan-5-ylmethyl)-amide:

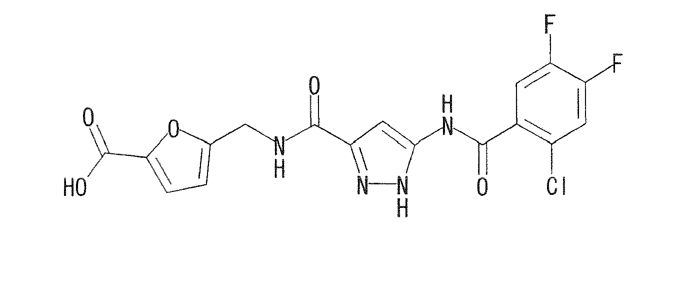

To a solution of 5-(2-chloro-4,5-difluoro-benzoylamino)-1H-pyrazole-3-carboxylic acid (2-ethoxycarbonyl-furan-5-ylmethyl)-amide (475 mg) produced in Example 1-1 in ethanol (10 mL) was added 4 N sodium hydroxide aqueous solution (2.6 mL). After stirring overnight at room temperature, 1 N hydrochloric acid (6 mL) was added to the reaction mixture. Ethanol was distilled off under reduced pressure, and the precipitated solid was collected by filtration to obtain the title compound (466 mg) as a white solid.

### Example 3-2

### Production of sodium 5-({[5-(2-chloro-4,5-difluoro-benzoylamino)-1H-pyrazole-3-carbonyl]-amino}-methyl)-4-methyl-thiazole-2-carboxylate:

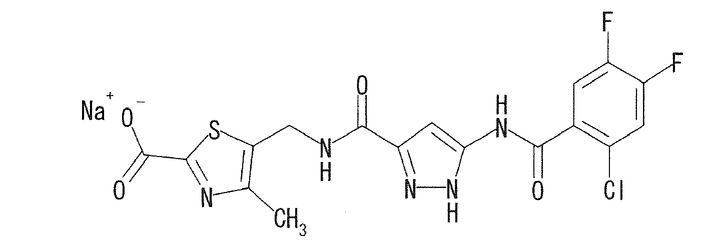

To a solution of 5-(2-chloro-4,5-difluoro-benzoylamino)-1H-pyrazole-3-carboxylic acid (2-ethoxycarbonyl-4-methyl-thiazol-5-ylmethyl)-amide (78 mg) produced in Example 1-10 in methanol (5 mL) was added 4 N sodium hydroxide aqueous solution (0.120 mL). After stirring overnight at room temperature, water was added to the reaction mixture, and pH was adjusted to 4 to 5 with 1 N hydrochloric acid. The precipitated solid was collected by filtration to obtain a colorless solid (41 mg).

The resulting solid (32 mg) was suspended in water and dissolved in sodium hydrogencarbonate (5.6 mg) and tetrahydrofuran. The reaction mixture was washed with ethyl acetate, and the aqueous layer was concentrated. 2-Propanol was added to the residue, and the precipitated solid was collected by filtration to obtain the title compound (22 mg) as a colorless solid.

### Example 3-3

### Production of 5-(2-chloro-4,5-difluoro-benzoylamino)-1H-pyrazole-3-carboxylic acid (3-carboxy-isoxazol-5-ylmethyl)-amide:

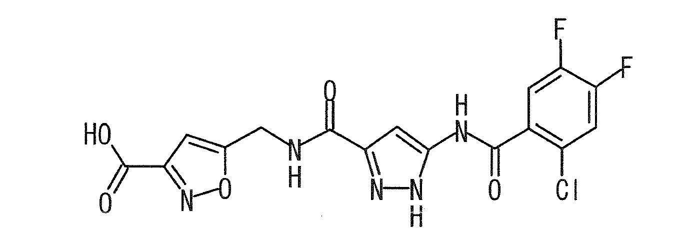

To a solution of 5-(2-chloro-4,5-difluoro-benzoylamino)-1H-pyrazole-3-carboxylic acid (3-ethoxycarbonylisoxazol-5-ylmethyl)-amide (255 mg) produced in Example 1-23 in methanol (2.5 mL) was added 2 N sodium hydroxide aqueous solution (0.56 mL) with ice cooling. After stirring at room temperature for 1 hour, 1 N hydrochloric acid was added dropwise. Water was added to the resulting suspension, the mixture was stirred at room temperature, and the precipitated solid was collected by filtration and dried at 60°C under reduced pressure to obtain the title compound (222 mg) as a white solid.

### Example 3-4

### Production of 5-(2-chloro-4,5-difluoro-benzoylamino)-1H-pyrazole-3-carboxylic acid (4-acetoxymethyl-2-methyl-oxazol-5-ylmethyl)-amide:

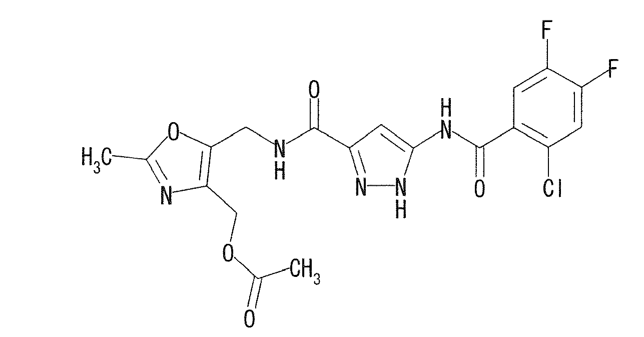

To a solution of 5-(2-chloro-4,5-difluoro-benzoylamino) -1H-pyrazole-3-carboxylic acid (4-hydroxymethyl-2-methyl-oxazol-5-ylmethyl)-amide (100 mg) produced in Example 1-35 in N-methylpyrrolidone (3 mL) was added triethylamine (0.050 mL) followed by addition of acetic anhydride (0.0243 mL) with ice cooling. After stirring overnight at room temperature, water was added to the reaction mixture, and the mixture was extracted with ethyl acetate. The organic layer was successively washed with water and saturated brine, dried over anhydrous sodium sulfate, and then concentrated. The residue was purified by silica gel column chromatography (hexane/ethyl acetate, 1:3 → 1:4) to obtain the title compound (92 mg) as a colorless solid.

### Example 3-5

### Production of 5-(2-chloro-4,5-difluoro-benzoylamino)-1H-pyrazole-3-carboxylic acid (4-benzoyloxymethyl-2-methyl-oxazol-5-ylmethyl)-amide:

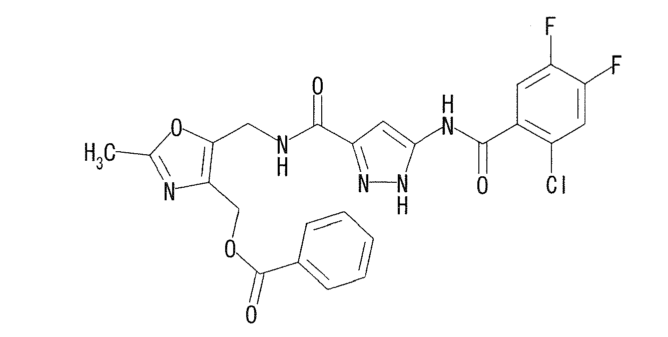

In the same manner as in Example 3-4, the title compound (89 mg) was obtained from 5-(2-chloro-4,5-difluoro-benzoylamino)-1H-pyrazole-3-carboxylic acid (4-hydroxymethyl-2-methyl-oxazol-5-ylmethyl)-amide (200 mg) produced in Example 1-35 and benzoyl chloride (0.060 mL) as a colorless solid.

### Example 3-6

### Production of 5-(2-chloro-4,5-difluoro-benzoylamino)-1H-pyrazole-3-carboxylic acid (4-carboxy-2-methyl-oxazol-5-ylmethyl)-amide:

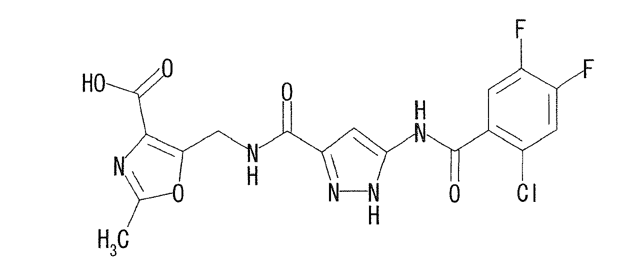

In the same manner as in Example 3-1, the title compound (180 mg) was obtained from 5-(2-chloro-4,5-difluoro-benzoylamino)-1H-pyrazole-3-carboxylic acid (4-ethoxycarbonyl-2-methyl-oxazol-5-ylmethyl)-amide (221 mg) produced in Example 1-34 as a white solid.

### Example 3-7

### Production of 5-(2-chloro-4,5-difluoro-benzoylamino)-1H-pyrazole-3-carboxylic acid (2-carboxymethoxymethyl-4-methyl-oxazol-5-ylmethyl)-amide:

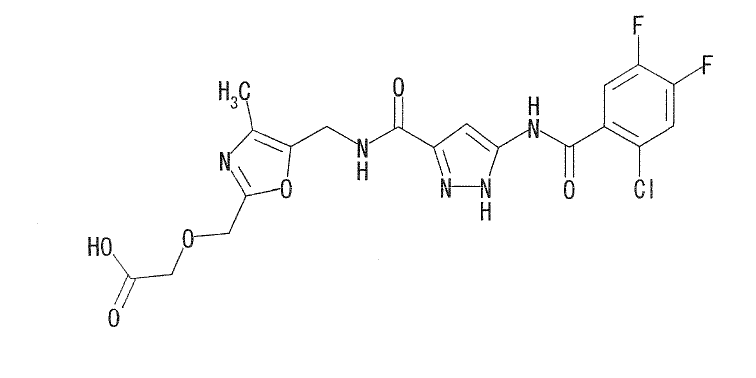

In the same manner as in Example 3-1, the title compound (685 mg) was obtained from 5-(2-chloro-4,5-difluoro-benzoylamino)-1H-pyrazole-3-carboxylic acid (2-ethoxycarbonylmethoxymethyl-4-methyl-oxazol-5-ylmethyl)-amide (698 mg) produced in Example 1-48 as a white solid.

### Example 3-8

### Production of 5-(2-chloro-4,5-difluoro-benzoylamino)-1H-pyrazole-3-carboxylic acid (2-diethylcarbamoyl methoxymethyl-4-methyl-oxazol-5-ylmethyl)-amide:

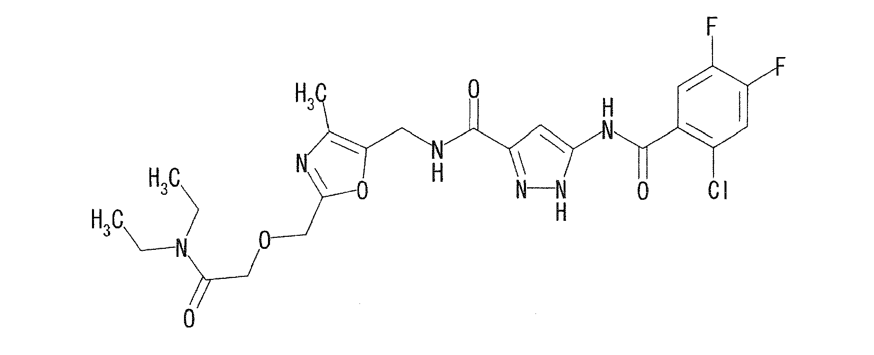

To a solution of 5-(2-chloro-4,5-difluoro-benzoylamino)-1H-pyrazole-3-carboxylic acid (2-carboxymethoxymethyl-4-methyl-oxazol-5-ylmethyl)-amide (117 mg) produced in Example 3-7 and 1-hydroxybenzotriazole monohydrate (56 mg) in N,N-dimethylformamide (1 mL) was added EDC hydrochloride (56 mg). After stirring at room temperature for 1 hour, diethylamine (0.026 mL) was added. After stirring overnight at room temperature, aqueous sodium hydrogencarbonate was added to the reaction mixture, and the mixture was extracted with ethyl acetate. The organic layer was washed with saturated brine, dried over anhydrous sodium sulfate, and then concentrated. The residue was purified by silica gel column chromatography (chloroform/ethyl acetate, 8:1) to obtain the title compound (25 mg) as a colorless oily matter.

### Example 3-9

### Production of 5-(2-chloro-4,5-difluoro-benzoylamino)-1H-pyrazole-3-carboxylic acid [2-(benzylcarbamoyl-methoxymethyl)-4-methyl-oxazol-5-ylmethyl]-amide:

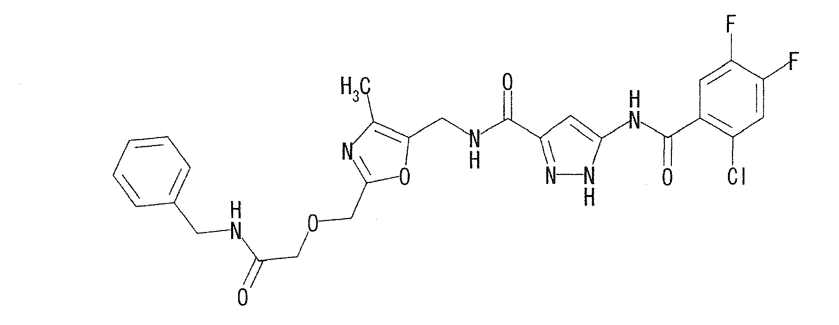

To a solution of 5-(2-chloro-4,5-difluoro-benzoylamino)-1H-pyrazole-3-carboxylic acid (2-carboxymethoxymethyl-4-methyl-oxazol-5-ylmethyl)-amide (100 mg) produced in Example 3-7, benzylamine (0.025 mL), and 1-hydroxybenzotriazole monohydrate (40 mg) in N,N-dimethylformamide (1 mL) was added EDC hydrochloride (45 mg). After stirring overnight at room temperature, aqueous sodium hydrogencarbonate was added to the reaction mixture, and the mixture was extracted with ethyl acetate. The organic layer was successively washed with water and saturated brine, dried over anhydrous sodium sulfate, and then concentrated. The residue was purified by silica gel column chromatography (chloroform/methanol, 7:1) to obtain the title compound (77 mg) as a colorless solid.

### Example 3-10

### Production of 5-(2-chloro-4,5-difluoro-benzoylamino)-1H-pyrazole-3-carboxylic acid [4-methyl-2-(2-morpholin-4-yl-2-oxo-ethoxymethyl)-oxazol-5-ylmethyl]-amide:

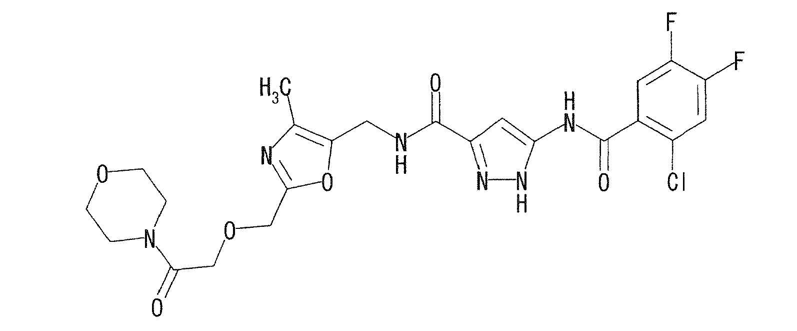

In the same manner as in Example 3-9, the title compound (65 mg) was obtained from 5-(2-chloro-4,5-difluoro-benzoylamino)-1H-pyrazole-3-carboxylic acid (2-carboxymethoxymethyl-4-methyl-oxazol-5-ylmethyl)-amide (100 mg) produced in Example 3-7 as a colorless solid.

### Example 3-11

### Production of 5-(2-chloro-4,5-difluoro-benzoylamino)-1H-pyrazole-3-carboxylic acid {4-methyl-2-[(toluene-4-sulfonylamino)-methyl]-oxazol-5-ylmethyl}-amide:

### (Step 1)

### Production of 5-(tert-butyl-dimethyl-silanyloxymethyl)-2-(1,3-dioxo-1,3-dihydro-isoindol-2-ylmethyl)-4-methyl-oxazole:

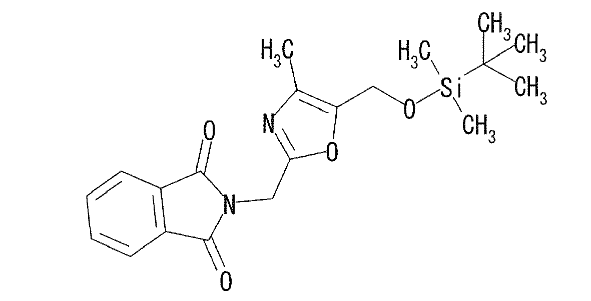

To a solution of 5-(tert-butyl-dimethyl-silanyloxymethyl)-2-hydroxymethyl-4-methyl-oxazole (2.080 g) produced in Step 4 of Reference Example 42 in chloroform (20 mL) was added triethylamine (1.23 mL) followed by addition of methanesulfonyl chloride (0.66 mL) with ice cooling. After stirring overnight at room temperature, water was added to the reaction mixture, the organic layer was separated, and the aqueous layer was extracted with chloroform. The combined organic layers were washed with saturated brine, dried over anhydrous sodium sulfate, and then concentrated to obtain 5-(tert-butyl-dimethyl-silanyloxymethyl)-2-chloromethyl-4-methyl-oxazole.

Subsequently, to a solution of 5-(tert-butyldimethyl-silanyloxymethyl)-2-chloromethyl-4-methyl-oxazole in N,N-dimethylformamide (10 mL) was added potassium phthalimide (1.575 g). After stirring at 70°C for 7 hours, water was added to the reaction mixture, and the precipitated solid was collected by filtration to obtain the title compound (2.583 g) as an ocher solid.

### (Step 2)

### Production of 2-aminomethyl-5-(tert-butyl-dimethyl-silanyloxymethyl)-4-methyl-oxazole:

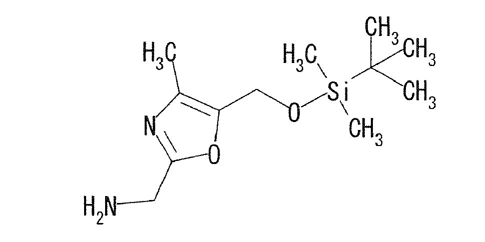

To a solution of 5-(tert-butyl-dimethyl-silanyloxymethyl)-2-(1,3-dioxo-1,3-dihydro-isoindol-2-ylmethyl)-4-methyl-oxazole (2.583 g) in methanol (25 mL) was added hydrazine monohydrate (1 mL). After stirring at 85°C for 4 hours and 30 minutes, the precipitated solid was filtered off, and the filtrate was concentrated. Chloroform was added to the residue, the precipitated solid was filtered off, and the filtrate was concentrated to obtain the title compound (1.897 g) as a yellow oily matter.

### (Step 3)

### Production of 2-(tert-butoxycarbonylamino-methyl)-5-(tert-butyl-dimethyl-silanyloxymethyl)-4-methyl-oxazole:

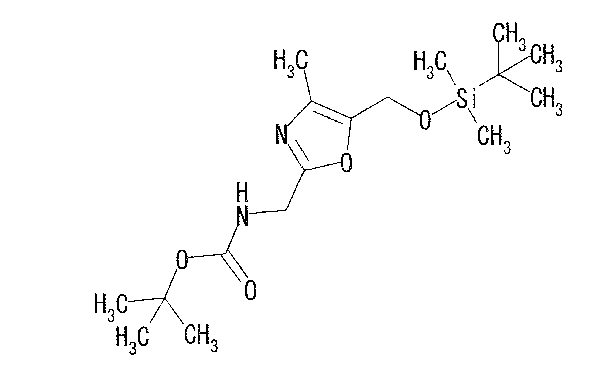

To a solution of 2-aminomethyl-5-(tert-butyldimethyl-silanyloxymethyl)-4-methyl-oxazole (1.897 g) in chloroform (20 mL), a solution of di-tert-butyl dicarbonate (1.85 g) in chloroform (5 mL) was added with ice cooling. After stirring overnight at room temperature, the reaction mixture was concentrated, and the residue was dissolved in ethyl acetate. The organic layer was washed with saturated brine, dried over anhydrous sodium sulfate, and then concentrated. The residue was purified by silica gel column chromatography (hexane/ethyl acetate, 4:1 → 2:1 → 1:1) to obtain the title compound (1.642 g) as an orange color solid.

### (Step 4)

### Production of 2-(tert-butoxycarbonylamino-methyl)-5-hydroxymethyl-4-methyl-oxazole:

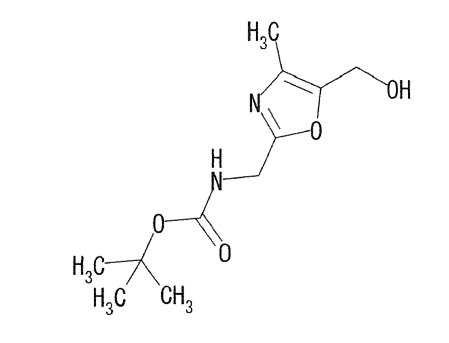

In the same manner as in Step 6 of Reference Example 42, the title compound (1.017 g) was obtained from 2-(tert-butoxycarbonylamino-methyl)-5-(tert-butyl-dimethyl-silanyloxymethyl)-4-methyl-oxazole (1.642 g) as a yellow oily matter.

### (Step 5)

### Production of 5-azidomethyl-2-(tert-butoxycarbonylaminomethyl)-4-methyl-oxazole:

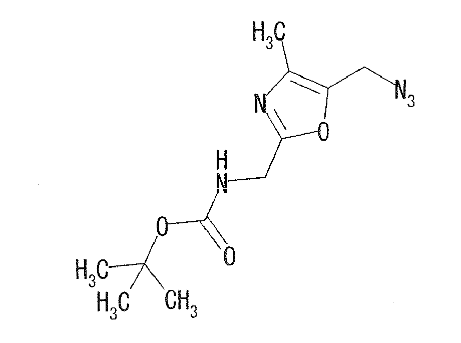

In the same manner as in Step 4 of Reference Example 3, the title compound (1.026 g) was obtained from 2-(tert-butoxycarbonylamino-methyl)-5-hydroxymethyl-4-methyl-oxazole (1.017 g) as a brown oily matter.

### (Step 6)

### Production of 5-aminomethyl-2-(tert-butoxycarbonylaminomethyl)-4-methyl-oxazole:

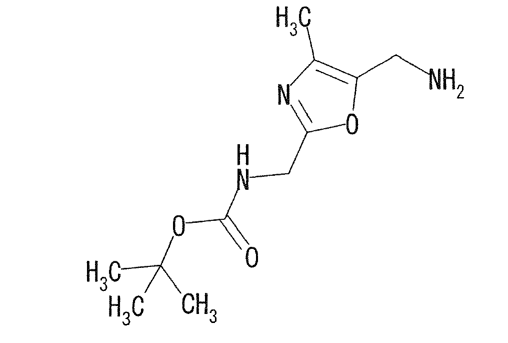

To a solution of 5-azidomethyl-2-(tert-butoxycarbonylamino-methyl)-4-methyl-oxazole (1.026 g) in tetrahydrofuran (10 mL), a solution of triphenylphosphine (1.125 g) in tetrahydrofuran (5 mL) was added at 50°C. After 2 hours, water (0.4 mL) was added and stirred at 50°C for 1 hour and then at room temperature overnight. The reaction mixture was concentrated, and the residue was purified by silica gel column chromatography (chloroform/methanol/28% aqueous ammonia, 10:1:0 → 10:1:0.1 → 10:1.5:0.2) to obtain the title compound (898 mg) as brown oily matter.

### (Step 7)

### Production of 5-(2-chloro-4,5-difluoro-benzoylamino)-1H-pyrazole-3-carboxylic acid [2-(tert-butoxycarbonylaminomethyl)-4-methyl-oxazol-5-ylmethyl]-amide:

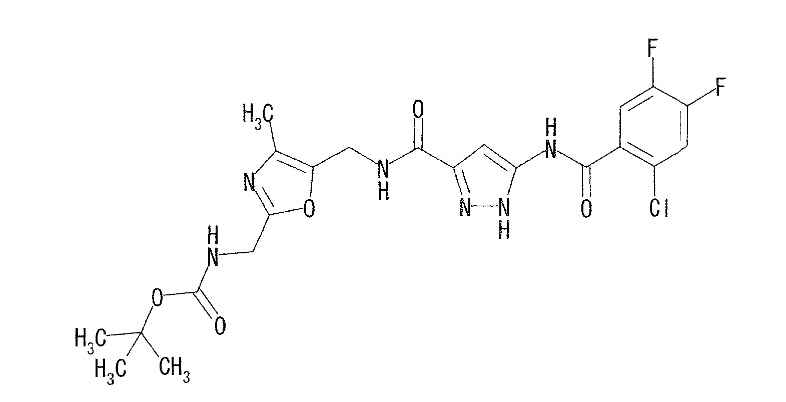

In the same manner as in Example 1-1, the title compound (1.611 g) was obtained from 5-aminomethyl-2-(tert-butoxycarbonylamino-methyl)-4-methyl-oxazole (898 mg) as a yellow solid.

### (Step 8)

### Production of 5-(2-chloro-4,5-difluoro-benzoylamino)-1I-i-pyrazole-3-carboxylic acid (2-aminomethyl-4-methyl-oxazol-5-ylmethyl)-amide trihydrochloride:

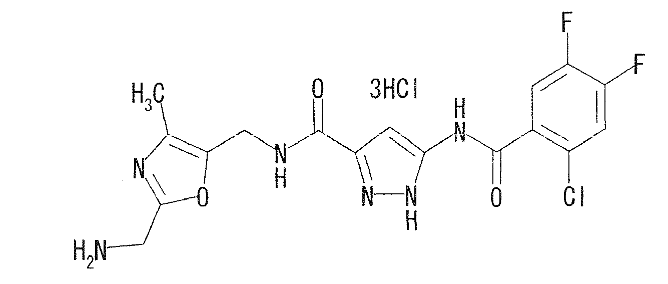

To a solution of 5-(2-chloro-4,5-difluoro-benzoylamino)-1H-pyrazole-3-carboxylic acid [2-(tert-butoxycarbonylamino-methyl)-4-methyl-oxazol-5-ylmethyl]-amide (1.51 g) in methanol (30 mL) was added 4 N hydrochloric acid/ethyl acetate (7 mL). After stirring overnight at room temperature, the reaction mixture was concentrated. Ethyl acetate was added to the residue, and the precipitated solid was collected by filtration to obtain the title compound (1.28 g) as a light yellow solid.

### (Step 9)

### Production of 5-(2-chloro-4,5-difluoro-benzoylamino)-2H-pyrazole-3-carboxylic acid {4-methyl-2-[(toluene-4-sulfonylamino)-methyl]-oxazol-5-ylmethyl}-amide:

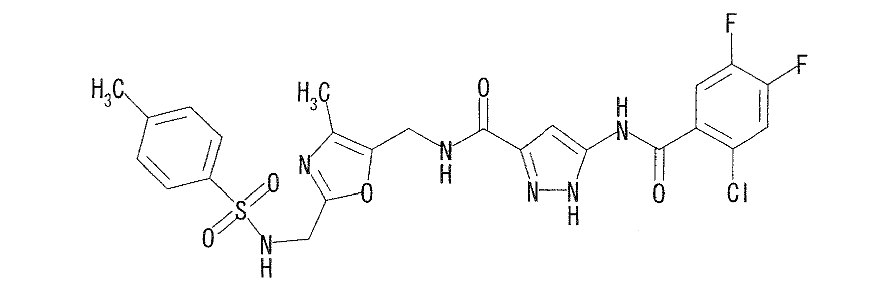

To a suspension of 5-(2-chloro-4,5-difluoro-benzoylamino)-1H-pyrazole-3-carboxylic acid (2-aminomethyl-4-methyl-oxazol-5-ylmethyl)-amide trihydrochloride (100 mg) in tetrahydrofuran (1 mL) was added triethylamine (0.08 mL) followed by addition of p-toluenesulfonyl chloride (36 mg) with ice cooling. After stirring overnight at room temperature, the reaction mixture was concentrated. Methanol and aqueous sodium hydrogencarbonate were added to the residue, and the precipitated solid was collected by filtration to obtain the title compound (88 mg) as a yellow solid.

### Example 3-12

### Production of 5-(2-chloro-4,5-difluoro-benzoylamino)-1H-pyrazole-3-carboxylic acid [2-(acetylamino-methyl)-4-methyl-oxazol-5-ylmethyl]-amide dihydrochloride:

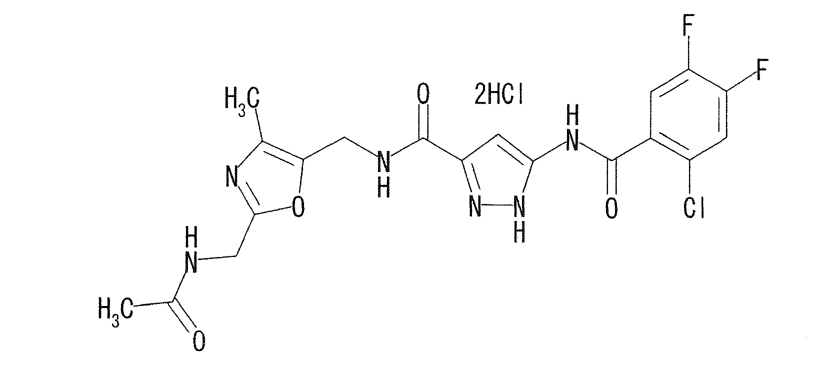

To a solution of 5-(2-chloro-4,5-difluoro-benzoylamino)-1H-pyrazole-3-carboxylic acid (2-aminomethyl-4-methyl-oxazol-5-ylmethyl)-amide trihydrochloride (100 mg) produced in Step 8 of Example 3-11, 1-hydroxybenzotriazole monohydrate (36 mg), triethylamine (0.08 mL), and acetic acid (0.012 mL) in N,N-dimethylformamide (1 mL) was added EDC hydrochloride (40 mg). After stirring overnight at room temperature, aqueous sodium hydrogencarbonate was added to the reaction mixture, and the mixture was extracted with a mixed solvent of ethyl acetate/tetrahydrofuran. The organic layer was successively washed with water and saturated brine, dried over anhydrous sodium sulfate, and then concentrated. The residue was purified by silica gel column chromatography (chloroform/methanol, 30:1 → 8:1) to obtain 5-(2-chloro-4,5-difluoro-benzoylamino)-1H-pyrazole-3-carboxylic acid [2-(acetylamino-methyl)-4-methyl-oxazol-5-ylmethyl]-amide.

Subsequently, to a solution of 5-(2-chloro-4,5-difluoro-benzoylamino)-1H-pyrazole-3-carboxylic acid [2-(acetylamino-methyl)-4-methyl-oxazol-5-ylmethyl]-amide in ethanol (1 mL) was added 4 N hydrochloric acid/ethyl acetate (0.5 mL). Ethyl acetate was added to the reaction mixture, and the precipitated solid was collected by filtration to obtain the title compound (37 mg) as a yellow solid.

### Example 3-13

### Production of 5-(2-chloro-4,5-difluoro-benzoylamino)-1H-pyrazole-3-carboxylic acid {2-[(4-chloro-benzoylamino)-methyl]-4-methyl-oxazol-5-ylmethyl}-amide:

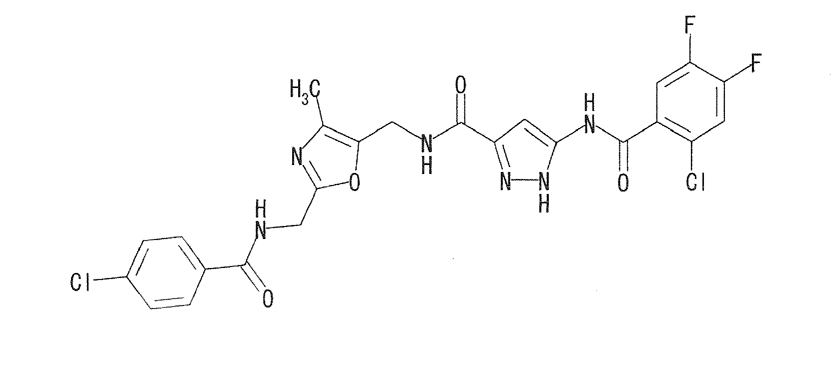

In the same manner as in Example 3-12, the title compound (88 mg) was obtained from 5-(2-chloro-4,5-difluoro-benzoylamino)-1H-pyrazole-3-carboxylic acid (2-aminomethyl-4-methyl-oxazol-5-ylmethyl)-amide trihydrochloride (100 mg) produced in Step 8 of Example 3-11 and 4-chlorobenzoic acid (33 mg) as a yellow solid.

### Example 3-14

### Production of sodium 5-({[5-(2-chloro-4,5-difluoro-benzoylamino)-1H-pyrazole-3-carbonyl]-amino}-methyl)-4-methyl-oxazole-2-carboxylate:

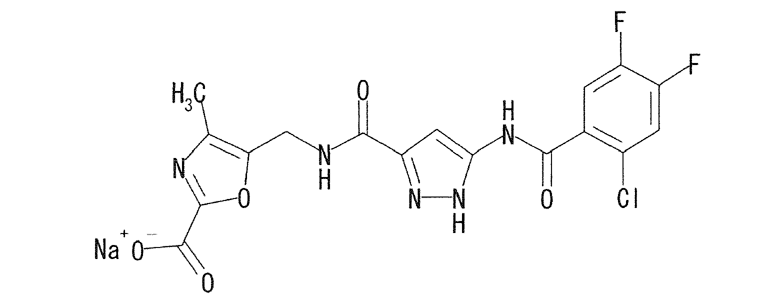

To a suspension of 5-(2-chloro-4,5-difluoro-benzoylamino)-1H-pyrazole-3-carboxylic acid (2-methoxycarbonyl-4-methyl-oxazol-5-ylmethyl)-amide (409 mg) produced in Example 1-50 in acetone (2 mL) was added 4 N sodium hydroxide aqueous solution (0.15 mL), and the mixture was stirred at room temperature for 1 day. The precipitated solid was collected by filtration, washed with acetone, and dried to obtain the title compound (280 mg) as a light yellow solid.

### Example 3-15

### Production of 5-(2-chloro-4,5-difluoro-benzoylamino)-1H-pyrazole-3-carboxylic acid [4-methyl-2-(piperidine-1-carbonyl)-oxazol-5-ylmethyl]-amide:

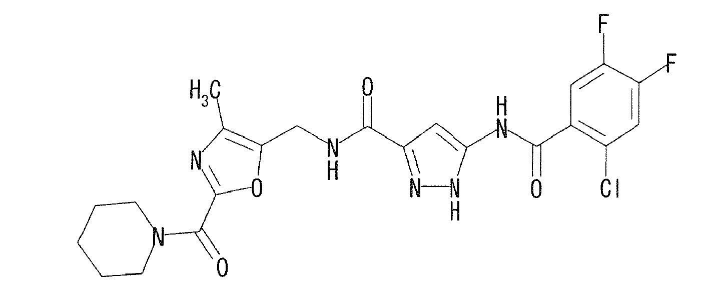

To a solution of sodium 5-({[5-(2-chloro-4,5-difluoro-benzoylamino)-1H-pyrazole-3-carbonyl]-amino}-methyl)-4-methyl-oxazole-2-carboxylate (66 mg) produced in Example 3-14, piperidine (0.014 mL), 4 N hydrochloric acid (0.036 mL), and 1-hydroxybenzotriazole monohydrate (29 mg) in N,N-dimethylformamide (0.7 mL) was added EDC hydrochloride (29 mg). After stirring overnight at room temperature, aqueous sodium hydrogencarbonate was added to the reaction mixture, and the mixture was extracted with ethyl acetate. The organic layer was successively washed with water and saturated brine, dried over anhydrous sodium sulfate, and then concentrated. The residue was purified by silica gel column chromatography (chloroform/methanol, 10:1) to obtain the title compound (25 mg) as a light yellow solid.

### Example 3-16

### Production of 5-(2-chloro-4,5-difluoro-benzoylamino)-1H-pyrazole-3-carboxylic acid (2-benzylcarbamoyl-4-methyl-oxazol-5-ylmethyl)-amide:

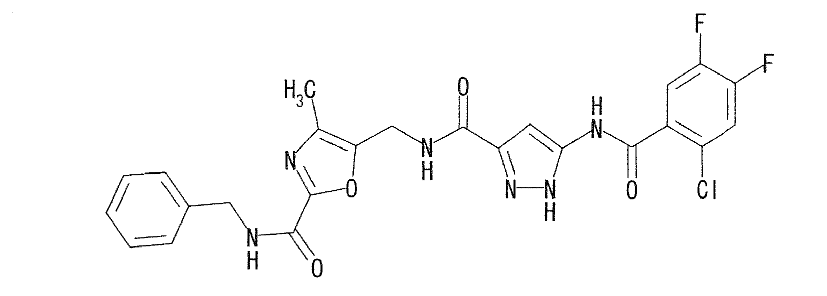

In the same manner as in Example 3-15, the title compound (64 mg) was obtained from sodium 5-({[5-(2-chloro-4,5-difluoro-benzoylamino)-1H-pyrazole-3-carbonyl]-amino}-methyl)-4-methyl-oxazole-2-carboxylate (66 mg) produced in Example 3-14 as a white solid.

### Example 3-17

### Production of 5-(2-chloro-4,5-difluoro-benzoylamino)-1H-pyrazole-3-carboxylic acid (2-benzyloxycarbonyl-4-methyl-oxazol-5-ylmethyl)-amide:

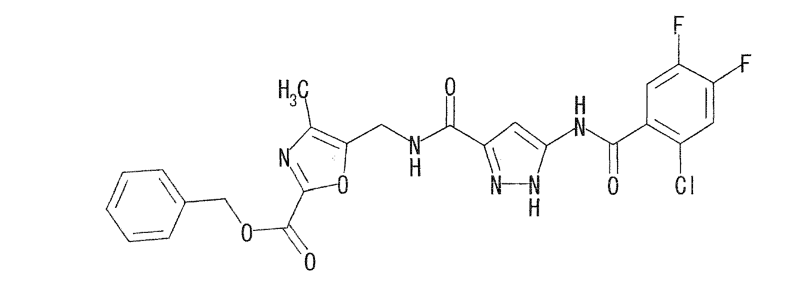

To a solution of sodium 5-({[5-(2-chloro-4,5-difluoro-benzoylamino)-1H-pyrazole-3-carbonyl]-amino}-methyl)-4-methyl-oxazole-2-carboxylate (66 mg) produced in Example 3-14 in N,N-dimethylformamide, 1,8-diazabicyclo[5,4,0]undec-7-ene (0.021 mL) and benzyl chloride (0.017 mL) were successively added at room temperature, and the mixture was stirred for 1 hour. Water and ethyl acetate were added to the reaction mixture, and the organic layer was separated, successively washed with water and saturated brine, dried over anhydrous sodium sulfate, and then concentrated. The residue was purified by silica gel column chromatography (chloroform/methanol, 10:1). The resulting residue (83 mg) dissolved in ethyl acetate, the mixture was allowed to stand overnight, and the precipitated solid was collected by filtration and dried to obtain the title compound (30 mg) as a light yellow solid.

### Example 3-18

### Production of 5-(2-chloro-4,5-difluoro-benzoylamino)-1H-pyrazole-3-carboxylic acid (5-carboxy-4-methyl-oxazol-2-ylmethyl)-amide:

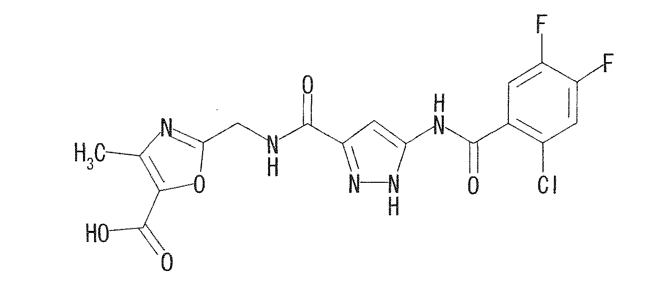

In the same manner as in Example 3-1, the title compound (368 mg) was obtained from 5-(2-chloro-4,5-difluoro-benzoylamino)-1H-pyrazole-3-carboxylic acid (5-ethoxycarbonyl-4-methyl-oxazol-2-ylmethyl)-amide (498 mg) produced in Example 1-51 as a yellow solid.

### Example 3-19

### Production of 5-(2-chloro-4,5-difluoro-benzoylamino)-1H-pyrazole-3-carboxylic acid (5-carboxymethyl-[1,3,4]oxadiazol-2-ylmethyl)-amide:

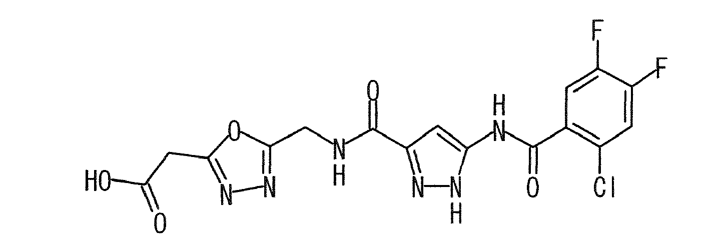

To a solution of 5-(2-chloro-4,5-difluoro-benzoylamino)-1H-pyrazole-3-carboxylic acid (5-ethoxycarbonyl-[1,3,4]oxadiazol-2-ylmethyl)-amide (200 mg) produced in Example 1-62 in methanol (2.0 mL) was added 2 N sodium hydroxide aqueous solution (0.42 mL) with ice cooling. After stirring at room temperature for 1 hour, 1 N hydrochloric acid was added dropwise. The reaction solution was concentrated, water was added to the residue, and the mixture was stirred at room temperature. The precipitated solid was collected by filtration and dried to obtain the title compound (156 mg) as a light yellow solid.

### Example 3-20

### Production of 5-(2-chloro-4,5-difluoro-benzoylamino)-1H-pyrazole-3-carboxylic acid (5-carbamoylmethyl-[1,3,4]oxadiazol-2-ylmethyl)-amide:

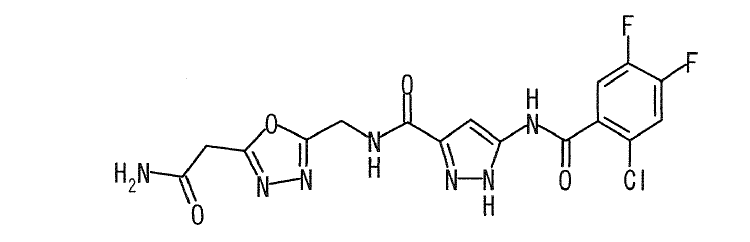

To a solution of 5-(2-chloro-4,5-difluoro-benzoylamino)-1H-pyrazole-3-carboxylic acid (5-ethoxycarbonyl-[1,3,4]oxadiazol-2-ylmethyl)-amide (202 mg) produced in Example 1-62 in 7 N ammonia/methanol (2.5 mL) was added a sodium methoxide/methanol solution (91 mg) with ice cooling. After stirring at room temperature for 2 hours, 1 N hydrochloric acid was added to the reaction mixture followed by addition of water, and the precipitated solid was collected by filtration to obtain the title compound (182 mg) as a white solid.

### Example 3-21

### Production of 5-(4,5-difluoro-2-methyl-benzoylamino)-1H-pyrazole-3-carboxylic acid (4-carboxy-2-methyl-oxazol-5-ylmethyl)-amide:

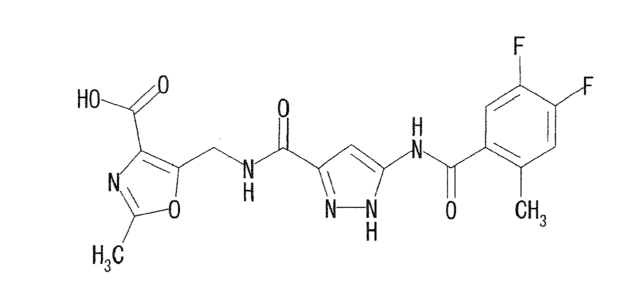

In the same manner as in Example 3-1, the title compound (301 mg) was obtained from 5-(4,5-difluoro-2-methyl-benzoylamino)-1H-pyrazole-3-carboxylic acid (4-ethoxycarbonyl-2-methyl-oxazol-5-ylmethyl)-amide (347 mg) produced in Example 2-1 as a light yellow solid.

### Example 3-22

### Production of 5-(2-chloro-4,5-difluoro-benzoylamino)-1H-pyrazole-3-carboxylic acid [2-methyl-4-(thiophene-2-carbonyloxymethyl)-oxazol-5-ylmethyl]-amide:

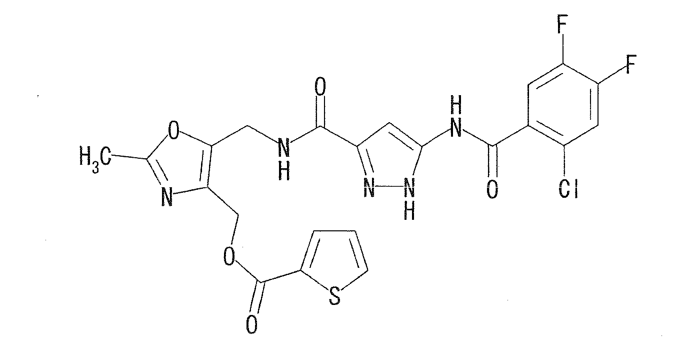

In the same manner as in Example 3-4, the title compound (120 mg) was obtained from 5-(2-chloro-4,5-difluoro-benzoylamino)-1H-pyrazole-3-carboxylic acid (4-hydroxymethyl-2-methyl-oxazol-5-ylmethyl)-amide (200 mg) produced in Example 1-35 and thiophene-2-carbonyl chloride (0.055 mL) as a yellow solid.

### Example 3-23

### Production of 5-(2-chloro-4,5-difluoro-benzoylamino)-1H-pyrazole-3-carboxylic acid (4-cyclohexanecarbonyloxymethyl-2-methyl-oxazol-5-ylmethyl)-amide:

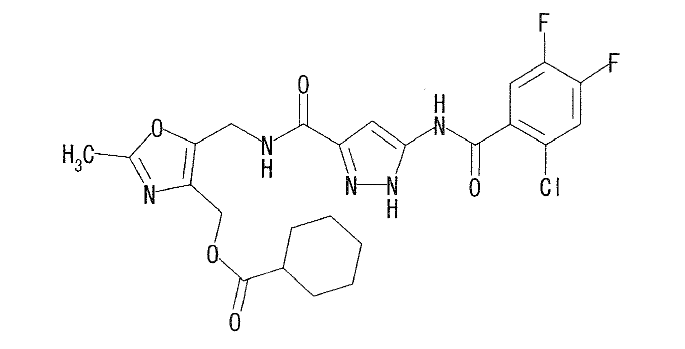

In the same manner as in Example 3-4, the title compound (182 mg) was obtained from 5-(2-chloro-4,5-difluoro-benzoylamino)-1H-pyrazole-3-carboxylic acid (4-hydroxymethyl-2-methyl-oxazol-5-ylmethyl)-amide (200 mg) produced in Example 1-35 and cyclohexanecarbonyl chloride (0.070 mL) as a white solid.

### Example 3-24

### Production of 5-(2-chloro-4,5-difluoro-benzoylamino)-1H-pyrazole-3-carboxylic acid (2-hydroxymethyl-4-methyl-oxazol-5-ylmethyl)-amide hydrochloride:

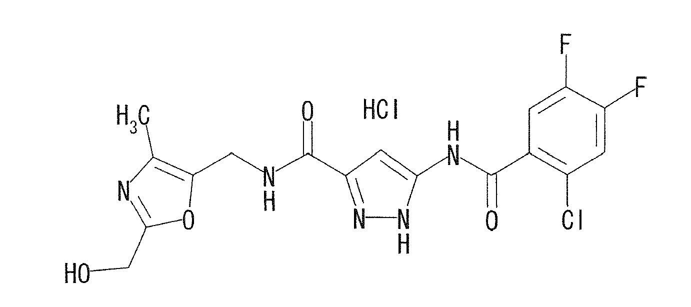

To a solution of 5-(2-chloro-4,5-difluoro-benzoylamino)-1H-pyrazole-3-carboxylic acid (4-methyl-2-trityloxymethyl-oxazol-5-ylmethyl)-amide (56.7 mg) produced in Example 1-43 in ethyl acetate (1 mL) were added 4 N hydrochloric acid/ethyl acetate (0.3 mL) and methanol (1 mL). After stirring overnight at room temperature, the reaction mixture was concentrated. Ethyl acetate was added to the residue, and the precipitated solid was collected by filtration to obtain the title compound (1.59 g) as a light yellow solid.

### Example 3-25

### Production of 5-({[5-(2-chloro-4,5-difluoro-benzoylamino)-1H-pyrazole-3-carbonyl]-amino}-methyl)-4-methyl-oxazole-2-carboxylic acid benzyl-methyl-amide:

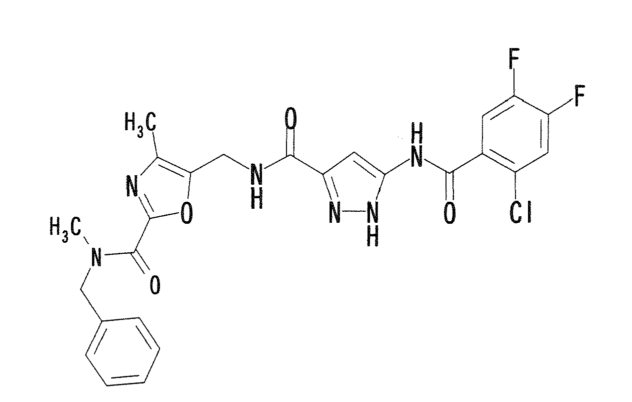

According to the method described in Example 3-15, the title compound (61 mg) was obtained from the compound (79 mg) of Example 3-14.

The structural formulas and ¹H-NMR spectrum data of the compounds of Examples 3-1 to 3-25 are shown in the following Tables 17 to 21.

¹H-NMR spectra were measured using tetramethylsilane in CDCl₃ or DMSO-*d₆* as the internal standard, and all the δ values are expressed in ppm.

Symbols in the tables denote as follows:
s: Singlet
d: Doublet
t: Triplet
dd: Double doublet
ddd: Double double doublet
brs: Broad singlet
m: Multiplet
J: Coupling constant

**Table 17**

| Example | Structural formula | **NMR** |
|---|---|---|
| 3-1 | 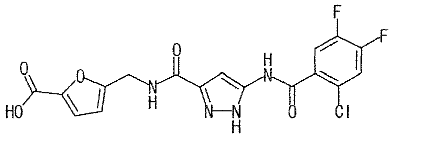 | ¹H-NMR (DMSO-*d*₆) δ: 13.13 (2H, br s), 11.17 (1H, br s), 9.14 (1H, br s), 7.84-7.78 (2H, m), 7.26 (1H, br s), 7.14 (1H, d, *J* = 3.2 Hz), 6.45 (1H, d, *J* = 3.2 Hz), 4.47 (2H, d, *J* = 5.6 Hz). |
| 3-2 | 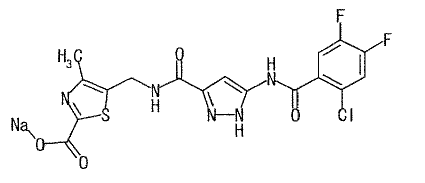 | ¹H-NMR (DMSO-*d*₆) δ: 13.30 (1H, br s), 11.14 (1H, br s), 9.17 (1H, br s), 7.85-7.79 (2H, m), 7.30 (1H, br s), 4.48 (2H, d, *J* = 5.5 Hz). 2.34 (3H, s). |
| 3-3 | 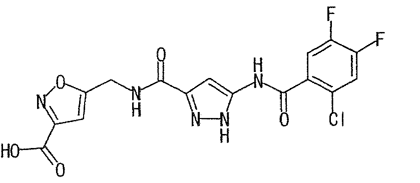 | ¹H-NMR (DMSO-*d*₆) δ: 13.44 (1H, s), 11.21 (1H, s), 9.26 (1H, s), 7.87-7.81 (2H, m), 7.27 (1H, s), 6.68 (1H, s), 4.64 (2H, d, *J* = 5.8 Hz). |
| 3-4 | 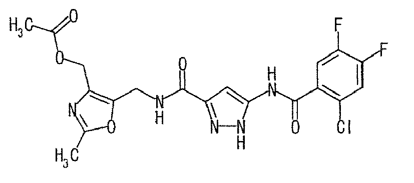 | ¹-NMR (DMSO-*d*₆) δ: 13.27 (1H, br s), 11.15 (1H, br s), 9.07 (1H, br s), 7.86-7.79 (2H, m), 7.29 (1H, br s), 4.98 (2H, s), 4.49 (2H, d, *J* = 5.7 Hz), 2.37 (3H, s), 2.01 (3H, s). |
| 3-5 | 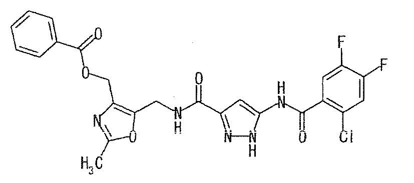 | ¹H-NMR (DMSQ-*d*₆) δ: 13.28 (1H, br s), 11.16 (1H, br s), 9.11 (1H, br s), 7.96-7.93 (2H, m), 7.86-7.79 (2H, m), 7.68-7.63 (1H, m), 7.53-7.49 (2H, m), 7.31 (1H, br s), 5.29 (2H, s), 4.57 (2H, d, *J* = 5.5 Hz), 2.39 (3H, s). |
| 3-6 | 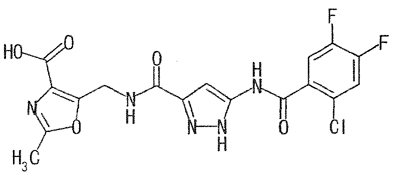 | ¹H-NMR (DMSO-*d*₆) δ: 13.18 (2H, br s), 11.17 (1H, br s). 9.06 (1H, br s), 7.86-7.79 (2H, m), 7.28 (1H, s), 4.78 (2H, d, *J* = 5.3 Hz), 2.40 (3H, s). |

**Table 18**

| Example | Structural formula | **NMR** |
|---|---|---|
| 3-7 | 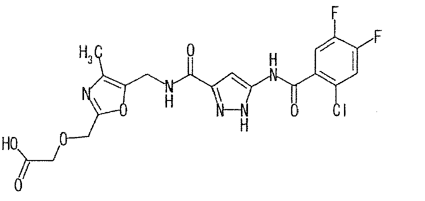 | ¹H-NMR (DMSO-*d*₆) δ: 13.02 (2H, br s), 11.17 (1H, br s), 9.08 (1H, br s), 7.86-7.80 (2H, m), 7.26 (1H, s), 4.55 (2H, s), 4.44 (2H, d, *J* = 5.6 Hz), 4.10 (2H, s), 2.13 (3H, s). |
| 3-8 | 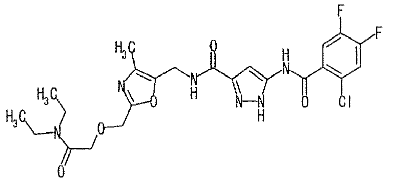 | ¹H-NMR (DMSO-*d*₆) δ: 13.27 (1H, br s), 11.14 (1H, br s), 9.11 (1H, t, *J* = 5.6 Hz), 7.85-7.79 (2H, m), 7.32 (1H, s), 4.54 (2H, s), 4.44 (2H, d, *J* = 5.6 Hz), 4.19 (2H, s), 3.26-3.15 (4H, m), 2.13 (3H, s), 1.06-0.98 (6H, m). |
| 3-9 | 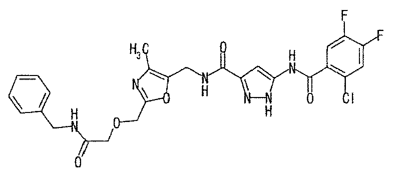 | ¹H-NMR (DMSO-*d*₆) δ: 13.26 (1H, br s), 11.14 (1H, br s), 9.10 (1H, br s), 8.38 (1H, t, *J* = 5.8 Hz), 7.85-7.78 (2H, m), 7.32-7.20 (6H, m), 4.58 (2H, s), 4.44 (2H, d, *J* = 5.3 Hz), 4.29 (2H, d, *J* = 5.8 Hz), 4.03 (2H, s,), 2.13 (3H, s). |
| 3-10 | 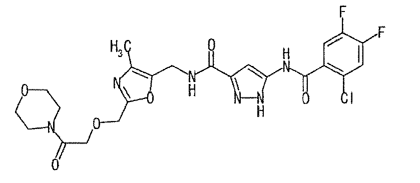 | ¹H-NMR (DMSO*-d*₆) δ: 13.26 (1H, br s), 11.14 (1H, br s), 9.10 (1H, br s), 7.85-7.78 (2H, m), 7.32 (1H, s), 4.54 (2H, s), 4.44 (2H, d, *J* = 4.6 Hz), 4.24 (2H, s), 3.55-3.52 (4H, m), 3.42-3.29 (4H, m), 2.13 (3H, s). |
| 3-11 | 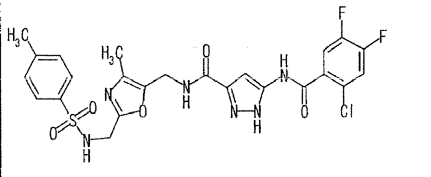 | ¹H-NMR (DMSO-*d*₆) δ: 13.27 (1H, br s), 11.14 (1H, br s), 9.05 (1H, t, *J* = 5.6 Hz), 8.29 (1H, t, *J* = 6.1 Hz), 7.85-7.79 (2H, m), 7.61 (2H, d, *J* = 8.1 Hz), 7.33 (2H, d, *J* = 8.1 Hz), 7.32 (1H, s), 4.32 (2H, d, *J* = 5.6 Hz), 4.02 (2H, d, *J* = 6.1Hz), 2.35 (3H, s), 2.03 (3H, s). |
| 3-12 | 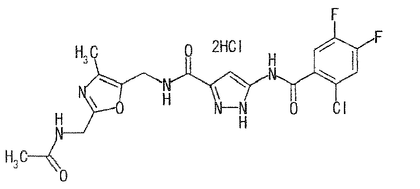 | ¹H-NMR (DMSO-*d*₆) δ: 11.17 (1H, br s), 9.06 (1H, br s), 8.47 (1H, br s), 7.86-7.80 (2H, m), 7.24 (1H, s), 4.40 (2H, d, *J* = 5.6 Hz), 4.28 (2H, d, *J* = 5.8 Hz), 2.10 (3H, s), 1.85 (3H, s). |

**Table 19**

| Example | Structural formula | **NMR** |
|---|---|---|
| 3-13 | 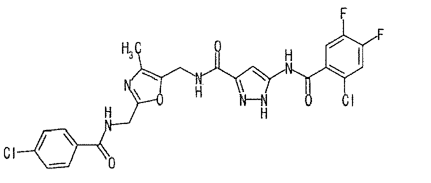 | ¹H-NMR (OMSO-*d*₆) δ: 13.25 (1H, br s), 11.14 (1H, br s), 9.20 (1H, t, *J* = 5.8 Hz), 9.09 (1H, t, *J* = 5.3 Hz), 7.89 (2H, d, *J* = 8.6 Hz), 7.86-7.79 (2H, m), 7.55 (2H, d, *J* = 8.6 Hz), 7.31 (1H, s), 4.50 (2H, d, *J* = 5.8 Hz), 4.41 (2H, d, *J* = 5.3 Hz), 2.11 (3H, s). |
| 3-14 | 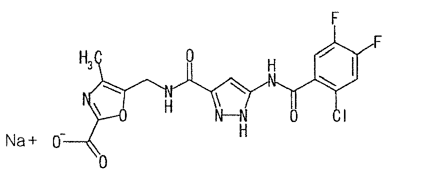 | ¹H-NMR (OMSO-*d*₆) δ: 13.30 (1H, s), 11.12 (1H, s), 9.15 (1H, br s), 7.87-7.77 (2H, m), 7.31 (1H, s), 4.41 (2H, d, J = 5.1 Hz), 2.10 (3H, s). |
| 3-15 | 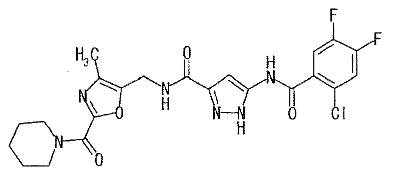 | ¹-NMR (DMSO-*d*₆) δ: 13.28 (1H, br s), 11.14 (1H, br s), 9.15 (1H, t. *J* = 5.3Hz), 7.85-7.78 (2H, m), 7.32 (1H, d, *J* = 1.8 Hz), 4.50 (2H, d, *J* = 5.3 Hz), 3.87-3.84 (2H, m), 3.60-3.56 (2H, m), 2.20 (3H, s), 1.64-1.60 (2H, m), 1.56-1.50 (4H, m). |
| 3-16 | 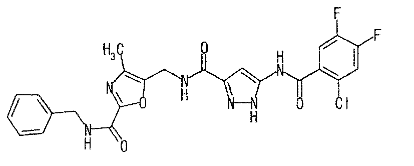 | ¹H-NMR (DMSO-*d*₆) δ: 13.27 (1H, s), 11.14 (1H, s), 9.38 (1H, t, *J* = 6.2 Hz), 9.16 (1H, t, *J* = 5.3 Hz), 7.85-7.78 (2H, m), 7.33-7.20 (6H, m), 4.51 (2H, d, *J* = 5.3 Hz), 4.40 (2H, d, *J* = 6.2 Hz), 2.22 (3H, s). |
| 3-17 | 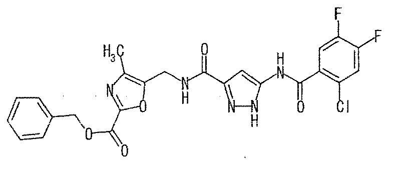 | ¹H-NMR (DMSO-*d*₆) δ: 13.28 (1H, d, *J* = 2.2 Hz), 11.14 (1H, s), 9.19 (1H, t, *J* = 5.6 Hz), 7.86-7.77 (2H, m), 7.48-7.34 (5H, m), 7.31 (1H, d, *J* = 2.2 Hz), 5.36 (2H, s), 4.52 (2H, d. *J* = 5.6 Hz), 2.22 (3H, s). |
| 3-18 | 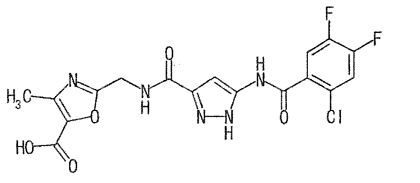 | ¹H-NMR (DMSO-*d*₆) δ: 13.36 (2H, br s), 11.20 (1H, br s), 9.27 (1H, br s), 7.87-7.81 (2H, m), 7.30 (1H, br s), 4.56 (2H, d, *J* = 5.8 Hz), 2.35 (3H. s). |

**Table 20**

| Example | Structural formula | **NMR** |
|---|---|---|
| 3-19 | 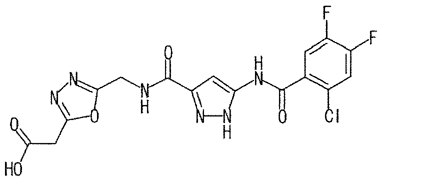 | ¹H-NMR (DMSO-*d*₆) δ: 13.26 (1H, s), 11.21 (1H, s), 9.32 (1H, s), 7.87-7.81 (2H, m), 7.30 (1H, s), 4.66 (2H, d, *J* = 5.7 Hz), 4.07 (2H, s). |
| 3-20 | 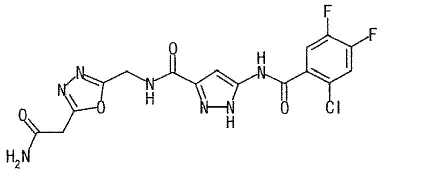 | ¹H-NMR (DMSO-*d*₆) δ: 13.34 (1H, s), 11.18 (1 H, s), 9.36 (1 H, t, *J* = 5.6 Hz), 7.86-7.80 (2H, m), 7.70 (1 H, s), 7.37 (1 H, s), 7.25 (1H, s), 4.67 (2H, d, *J* = 5.6 Hz), 3.81 (2H, s). |
| 3-21 | 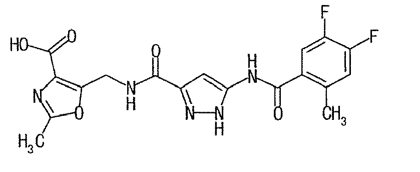 | ¹H-NMR (DMSO-*d*₆) δ: 13.15 (2H, br s), 10.98 (1 H, br s), 9.02 (1H, br s), 7.61-7.56 (1H, m), 7.40 (1H, dd, *J* = 11.8, 7.9 Hz), 7.22 (1H, br s), 4.78 (2H, d, *J* = 5.1 Hz), 2.40 (3H, s), 2.35 (3H, s). |
| 3-22 | 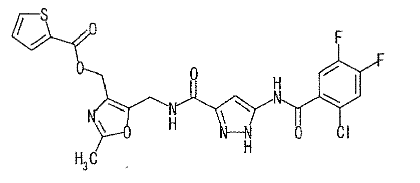 | ¹H-NMR (DMSO-*d*₆) δ: 13.27 (1H, br s), 11.14 (1H, br s), 9.12 (1H, br s), 7.95 (1H, dd, *J* = 5.1, 1.3 Hz), 7.86-7.79 (3H, m), 7.31 (1H, br s), 7.21 (1H, dd, *J* = 5.1, 3.7 Hz), 5.26 (2H, s), 4.56 (2H, d, *J* = 5.3 Hz), 2.39 (3H, s). |
| 3-23 | 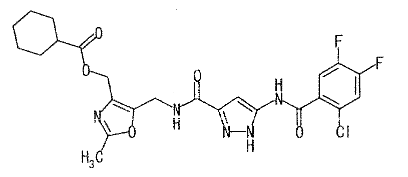 | ¹H-NMR (OMSO-*d*₆) δ: 13.26 (1H, br s), 11.15 (1H, br s), 9.07 (1H, br s), 7.86-7.78 (2H, m), 7.29 (1H, br s), 4.97 (2H, s), 4.49 (2H, d, *J* = 5.7 Hz), 2.37 (3H, s), 2.30-2.22 (1H, m), 1.78-1.53 (5H, m), 1.35-1.13 (5H, m). |
| 3-24 | 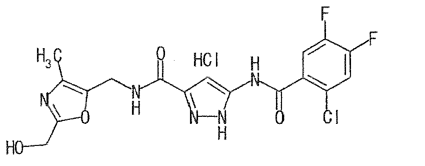 | ¹H-NMR (DMSO-*d*₆) δ: 11.19 (1H, br s), 9.10 (1H, t, *J* = 5.3 Hz), 7.87-7.80 (2H, m), 7.25 (1H, s), 4.44-4.41 (4H, m), 2.12 (3H, s). |

**Table 21**

| Example | Structural formula | **NMR** |
|---|---|---|
| 3-25 | 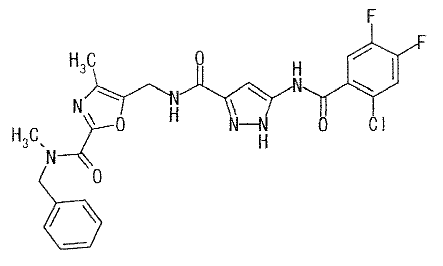 | ¹H-NMR (DMSO-D₆) δ: 13.30(1.0H, s), 11.16(1.0H. s). 9.19(1.0H, s), 7.95-7.80(2H, m), 7.45-7.24(5H, m), 5.08 (1.0H. s), 4.67 (1.0H. s), 4.51 (2.0H, m), 3.27 (1.5H, s), 2.88 (1.5H, s), 2.22 (1.5H, s), 2.18(1.5H. s) |

### Test Examples

### 1. Test Example (1): Method for determining liver glycogen phosphorylase activity

The glycogen phosphorylase activity was determined by a method using a forward reaction system.

The following method was used to determine the glycogen phosphorylase activity using a forward reaction system. Glucose-1-phosphate generated from glycogen by glycogen phosphorylase was converted to glucono-δ-lactone-6-phosphate by transphosphorylation and dehydrogenation reactions using phosphoglucomutase and glucose-6-phosphate dehydrogenase (G6PDH). At this time, NADPH generated from NADP in the dehydrogenation reaction by G6PDH was detected.

An enzyme solution of human liver glycogen phosphorylase was obtained by diluting with 100 mmol/L BES buffer (pH 6.8, containing 2 mmol/L EDTA) a suspension disrupted Sf9 cells in which a recombinant human liver glycogen phosphorylase was forcibly expressed. Phosphate buffer (16 mmol/L KH₂PO₄, containing 24 mmol/L Na₂HPO₄) was used as a substrate solution. A mixture of 8 U/mL phosphoglucomutase and 60 U/mL G6PDH was prepared with BES buffer. A reaction buffer (1.4 mmol/L NADP, 30 mmol/L MgCl₂, 8 µmol/L glucose-1,6-diphosphate, 8 mg/mL glycogen, 40 mmol/L BES, containing 0.8 mmol/L EDTA) and a glucose solution (75 mmol/L glucose and 100 mmol/L BES, containing 2 mmol/L EDTA) were prepared. A test substance was dissolved in 1% DMSO aqueous solution.

To a mixture of the glucose solution (20 µL), the substrate solution (20 µL), the reaction buffer (100 µL), and the test substance (20 µL), the recombinant human liver glycogen phosphorylase solution (20 µL), and a mixture (20 µL) of phosphoglucomutase and G6PDH were added to initiate an enzymatic reaction. To a control, 1% DMSO aqueous solution was added instead of the test substance. A solution to which the substrate was not added was used as a blank. After initiation of the reaction, absorbance at 340 nm was measured immediately. The reaction was performed at room temperature for 75 minutes, and then absorbance at 340 nm was measured again. The enzyme activity was obtained by deducting the change in absorbance of the blank for 75 minutes from the change in absorbance over 75 minutes. The inhibition rate (%) of the test substance was calculated by "(1 - (enzyme activity of test substance)/(enzyme activity of control)) × 100". IC50 value was calculated from a concentration obtained by calculating a line equation of concentration points with the inhibition rate of 50% therebetween and obtaining the intersection with the inhibition rate of 50%.

### 2. Test Example (2): Method of measuring plasma glucose concentrations

Effect of the compound of the present invention on plasma glucose concentrations is examined using db/db mice, an obese diabetes model. Plasma glucose concentrations in db/db mice (for example, 9 to 18 weeks old) are measured, and the animals are divided into groups each consisting of 5 or 8 animals so that the mean plasma glucose concentrations should match. After fasting for 4 hours, db/db mice are orally administered an example compound or a vehicle (0.5% methylcellulose), and plasma glucose concentrations are measured at 1 and 3 hours after administration. The blood sugar lowering action of the example compound is evaluated at each time point by performing a statistical test between the vehicle treatment group and the example compound treatment group (Dunnett's test).

The biological activity data of the example compounds are shown in the following Tables 22 to 25.

"IC₅₀" represents an enzyme inhibitory activity against human liver glycogen phosphorylase. A compound with IC₅₀ of less than 0.1 µmol/L is denoted by +++, a compound with IC₅₀ of not less than 0.1 µmol/L and not greater than 0.3 µmol/L is denoted by ++, and a compound with IC₅₀ exceeding 0.3 µmol/L is denoted by +.

**Table 22**

| Example | **IC50** | Example | **IC50** | Example | **IG50** | Example | **IC50** |
|---|---|---|---|---|---|---|---|
| **1-1** | **+++** | **1-11** | **+++** | **1-21** | **+++** | **1-31** | **+++** |
| **1-2** | **++** | **1-12** | **++** | **1-22** | **+++** | **1-32** | **+++** |
| **1-3** | **++** | **1-13** | **+++** | **1-23** | **+++** | **1-33** | **+++** |
| **1-4** | **+++** | **1-14** | **+++** | **1-24** | **+++** | **1-34** | **+++** |
| **1-5** | **+++** | **1-15** | **+++** | **1-25** | **+++** | **1-35** | **+++** |
| **1-6** | **++** | **1-16** | **+++** | **1-26** | **+++** | **1-36** | **+++** |
| **1-7** | **+++** | **1-17** | **++** | **1-27** | **+++** | **1-37** | **+++** |
| **1-8** | **+++** | **1-18** | **+++** | **1-28** | **+++** | **1-38** | **+++** |
| **1-9** | **+++** | **1-19** | **+++** | **1-29** | **+++** | **1-39** | **+++** |
| **1-10** | **+++** | **1-20** | **+++** | **1-30** | **+++** | **1-40** | **+++** |

**Table 23**

| Example | **IC50** | Example | **IC50** | Example | **IC50** |
|---|---|---|---|---|---|
| **1-41** | **+++** | **1-51** | **+++** | **1-61** | **+++** |
| **1-42** | **+++** | **1-52** | **+++** | **1-62** | **++** |
| **1-43** | **+** | **1-53** | **+++** | **1-63** | **+++** |
| **1-44** | **+++** | **1-54** | **+++** | **1-64** | **+++** |
| **1-45** | **+++** | **1-55** | **+** | **1-65** | **+++** |
| **1-46** | **+++** | **1-56** | **+++** | **1-66** | **+++** |
| **1-47** | **+++** | **1-57** | **+++** | **1-67** | **+++** |
| **1-48** | **+++** | **1-58** | **+++** | **1-68** | **+++** |
| **1-49** | **+++** | **1-59** | **+++** | **1-69** | **+++** |
| **1-50** | **+++** | **1-60** | **+++** | **1-70** | **+++** |

**Table 24**

| Example | **IC50** | Example | **IC50** |
|---|---|---|---|
| **1-71** | **+++** | **1-81** | **+++** |
| **1-72** | **+++** | **1-82** | **+++** |
| **1-73** | **+++** | **1-83** | **+++** |
| **1-74** | **+++** | **1-84** | **+++** |
| **1-75** | **+++** | **1-85** | **+++** |
| **1-76** | **+++** | **1-86** | **+++** |
| **1-77** | **++** | **1-87** | **+++** |
| **1-78** | **+++** | **1-88** | **+++** |
| **1-79** | **+++** | **2-1** | **+++** |
| **1-80** | **+++** | | |

**Table 25**

| Example | **IC50** | Example | **IC50** | Example | **IC50** |
|---|---|---|---|---|---|
| **3-1** | **+++** | **3-11** | **+++** | **3-21** | **+++** |
| **3-2** | **+++** | **3-12** | **+++** | **3-22** | **+++** |
| **3-3** | **++** | **3-13** | **+++** | **3-23** | **+++** |
| **3-4** | **+++** | **3-14** | **+++** | **3-24** | **+++** |
| **3-5** | **+++** | **3-15** | **+++** | **3-25** | **+++** |
| **3-6** | **+++** | **3-16** | **+++** | | |
| **3-7** | **+++** | **3-17** | **+++** | | |
| **3-8** | **+++** | **3-18** | **+++** | | |
| **3-9** | **+++** | **3-19** | **++** | | |
| **3-10** | **+++** | **3-20** | **++** | | |

### Industrial Applicability

As shown in the above test results, the compound of the present invention and pharmacologically acceptable salts thereof potently inhibited human liver glycogen phosphorylase. Therefore, the compound of the present invention is useful as a therapeutic agent for diabetes having an inhibitory effect on human liver glycogen phosphorylase.

## Claims

1. A pyrazole compound represented by the following general formula (I) or a salt thereof or a solvate thereof: 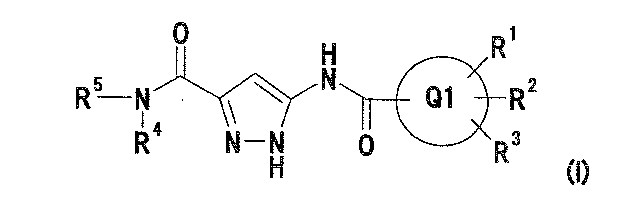 [wherein
ring Q1 represents
(1) an aryl group, or
(2) a monocyclic aromatic heterocyclic ring group;
R¹, R², and R³ are the same or different and each
represent
(1) a halogen atom, or
(2) a C₁₋₆ alkyl group;
R⁴ represents
(1) a hydrogen atom, or
(2) a C₁₋₆ alkyl group;
R⁵ represents a substituent represented by the following general formula (II): 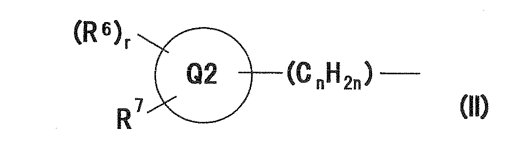 {wherein
n is an integer of 1 to 4;
r is 0 or an integer of 1 or 2;
ring Q2 represents a monocyclic aromatic heterocyclic ring group;
when r is 1, R⁶ represents
(1) a C₁₋₆ alkyl group, or
(2) a C₃₋₈ cycloalkyl group;
when r is 2, R⁶'s are the same or different and each
represent
(1) a C₁₋₆ alkyl group, or
(2) a C₃₋₈ cycloalkyl group;
R⁷ represents a substituent selected from the following
(1) to (11);
(1) - COOR⁸
(R⁸ represents a hydrogen atom, a C₁₋₆ alkyl group, or an aralkyl group),
(2) -S(O)₁-NR^{9a}R^{9b}
(1 is an integer of 1 or 2, and R^{9a} and R^{9b} are the same or different and each represent a hydrogen atom or a C₁₋₆ alkyl group),
(3) -O-R¹⁰
(R¹⁰ represents an aralkyl group),
(4) a monocyclic aromatic heterocyclic ring group substituted with 1 to 3 substituents that are the same or different and selected from the following group A;
[group A]
a. a C₁₋₆ alkyl group which may be substituted with 1 to 3 halogen atoms that are the same or different, and
b. a halogen atom,
(5) an aryl group substituted with 1 to 5 substituents that are the same or different and selected from the following group B:
[group B]
a. a C₁₋₆ alkyl group which may be substituted with 1 to 3 halogen atoms that are the same or different,
b. a halogen atom,
c. a cyano group,
d. -NR^{11a}R^{11b}
(R^{11a} and R^{11b} are the same or different and each represent a hydrogen atom or a C₁₋₆ alkyl group),
e. -S(O)ₘ-R¹²
(m is an integer of 1 or 2, and R¹² represents a C₁₋₆ alkyl group),
f. -O-R¹³
(R¹³ represents a hydrogen atom or a C₁₋₆ alkyl group),
g. a nitro group, and
h. an aryl group,
(6) a C₃₋₈ cycloalkyl group,
(7) an adamantyl group,
(8) -S-R¹⁴
(R¹⁴ represents a C₁₋₆ alkyl group or an aryl group),
(9) CONR^{15a}R^{15b}
(R^{15a} represents a hydrogen atom or a C₁₋₆ alkyl group, and R^{15b} represents an aralkyl group, or R^{15a} and R^{15b}, together with an adjacent nitrogen atom, may form a monocyclic nonaromatic heterocyclic ring),
(10) a C₁₋₆ alkyl group substituted with 1 to 3 substituents that are the same or different and selected from the following group C:
[group C]
a. -COOR¹⁶
(R¹⁶ represents a hydrogen atom, a C₁₋₆ alkyl group, or an aralkyl group),
b. -CONR^{17a}R^{17b}
(R^{17a} and R^{17b} are the same or different and each represent a hydrogen atom, a C₁₋₆ alkyl group, or an aralkyl group, or R^{17a} and R^{17b}, together with an adjacent nitrogen atom, may form a monocyclic nonaromatic heterocyclic ring),
c. a monocyclic aromatic heterocyclic ring group which may be substituted with 1 to 3 substituents that are the same or different and selected from the above group A,
d. an aryl group which may be substituted with 1 to 5 substituents that are the same or different and selected from the above group B,
e. -S-R¹⁸
(R¹⁸ represents a hydrogen atom, a C₁₋₆ alkyl group, or an aryl group),
f. -O-R¹⁹
(R¹⁹ represents a hydrogen atom, an aryl group, an aralkyl group, a C₁₋₆ alkyl group, or a C₁₋₆ alkyl group substituted with 1 to 3 substituents that are the same or different and selected from the following group D)
[group D]
a'. a halogen atom,
b'. -COOR²⁰
(R²⁰ represents a hydrogen atom or a C₁₋₆ alkyl group),
and
c'. - CONR^{21a}R^{21b}
(R^{21a} and R^{21b} are the same or different and each represent a hydrogen atom, a C₁₋₆ alkyl group, or an aralkyl group, or R^{21a} and R^{21b}, together with an adjacent nitrogen atom, may form a monocyclic nonaromatic heterocyclic ring),
g. -O-CO-R²²
(R²² represents a C₁₋₆ alkyl group, an aryl group, a C₃₋₈ cycloalkyl group, or a monocyclic aromatic heterocyclic ring group),
h. -NR²³-CO-R²⁴
(R²³ represents a hydrogen atom or a C₁₋₆ alkyl group, and R²⁴ represents a C₁₋₆ alkyl group or an aryl group [the aryl group may be substituted with 1 to 5 halogen atoms that are the same or different]), and
i. -NR²⁵-S(O)ₚ-R²⁶
(R²⁵ represents a hydrogen atom or a C₁₋₆ alkyl group, p is an integer of 1 or 2, and R²⁶ represents an aryl group which may be substituted with 1 to 5 C₁₋₆ alkyl groups that are the same or different), and
(11) a polycyclic aromatic heterocyclic ring group} or R⁵ and R⁴, together with an adjacent nitrogen atom, may form a substituent represented by the following general formula (III): 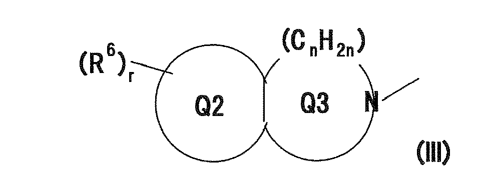 (wherein
ring Q3 represents a monocyclic nonaromatic heterocyclic ring;
ring Q2, n, r and R⁶ have the same meanings as defined above)].

2. The pyrazole compound represented by the following general formula (IV) or a salt thereof or a solvate thereof according to claim 1: 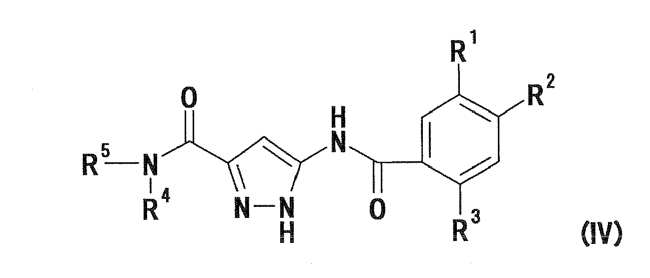 [wherein
R¹ and R² are the same or different and each represent a halogen atom;
R³ represents a halogen atom or a C₁₋₆ alkyl group;
R⁴ and R⁵ have the same meanings as defined in claim 1].

3. The pyrazole compound represented by the following general formula (V) or a salt thereof or a solvate thereof according to claim 2: 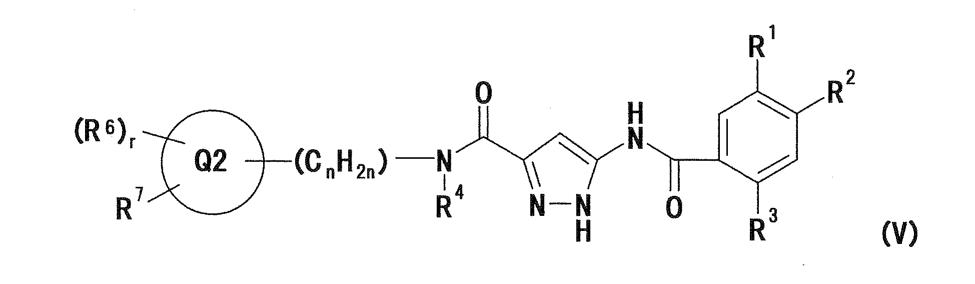 [wherein
R¹ and R² are the same or different and each represent a halogen atom;
R³ represents a halogen atom or a C₁₋₄ alkyl group;
R⁴ represents a hydrogen atom;
n is an integer of 1;
r is 0 or an integer of 1 or 2;
ring Q2 represents a monocyclic aromatic heterocyclic
ring group;
when r is 1, R⁶ represents
(1) a C₁₋₄ alkyl group, or
(2) a C₃₋₈ cycloalkyl group;
when r is 2, R⁶'s are the same or different and each
represent
(1) a C₁₋₄ alkyl group;
R⁷ represents a substituent selected from the following
(1) to (11);
(1) -COOR⁸
(R⁸ represents a hydrogen atom, a C₁₋₄ alkyl group, or an aralkyl group),
(2) -S(O)₁-NR^{9a}R^{9b}
(l is an integer of 1 or 2, and R^{9a} and R^{9b} are the same or different and each represent a C₁₋₄ alkyl group),
(3) -O-R¹⁰
(R¹⁰ represents an aralkyl group),
(4) a monocyclic aromatic heterocyclic ring group substituted with 1 to 3 substituents that are the same or different and selected from the following group A:
[group A]
a. a C₁₋₄ alkyl group which may be substituted with 1 to 3 halogen atoms that are the same or different, and
b. a halogen atom,
(5) an aryl group substituted with 1 to 5 substituents that are the same or different and selected from the following group B:
[group B]
a. a C₁₋₄ alkyl group which may be substituted with 1 to 3 halogen atoms that are the same or different,
b. a halogen atom,
c. a cyano group,
d. -NR^{11a}R^{11b}
(R^{11a} and R^{11b} are the same or different and each represent a C₁₋₄ alkyl group,
e. -S(O)ₘ-R¹²
(m is an integer of 1 or 2, and R¹² represents a C₁₋₄ alkyl group),
f. -O-R¹³
(R¹³ represents a hydrogen atom or a C₁₋₄ alkyl group),
g. a nitro group, and
h. an aryl group,
(6) a C₃₋₈ cycloalkyl group,
(7) an adamantyl group,
(8) -S-R¹⁴
(R¹⁴ represents an aryl group),
(9) -CONR^{15a}R^{15b}
(R^{15a} represents a hydrogen atom, and R^{15b} represents an aralkyl group, or R^{15a} and R^{15b}, together with an adjacent nitrogen atom, may form a monocyclic nonaromatic saturated heterocyclic ring),
(10) a C₁₋₄ alkyl group substituted with 1 to 3 substituents that are the same or different and selected from the following group C:
[group C]
a. -COOR¹⁶
(R¹⁶ represents a hydrogen atom or a C₁₋₄ alkyl group),
b. -CONR^{17a}R^{17b}
(R^{17a} and R^{17b} represent a hydrogen atom),
c. a monocyclic aromatic heterocyclic ring group which may be substituted with 1 to 3 substituents that are the same or different and selected from the above group A,
d. an aryl group which may be substituted with 1 to 5 substituents that are the same or different and selected from the above group B,
e. -S-R¹⁸
(R¹⁸ represents a C₁₋₄ alkyl group),
f. -O-R¹⁹
(R¹⁹ represents a hydrogen atom, an aryl group, an aralkyl group, a C₁₋₄ alkyl group, or a C₁₋₄ alkyl group substituted with 1 to 3 substituents that are the same or different and selected from the following group D)
[group D]
a'. a halogen atom,
b'. -COOR²⁰
(R²⁰ represents a hydrogen atom or a C₁₋₄ alkyl group),
and
c'. - CONR^{21a}R^{21b}
(R^{21a} and R^{21b} are the same or different and each represent a hydrogen atom, a C₁₋₄ alkyl group, or an aralkyl group, or R^{21a} and R^{21b}, together with an adjacent nitrogen atom, may form a monocyclic nonaromatic saturated heterocyclic ring),
g. -O-CO-R²²
(R²² represents a C₁₋₄ alkyl group, an aryl group, a C₃₋₈ cycloalkyl group, or a monocyclic aromatic heterocyclic ring group),
h. -NR²³-CO-R²⁴
(R²³ represents a hydrogen atom, and R²⁴ represents a C₁₋₄ alkyl group or an aryl group [the aryl group may be substituted with 1 to 5 halogen atoms that are the same or different]), and
i. -NR²⁵-S(O)ₚ-R²⁶
(R²⁵ represents a hydrogen atom, p is an integer of 1 or 2, and R²⁶ represents an aryl group which may be substituted with 1 to 5 C₁₋₄ alkyl groups that are the same or different),
(11) a polycyclic aromatic heterocyclic ring group obtained by condensation of a 5- or 6-membered monocyclic aromatic heterocyclic ring and 1 or 2 benzene rings, or R⁴ and R⁷, together with an adjacent nitrogen atom and ring Q2, may form a substituent represented by the following general formula (VI): 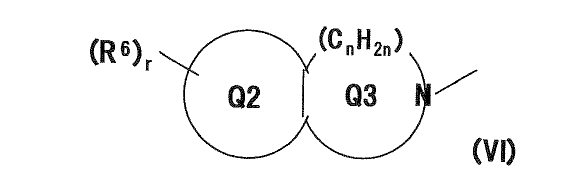 (wherein
ring Q3 represents a monocyclic nonaromatic heterocyclic ring;
ring Q2, n, r and R⁶ have the same meanings as defined above)].

4. The pyrazole compound or a salt thereof or a solvate thereof according to claim 3, wherein R¹, R², R³, R⁴, R⁶, R⁷, n, r, and ring Q2 in the general formula (V) each represent as follows:
R¹ and R² represent a fluorine atom;
R³ represents a chlorine atom or a methyl group;
R⁴ represents a hydrogen atom;
n is an integer of 1;
r is 0 or an integer of 1 or 2;
ring Q2 represents a furyl group, a thienyl group, a thiazolyl group, an imidazolyl group, an isoxazolyl group, an oxadiazolyl group, a triazolyl group, a pyrazolyl group, a thiadiazolyl group, an oxazolyl group, or a pyrimidinyl group;
when r is 1, R⁶ represents
(1) a C₁₋₄ alkyl group, or
(2) a C₃₋₈ cycloalkyl group;
when r is 2, R⁶'s are the same or different and each
represent
(1) a C₁₋₄ alkyl group;
R⁷ represents a substituent selected from the following (1) to (11) :
(1) -COOR⁸
(R⁸ represents a hydrogen atom, a C₁₋₄ alkyl group, or a benzyl group),
(2) -S(O)₁-NR^{9a}R^{9b}
(1 is an integer of 2, and R^{9a} and R^{9b} are the same or different and each represent a C₁₋₄ alkyl group),
(3) -O-R¹⁰
(R¹⁰ represents a benzyl group),
(4) a monocyclic aromatic heterocyclic ring group substituted with 1 to 3 substituents that are the same or different and selected from the following group A (the aromatic heterocyclic ring group is a thiazolyl group, a thienyl group, an isoxazolyl group, or a pyridyl group),
[group A]
a. a C₁₋₄ alkyl group which may be substituted with 1 to 3 halogen atoms that are the same or different, and
b. a halogen atom,
(5) a phenyl group substituted with 1 to 5 substituents that are the same or different and selected from the following group B:
[group B]
a. a C₁₋₄ alkyl group which may be substituted with 1 to 3 halogen atoms that are the same or different,
b. a halogen atom,
c. a cyano group,
d. -NR^{11a}R^{11b}
(R^{11a} and R^{11b} are the same or different and each represent a C₁₋₄ alkyl group,
e. -S(O)ₘ-R¹²
(m is an integer of 2, and R¹² represents a C₁₋₄ alkyl group),
f. -O-R¹³
(R¹³ represents a hydrogen atom or a C₁₋₄ alkyl group),
g. a nitro group, and
h. a phenyl group,
(6) a C₃₋₈ cycloalkyl group,
(7) an adamantyl group,
(8) -S-R¹⁴
(R¹⁴ represents a phenyl group),
(9) -CONR^{15a}R^{15b}
(R^{15a} represents a hydrogen atom, and R^{15b} represents a benzyl group, or R^{15a} and R^{15b}, together with an adjacent nitrogen atom, may form a piperidine ring),
(10) a C₁₋₄ alkyl group substituted with 1 to 3 substituents that are the same or different and selected from the following group C:
[group C]
a. - COOR¹⁶
(R¹⁶ represents a hydrogen atom or a C₁₋₄ alkyl group),
b. -CONR^{17a}R^{17b}
(R^{17a} and R^{17b} represent a hydrogen atom),
c. a thiazolyl group which may be substituted with 1 or 2 C₁₋₄ alkyl groups that are the same or different,
d. a phenyl group which may be substituted with 1 to 5 substituents that are the same or different and selected from halogen atoms and C₁₋₄ alkyl groups,
e. -S-R¹⁸
(R¹⁸ represents a C₁₋₄ alkyl group),
f. -O-R¹⁹
(R¹⁹ represents a hydrogen atom, a phenyl group, a benzyl group, a C₁₋₄ alkyl group, or a C₁₋₄ alkyl group substituted with 1 to 3 substituents that are the same or different and selected from the following group D)
[group D]
a'. a halogen atom,
b'. -COOR²⁰
(R²⁰ represents a hydrogen atom or a C₁₋₄ alkyl group),
c'. - CONR^{21a}R^{21b}
(R^{21a} and R^{21b} are the same or different and each represent a hydrogen atom, a C₁₋₄ alkyl group, or a benzyl group, or R^{21a} and R^{21b}, together with an adjacent nitrogen atom, may form a morpholine ring),
g. -O-CO-R²²
(R²² represents a C₁₋₄ alkyl group, a phenyl group, a C₃₋₈ cycloalkyl group, or a thienyl group),
h. -NR²³- CO-R²⁴
(R²³ represents a hydrogen atom, and R²⁴ represents a C₁₋₄ alkyl group or a phenyl group [the phenyl group may be substituted with 1 to 5 halogen atoms that are the same or different]), and
i. -NR²⁵-S(O)ₚ-R²⁶
(R²⁵ represents a hydrogen atom, p is an integer of 2, and R²⁶ represents a phenyl group which may be substituted with 1 to 5 C₁₋₄ alkyl groups that are the same or different),
(11) a polycyclic aromatic heterocyclic ring group obtained by condensation of a 5- or 6-membered monocyclic aromatic heterocyclic ring and 1 benzene ring, or R⁴ and R⁷, together with an adjacent nitrogen atom and ring Q2, may form a substituent represented by the following general formula (VII): 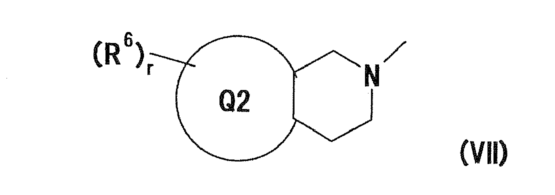 (wherein
ring Q2, r and R⁶ have the same meanings as defined above).

5. The pyrazole compound or a salt thereof or a solvate thereof according to claim 1 selected from the group consisting of the following (1-1) to (1-88), (2-1), and (3-1) to (3-25):
(1-1)
5-(2-chloro-4,5-difluoro-benzoylamino)-1H-pyrazole-3-carboxylic acid (5-ethoxycarbonyl-furan-2-ylmethyl)-amide:
(1-2)
5-(2-chloro-4,5-difluoro-benzoylamino)-1H-pyrazole-3-carboxylic acid (3-diethylsulfamoyl-thiophen-2-ylmethyl)-amide:
(1-3)
5-(2-chloro-4,5-difluoro-benzoylamino)-1H-pyrazole-3-carboxylic acid (3-benzyloxy-thiophen-2-ylmethyl)-amide:
(1-4) 5-(2-chloro-4,5-difluoro-benzoylamino)-1H-pyrazole-3-carboxylic acid [5-(2-methyl-thiazol-4-yl)-thiophen-2-ylmethyl]-amide:
(1-5)
5-(2-chloro-4,5-difluoro-benzoylamino)-1H-pyrazole-3-carboxylic acid [2-(4-chloro-phenyl)-thiazol-4-ylmethyl]-amide:
(1-6)
5-(2-chloro-4,5-difluoro-benzoylamino)-1H-pyrazole-3-carboxylic acid [4-methyl-2-(4-trifluoromethyl-phenyl)-thiazol-5-ylmethyl]-amide:
(1-7)
5-(2-chloro-4,5-difluoro-benzoylamino)-1H-pyrazole-3-carboxylic acid [4-methyl-2-(5-methyl-thiophen-2-yl)-thiazol-5-ylmethyl]-amide:
(1-8)
5-(2-chloro-4,5-difluoro-benzoylamino)-1H-pyrazole-3-carboxylic acid [2-(5-chlorothiophen-2-yl)-4-methyl-thiazol-5-ylmethyl]-amide:
(1-9)
5-(2-chloro-4,5-difluoro-benzoylamino)-1H-pyrazole-3-carboxylic acid (2-benzo[b]thiophen-2-yl-4-methyl-thiazol-5-ylmethyl)-amide:
(1-10)
5-(2-chloro-4,5-difluoro-benzoylamino)-1H-pyrazole-3-carboxylic acid (2-ethoxycarbonyl-4-methyl-thiazol-5-ylmethyl)-amide:
(1-11)
5-(2-chloro-4,5-difluoro-benzoylamino)-1H-pyrazole-3-carboxylic acid (4-adamantan-1-yl-2-methyl-thiazol-5-ylmethyl)-amide:
(1-12)
5-(2-chloro-4,5-difluoro-benzoylamino)-1H-pyrazole-3-carboxylic acid (2-methyl-6,7-dihydro-4H-thiazolo[5,4-c]pyridin-5-yl)-amide:
(1-13)
5-(2-chloro-4,5-difluoro-benzoylamino)-1H-pyrazole-3-carboxylic acid (5-ethoxycarbonyl-4-methyl-1H-imidazol-2-ylmethyl)-amide:
(1-14)
5-(2-chloro-4,5-difluoro-benzoylamino)-1H-pyrazole-3-carboxylic acid [3-(4-cyano-phenyl)-5-methylisoxazol-4-ylmethyl]-amide:
(1-15)
5-(2-chloro-4,5-difluoro-benzoylamino)-1H-pyrazole-3-carboxylic acid [3-(4-dimethylamino-phenyl)-5-methylisoxazol-4-ylmethyl]-amide:
(1-16)
5-(2-chloro-4,5-difluoro-benzoylamino)-1H-pyrazole-3-carboxylic acid [3-(4-methanesulfonyl-phenyl)-5-methylisoxazol-4-ylmethyl]-amide:
(1-17) 5-(2-chloro-4,5-difluoro-benzoylamino)-1H-pyrazole-3-carboxylic acid [3-(2-fluoro-phenyl)-5-methylisoxazol-4-ylmethyl]-amide:
(1-18)
5-(2-chloro-4,5-difluoro-benzoylamino)-1H-pyrazole-3-carboxylic acid (5,3'-dimethyl-[3,5']biisoxazolyl-4'-ylmethyl)-amide:
(1-19)
5-(2-chloro-4,5-difluoro-benzoylamino)-1H-pyrazole-3-carboxylic acid [3-methyl-5-(6-methyl-pyridin-2-yl)-isoxazol-4-ylmethyl]-amide:
(1-20)
5-(2-chloro-4,5-difluoro-benzoylamino)-1H-pyrazole-3-carboxylic acid [5-(2-chloro-pyridin-4-yl)-3-methylisoxazol-4-ylmethyl]-amide:
(1-21)
5-(2-chloro-4,5-difluoro-benzoylamino)-1H-pyrazole-3-carboxylic acid [3-methyl-5-(6-methyl-pyridin-3-yl)-isoxazol-4-ylmethyl]-amide:
(1-22)
5-(2-chloro-4,5-difluoro-benzoylamino)-1H-pyrazole-3-carboxylic acid [5-(2,4-dimethyl-thiazol-5-yl)-3-methylisoxazol-4-ylmethyl]-amide:
(1-23)
5-(2-chloro-4,5-difluoro-benzoylamino)-1H-pyrazole-3-carboxylic acid (3-ethoxycarbonylisoxazol-5-ylmethyl)-amide:
(1-24)
5-(2-chloro-4,5-difluoro-benzoylamino)-1H-pyrazole-3-carboxylic acid [2-methyl-5-(2-methylsulfanyl-ethyl)-oxazol-4-ylmethyl]-amide:
(1-25)
5-(2-chloro-4,5-difluoro-benzoylamino)-1H-pyrazole-3-carboxylic acid [4-(4-fluoro-phenyl)-2-methyl-oxazol-5-ylmethyl]-amide;
(1-26)
5-(2-chloro-4,5-difluoro-benzoylamino)-1H-pyrazole-3-carboxylic acid [4-(3-fluoro-phenyl)-2-methyl-oxazol-5-ylmethyl]-amide:
(1-27)
5-(2-chloro-4,5-difluoro-benzoylamino)-1H-pyrazole-3-carboxylic acid [4-(2-fluoro-phenyl)-2-methyl-oxazol-5-ylmethyl]-amide:
(1-28)
5-(2-chloro-4,5-difluoro-benzoylamino)-1H-pyrazole-3-carboxylic acid [4-(4-chloro-phenyl)-2-methyl-oxazol-5-ylmethyl]-amide:
(1-29)
5-(2-chloro-4,5-difluoro-benzoylamino)-1H-pyrazole-3-carboxylic acid [4-(4-methyl-phenyl)-2-methyl-oxazol-5-ylmethyl]-amide:
(1-30) 5-(2-chloro-4,5-difluoro-benzoylamino)-1H-pyrazole-3-carboxylic acid [4-(3-methyl-phenyl)-2-methyl-oxazol-5-ylmethyl]-amide;
(1-31)
5-(2-chloro-4,5-difluoro-benzoylamino)-1H-pyrazole-3-carboxylic acid [4-(3-methoxy-phenyl)-2-methyl-oxazol-5-ylmethyl]-amide:
(1-32)
5-(2-chloro-4,5-difluoro-benzoylamino)-1H-pyrazole-3-carboxylic acid (4-biphenyl-4-yl-2-methyl-oxazol-5-ylmethyl)-amide:
(1-33)
5-(2-chloro-4,5-difluoro-benzoylamino)-1H-pyrazole-3-carboxylic acid (2-methyl-4-phenethyl-oxazol-5-ylmethyl)-amide:
(1-34)
5-(2-chloro-4,5-difluoro-benzoylamino)-1H-pyrazole-3-carboxylic acid (4-ethoxycarbonyl-2-methyl-oxazol-5-ylmethyl)-amide:
(1-35)
5-(2-chloro-4,5-difluo-ro-benzoylamino)-1H-pyrazole-3-carboxylic acid (4-hydroxymethyl-2-methyl-oxazol-5-ylmethyl)-amide:
(1-36)
5-(2-chloro-4,5-difluoro-benzoylamino)-1H-pyrazole-3-carboxylic acid (4-benzyl-2-methyl-oxazol-5-ylmethyl)-amide p-toluenesulfonate:
(1-37)
5-(2-chloro-4,5-difluoro-benzoylamino)-1H-pyrazole-3-carboxylic acid (4-cyclopropyl-2-methyl-oxazol-5-ylmethyl)-amide:
(1-38)
5-(2-chloro-4,5-difluoro-benzoylamino)-1H-pyrazole-3-carboxylic acid (4-cyclohexyl-2-methyl-oxazol-5-ylmethyl)-amide;
(1-39)
5-(2-chloro-4,5-difluoro-benzoylamino)-1H-pyrazole-3-carboxylic acid (2-cyclopropyl-4-methyl-oxazol-5-ylmethyl)-amide:
(1-40)
5-(2-chloro-4,5-difluoro-benzoylamino)-1H-pyrazole-3-carboxylic acid (2-cyclohexyl-4-methyl-oxazol-5-ylmethyl)-amide:
(1-41)
5-(2-chloro-4,5-difluoro-benzoylamino)-1H-pyrazole-3-carboxylic acid (2-benzyl-4-methyl-oxazol-5-ylmethyl)-amide:
(1-42)
5-(2-chloro-4,5-difluoro-benzoylamino)-1H-pyrazole-3-carboxylic acid (4-methyl-2-phenethyl-oxazol-5-ylmethyl)-amide:
(1-43) 5-(2-chloro-4,5-difluoro-benzoylamino)-1H-pyrazole-3-carboxylic acid (4-methyl-2-trityloxymethyl-oxazol-5-ylmethyl)-amide:
(1-44)
5-(2-chloro-4,5-difluoro-benzoylamino)-1H-pyrazole-3-carboxylic acid (4-methyl-2-phenoxymethyl-oxazol-5-ylmethyl)-amide:
(1-45)
5-(2-chloro-4,5-difluoro-benzoylamino)-1H-pyrazole-3-carboxylic acid (2-methoxymethyl-4-methyl-oxazol-5-ylmethyl)-amide:
(1-46)
5-(2-chloro-4,5-difluoro-benzoylamino)-1H-pyrazole-3-carboxylic acid [4-methyl-2-(2,2,2-trifluoro-ethoxymethyl)-oxazol-5-ylmethyl)]-amide:
(1-47)
5-(2-chloro-4,5-difluoro-benzoylamino)-1H-pyrazole-3-carboxylic acid (2-benzyloxymethyl-4-methyl-oxazol-5-ylmethyl)-amide:
(1-48)
5-(2-chloro-4,5-difluoro-benzoylamino)-1H-pyrazole-3-carboxylic acid (2-ethoxycarbonylmethoxymethyl-4-methyl-oxazol-5-ylmethyl)-amide:
(1-49)
5-(2-chloro-4,5-difluoro-benzoylamino)-2H-pyrazole-3-carboxylic acid (4-methyl-2-phenylsulfanyl-oxazol-5-ylmethyl)-amide:
(1-50)
5-(2-chloro-4,5-difluoro-benzoylamino)-1H-pyrazole-3-carboxylic acid (2-methoxycarbonyl-4-methyl-oxazol-5-ylmethyl)-amide:
(1-51)
5-(2-chloro-4,5-difluoro-benzoylamino)-1H-pyrazole-3-carboxylic acid (5-ethoxycarbonyl-4-methyl-oxazol-2-ylmethyl)-amide:
(1-52)
5-(2-chloro-4,5-difluoro-benzoylamino)-1H-pyrazole-3-carboxylic acid [5-(4-chloro-phenyl)-[1,3,4]oxadiazol-2-ylmethyl]-amide:
(1-53)
5-(2-chloro-4,5-difluoro-benzoylamino)-1H-pyrazole-3-carboxylic acid [5-(2-methyl-phenyl)-[1,3,4]oxadiazol-2-ylmethyl]-amide:
(1-54)
5-(2-chloro-4,5-difluoro-benzoylamino)-1H-pyrazole-3-carboxylic acid [5-(4-dimethylamino-phenyl)-[1,3,4]oxadiazol-2-ylmethyl]-amide:
(1-55)
5-(2-chloro-4,5-difluoro-benzoylamino)-1H-pyrazole-3-carboxylic acid [5-(4-trifluoromethyl-phenyl)-[1,3,4]oxadiazol-2-ylmethyl]-amide:
(1-56) 5-(2-chloro-4,5-difluoro-benzoylamino)-1H-pyrazole-3-carboxylic acid [5-(3-nitro-phenyl)-[1,3,4]oxadiazol-2-ylmethyl]-amide:
(1-57)
5-(2-chloro-4,5-difluoro-benzoylamino)-1H-pyrazole-3-carboxylic acid [5-(3-hydroxy-phenyl)-[1,3,4]oxadiazol-2-ylmethyl]-amide:
(1-58)
5-(2-chloro-4,5-difluoro-benzoylamino)-1H-pyrazole-3-carboxylic acid [5-(2-hydroxy-phenyl)-[1,3,4]oxadiazol-2-ylmethyl]-amide:
(1-59)
5-(2-chloro-4,5-difluoro-benzoylamino)-1H-pyrazole-3-carboxylic acid [5-(3-chloro-phenyl)-[1,3,4]oxadiazol-2-ylmethyl]-amide:
(1-60)
5-(2-chloro-4,5-difluoro-benzoylamino)-1H-pyrazole-3-carboxylic acid [5-(3-methoxy-phenyl)-[1,3,4]oxadiazol-2-ylmethyl]-amide:
(1-61)
5-(2-chloro-4,5-difluoro-benzoylamino)-1H-pyrazole-3-carboxylic acid [5-(2-methoxy-phenyl)-[1,3,4]oxadiazol-2-ylmethyl]-amide:
(1-62)
5-(2-chloro-4,5-difluoro-benzoylamino)-1H-pyrazole-3-carboxylic acid (5-ethoxycarbonylmethyl-[1,3,4]oxadiazol-2-ylmethyl)-amide:
(1-63)
5-(2-chloro-4,5-difluoro-benzoylamino)-1H-pyrazole-3-carboxylic acid [1-(4-chloro-benzyl)-5-methyl-1H-[1,2,3]triazol-4-ylmethyl]-amide:
(1-64)
5-(2-chloro-4,5-difluoro-benzoylamino)-1H-pyrazole-3-carboxylic acid [5-methyl-1-(4-methyl-benzyl)-1H-[1,2,3]triazol-4-ylmethyl]-amide:
(1-65)
5-(2-chloro-4,5-difluoro-benzoylamino)-1H-pyrazole-3-carboxylic acid [5-methyl-1-(2-methyl-thiazol-4-ylmethyl)-1H-[1,2,3]triazol-4-ylmethyl]-amide:
(1-66)
5-(2-chloro-4,5-difluoro-benzoylamino)-1H-pyrazole-3-carboxylic acid [3,5-dimethyl-1-(3-methyl-phenyl)-1H-pyrazol-4-ylmethyl]-amide:
(1-67)
5-(2-chloro-4,5-difluoro-benzoylamino)-1H-pyrazole-3-carboxylic acid [5-cyclopropyl-1-(4-fluoro-phenyl)-1H-pyrazol-4-ylmethyl]-amide:
(1-68)
5-(2-chloro-4,5-difluoro-benzoylamino)-1H-pyrazole-3-carboxylic acid (4-cyclopropyl-2-methyl-pyrimidin-5-ylmethyl)-amide:
(1-69) 5-(2-chloro-4,5-difluoro-benzoylamino)-1H-pyrazole-3-carboxylic acid [4-(4-methoxy-phenyl)-[1,2,3]thiadiazol-5-ylmethyl]-amide:
(1-70)
5-(2-chloro-4,5-difluoro-benzoylamino)-1H-pyrazole-3-carboxylic acid [5-methyl-3-(3-methyl-[1,2,4]oxadiazol-5-yl)-isoxazol-4-ylmethyl]-amide:
(1-71)
5-(2-chloro-4,5-difluoro-benzoylamino)-1H-pyrazole-3-carboxylic acid [2-methyl-4-(3-methyl-[1,2,4]oxadiazol-5-yl)-oxazol-5-ylmethyl]-amide:
(1-72)
5-(2-chloro-4,5-difluoro-benzoylamino)-1H-pyrazole-3-carboxylic acid [5-(3-fluoro-phenyl)-[1,3,4]oxadiazol-2-ylmethyl]-amide:
(1-73)
5-(2-chloro-4,5-difluoro-benzoylamino)-1H-pyrazole-3-carboxylic acid [5-(2-methyl-phenyl)-[1,3,4]oxadiazol-2-ylmethyl]-amide:
(1-74)
5-(2-chloro-4,5-difluoro-benzoylamino)-1H-pyrazole-3-carboxylic acid (5-benzyl-[1,3,4]oxadiazol-2-ylmethyl)-amide:
(1-75)
5-(2-chloro-4,5-difluoro-benzoylamino)-1H-pyrazole-3-carboxylic acid [5-(1-methyl-1H-pyrrol-2-yl)-[1,3,4]oxadiazol-2-ylmethyl]-amide:
(1-76)
5-(2-chloro-4,5-difluoro-benzoylamino)-1H-pyrazole-3-carboxylic acid [5-(5-methyl-2H-pyrazol-3-yl)-[1,3,4]oxadiazol-2-ylmethyl]-amide:
(1-77)
5-(2-chloro-4,5-difluoro-benzoylamino)-1H-pyrazole-3-carboxylic acid (5-benzo[b]thiophen-2-yl-[1,3,4]oxadiazol-2-ylmethyl)-amide:
(1-78)
5-(2-chloro-4,5-difluoro-benzoylamino)-1H-pyrazole-3-carboxylic acid [5-(4-methyl-[1,2,3]thiadiazol-5-yl)-[1,3,4]oxadiazol-2-ylmethyl]-amide:
(1-79)
5-(2-chloro-4,5-difluoro-benzoylamino)-1H-pyrazole-3-carboxylic acid [4-(3-fluoro-phenyl)-2-methyl-pyrimidin-5-ylmethyl]-amide:
(1-80)
5-(2-chloro-4,5-difluoro-benzoylamino)-1H-pyrazole-3-carboxylic acid (2-cyclopropyl-4-methyl-pyrimidin-5-ylmethyl)-amide:
(1-81)
5-(2-chloro-4,5-difluoro-benzoylamino)-1H-pyrazole-3-carboxylic acid (4-cyclopropyl-pyrimidin-5-ylmethyl)-amide:
(1-82) 5-(2-chloro-4,5-difluoro-benzoylamino)-1H-pyrazole-3-carboxylic acid (2,4-dicyclopropyl-pyrimidin-5-ylmethyl)-amide hydrochloride:
(1-83)
5-(2-chloro-4,5-difluoro-benzoylamino)-1H-pyrazole-3-carboxylic acid [4-(2,4-dimethyl-oxazol-5-yl)-2-methyl-pyrimidin-5-ylmethyl]-amide:
(1-84)
5-(2-chloro-4,5-difluoro-benzoylamino)-1H-pyrazole-3-carboxylic acid [4-(2,5-dimethyl-furan-3-yl)-[1,2,3]thiadiazol-5-ylmethyl]-amide:
(1-85)
5-(2-chloro-4,5-difluoro-benzoylamino)-1H-pyrazole-3-carboxylic acid [5-(3-chloro-phenyl)-[1,3,4]thiadiazol-2-ylmethyl]-amide:
(1-86)
5-(2-chloro-4,5-difluoro-benzoylamino)-1H-pyrazole-3-carboxylic acid [2-(5-ethyl-1H-[1,2,4]triazol-3-ylmethyl)-4-methyl-oxazol-5-ylmethyl]-amide hydrochloride:
(1-87)
5-(2-chloro-4,5-difluoro-benzoylamino)-1H-pyrazole-3-carboxylic acid [5-(4-chloro-phenyl)-1H-[1,2,4]triazol-3-ylmethyl]-amide:
(1-88) 5-(2-chloro-4,5-difluoro-benzoylamino)-1H-pyrazole-3-carboxylic acid [5-(3,4-dimethoxy-benzyl)-1H-[1,2,4]triazol-3-ylmethyl]-amide:
(2-1)
5-(4,5-difluoro-2-methyl-benzoylamino)-1H-pyrazole-3-carboxylic acid (4-ethoxycarbonyl-2-methyl-oxazol-5-ylmethyl)-amide:
(3-1)
5-(2-chloro-4,5-difluoro-benzoylamino)-1H-pyrazole-3-carboxylic acid (5-carboxy-furan-2-ylmethyl)-amide:
(3-2)
sodium 5-({[5-(2-chloro-4,5-difluoro-benzoylamino)-1H-pyrazole-3-carbonyl]-amino}-methyl)-4-methyl-thiazole-2-carboxylate:
(3-3)
5-(2-chloro-4,5-difluoro-benzoylamino)-1H-pyrazole-3-carboxylic acid (3-carboxy-isoxazol-5-ylmethyl)-amide:
(3-4)
5-(2-chloro-4,5-difluoro-benzoylamino)-1H-pyrazole-3-carboxylic acid (4-acetoxymethyl-2-methyl-oxazol-5-ylmethyl)-amide:
(3-5)
5-(2-chloro-4,5-difluoro-benzoylamino)-1H-pyrazole-3-carboxylic acid (4-benzoyloxymethyl-2-methyl-oxazol-5-ylmethyl)-amide:
(3-6) 5-(2-chloro-4,5-difluoro-benzoylamino)-1H-pyrazole-3-carboxylic acid (4-carboxy-2-methyl-oxazol-5-ylmethyl)-amide:
(3-7)
5-(2-chloro-4,5-difluoro-benzoylamino)-1H-pyrazole-3-carboxylic acid (2-carboxymethoxymethyl-4-methyl-oxazol-5-ylmethyl)-amide:
(3-8)
5-(2-chloro-4,5-difluoro-benzoylamino)-1H-pyrazole-3-carboxylic acid (2-diethylcarbamoylmethoxymethyl-4-methyl-oxazol-5-ylmethyl)-amide:
(3-9)
5-(2-chloro-4,5-difluoro-benzoylamino)-1H-pyrazole-3-carboxylic acid [2-(benzylcarbamoyl-methoxymethyl)-4-methyl-oxazol-5-ylmethyl]-amide:
(3-10)
5-(2-chloro-4,5-difluoro-benzoylamino)-1H-pyrazole-3-carboxylic acid [4-methyl-2-(2-morpholin-4-yl-2-oxo-ethoxymethyl)-oxazol-5-ylmethyl]-amide:
(3-11)
5-(2-chloro-4,5-difluoro-benzoylamino)-1H-pyrazole-3-carboxylic acid {4-methyl-2-[(toluene-4-sulfonylamino)-methyl]-oxazol-5-ylmethyl}-amide:
(3-12)
5-(2-chloro-4,5-difluoro-benzoylamino)-1H-pyrazole-3-carboxylic acid [2-(acetylamino-methyl)-4-methyl-oxazol-5-ylmethyl]-amide dihydrochloride:
(3-13)
5-(2-chloro-4,5-difluoro-benzoylamino)-1H-pyrazole-3-carboxylic acid {2-[(4-chloro-benzoylamino)-methyl]-4-methyl-oxazol-5-ylmethyl}-amide:
(3-14)
sodium 5-({[5-(2-chloro-4,5-difluoro-benzoylamino)-1H-pyrazole-3-carbonyl]-amino}-methyl)-4-methyl-oxazole-2-carboxylate:
(3-15)
5-(2-chloro-4,5-difluoro-benzoylamino)-1H-pyrazole-3-carboxylic acid [4-methyl-2-(piperidine-1-carbonyl)-oxazol-5-ylmethyl]-amide:
(3-16)
5-(2-chloro-4,5-difluoro-benzoylamino)-1H-pyrazole-3-carboxylic acid (2-benzylcarbamoyl-4-methyl-oxazol-5-ylmethyl)-amide:
(3-17)
5-(2-chloro-4,5-difluoro-benzoylamino)-1H-pyrazole-3-carboxylic acid (2-benzyloxycarbonyl-4-methyl-oxazol-5-ylmethyl)-amide:
(3-18)
5-(2-chloro-4,5-difluoro-benzoylamino)-1H-pyrazole-3-carboxylic acid (5-carboxy-4-methyl-oxazol-2-ylmethyl)-amide:
(3-19) 5-(2-chloro-4,5-difluoro-benzoylamino)-1H-pyrazole-3-carboxylic acid (5-carboxymethyl-[1,3,4]oxadiazol-2-ylmethyl)-amide:
(3-20)
5-(2-chloro-4,5-difluoro-benzoylamino)-1H-pyrazole-3-carboxylic acid (5-carbamoylmethyl-[1,3,4]oxadiazol-2-ylmethyl)-amide:
(3-21)
5-(4,5-difluoro-2-methyl-benzoylamino)-1H-pyrazole-3-carboxylic acid (4-carboxy-2-methyl-oxazol-5-ylmethyl)-amide:
(3-22)
5-(2-chloro-4,5-difluoro-benzoylamino)-1H-pyrazole-3-carboxylic acid [2-methyl-4-(thiophene-2-carbonyloxymethyl)-oxazol-5-ylmethyl]-amide:
(3-23)
5-(2-chloro-4,5-difluoro-benzoylamino)-1H-pyrazole-3-carboxylic acid (4-cyclohexanecarbonyloxymethyl-2-methyl-oxazol-5-ylmethyl)-amide:
(3-24)
5-(2-chloro-4,5-difluoro-benzoylamino)-1H-pyrazole-3-carboxylic acid (2-hydroxymethyl-4-methyl-oxazol-5-ylmethyl)-amide hydrochloride:
(3-25)
5-(2-chloro-4,5-difluoro-benzoylamino)-1H-pyrazole-3-carboxylic acid [2-(benzyl-methyl-carbamoyl)-4-methyl-oxazol-5-ylmethyl]-amide:

6. A pharmaceutical composition comprising the pyrazole compound or a salt thereof or a solvate thereof according to any one of claims 1 to 5.

7. A liver glycogen phosphorylase inhibitor comprising the pyrazole compound or a salt thereof or a solvate thereof according to any one of claims 1 to 5.

8. A blood sugar level lowering agent comprising the pyrazole compound or a salt thereof or a solvate thereof according to any one of claims 1 to 5.

9. A therapeutic or prophylactic agent for diabetes comprising the pyrazole compound or a salt thereof or a solvate thereof according to any one of claims 1 to 5.

10. A method for inhibiting liver glycogen phosphorylase comprising administration of a pharmaceutically effective amount of the pyrazole compound or a salt thereof or a solvate thereof according to any one of claims 1 to 5.

11. A method for lowering a blood sugar level comprising administration of a pharmaceutically effective amount of the pyrazole compound or a salt thereof or a solvate thereof according to any one of claims 1 to 5.

12. A method for therapeutic or prophylactic treatment of diabetes comprising administration of a pharmaceutically effective amount of the pyrazole compound or a salt thereof or a solvate thereof according to any one of claims 1 to 5.

13. Use of the pyrazole compound or a salt thereof or a solvate thereof according to any one of claims 1 to 5 for production of a liver glycogen phosphorylase inhibitor.

14. Use of the pyrazole compound or a salt thereof or a solvate thereof according to any one of claims 1 to 5 for production of a blood sugar level lowering agent.

15. Use of the pyrazole compound or a salt thereof or a solvate thereof according to any one of claims 1 to 5 for production of a therapeutic or prophylactic agent for diabetes.
